(19) Europäisches Patentamt
European Patent Office
Office européen des brevets

(11) EP 1 433 778 A1

(12) **EUROPEAN PATENT APPLICATION**

(43) Date of publication:
**30.06.2004 Bulletin 2004/27**

(21) Application number: **02028699.3**

(22) Date of filing: **23.12.2002**

(51) Int Cl.[7]: **C07C 255/25**, A61K 31/16, A61P 43/00, G01N 33/00, C07C 271/22, C07C 323/44, C07C 335/16, C07C 307/10, C07C 255/24, C07C 255/58, C07D 317/66, C07C 335/12, C07C 335/18, C07C 311/10, C07D 333/34, C07D 215/36, C07D 209/42, C07D 333/38, C07D 209/18, C07D 333/60, C07D 409/12, C07D 207/16

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(71) Applicant: **Jerini AG**
**10115 Berlin (DE)**

(72) Inventors:
- **Knolle, Jochen**
**10115 Berlin (DE)**
- **Schutkowski, Mike**
**06268 Ziegelroda (DE)**
- **Hummel, Gerd**
**13357Berlin (DE)**

(74) Representative: **Bohmann, Armin K., Dr.**
**Bohmann & Loosen,**
**Anwaltssozietät,**
**Sonnenstrasse 8**
**80331 München (DE)**

(54) **Use of nitriles as rotamase inhibitors**

(57) The present invention is related to the use of a compound as an inhibitor to a rotamase, whereby the compound has the structure of formula (I):

$$A\text{-}B\text{-}C\text{-}D \qquad (I)$$

wherein:
A is selected from the group comprising

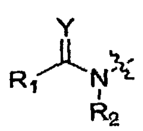 ; 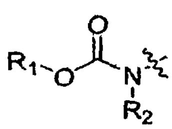 ; 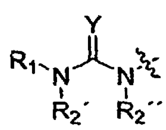 ; 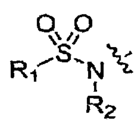 ; 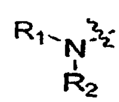 ;

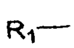 and 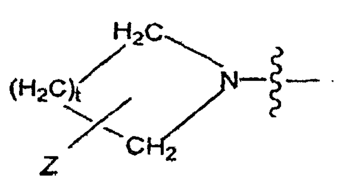

B is either present or absent, but if B is present B is

EP 1 433 778 A1

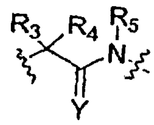

C is

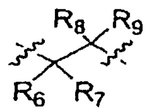

or

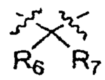

D is selected from the group comprising $-(CH_2)_rC(O)H$, $-(CH_2)_rC \equiv N$, $-(CH_2)_rNHNHC(O)NR_{A'}R_{A''}$, $-C(CH_2)_rC(O)(CH_2)_rC(O)OR_{A'}$, $-(CH_2)_rC(O)(CH_2)_rC(O)NR_{A'}R_{A''}$, $-(CH_2)_rCH(OH)(CH_2)_rC(O)U$, $-(CH_2)_rC(O)W-(CH_2)_rC(O)CH_2W$, $-(CH_2)_rC(O)$haloalkyl, and $-(CH_2)_rC(O)(CH_2)_rCHN_2$.

## Description

The present invention is related to new compounds and the use of said compounds as an inhibitor to rotamases, for the manufacture of medicaments, for removing a rotamase from a sample, for identifying a rotamase in a sample, for the manufacture of an affinity device, in drug potentiation applications and methods of treating a patient.

Rotamases, also referred to as peptidyl-prolyl cis-trans isomerases (PPIases) are a family of enzymes important in protein folding, assembly and transport. They act as catalysts to promote isomerization about the peptidyl-prolyl bond, which can have profound effects on protein function.

PPIases are divided into three classes, cyclophilins, FK-506 binding proteins (FKBPs) and the Pin1/parvulin class. While cyclophilins and FKBPs are distinguished by their ability to bind immunosuppressant molecules cyclosporin and FK-506, respectively, the Pin1/parvulin class binds neither of these immunosuppressants and is structurally unrelated to the other two classes. Known members of the Pin1/parvulin class include Pins 1 - 3 (Lu et al., Nature 380: 544-547, 1996), Pin-L (Campbell et al., Genomics 44:157-162, 1997), parvulin (Rahfeld et al., FEBS Letts 352:180-184, 1994), dodo (Maleszka et al., Proc Natl Acad Sci USA 93:447-451, 1996) and Essl/Pft1 (Hanes et al., Yeast 5:55-72, 1989; and Hani et al., FEBS Letts 365:198-202, 1995).

Recent research suggests that members of the Pin1/parvulin class are essential modulators of the cell cycle, and mitosis in particular. Lu et al., Nature 380:544-547, 1996 reports that depletion of Pin1/Ess1 in yeast or human cells induces mitotic arrest followed by apoptosis, indicating that enzymes in this class serve an essential function in cell division and proliferation.

Accordingly, compounds inhibiting rotamases can serve as agents for the treatment of a variety of disorders which are characterized by an inappropriate cell proliferation including cancer and infectious diseases.

In the prior art a huge number of compounds are described which are active as inhibitors to rotamase. The respective compounds are, among others, peptide derivatives such as amino methylene-peptides which are described in European patent EP 0 610 743, or non-peptidic or non-peptidomimetic molecules.

Given the importance of rotamase there is an ongoing need in the art to provide further compounds which are suitable as inhibitors to rotamases and thus suitable to be used as a medicament for those diseases wherein a rotamase is involved in the pathological mechanism.

Accordingly, the problem underlying the present invention is to provide compounds which inhibit a rotamase. A further problem underlying the present invention is to provide new compounds for the treatment of diseases the pathophysiology of which involves an imbalanced or undesired activity of a rotamase. A still further problem underlying the present invention is to provide means for the isolation and/or identification of rotamases.

In a first aspect the problem underlying the present invention is solved by a compound having the structure of formula (I)

A-B-C-D **(I)**

wherein:

A is selected from the group comprising

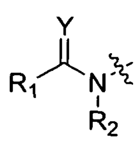 ; 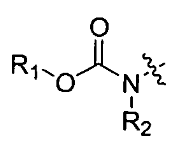 ; 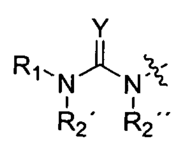

; 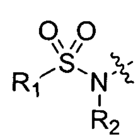 ; 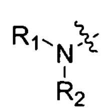 ; 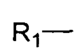

and

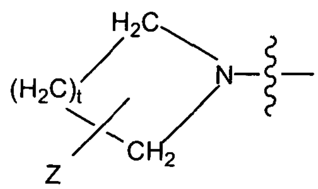

B is either present or absent, but if B is present, B is

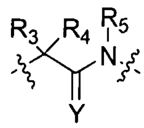

C is

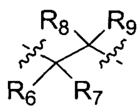 or 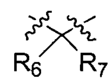

D is selected from the group comprising $-(CH_2)_rC(O)H$, $-(CH_2)_rC \equiv N$, $-(CH_2)_rNHNHC(O)NR_{A'}R_{A''}$, $-C(CH_2)_{r'}C(O)(CH_2)_{r'}C(O)OR_{A'}$, $-(CH_2)_rC(O)(CH_2)_{r'}C(O)NR_{A'}R_{A''}$, $-(CH_2)_rCH(OH)(CH_2)_{r'}C(O)U$, $-(CH_2)_rC(O)W$ $-(CH_2)_rC(O)CH_2W$, $-(CH_2)_rC(O)$haloalkyl, and $-(CH_2)_rC(O)(CH_2)_{r'}CHN_2$;
whereby U is $-OR_{A'}$ or $-NR_{A'}R_{A''}$; and
W is $-OR_{A'}$, $-SR_{A'}$, $-NR_{A'}R_{A''}$, or a heterocyclic moiety;
whereby r and r' are any integer from 0 to 5 and any r and r'mentioned are independently selected from any other r and r'mentioned or present; and whereby $R_{A'}$ and $R_{A''}$ are selected independently from the group comprising H, alkyl, cycloalkyl, aryl, heteroaryl and heterocyclyl and derivatives of any of these groups;

Y is O, S, or $NR_a$ wherein $R_a$ is selected from the group comprising H, alkyl, cycloalkyl, aryl, heteroaryl and heterocyclyl and derivatives of any of these groups;

t is 1, 2 or 3;

Z is

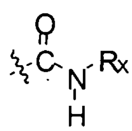

, whereby $R_x$ is selected from the group comprising amino acids, peptides and alkyl;
$R_1$, $R_3$, $R_4$, $R_6$, $R_7$, $R_8$, and $R_9$ are each individually and independently selected from the group comprising H, halogen, alkyl, cycloalkyl, aryl, heterocyclyl, heteroaryl and derivatives of any of these groups;
$R_2$, $R_2'$, $R_2''$, $R_5$ are each independently selected from the group consisting of H, alkyl, cycloalkyl, aryl, heteroaryl and heterocyclyl and derivatives of any of these groups or is a pharmaceutically acceptable salt or prodrug thereof; and
whereby "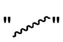" denotes any linkage to A, B, C and D, respectively.

In a preferred embodiment each of $R_1$, $R_3$, $R_4$, $R_6$, $R_7$, $R_8$ and $R_9$ is individually and independently a derivative of any of alkyl, cycloalkyl, aryl, heterocyclyl, or heteroaryl,

whereby any of these groups is individually and independently substituted by one or more groups of the formula $R_b$,

whereby $R_b$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, carbamoyl derivative, alkanoylamino, aroylamino, alkylthio, alkylthio derivatives, arylthio, arylthio derivatives, ureido, ureido derivatives, alkoxycarbonylamino, aryloxycarbonylamino, alkylcarbamoyloxy, arylcarbamoyloxy, alkylsulfonylamino, arylsulfonylamino, alkylaminosulfonyl, arylaminosulfonyl, amino, amino derivatives

and preferably $R_b$ is further substituted by one or more $R_c$,

whereby $R_c$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

In a further embodiment of the inventive compounds the carbamoyl group is derivatized, preferably the nitrogen atom is independently mono- or di-substituted by alkyl, aryl, heterocyclyl or heteroaryl; and/or

the alkylthio group is derivatized, preferably the sulfur atom is oxidized to a sulfoxide or sulfone; and/or

the arylthio group is derivatized, preferably the sulfur atom is oxidized to a sulfoxide or sulfone; and/or

the ureido group is derivatized, preferably either the nitrogen atom is independently mono- or di-substituted by a group which is selected from the group comprising alkyl, aryl, heterocyclyl or heteroaryl, alkoxycarbonylamino, aryloxycarbonylamino, alklycarbamoyloxy, arylcarbamoyloxy, alkylsulfonylamino, arylsulfonylamino, alkylaminosulfonyl, arylaminosulfonyl; and/or

the amino group is derivatized, preferably the nitrogen atom is independently mono- or di-substituted by alkyl, aryl, heterocyclyl or heteroaryl, halogen, hydroxy, oxo, carboxy, cyano, nitro, amidino and guanidino.

In a particularly preferred embodiment B is present and has the meaning as defined in any of the preceding claims,

C is

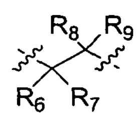

with $R_6$ to $R_9$ having the meaning as defined in any aspect or embodiment as specified herein.

In a more preferred embodiment D is selected from the group comprising $-(CH_2)_rC(O)H$, $—(CH_2)_rC \equiv N$, $—(CH_2)_rNHNHC(O)NR_{A'}R_{A''}$, $-C(CH_2)_rC(O)(CH_2)_{r'}C(O)OR_{A'}$, $—(CH_2)_rC(O)(CH_2)_{r'}C(O)NR_{A'}R_{A''}$, $-(CH_2)_rCH(OH)(CH_2)_{r'}C(O)U$, $—(CH_2)_rC(O)W$,

whereby U is $—OR_{A'}$ or $—NR_{A'}R_{A''}$; W is $—OR_{A'}$, $-SR_{A'}$, $-NR_{A'}R_{A''}$, or a heterocyclic moiety; r and r' are independently any integer from 0 to 5; and $R_{A'}$ and $R_{A''}$ are selected independently from the group comprising H, alkyl, phenyl, benzyl, and phenethyl.

In an even more preferred embodiment D is $—(CH_2)_rC \equiv N$ and r is any integer from 0 to 3.

In a further preferred embodiment

B is

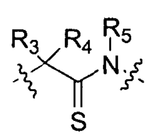

In a particularly preferred embodiment using the substituent B as defined above, D is selected from the group comprising $-(CH_2)_rC(O)H$, $—(CH_2)_rC \equiv N$, $—(CH_2)_rNHNHC(O)NR_{A'}R_{A''}$, $-C(CH_2)_rC(O)(CH_2)_{r'}C(O)OR_{A'}$, $—(CH_2)_rC(O)(CH_2)_{r'}C(O)NR_{A'}R_{A'}$, $-(CH_2)_rCH(OH)(CH_2)_{r'}C(O)U$, $—(CH_2)_rC(O)W$, whereby U is $—OR_{A'}$ or $—NR_{A'}R_{A''}$, W is $—OR_{A'}$, $-SR_{A'}$, $-NR_{A'}R_{A''}$, or a heterocyclic moiety; r and r' are independently any integer from 0 to 5; and $R_{A'}$ and $R_{A''}$ are selected independently from the group comprising H, alkyl, phenyl, benzyl, and phenethyl.

In an even more preferred embodiment D is $—(CH_2)_rC \equiv N$ and r is any integer from 0 to 3.

In a further embodiment of the compounds according to the present invention

A is

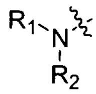 ; 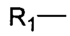 ; 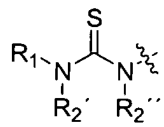

or a pharmaceutically acceptable salt or prodrug thereof.

In a preferred embodiment of the inventive compounds having A substituted as described above, D is selected from the group comprising $-(CH_2)_rC(O)H$, $—(CH_2)_rC \equiv N$, $—(CH_2)_rNHNHC(O)NR_{A'}R_{A''}$, $-C(CH_2)_rC(O)(CH_2)_{r'}C(O)OR_{A'}$, $—(CH_2)_rC(O)(CH_2)_{r'}C(O)NR_{A'}R_{A''}$, $-(CH_2)_rCH(OH)(CH_2)_{r'}C(O)U$, $—(CH_2)_rC(O)W$; whereby U is $—OR_{A'}$ or $—NR_{A'}R_{A''}$, W is $—OR_{A'}$, $-SR_{A'}$, $-NR_{A'}R_{A''}$, or a heterocyclic moiety; $R_{A'}$ and $R_{A''}$ are independently selected from H, alkyl, phenyl, benzyl, and phenethyl; and r and r' are independently any integer from 0 to 5. In an even more preferred embodiment D is $—(CH_2)_rC \equiv N$ and r is any integer from 0 to 3.

In a further embodiment of the compounds according to the present invention B is absent and the other residues have the same meaning as discussed in any of the aforementioned aspects and embodiments, respectively, of the present invention.

In an even preferred embodiment with B being absent, D is selected from the group comprising $-(CH_2)_rC(O)H$, $—(CH_2)_rC \equiv N$, $—(CH_2)_rNHNHC(O)NR_{A'}R_{A''}$, $-C(CH_2)_rC(O)(CH_2)_{r'}C(O)OR_{A'}$, $-(CH_2)_rC(O)(CH_2)_{r'}C(O)NR_{A'}R_{A''}$, $-(CH_2)_rCH(OH)(CH_2)_{r'}C(O)U$, $—(CH_2)_rC(O)W$;
whereby U is $—OR_{A'}$ or $—NR_{A'}R_{A''}$, W is $—OR_{A'}$, $-SR_{A'}$, $-NR_{A'}R_{A''}$, or a heterocyclic moiety; $R_{A'}$ and $R_{A''}$ are independently selected from H, alkyl, phenyl, benzyl, and phenethyl; and r and r' are independently any integer from 0 to 5. In a more preferred embodiment D is $—(CH_2)_rC \equiv N$ and r is any integer from 0 to 3.

Particularly preferred compounds according to the present invention are the compounds specified in the following table:

3-(1*H*-Indol-3-yl)-2-acetylamino-propionic acid cyanomethyl-amide,
3-(1*H*-Indol-3-yl)-2-[3-(4-trifluoromethyl-phenyl)-ureido]-propionic acid cyanomethyl-amide,
3-(1*H*-Indol-3-yl)-2-[3-(4-trifluoromethylsulfanyl-phenyl)-ureido]-propionic acid cyanomethyl-amide,
3-(1*H*-Indol-3-yl)-2-[3-(4-trifluoromethoxy-phenyl)-ureido]-propionic acid cyanomethyl-amide,
3-(1*H*-Indol-3-yl)-2-[3-(4-cyano-phenyl)-ureido]-propionic acid cyanomethyl-amide,
3-(1*H*-Indol-3-yl)-2-(3-benzyl-ureido)-propionic acid cyanomethyl-amide,
3-(1*H*-Indol-3-yl)-2-(3-o-tolyl-ureido)-propionic acid cyanomethyl-amide,
3-(1*H*-Indol-3-yl)-2-[3-(S)-(1-phenyl-ethyl)-ureido]-propionic acid cyanomethyl-amide,
3-(1*H*-Indol-3-yl)-2-[3-(2,6-dimethyl-phenyl)-ureido]-propionic acid cyanomethyl-amide,
3-(1*H*-Indol-3-yl)-2-[3-(3-methyl-benzyl)-ureido]-propionic acid cyanomethyl-amide,
3-(1*H*-Indol-3-yl)-2-[3-(1,1,3,3-tetramethyl-butyl)-ureido]-propionic acid cyanomethyl-amide,
3-(1*H*-Indol-3-yl)-2-(3-indan-5-yl-ureido)-propionic acid cyanomethyl-amide,
3-(1*H*-Indol-3-yl)-2-[3-(2-phenyl-cyclopropyl)-ureido]-propionic acid cyanomethyl-amide,
3-(1*H*-Indol-3-yl)-2-(3-adamantan-1-yl-ureido)-propionic acid cyanomethyl-amide,
3-(1*H*-Indol-3-yl)-2-(3-biphenyl-4-yl-ureido)-propionic acid cyanomethyl-amide,
3-(1*H*-Indol-3-yl)-2-[3-(4-phenoxy-phenyl)-ureido]-propionic acid cyanomethyl-amide,
3-(1*H*-Indol-3-yl)-2-[3-(4-nitro-phenyl)-ureido]-propionic acid cyanomethyl-amide,
3-(1*H*-Indol-3-yl)-2-(3-cyclohexyl-ureido)-propionic acid cyanomethyl-amide,
3-(1*H*-Indol-3-yl)-2-(3-benzo[1,3]dioxol-5-yl-ureido)-propionic acid cyanomethyl-amide,
3-(1*H*-Indol-3-yl)-2-[3-(2-fluoro-benzyl)-ureido]-propionic acid cyanomethyl-amide,
3-(1*H*-Indol-3-yl)-2-[3-(4-methyl-benzyl)-ureido]-propionic acid cyanomethyl-amide,
3-(1*H*-Indol-3-yl)-2-(3-phenethyl-ureido)-ureido]-propionic acid cyanomethyl-amide,
3-(1*H*-Indol-3-yl)-2-[3-(3,4,5-trimethoxy-phenyl)-ureido]-propionic acid cyanomethyl-amide,
3-(1*H*-Indol-3-yl)-2-(3-ethyl-thioureido)-propionic acid cyanomethyl-amide,
3-(1*H*-Indol-3-yl)-2-(3-isopropyl-thioureido)-propionic acid cyanomethyl-amide,
3-(1*H*-Indol-3-yl)-2-[3-(4-nitro-phenyl)-thioureido]-propionic acid cyanomethyl-amide,
3-(1*H*-Indol-3-yl)-2-(3-phenyl-thioureido)-propionic acid cyanomethyl-amide,
3-(1*H*-Indol-3-yl)-2-[3-(4-trifluoromethoxy-phenyl)-thioureido]-propionic acid cyanomethyl-amide,
3-(1*H*-Indol-3-yl)-2-{3-[4-(2,2,2-trifluoro-ethyl)-phenyl]-thioureido}-propionic acid cyanomethyl-amide,
3-(1*H*-Indol-3-yl)-2-[3-(4-methoxy-phenyl)-thioureido]-propionic acid cyanomethyl-amide,
3-(1*H*-Indol-3-yl)-2-(7,7-dimethyl-2-oxo-bicyclo[2.2.1]hept-1-ylmethanesulfonylamino)-propionic acid cyanomethyl-amide,

3-(1*H*-Indol-3-yl)-2-(thiophene-2-sulfonylamino)-propionic acid cyanomethyl-amide,
3-(1*H*-Indol-3-yl)-2-(4-trifluoromethoxy-benzenesulfonylamino)-propionic acid cyanomethyl-amide,
3-(1*H*-Indol-3-yl)-2-(4-*tert*-butyl-benzenesulfonylamino)-propionic acid cyanomethyl-amide,
3-(1*H*-Indol-3-yl)-2-(4-chloro-benzenesulfonylamino)-propionic acid cyanomethyl-amide,
3-(1*H*-Indol-3-yl)-2-(4-methoxy-benzenesulfonylamino)-propionic acid cyanomethyl-amide,
3-(1*H*-Indol-3-yl)-2-(quinoline-6-sulfonylamino)-propionic acid cyanomethyl-amide, 3-(1*H*-Indol-3-yl)-2-benzenesulfonylamino-propionic acid cyanomethyl-amide,
1-Methyl-1*H*-indole-2-carboxylic acid [1-(cyanomethyl-carbamoyl)-2-(1*H*-indol-3-yl)-ethyl]-amide,
2-Propyl-pentanoic acid [1-(cyanomethyl-carbamoyl)-2-(1*H*-indol-3-yl)-ethyl]-amide,
1-Methyl-cyclopropanecarboxylic acid [1-(cyanomethyl-carbarnoyl)-2-(1*H*-indol-3-yl)-ethyl]-amide,
Thiophene-2-carboxylic acid [1-(cyanomethyl-carbarnoyl)-2-(1*H*-indol-3-yl)-ethyl]-amide,
N-[1-(Cyanomethyl-carbamoyl)-2-(1*H*-indol-3-yl)-ethyl]-3-trifluoromethyl-benzamide,
Biphenyl-2-carboxylic acid [1-(cyanomethyl-carbamoyl)-2-(1*H*-indol-3-yl)-ethyl]-amide,
4-Acetylamino-*N*-[1-(cyanomethyl-carbamoyl)-2-(1H-indol-3-yl)-ethyl]-benzamide,
3-(1*H*-Indol-3-yl)-2-(2-1*H*-indol-3-yl-acetylamino)-propionic acid cyanomethyl-amide,
3-(1*H*-Indol-3-yl)-2-(3-1*H*-indol-3-yl-propionylamino)-propionic acid cyanomethyl-amide,
*N*-[1-(Cyanomethyl-carbamoyl)-2-(1*H*-indol-3-yl)-ethyl]-4-(1*H*-indol-3-yl)-butyramide,
N-[1-(Cyanomethyl-carbamoyl)-2-(1*H*-indol-3-yl)-ethyl]-benzamide,
3-Chloromethyl-*N*-[1-(cyanomethyl-carbamoyl)-2-(1*H*-indol-3-yl)-ethyl]-benzamide,
4-Chloromethyl-*N*-[1-(cyanomethyl-carbamoyl)-2-(1*H*-indol-3-yl)-ethyl]-benzamide,
*N*-[1-(Cyanomethyl-carbamoyl)-2-(1*H*-indol-3-yl)-ethyl]-2-fluoro-benzamide,
*N*-[1-(Cyanomethyl-carbamoyl)-2-(1*H*-indol-3-yl)-ethyl]-2-nitro-benzamide,
*N*-[1-(Cyanomethyl-carbamoyl)-2-(1*H*-indol-3-yl)-ethyl]-acrylamide,
3-(1*H*-Indol-3-yl)-2,2-dimethyl-propionic acid cyanomethyl-amide,
3-(1*H*-Indol-3-yl)-2-(2-methoxy-acetylamino)-propionic acid cyanomethyl-amide,
*N*-[1-(Cyanomethyl-carbamoyl)-2-(1*H*-indol-3-yl)-ethyl]-oxalamic acid methyl ester,
*N*-[1-(Cyanomethyl-carbamoyl)-2-(1*H*-indol-3-yl)-ethyl]-phthalamic acid,
*N*-[1-(Cyanomethyl-carbamoyl)-2-(1*H*-indol-3-yl)-ethyl]-succinamic acid,
3-[1-(Cyanomethyl-carbamoyl)-2-(1*H*-indol-3-yl)-ethylcarbamoyl]-acrylic acid,
[1-(Cyanomethyl-carbamoyl)-2-(1*H*-indol-3-yl)-ethyl]-carbamic acid isobutyl ester,
[1-(Cyanomethyl-carbamoyl)-2-(1*H*-indol-3-yl)-ethyl]-carbamic acid butyl ester,
[1-(Cyanomethyl-carbamoyl)-2-(1*H*-indol-3-yl)-ethyl]-carbamic acid hexyl ester,
[1-(Cyanomethyl-carbamoyl)-2-(1*H*-indol-3-yl)-ethyl]-carbamic acid *tert*-butyl ester,
[1-(Cyanomethyl-carbamoyl)-2-(1*H* indol-3-yl)-ethyl]-carbamic acid methyl ester,
[1-(Cyanomethyl-carbamoyl)-2-(1*H*-indol-3-yl)-ethyl]-carbamic acid ethyl ester,
[1-(Cyanomethyl-carbamoyl)-2-(1*H*-indol-3-yl)-ethyl]-carbamic acid 9*H*-fluoren-9-ylmethyl ester,
3-(1*H*-Indol-3-yl)-2-acetylamino-propionic acid cyanoethyl-amide,
3-(1*H*-Indol-3-yl)-2-[3-(4-trifluoromethyl-phenyl)-ureido]-propionic acid cyanoethyl-amide,
3-(1*H*-Indol-3-yl)-2-[3-(4-trifluoromethylsulfanyl-phenyl)-ureido]-propionic acid cyanoethyl-amide,
3-(1*H*-Indol-3-yl)-2-[3-(4-trifluoromethoxy-phenyl)-ureido]-propionic acid cyanoethyl-amide,
3-(1*H*-Indol-3-yl)-2-[3-(4-cyano-phenyl)-ureido]-propionic acid cyanoethyl-amide,
3-(1*H*-Indol-3-yl)-2-(3-benzyl-ureido)-propionic acid cyanoethyl-amide,
3-(1*H*-Indol-3-yl)-2-(3-o-tolyl-ureido)-propionic acid cyanoethyl-amide,
3-(1*H*-Indol-3-yl)-2-[3-(*S*)-(1-phenyl-ethyl)-ureido]-propionic acid cyanoethyl-amide,
3-(1*H*-Indol-3-yl)-2-[3-(2,6-dimethyl-phenyl)-ureido]-propionic acid cyanoethyl-amide,
3-(1*H*-Indol-3-yl)-2-[3-(3-methyl-benzyl)-ureido]-propionic acid cyanoethyl-amide,
3-(1*H*-Indol-3-yl)-2-[3-(1,1,3,3-tetramethyl-butyl)-ureido]-propionic acid cyanoethyl-amide,
3-(1*H*-Indol-3-yl)-2-(3-indan-5-yl-ureido)-propionic acid cyanoethyl-amide,
3-(1*H*-Indol-3-yl)-2-[3-(2-phenyl-cyclopropyl)-ureido]-propionic acid cyanoethyl-amide,
3-(1*H*-Indol-3-yl)-2-(3-adamantan-1-yl-ureido)-propionic acid cyanoethyl-amide,
3-(1*H*-Indol-3-yl)-2-(3-biphenyl-4-yl-ureido)-propionic acid cyanoethyl-amide,
3-(1*H*-Indol-3-yl)-2-[3-(4-phenoxy-phenyl)-ureido]-propionic acid cyanoethyl-amide,
3-(1*H*-Indol-3-yl)-2-[3-(4-nitro-phenyl)-ureido]-propionic acid cyanoethyl-amide,
3-(1*H*-Indol-3-yl)-2-(3-cyclohexyl-ureido)-propionic acid cyanoethyl-amide,
3-(1*H*-Indol-3-yl)-2-(3-benzo[1,3]dioxol-5-yl-ureido)-propionic acid cyanoethyl-amide,
3-(1*H*-Indol-3-yl)-2-[3-(2-fluoro-benzyl)-ureido]-propionic acid cyanoethyl-amide,
3-(1*H*-Indol-3-yl)-2-[3-(4-methyl-benzyl)-ureido]-propionic acid cyanoethyl-amide,
3-(1*H*-Indol-3-yl)-2-(3-phenethyl-ureido)-ureido]-propionic acid cyanoethyl-amide,

3-(1*H*-Indol-3-yl)-2-[3-(3,4,5-trimethoxy-phenyl)-ureido]-propionic acid cyanoethyl-amide,
3-(1*H*-Indol-3-yl)-2-(3-ethyl-thioureido)-propionic acid cyanoethyl-amide,
3-(1*H*-Indol-3-yl)-2-(3-isopropyl-thioureido)-propionic acid cyanoethyl-amide,
3-(1*H*-Indol-3-yl)-2-[3-(4-nitro-phenyl)-thioureido]-propionic acid cyanoethyl-amide,
3-(1*H*-Indol-3-yl)-2-(3-phenyl-thioureido)-propionic acid cyanoethyl-amide,
3-(1*H*-Indol-3-yl)-2-[3-(4-trifluoromethoxy-phenyl)-thioureido]-propionic acid cyanoethyl-amide,
3-(1*H*-Indol-3-yl)-2-{3-[4-(2,2,2-trifluoro-ethyl)-phenyl]-thioureido}-propionic acid cyanoethyl-amide,
3-(1*H*-Indol-3-yl)-2-[3-(4-methoxy-phenyl)-thioureido]-propionic acid cyanoethyl-amide,
3-(1*H*-Indol-3-yl)-2-(7,7-dimethyl-2-oxo-bicyclo[2.2.1]hept-1- ylmethanesulfonylamino)-propionic acid cyanoethyl-amide,
3-(1*H*-Indol-3-yl)-2-(thiophene-2-sulfonylamino)-propionic acid cyanoethyl-amide,
3-(1*H*-Indol-3-yl)-2-(4-trifluoromethoxy-benzenesulfonylamino)-propionic acid cyanoethyl-amide,
3-(1*H*-Indol-3-yl)-2-(4-tert-butyl-benzenesulfonylamino)-propionic acid cyanoethyl-amide,
3-(1*H*-Indol-3-yl)-2-(4-chloro-benzenesulfonylamino)-propionic acid cyanoethyl-amide,
3-(1*H*-Indol-3-yl)-2-(4-methoxy-benzenesulfonylamino)-propionic acid cyanoethyl-amide,
3-(1*H*-Indol-3-yl)-2-(quinoline-6-sulfonylamino)-propionic acid cyanoethyl-amide,
3-(1*H*-Indol-3-yl)-2-benzenesulfonylamino-propionic acid cyanoethyl-amide,
1-Methyl-1*H*-indole-2-carboxylic acid [1-(cyanoethyl-carbamoyl)-2-(1*H*-indol-3-yl)-ethyl]-amide
2-Propyl-pentanoic acid [1-(cyanoethyl-carbamoyl)-2-(1*H*-indol-3-yl)-ethyl]-amide,
1-Methyl-cyclopropanecarboxylic acid [1-(cyanoethyl-carbamoyl)-2-(1*H*-indol-3-yl)-ethyl]-amide,
Thiophene-2-carboxylic acid [1-(cyanoethyl-carbamoyl)-2-(1*H*-indol-3-yl)-ethyl]-amide,
*N*-[1-(Cyanoethyl-carbamoyl)-2-(1*H*-indol-3-yl)-ethyl]-3-trifluoromethyl-benzamide, Biphenyl-2-carboxylic acid [1-(cyanoethyl-carbamoyl)-2-(1*H*-indol-3-yl)-ethyl]-amide,
4-Acetylamino-*N*-[1-(cyanoethyl-carbamoyl)-2-(1*H*-indol-3-yl)-ethyl]-benzamide,
3-(1*H*-indol-3-yl)-2-(2-1*H*-indol-3-yl-acetylamino)-propionic acid cyanoethyl-amide,
3-(1*H*-Indol-3-yl)-2-(3-1*H*-indol-3-yl-propionylamino)-propionic acid cyanoethyl-amide,
*N*-[1-(Cyanoethyl-carbamoyl)-2-(1*H*-indol-3-yl)-ethyl]-4-(1*H*-indol-3-yl)-butyramide,
N-[1-(Cyanoethyl-carbamoyl)-2-(1*H*-indol-3-yl)-ethyl]-benzamide,
3-Chloromethyl-*N*-[1-(cyanoethyl-carbamoyl)-2-(1*H* indol-3-yl)-ethyl]-benzamide,
4-Chloromethyl-*N*-[1-(cyanoethyl-carbamoyl)-2-(1*H*-indol-3-yl)-ethyl]-benzamide,
*N*-[1-(Cyanoethyl-carbamoyl)-2-(1*H*-indol-3-yl)-ethyl]-2-fluoro-benzamide,
*N*-[1-(Cyanoethyl-carbamoyl)-2-(1*H*-indol-3-yl)-ethyl]-2-nitro-benzamide,
*N*-[1-(Cyanoethyl-carbamoyl)-2-(1*H*-indol-3-yl)-ethyl]-acrylamide,
3-(1*H*-Indol-3-yl)-2,2-dimethyl-propionic acid cyanoethyl-amide,
3-(1*H*-Indol-3-yl)-2-(2-methoxy-acetylamino)-propionic acid cyanoethyl-amide,
*N*-[1-(Cyanoethyl-carbamoyl)-2-(1*H*-indol-3-yl)-ethyl]-oxalamic acid methyl ester,
*N*-[1-(Cyanoethyl-carbamoyl)-2-(1*H*-indol-3-yl)-ethyl]-phthalamic acid,
*N*-[1-(Cyanoethyl-carbamoyl)-2-(1*H*-indol-3-yl)-ethyl]-succinamic acid,
3-[1-(Cyanoethyl-carbamoyl)-2-(1*H*-indol-3-yl)-ethylcarbamoyl]-acrylic acid,
[1-(Cyanoethyl-carbamoyl)-2-(1*H*-indol-3-yl)-ethyl]-carbamic acid isobutyl ester,
[1-(Cyanoethyl-carbamoyl)-2-(1*H*-indol-3-yl)-ethyl]-carbamic acid butyl ester,
[1-(Cyanomethyl-carbamoyl)-2-(1*H*-indol-3-yl)-ethyl]-carbamic acid cyanomethyl ester,
[1-(Cyanoethyl-carbamoyl)-2-(1*H*-indol-3-yl)-ethyl]-carbamic acid cyanomethyl ester,
[1-(Cyanomethyl-carbamoyl)-2-(1*H*-indol-3-yl)-ethyl]-carbamic acid but-3-enyl ester,
[1-(Cyanoethyl-carbamoyl)-2-(1*H*-indol-3-yl)-ethyl]-carbamic acid but-3-enyl ester,
[1-(Cyanomethyl-carbamoyl)-2-(1*H*-indol-3-yl)-ethyl]-carbamic acid 2-isopropyl-5-methyl-cyclohexyl ester,
[1-(Cyanoethyl-carbamoyl)-2-(1*H*-indol-3-yl)-ethyl]-carbamic acid 2-isopropyl-5-methyl-cyclohexyl ester,
[1-(Cyanoethyl-carbamoyl)-2-(1*H*-indol-3-yl)-ethyl]-carbamic acid hexyl ester,
[1-(Cyanoethyl-carbamoyl)-2-(1*H*-indol-3-yl)-ethyl]-carbamic acid *tert*-butyl ester,
[1-(Cyanoethyl-carbamoyl)-2-(1*H*-indol-3-yl)-ethyl]-carbamic acid methyl ester,
[1-(Cyanoethyl-carbamoyl)-2-(1*H*-indol-3-yl)-ethyl]-carbamic acid ethyl ester,
[1-(Cyanoethyl-carbamoyl)-2-(1*H*-indol-3-yl)-ethyl]-carbamic acid 9*H*-fluoren-9-ylmethylester,
3-(4-Hydroxy-phenyl)-2-acetylamino-propionic acid cyanomethyl-amide,
3-(4-Hydroxy-phenyl)-2-[3-(4-trifluoromethyl-phenyl)-ureido]-propionic acid cyanomethyl-amide,
3-(4-Hydroxy-phenyl)-2-[3-(4-trifluoromethylsulfanyl-phenyl)-ureido]-propionic acid cyanomethyl-amide,
3-(4-Hydroxy-phenyl)-2-[3-(4-trifluoromethoxy-phenyl)-ureido]-propionic acid cyanomethyl-amide,
3-(4-Hydroxy-phenyl)-2-[3-(4-cyano-phenyl)-ureido]-propionic acid cyanomethyl-amide,
3-(4-Hydroxy-phenyl)-2-(3-benzyl-ureido)-propionic acid cyanomethyl-amide,

3-(4-Hydroxy-phenyl)-2-(3-o-tolyl-ureido)-propionic acid cyanomethyl-amide,
3-(4-Hydroxy-phenyl)-2-[3-(*S*)-(1-phenyl-ethyl)-ureido]-propionic acid cyanomethyl-amide,
3-(4-Hydroxy-phenyl)-2-[3-(2,6-dimethyl-phenyl)-ureido]-propionic acid cyanomethyl-amide,
3-(4-Hydroxy-phenyl)-2-[3-(3-methyl-benzyl)-ureido]-propionic acid cyanomethyl-amide,
3-(4-Hydroxy-phenyl)-2-[3-(1,1,3,3-tetramethyl-butyl)-ureido]-propionic acid cyanomethyl-amide,
3-(4-Hydroxy-phenyl)-2-(3-indan-5-yl-ureido)-propionic acid cyanomethyl-amide,
3-(4-Hydroxy-phenyl)-2-[3-(2-phenyl-cyclopropyl)-ureido]-propionic acid cyanomethyl-amide,
3-(4-Hydroxy-phenyl)-2-(3-adamantan-1-yl-ureido)-propionic acid cyanomethyl-amide,
3-(4-Hydroxy-phenyl)-2-(3-biphenyl-4-yl-ureido)-propionic acid cyanomethyl-amide,
3-(4-Hydroxy-phenyl)-2-[3-(4-phenoxy-phenyl)-ureido]-propionic acid cyanomethyl-amide,
3-(4-Hydroxy-phenyl)-2-[3-(4-nitro-phenyl)-ureido]-propionic acid cyanomethyl-amide,
3-(4-Hydroxy-phenyl)-2-(3-cyclohexyl-ureido)-propionic acid cyanomethyl-amide,
3-(4-Hydroxy-phenyl)-2-(3-benzo[ 1,3]dioxol-5-yl-ureido)-propionic acid cyanomethyl-amide,
3-(4-Hydroxy-phenyl)-2-[3-(2-fluoro-benzyl)-ureido]-propionic acid cyanomethyl-amide,
3-(4-Hydroxy-phenyl)-2-[3-(4-methyl-benzyl)-ureido]-propionic acid cyanomethyl-amide,
3-(4-Hydroxy-phenyl)-2-(3-phenethyl-ureido) -ureido]-propionic acid cyanomethyl-amide,
3-(4-Hydroxy-phenyl)-2-[3-(3,4,5-trimethoxy-phenyl)-ureido]-propionic acid cyanomethyl-amide,
3-(4-Hydroxy-phenyl)-2-(3-ethyl-thioureido)-propionic acid cyanomethyl-amide,
3-(4-Hydroxy-phenyl)-2-(3-isopropyl-thioureido)-propionic acid cyanomethyl-amide,
3-(4-Hydroxy-phenyl)-2-[3-(4-nitro-phenyl)-thioureido]-propionic acid cyanomethyl-amide,
3-(4-Hydroxy-phenyl)-2-(3-phenyl-thioureido)-propionic acid cyanomethyl-amide,
3-(4-Hydroxy-phenyl)-2-[3-(4-trifluoromethoxy-phenyl)-thioureido]-propionic acid cyanomethyl-amide,
3-(4-Hydroxy-phenyl)-2-{3-[4-(2,2,2-trifluoro-ethyl)-phenyl]-thioureido}-propionic acid cyanomethyl-amide,
3-(4-Hydroxy-phenyl)-2-[3-(4-methoxy-phenyl)-thioureido]-propionic acid cyanomethyl-amide,
3-(4-Hydroxy-phenyl)-2-(7,7-dimethyl-2-oxo-bicyclo[2.2.1]hept-1- ylmethanesulfonylamino)-propionic acid cyanomethyl-amide,
3-(4-Hydroxy-phenyl)-2-(thiophene-2-sulfonylamino)-propionic acid cyanomethyl-amide,
3-(4-Hydroxy-phenyl)-2-(4-trifluoromethoxy-benzenesulfonylamino)-propionic acid cyanomethyl-amide,
3-(4-Hydroxy-phenyl)-2-(4-*tert*-butyl-benzenesulfonylamino)-propionic acid cyanomethyl-amide,
3-(4-Hydroxy-phenyl)-2-(4-chloro-benzenesulfonylamino)-propionic acid cyanomethyl-amide,
3-(4-Hydroxy-phenyl)-2-(4-methoxy-benzenesulfonylamino)-propionic acid cyanomethyl-amide,
3-(4-Hydroxy-phenyl)-2-(quinoline-6-sulfonylamino)-propionic acid cyanomethyl-amide,
3-(4-Hydroxy-phenyl)-2-benzenesulfonylamino-propionic acid cyanomethyl-amide,
1-Methyl-1H-indole-2-carboxylic acid [1-(cyanomethyl-carbamoyl)-2-(4-hydroxy-phenyl)-ethyl]-amide,
2-Propyl-pentanoic acid [1-(cyanomethyl-carbamoyl)-2-(4-hydroxy-phenyl)-ethyl]-amide,
1-Methyl-cyclopropanecarboxylic acid [1-(cyanomethyl-carbamoyl)-2-(4-hydroxy-phenyl)-ethyl]-amide,
Thiophene-2-carboxylic acid [1-(cyanomethyl-carbamoyl)-2-(4-hydroxy-phenyl)-ethyl]-amide,
*N*-[1-(Cyanomethyl-carbamoyl)-2-(4-hydroxy-phenyl)-ethyl]-3-trifluoromethyl-benzamide,
Biphenyl-2-carboxylic acid [1-( cyanomethyl-carbamoyl)-2-( 4-hydroxy-phenyl)-ethyl]-amide,
4-Acetylamino-*N*-[1-(cyanomethyl-carbamoyl)-2-(4-hydroxy-phenyl)-ethyl]-benzamide,
3-(4-Hydroxy-phenyl)-2-(2-1*H*-indol-3-yl-acetylamino)-propionic acid cyanomethyl-amide,
3-(4-Hydroxy-phenyl)-2-(3-1H indol-3-yl-propionylamino)-propionic acid cyanomethyl-amide,
*N*-[1-(Cyanomethyl-carbamoyl)-2-(4-hydroxy-phenyl)-ethyl]-4-(1*H*-indol-3-yl)-butyramide,
*N*-[1-(Cyanomethyl-carbamoyl)-2-(4-hydroxy-phenyl)-ethyl]-benzamide,
3-Chloromethyl-*N*-[1-(cyanomethyl-carbamoyl)-2-(4-hydroxy-phenyl)-ethyl]-benzamide,
4-Chloromethyl-*N*-[1-(cyanomethyl-carbamoyl)-2-(4-hydroxy-phenyl)-ethyl]-benzamide,
*N*-[1-(Cyanomethyl-carbamoyl)-2-(4-hydroxy-phenyl)-ethyl]-2-fluoro-benzamide,
*N*-[1-(Cyanomethyl-carbamoyl)-2-(4-hydroxy-phenyl)-ethyl]-2-nitro-benzamide,
*N*-[1-(Cyanomethyl-carbamoyl)-2-(4-hydroxy-phenyl)-ethyl]-acrylamide,
3-(4-Hydroxy-phenyl)-2,2-dimethyl-propionic acid cyanomethyl-amide,
3-(4-Hydroxy-phenyl)-2-(2-methoxy-acetylamino)-propionic acid cyanomethyl-amide,
*N*-[1-(Cyanomethyl-carbamoyl)-2-(4-hydroxy-phenyl)-ethyl]-oxalamic acid methyl ester,
*N*-[1-(Cyanomethyl-carbamoyl)-2-(4-hydroxy-phenyl)-ethyl]-phthalamic acid,
*N*-[1-(Cyanomethyl-carbamoyl)-2-(4-hydroxy-phenyl)-ethyl]-succinamic acid,
3-[1-(Cyanomethyl-carbamoyl)-2-(4-hydroxy-phenyl)-ethylcarbamoyl]-acrylic acid,
[1-(Cyanomethyl-carbamoyl)-2-(4-hydroxy-phenyl)-ethyl]-carbamic acid isobutyl ester,
[1-(Cyanomethyl-carbamoyl)-2-(4-hydroxy-phenyl)-ethyl]-carbamic acid butyl ester,
[1-(Cyanomethyl-carbamoyl)-2-(4-hydroxy-phenyl)-ethyl]-carbamic acid hexyl ester,

[1-(Cyanomethyl-carbamoyl)-2-(4-hydroxy-phenyl)-ethyl]-carbamic acid tert-butyl ester,
[1-(Cyanomethyl-carbamoyl)-2-(4-hydroxy-phenyl)-ethyl]-carbamic acid methyl ester,
[1-(Cyanomethyl-carbamoyl)-2-(4-hydroxy-phenyl)-ethyl]-carbamic acid ethyl ester,
[1-(Cyanomethyl-carbamoyl)-2-(4-hydroxy-phenyl)-ethyl]-carbamic acid 9*H*-fluoren-9-ylmethyl ester,
3-(4-Hydroxy-phenyl)-2-acetylamino-propionic acid cyanoethyl-amide,
3-(4-Hydroxy-phenyl)-2-[3-(4-trifluoromethyl-phenyl)-ureido]-propionic acid cyanoethyl-amide,
3-(4-Hydroxy-phenyl)-2-[3-(4-trifluoromethylsulfanyl-phenyl)-ureido]-propionic acid cyanoethyl-amide,
3-(4-Hydroxy-phenyl)-2-[3-(4-trifluoromethoxy-phenyl)-ureido]-propionic acid cyanoethyl-amide,
3-(4-Hydroxy-phenyl)-2-[3-(4-cyano-phenyl)-ureido]-propionic acid cyanoethyl-amide,
3-(4-Hydroxy-phenyl)-2-(3-benzyl-ureido)-propionic acid cyanoethyl-amide,
3-(4-Hydroxy-phenyl)-2-(3-o-tolyl-ureido)-propionic acid cyanoethyl-amide,
3-(4-Hydroxy-phenyl)-2-[3-(*S*)-(1-phenyl-ethyl)-ureido]-propionic acid cyanoethyl-amide,
3-(4-Hydroxy-phenyl)-2-[3-(2,6-dimethyl-phenyl)-ureido]-propionic acid cyanoethyl-amide,
3-(4-Hydroxy-phenyl)-2-[3-(3-methyl-benzyl)-ureido]-propionic acid cyanoethyl-amide,
3-(4-Hydroxy-phenyl)-2-[3-(1,1,3,3-tetramethyl-butyl)-ureido]-propionic acid cyanoethyl-amide,
3-(4-Hydroxy-phenyl)-2-(3-indan-5-yl-ureido)-propionic acid cyanoethyl-amide,
3-(4-Hydroxy-phenyl)-2-[3-(2-phenyl-cyclopropyl)-ureido]-propionic acid cyanoethyl-amide,
3-(4-Hydroxy-phenyl)-2-(3-adamantan-1-yl-ureido)-propionic acid cyanoethyl-amide,
3-(4-Hydroxy-phenyl)-2-(3-biphenyl-4-yl-ureido)-propionic acid cyanoethyl-amide,
3-(4-Hydroxy-phenyl)-2-[3-(4-phenoxy-phenyl)-ureido]-propionic acid cyanoethyl-amide,
3-(4-Hydroxy-phenyl)-2-[3-(4-nitro-phenyl)-ureido]-propionic acid cyanoethyl-amide,
3-(4-Hydroxy-phenyl)-2-(3-cyclohexyl-ureido)-propionic acid cyanoethyl-amide,
3-(4-Hydroxy-phenyl)-2-(3-benzo[1,3]dioxol-5-yl-ureido)-propionic acid cyanoethyl-amide,
3-(4-Hydroxy-phenyl)-2-[3-(2-fluoro-benzyl)-ureido]-propionic acid cyanoethyl-amide,
3-(4-Hydroxy-phenyl)-2-[3-(4-methyl-benzyl)-ureido]-propionic acid cyanoethyl-amide,
3-(4-Hydroxy-phenyl)-2-(3-phenethyl-ureido) -ureido]-propionic acid cyanoethyl-amide,
3-(4-Hydroxy-phenyl)-2-[3-(3,4,5-trimethoxy-phenyl)-ureido]-propionic acid cyanoethyl-amide,
3-(4-Hydroxy-phenyl)-2-(3-ethyl-thioureido)-propionic acid cyanoethyl-amide,
3-(4-Hydroxy-phenyl)-2-(3-isopropyl-thioureido)-propionic acid cyanoethyl-amide,
3-(4-Hydroxy-phenyl)-2-[3-(4-nitro-phenyl)-thioureido]-propionic acid cyanoethyl-amide,
3-(4-Hydroxy-phenyl)-2-(3-phenyl-thioureido)-propionic acid cyanoethyl-amide,
3-(4-Hydroxy-phenyl)-2-[3-(4-trifluoromethoxy-phenyl)-thioureido]-propionic acid cyanoethyl-amide,
3-(4-Hydroxy-phenyl)-2-{3-[4-(2,2,2-trifluoro-ethyl)-phenyl]-thioureido-propionic acid cyanoethyl-amide,
3-(4-Hydroxy-phenyl)-2-[3-(4-methoxy-phenyl)-thioureido]-propionic acid cyanoethyl-amide,
3-(4-Hydroxy-phenyl)-2-(7,7-dimethyl-2-oxo-bicyclo[2.2.1]hept-1-ylmethanesulfonylamino)-propionic acid cyanoethyl-amide,
3-(4-Hydroxy-phenyl)-2-(thiophene-2-sulfonylamino)-propionic acid cyanoethyl-amide,
3-(4-Hydroxy-phenyl)-2-(4-trifluoromethoxy-benzenesulfonylamino)-propionic acid cyanoethyl-amide,
3-(4-Hydroxy-phenyl)-2-(4-*tert*-butyl-benzenesulfonylamino)-propionic acid cyanoethyl-amide,
3-(4-Hydroxy-phenyl)-2-(4-chloro-benzenesulfonylamino)-propionic acid cyanoethyl-amide,
3-(4-Hydroxy-phenyl)-2-(4-methoxy-benzenesulfonylamino)-propionic acid cyanoethyl-amide,
3-(4-Hydroxy-phenyl)-2-(quinoline-6-sulfonylamino)-propionic acid cyanoethyl-amide,
3-(4-Hydroxy-phenyl)-2-benzenesulfonylamino-propionic acid cyanoethyl-amide,
1-Methyl-1H-indole-2-carboxylic acid [1-(cyanoethyl-carbamoyl)-2-(4-hydroxy-phenyl)-ethyl]-amide,
2-Propyl-pentanoic acid [1-(cyanoethyl-carbamoyl)-2-(4-hydroxy-phenyl)-ethyl]-amide,
1-Methyl-cyclopropanecarboxylic acid [1-(cyanoethyl-carbamoyl)-2-(4-hydroxy-phenyl)-ethyl]-amide,
Thiophene-2-carboxylic acid [1-(cyanoethyl-carbamoyl)-2-(4-hydroxy-phenyl)-ethyl]-amide,
*N*-[1-(Cyanoethyl-carbamoyl)-2-(4-hydroxy-phenyl)-ethyl]-3-trifluoromethyl-benzamide,
Biphenyl-2-carboxylic acid [1-(cyanoethyl-carbamoyl)-2-(4-hydroxy-phenyl)-ethyl]-amide,
4-Acetylamino-*N*-[1-(cyanoethyl-carbamoyl)-2-(4-hydroxy-phenyl)-ethyl]-benzamide,
3-(4-hydroxy-phenyl)-2-(2-1*H*-indol-3-yl-acetylamino)-propionic acid cyanoethyl-amide,
3-(4-Hydroxy-phenyl)-2-(3-1*H*-indol-3-yl-propionylamino)-propionic acid cyanoethyl-amide,
*N*-[1-(Cyanoethyl-carbamoyl)-2-(4-hydroxy-phenyl)-ethyl]-4-(1*H*-indol-3-yl)-butyramide,
N-[1-(Cyanoethyl-carbamoyl)-2-(4-hydroxy-phenyl)-ethyl]-benzamide,
3-Chloromethyl-*N*-[1-(cyanoethyl-carbamoyl)-2-(4-hydroxy-phenyl)-ethyl]-benzamide,
4-Chloromethyl-*N*-[1-(cyanoethyl-carbamoyl)-2-(4-hydroxy-phenyl)-ethyl]-benzamide,
*N*-[1-(Cyanoethyl-carbamoyl)-2-(4-hydroxy-phenyl)-ethyl]-2-fluoro-benzamide,
*N*-[1-(Cyanoethyl-carbamoyl)-2-(4-hydroxy-phenyl)-ethyl]-2-nitro-benzamide,

*N*-[1-(Cyanoethyl-carbamoyl)-2-(4-hydroxy-phenyl)-ethyl]-acrylamide,
3-(4-Hydroxy-phenyl)-2,2-dimethyl-propionic acid cyanoethyl-amide,
3-(4-Hydroxy-phenyl)-2-(2-methoxy-acetylamino)-propionic acid cyanoethyl-amide,
*N*-[1-(Cyanoethyl-carbamoyl)-2-(4-hydroxy-phenyl)-ethyl]-oxalamic acid methyl ester,
*N*-[1-(Cyanoethyl-carbamoyl)-2-(4-hydroxy-phenyl)-ethyl]-phthalamic acid,
*N*-[1-(Cyanoethyl-carbamoyl)-2-(4-hydroxy-phenyl)-ethyl]-succinamic acid,
3-[1-(Cyanoethyl-carbamoyl)-2-(4-hydroxy-phenyl)-ethylcarbamoyl]-acrylic acid,
[1-(Cyanoethyl-carbamoyl)-2-(4-hydroxy-phenyl)-ethyl]-carbamic acid isobutyl ester,
[1-(Cyanoethyl-carbamoyl)-2-(4-hydroxy-phenyl)-ethyl]-carbamic acid butyl ester,
[1-(Cyanomethyl-carbamoyl)-2-(4-hydroxy-phenyl)-ethyl]-carbamic acid cyanomethyl ester,
[1-(Cyanoethyl-carbamoyl)-2-(4-hydroxy-phenyl)-ethyl]-carbamic acid cyanomethyl ester,
[1-(Cyanomethyl-carbamoyl)-2-(4-hydroxy-phenyl)-ethyl]-carbamic acid but-3-enyl ester,
[1-(Cyanoethyl-carbamoyl)-2-(4-hydroxy-phenyl)-ethyl]-carbamic acid but-3-enyl ester,
[1-(Cyanomethyl-carbamoyl)-2-(4-hydroxy-phenyl)-ethyl]-carbamic acid 2-isopropyl-5-methyl-cyclohexyl ester,
[1-(Cyanoethyl-carbamoyl)-2-(4-hydroxy-phenyl)-ethyl]-carbamic acid 2-isopropyl-5-methyl-cyclohexyl ester,
[1-(Cyanoethyl-carbamoyl)-2-(4-hydroxy-phenyl)-ethyl]-carbamic acid hexyl ester,
[1-(Cyanoethyl-carbamoyl)-2-(4-hydroxy-phenyl)-ethyl]-carbamic acid tert-butyl ester,
[1-(Cyanoethyl-carbamoyl)-2-(4-hydroxy-phenyl)-ethyl]-carbamic acid methyl ester,
[1-(Cyanoethyl-carbamoyl)-2-(4-hydroxy-phenyl)-ethyl]-carbamic acid ethyl ester,
[1-(Cyanoethyl-carbamoyl)-2-(4-hydroxy-phenyl)-ethyl]-carbamic acid 9*H*-fluoren-9-ylmethylester,
3-Phenyl-2-acetylamino-propionic acid cyanomethyl-amide,
3-Phenyl-2-[3-(4-trifluoromethyl-phenyl)-ureido]-propionic acid cyanomethyl-amide,
3-Phenyl-2-[3-(4-trifluoromethylsulfanyl-phenyl)-ureido]-propionic acid cyanomethyl-amide,
3-Phenyl-2-[3-(4-trifluoromethoxy-phenyl)-ureido]-propionic acid cyanomethyl-amide,
3-Phenyl-2-[3-(4-cyano-phenyl)-ureido]-propionic acid cyanomethyl-amide,
3-Phenyl-2-(3-benzyl-ureido)-propionic acid cyanomethyl-amide,
3-Phenyl-2-(3-o-tolyl-ureido)-propionic acid cyanomethyl-amide,
3-Phenyl-2-[3-(*S*)-(1-phenyl-ethyl)-ureido]-propionic acid cyanomethyl-amide,
3-Phenyl-2-[3-(2,6-dimethyl-phenyl)-ureido]-propionic acid cyanomethyl-amide,
3-Phenyl-2-[3-(3-methyl-benzyl)-ureido]-propionic acid cyanomethyl-amide,
3-Phenyl-2-[3-(1,1,3,3-tetramethyl-butyl)-ureido]-propionic acid cyanomethyl-amide,
3-Phenyl-2-(3-indan-5-yl-ureido)-propionic acid cyanomethyl-amide,
3-Phenyl-2-[3-(2-phenyl-cyclopropyl)-ureido]-propionic acid cyanomethyl-amide,
3-Phenyl-2-(3-adamantan-1-yl-ureido)-propionic acid cyanomethyl-amide,
3-Phenyl-2-(3-biphenyl-4-yl-ureido)-propionic acid cyanomethyl-amide,
3-Phenyl-2-[3-(4-phenoxy-phenyl)-ureido]-propionic acid cyanomethyl-amide,
3-Phenyl-2-[3-(4-nitro-phenyl)-ureido]-propionic acid cyanomethyl-amide,
3-Phenyl-2-(3-cyclohexyl-ureido)-propionic acid cyanomethyl-amide,
3-Phenyl-2-(3-benzo[1,3]dioxol-5-yl-ureido)-propionic acid cyanomethyl-amide,
3-Phenyl-2-[3-(2-fluoro-benzyl)-ureido]-propionic acid cyanomethyl-amide,
3-Phenyl-2-[3-(4-methyl-benzyl)-ureido]-propionic acid cyanomethyl-amide,
3-Phenyl-2-(3-phenethyl-ureido) -ureido]-propionic acid cyanomethyl-amide,
3-Phenyl-2-[3-(3,4,5-trimethoxy-phenyl)-ureido]-propionic acid cyanomethyl-amide,
3-Phenyl-2-(3-ethyl-thioureido)-propionic acid cyanomethyl-amide,
3-Phenyl-2-(3-isopropyl-thioureido)-propionic acid cyanomethyl-amide,
3-Phenyl-2-[3-(4-nitro-phenyl)-thioureido]-propionic acid cyanomethyl-amide,
3-Phenyl-2-(3-phenyl-thioureido)-propionic acid cyanomethyl-amide,
3-Phenyl-2-[3-(4-trifluoromethoxy-phenyl)-thioureido]-propionic acid cyanomethyl-amide,
3-Phenyl-2-{3-[4-(2,2,2-triftuoro-ethyl)-phenyl]-thioureido}-propionic acid cyanomethyl-amide,
3-Phenyl-2-[3-(4-methoxy-phenyl)-thioureido]-propionic acid cyanomethyl-amide,
3-Phenyl-2-(7,7-dimethyl-2-oxo-bicyclo[2.2.1 ]hept-1-ylmethanesulfonylamino)-propionic acid cyanomethyl-amide,
3-Phenyl-2-(thiophene-2-sulfonylamino)-propionic acid cyanomethyl-amide,
3-Phenyl-2-(4-trifluoromethoxy-benzenesulfonylamino)-propionic acid cyanomethyl-amide,
3-Phenyl-2-(4-*tert*-butyl-benzenesulfonylamino)-propionic acid cyanomethyl-amide,
3-Phenyl-2-(4-chloro-benzenesulfonylamino)-propionic acid cyanomethyl-amide,
3-Phenyl-2-(4-methoxy-benzenesulfonylamino)-propionic acid cyanomethyl-amide,
3-Phenyl-2-(quinoline-6-sulfonylamino)-propionic acid cyanomethyl-amide,

3-Phenyl-2-benzenesulfonylamino-propionic acid cyanomethyl-amide,
1-Methyl-1H-indole-2-carboxylic acid [1-(cyanomethyl-carbamoyl)-2-phenyl-ethyl]-amide,
2-Propyl-pentanoic acid [1-(cyanomethyl-carbamoyl)-2-phenyl-ethyl]-amide,
1-Methyl-cyclopropanecarboxylic acid [1-(cyanomethyl-carbamoyl)-2-phenyl-ethyl]-amide,
Thiophene-2-carboxylic acid [1-(cyanomethyl-carbamoyl)-2-phenyl-ethyl]-amide,
*N*-[1-(Cyanomethyl-carbamoyl)-2-phenyl-ethyl]-3-trifluoromethyl-benzamide,
Biphenyl-2-carboxylic acid [1-(cyanomethyl-carbamoyl)-2-phenyl-ethyl]-amide,
4-Acetylamino-*N*-[1-(cyanomethyl-carbamoyl)-2-phenyl-ethyl]-benzamide,
3-Phenyl-2-(2-1*H*-indol-3-yl-acetylamino)-propionic acid cyanomethyl-amide,
3-Phenyl-2-(3-1*H*-indol-3-yl-propionylamino)-propionic acid cyanomethyl-amide,
*N*-[1-(Cyanomethyl-carbamoyl)-2-phenyl-ethyl]-4-(1*H*-indol-3-yl)-butyramide,
N-[1-(Cyanomethyl-carbamoyl)-2-phenyl-ethyl]-benzamide,
3-Chloromethyl-*N*-[1-(cyanomethyl-carbamoyl)-2-phenyl-ethyl]-benzamide,
4-Chloromethyl-*N*-[1-(cyanomethyl-carbamoyl)-2-phenyl-ethyl]-benzamide,
*N*-[1-(Cyanomethyl-carbamoyl)-2-phenyl-ethyl]-2-fluoro-benzamide,
*N*-[1-(Cyanomethyl-carbamoyl)-2-phenyl-ethyl]-2-nitro-benzamide,
*N*-[1-(Cyanomethyl-carbamoyl)-2-phenyl-ethyl]-acrylamide,
3-Phenyl-2,2-dimethyl-propionic acid cyanomethyl-amide,
3-Phenyl-2-(2-methoxy-acetylamino)-propionic acid cyanomethyl-amide,
*N*-[1-(Cyanomethyl-carbamoyl)-2-phenyl-ethyl]-oxalamic acid methyl ester,
*N*-[1-(Cyanomethyl-carbamoyl)-2-phenyl-ethyl]-phthalamic acid,
*N*-[1-(Cyanomethyl-carbamoyl)-2-phenyl-ethyl]-succinamic acid,
3-[1-(Cyanomethyl-carbamoyl)-2-phenyl-ethylcarbamoyl]-acrylic acid,
[1-(Cyanomethyl-carbamoyl)-2-phenyl-ethyl]-carbamic acid isobutyl ester,
[1-(Cyanomethyl-carbamoyl)-2-phenyl-ethyl]-carbamic acid butyl ester,
[1-(Cyanomethyl-carbamoyl)-2-phenyl-ethyl]-carbamic acid hexyl ester,
[1-(Cyanomethyl-carbamoyl)-2-phenyl-ethyl]-carbamic acid tert-butyl ester,
[1-(Cyanomethyl-carbamoyl)-2-phenyl-ethyl]-carbamic acid methyl ester,
[1-(Cyanomethyl-carbamoyl)-2-phenyl-ethyl]-carbamic acid ethyl ester,
[1-(Cyanomethyl-carbamoyl)-2-phenyl-ethyl]-carbamic acid 9*H*-fluoren-9-ylmethyl ester,
3-Phenyl-2-acetylamino-propionic acid cyanoethyl-amide,
3-Phenyl-2-[3-(4-trifluoromethyl-phenyl)-ureido]-propionic acid cyanoethyl-amide
3-Phenyl-2-[3-(4-trifluoromethylsulfanyl-phenyl)-ureido]-propionic acid cyanoethyl-amide,
3-Phenyl-2-[3-(4-trifluoromethoxy-phenyl)-ureido]-propionic acid cyanoethyl-amide,
3-Phenyl-2-[3-(4-cyano-phenyl)-ureido]-propionic acid cyanoethyl-amide,
3-Phenyl-2-(3-benzyl-ureido)-propionic acid cyanoethyl-amide,
3-Phenyl-2-(3-o-tolyl-ureido)-propionic acid cyanoethyl-amide,
3-Phenyl-2-[3-(*S*)-(1-phenyl-ethyl)-ureido]-propionic acid cyanoethyl-amide,
3-Phenyl-2-[3-(2,6-dimethyl-phenyl)-ureido]-propionic acid cyanoethyl-amide,
3-Phenyl-2-(3-(3-methyl-benzyl)-ureido]-propionic acid cyanoethyl-amide,
3-Phenyl-2-[3-(1,1,3,3-tetramethyl-butyl)-ureido]-propionic acid cyanoethyl-amide,
3-Phenyl-2-(3-indan-5-yl-ureido)-propionic acid cyanoethyl-amide,
3-Phenyl-2-[3-(2-phenyl-cyclopropyl)-ureido]-propionic acid cyanoethyl-amide,
3-Phenyl-2-(3-adamantan-1-yl-ureido)-propionic acid cyanoethyl-amide,
3-Phenyl-2-(3-biphenyl-4-yl-ureido)-propionic acid cyanoethyl-amide,
3-Phenyl-2-[3-(4-phenoxy-phenyl)-ureido]-propionic acid cyanoethyl-amide,
3-Phenyl-2-[3-(4-nitro-phenyl)-ureido]-propionic acid cyanoethyl-amide,
3-Phenyl-2-(3-cyclohexyl-ureido)-propionic acid cyanoethyl-amide,
3-Phenyl-2-(3-benzo[1,3]dioxol-5-yl-ureido)-propionic acid cyanoethyl-amide,
3-Phenyl-2-[3-(2-fluoro-benzyl)-ureido]-propionic acid cyanoethyl-amide,
3-Phenyl-2-[3-(4-methyl-benzyl)-ureido]-propionic acid cyanoethyl-amide,
3-Phenyl-2-(3-phenethyl-ureido) -ureido]-propionic acid cyanoethyl-amide,
3-Phenyl-2-[3-(3,4,5-trimethoxy-phenyl)-ureido]-propionic acid cyanoethyl-amide,
3-Phenyl-2-(3-ethyl-thioureido)-propionic acid cyanoethyl-amide,
3-Phenyl-2-(3-isopropyl-thioureido)-propionic acid cyanoethyl-amide,
3-Phenyl-2-[3-(4-nitro-phenyl)-thioureido]-propionic acid cyanoethyl-amide,
3-Phenyl-2-(3-phenyl-thioureido)-propionic acid cyanoethyl-amide,
3-Phenyl-2-[3-(4-trifluoromethoxy-phenyl)-thioureido]-propionic acid cyanoethyl-amide,

3-Phenyl-2-{3-[4-(2,2,2-trifluoro-ethyl)-phenyl]-thioureido}-propionic acid cyanoethyl-amide,
3-Phenyl-2-[3-(4-methoxy-phenyl)-thioureido]-propionic acid cyanoethyl-amide,
3-Phenyl-2-(7,7-dimethyl-2-oxo-bicyclo[2.2.1 ]hept-1-ylmethanesulfonylamino)-propionic acid cyanoethyl-amide,
3-Phenyl-2-(thiophene-2-sulfonylamino)-propionic acid cyanoethyl-amide,
3-Phenyl-2-(4-trifluoromethoxy-benzenesulfonylamino)-propionic acid cyanoethyl-amide,
3-Phenyl-2-(4-*tert*-butyl-benzenesulfonylamino)-propionic acid cyanoethyl-amide,
3-Phenyl-2-(4-chloro-benzenesulfonylamino)-propionic acid cyanoethyl-amide,
3-Phenyl-2-(4-methoxy-benzenesulfonylamino)-propionic acid cyanoethyl-amide,
3-Phenyl-2-(quinoline-6-sulfonylamino)-propionic acid cyanoethyl-amide,
3-Phenyl-2-benzenesulfonylamino-propionic acid cyanoethyl-amide,
1-Methyl-1H-indole-2-carboxylic acid [1-(cyanoethyl-carbamoyl)-2-phenyl-ethyl]-amide,
2-Propyl-pentanoic acid [1-(cyanoethyl-carbamoyl)-2-phenyl-ethyl]-amide,
1-Methyl-cyclopropanecarboxylic acid [1-(cyanoethyl-carbamoyl)-2-phenyl-ethyl]-amide,
Thiophene-2-carboxylic acid [1-(cyanoethyl-carbamoyl)-2-phenyl-ethyl]-amide,
*N*-[1-(Cyanoethyl-carbamoyl)-2-phenyl-ethyl]-3-trifluoromethyl-benzamide,
Biphenyl-2-carboxylic acid [1-(cyanoethyl-carbamoyl)-2-phenyl-ethyl]-amide,
4-Acetylamino-*N*-[1-(cyanoethyl-carbamoyl)-2-phenyl-ethyl]-benzamide,
3-Phenyl-2-(2-1*H*-indol-3-yl-acetylamino)-propionic acid cyanoethyl-amide,
3-Phenyl-2-(3-1*H*-indol-3-yl-propionylamino)-propionic acid cyanoethyl-amide,
*N*-[1-(Cyanoethyl-carbamoyl)-2-phenyl-ethyl]-4-(1*H*-indol-3-yl)-butyramide,
*N*-[1-(Cyanoethyl-carbamoyl)-2-phenyl-ethyl]-benzamide,
3-Chloromethyl-*N*-[1-(cyanoethyl-carbamoyl)-2-phenyl-ethyl]-benzamide,
4-Chloromethyl-*N*-[1-(cyanoethyl-carbamoyl)-2-phenyl-ethyl)-benzamide,
*N*-[1-(Cyanoethyl-carbamoyl)-2-phenyl-ethyl]-2-fluoro-benzamide,
*N*-[1-(Cyanoethyl-carbamoyl)-2-phenyl-ethyl]-2-nitro-benzamide,
*N*-[1-(Cyanoethyl-carbamoyl)-2-phenyl-ethyl]-acrylamide,
3-Phenyl-2,2-dimethyl-propionic acid cyanoethyl-amide,
3-Phenyl-2-(2-methoxy-acetylamino)-propionic acid cyanoethyl-amide,
*N*-[1-(Cyanoethyl-carbamoyl)-2-phenyl-ethyl]-oxalamic acid methyl ester,
*N*-[1-(Cyanoethyl-carbamoyl)-2-phenyl-ethyl]-phthalamic acid,
*N*-[1-(Cyanoethyl-carbamoyl)-2-phenyl-ethyl]-succinamic acid,
3-[1-(Cyanoethyl-carbamoyl)-2-phenyl-ethylcarbamoyl)-acrylic acid,
[1-(Cyanoethyl-carbamoyl)-2-phenyl-ethyl]-carbamic acid isobutyl ester,
[1-(Cyanoethyl-carbamoyl)-2-phenyl-ethyl]-carbamic acid butyl ester,
[1-(Cyanomethyl-carbamoyl)-2-phenyl-ethyl]-carbamic acid cyanomethyl ester,
[1-(Cyanoethyl-carbamoyl)-2-phenyl-ethyl]-carbamic acid cyanomethyl ester,
[1-(Cyanomethyl-carbamoyl)-2-phenyl-ethyl]-carbamic acid but-3-enyl ester,
[1-(Cyanoethyl-carbamoyl)-2-phenyl-ethyl]-carbamic acid but-3-enyl ester,
[1-(Cyanomethyl-carbamoyl)-2-phenyl-ethyl]-carbamic acid 2-isopropyl-5-methyl-cyclohexyl ester,
[1-(Cyanoethyl-carbamoyl)-2-phenyl-ethyl]-carbamic acid 2-isopropyl-5-methyl-cyclohexyl ester,
[1-(Cyanoethyl-carbamoyl)-2-phenyl-ethyl]-carbamic acid hexyl ester,
[1-(Cyanoethyl-carbamoyl)-2-phenyl-ethyl]-carbamic acid tert-butyl ester,
[1-(Cyanoethyl-carbamoyl)-2-phenyl-ethyl]-carbamic acid methyl ester,
[1-(Cyanoethyl-carbamoyl)-2-phenyl-ethyl]-carbamic acid ethyl ester,
[1-(Cyanoethyl-carbamoyl)-2-phenyl-ethyl]-carbamic acid 9*H*-fluoren-9-ylmethylester,
3-Methylsulfanyl-2-acetylamino-propionic acid cyanomethyl-amide,
3-Methylsulfanyl-2-[3-(4-trifluoromethyl-phenyl)-ureido]-propionic acid cyanomethyl-amide,
3-Methylsulfanyl-2-[3-(4-trifluoromethylsulfanyl-phenyl)-ureido]-propionic acid cyanomethyl-amide,
3-Methylsulfanyl-2-[3-(4-trifluoromethoxy-phenyl)-ureido]-propionic acid cyanomethyl-amide,
3-Methylsulfanyl-2-[3-(4-cyano-phenyl)-ureido]-propionic acid cyanomethyl-amide,
3-Methylsulfanyl-2-(3-benzyl-ureido)-propionic acid cyanomethyl-amide,
3-Methylsulfanyl-2-(3-o-tolyl-ureido)-propionic acid cyanomethyl-amide,
3-Methylsulfanyl-2-[3-(*S*)-(1-phenyl-ethyl)-ureido]-propionic acid cyanomethyl-amide,
3-Methylsulfanyl-2-[3-(2,6-dimethyl-phenyl)-ureido]-propionic acid cyanomethyl-amide,
3-Methylsulfanyl-2-[3-(3-methyl-benzyl)-ureido]-propionic acid cyanomethyl-amide,
3-Methylsulfanyl-2-[3-(1,1,3,3-tetramethyl-butyl)-ureido]-propionic acid cyanomethyl-amide,
3-Methylsulfanyl-2-(3-indan-5-yl-ureido)-propionic acid cyanomethyl-amide,
3-Methylsulfanyl-2-[3-(2-phenyl-cyclopropyl)-ureido]-propionic acid cyanomethyl-amide,

3-Methylsulfanyl-2-(3-adamantan-1-yl-ureido)-propionic acid cyanomethyl-amide,
3-Methylsulfanyl-2-(3-biphenyl-4-yl-ureido)-propionic acid cyanomethyl-amide,
3-Methylsulfanyl-2-[3-(4-phenoxy-phenyl)-ureido]-propionic acid cyanomethyl-amide,
3-Methylsulfanyl-2-[3-(4-nitro-phenyl)-ureido]-propionic acid cyanomethyl-amide,
3-Methylsulfanyl-2-(3-cyclohexyl-ureido)-propionic acid cyanomethyl-amide,
3-Methylsulfanyl-2-(3-benzo[1,3]dioxol-5-yl-ureido)-propionic acid cyanomethyl-amide,
3-Methylsulfanyl-2-[3-(2-fluoro-benzyl)-ureido]-propionic acid cyanomethyl-amide,
3-Methylsulfanyl-2-[3-(4-methyl-benzyl)-ureido]-propionic acid cyanomethyl-amide,
3-Methylsulfanyl-2-(3-phenethyl-ureido) -ureido]-propionic acid cyanomethyl-amide,
3-Methylsulfanyl-2-[3-(3,4,5-trimethoxy-phenyl)-ureido]-propionic acid cyanomethyl-amide,
3-Methylsulfanyl-2-(3-ethyl-thioureido)-propionic acid cyanomethyl-amide,
3-Methylsulfanyl-2-(3-isopropyl-thioureido)-propionic acid cyanomethyl-amide,
3-Methylsulfanyl-2-[3-(4-nitro-phenyl)-thioureido]-propionic acid cyanomethyl-amide,
3-Methylsulfanyl-2-(3-phenyl-thioureido)-propionic acid cyanomethyl-amide,
3-Methylsulfanyl-2-[3-(4-trifluoromethoxy-phenyl)-thioureido]-propionic acid cyanomethyl-amide,
3-Methylsulfanyl-2-{3-[4-(2,2,2-trifluoro-ethyl)-phenyl]-thioureido}-propionic acid cyanomethyl-amide,
3-Methylsulfanyl-2-[3-(4-methoxy-phenyl)-thioureido]-propionic acid cyanomethyl-amide,
3-Methylsulfanyl-2-(7,7-dimethyl-2-oxo-bicyclo[2.2.1]hept-1-ylmethanesulfonylamino)-propionic acid cyanomethyl-amide,
3-Methylsulfanyl-2-(thiophene-2-sulfonylamino)-propionic acid cyanomethyl-amide,
3-Methylsulfanyl-2-(4-trifluoromethoxy-benzenesulfonylamino)-propionic acid cyanomethyl-amide,
3-Methylsulfanyl-2-(4-*tert*-butyl-benzenesulfonylamino)-propionic acid cyanomethyl-amide,
3-Methylsulfanyl-2-(4-chloro-benzenesulfonylamino)-propionic acid cyanomethyl-amide,
3-Methylsulfanyl-2-(4-methoxy-benzenesulfonylamino)-propionic acid cyanomethyl-amide,
3-Methylsulfanyl-2-(quinoline-6-sulfonylamino)-propionic acid cyanomethyl-amide,
3-Methylsulfanyl-2-benzenesulfonylamino-propionic acid cyanomethyl-amide,
1-Methyl-1H-indole-2-carboxylic acid [1-(cyanomethyl-carbamoyl)-2-methylsulfanyl-ethyl]-amide,
2-Propyl-pentanoic acid [1-(cyanomethyl-carbamoyl)-2-methylsulfanyl-ethyl]-amide,
1-Methyl-cyclopropanecarboxylic acid [1-(cyanomethyl-carbamoyl)-2-methylsulfanyl-ethyl]-amide,
Thiophene-2-carboxylic acid [1-(cyanomethyl-carbamoyl)-2-methylsulfanyl-ethyl]-amide,
*N*-[1-(Cyanomethyl-carbamoyl)-2-methylsulfanyl-ethyl]-3-trifluoromethyl-benzamide,
Biphenyl-2-carboxylic acid [1-(cyanomethyl-carbamoyl)-2-methylsulfanyl-ethyl]-amide,
4-Acetylamino-*N*-[1-(cyanomethyl-carbamoyl)-2-methylsulfanyl-ethyl]-benzamide,
3-Methylsulfanyl-2-(2-1*H*-indol-3-yl-acetylamino)-propionic acid cyanomethyl-amide,
3-Methylsulfanyl-2-(3-1*H*-indol-3-yl-propionylamino)-propionic acid cyanomethyl-amide,
*N*-[1-(Cyanomethyl-carbamoyl)-2-methylsulfanyl-ethyl]-4-(1*H*-indol-3-yl)-butyramide,
N-[1-(Cyanomethyl-carbamoyl)-2-methylsulfanyl-ethyl]-benzamide,
3-Chloromethyl-*N*-[1-(cyanomethyl-carbamoyl)-2-methylsulfanyl-ethyl]-benzamide,
4-Chloromethyl-*N*-[1-(cyanomethyl-carbamoyl)-2-methylsulfanyl-ethyl]-benzamide,
*N*-[1-(Cyanomethyl-carbamoyl)-2-methylsulfanyl-ethyl]-2-fluoro-benzamide,
*N*-[1-(Cyanomethyl-carbamoyl)-2-methylsulfanyl-ethyl]-2-nitro-benzamide,
*N*-[1-(Cyanomethyl-carbamoyl)-2-methylsufanyl-ethyl]-acrylamide,
3-Methylsulfanyl-2,2-dimethyl-propionic acid cyanomethyl-amide,
3-Methylsulfanyl-2-(2-methoxy-acetylamino)-propionic acid cyanomethyl-amide,
*N*-[1-(Cyanomethyl-carbamoyl)-2-methylsulfanyl-ethyl]-oxalamic acid methyl ester,
*N*-[1-(Cyanomethyl-carbamoyl)-2-methylsulfanyl-ethyl]-phthalamic acid,
*N*-[1-(Cyanomethyl-carbamoyl)-2-methylsulfanyl-ethyl]-succinamic acid,
3-[1-(Cyanomethyl-carbamoyl)-2-methylsulfanyl-ethylcarbamoyl]-acrylic acid,
[1-(Cyanomethyl-carbamoyl)-2-methylsulfanyl-ethyl]-carbamic acid isobutyl ester,
[1-(Cyanomethyl-carbamoyl)-2-methylsulfanyl-ethyl]-carbamic acid butyl ester,
[1-(Cyanomethyl-carbamoyl)-2-methylsulfanyl-ethyl]-carbamic acid hexyl ester,
[1-(Cyanomethyl-carbamoyl)-2-methylsulfanyl-ethyl]-carbamic acid tert-butyl ester,
[1-(Cyanomethyl-carbamoyl)-2-methylsulfanyl-ethyl]-carbamic acid methyl ester,
[1-(Cyanomethyl-carbamoyl)-2-methylsulfanyl-ethyl]-carbamic acid ethyl ester,
[1-(Cyanomethyl-carbamoyl)-2-methylsulfanyl-ethyl]-carbamic acid 9*H*-fluoren-9-ylmethyl ester,
3-Methylsulfanyl-2-acetylamino-propionic acid cyanoethyl-amide,
3-Methylsulfanyl-2-[3-(4-trifluoromethyl-phenyl)-ureido]-propionic acid cyanoethyl-amide,
3-Methylsulfanyl-2-[3-(4-trifluoromethylsulfanyl-phenyl)-ureido]-propionic acid cyanoethyl-amide,

3-Methylsulfanyl-2-[3-(4-trifluoromethoxy-phenyl)-ureido]-propionic acid cyanoethyl-amide,
3-Methylsulfanyl-2-[3-(4-cyano-phenyl)-ureido]-propionic acid cyanoethyl-amide,
3-Methylsulfanyl-2-(3-benzyl-ureido)-propionic acid cyanoethyl-amide,
3-Methylsulfanyl-2-(3-o-tolyl-ureido)-propionic acid cyanoethyl-amide,
3-Methylsulfanyl-2-[3-(*S*)-(1-phenyl-ethyl)-ureido]-propionic acid cyanoethyl-amide,
3-Methylsulfanyl-2-[3-(2,6-dimethyl-phenyl)-ureido]-propionic acid cyanoethyl-amide,
3-Methylsulfanyl-2-[3-(3-methyl-benzyl)-ureido]-propionic acid cyanoethyl-amide,
3-Methylsulfanyl-2-[3-(1,1,3,3-tetramethyl-butyl)-ureido]-propionic acid cyanoethyl-amide,
3-Methylsulfanyl-2-(3-indan-5-yl-ureido)-propionic acid cyanoethyl-amide,
3-Methylsulfanyl-2-[3-(2-phenyl-cyclopropyl)-ureido]-propionic acid cyanoethyl-amide,
3-Methylsulfanyl-2-(3-adamantan-1-yl-ureido)-propionic acid cyanoethyl-amide,
3-Methylsulfanyl-2-(3-biphenyl-4-yl-ureido)-propionic acid cyanoethyl-amide,
3-Methylsulfanyl-2-[3-(4-phenoxy-phenyl)-ureido]-propionic acid cyanoethyl-amide,
3-Methylsulfanyl-2-[3-(4-nitro-phenyl)-ureido]-propionic acid cyanoethyl-amide,
3-Methylsulfanyl-2-(3-cyclohexyl-ureido)-propionic acid cyanoethyl-amide,
3-Methylsulfanyl-2-(3-benzo[1,3]dioxol-5-yl-ureido)-propionic acid cyanoethyl-amide,
3-Methylsulfanyl-2-[3-(2-fluoro-benzyl)-ureido]-propionic acid cyanoethyl-amide,
3-Methylsulfanyl-2-[3-(4-methyl-benzyl)-ureido]-propionic acid cyanoethyl-amide,
3-Methylsulfanyl-2-(3-phenethyl-ureido) -ureido]-propionic acid cyanoethyl-amide,
3-Methylsulfanyl-2-[3-(3,4,5-trimethoxy-phenyl)-ureido]-propionic acid cyanoethyl-amide,
3-Methylsulfanyl-2-(3-ethyl-thioureido)-propionic acid cyanoethyl-amide,
3-Methylsulfanyl-2-(3-isopropyl-thioureido)-propionic acid cyanoethyl-amide,
3-Methylsulfanyl-2-[3-(4-nitro-phenyl)-thioureido]-propionic acid cyanoethyl-amide,
3-Methylsulfanyl-2-(3-phenyl-thioureido)-propionic acid cyanoethyl-amide,
3-Methylsulfanyl-2-[3-(4-trifluoromethoxy-phenyl)-thioureido]-propionic acid cyanoethyl-amide,
3-Methylsulfanyl-2-{3-[4-(2,2,2-trifluoro-ethyl)-phenyl]-thioureido}-propionic acid cyanoethyl-amide,
3-Methylsulfanyl-2-[3-(4-methoxy-phenyl)-thioureido]-propionic acid cyanoethyl-amide,
3-Methylsulfanyl-2-(7,7-dimethyl-2-oxo-bicyclo[2.2.1]hept-1-ylmethanesulfonylamino)-propionic acid cyanoethyl-amide,
3-Methylsulfanyl-2-(thiophene-2-sulfonylamino)-propionic acid cyanoethyl-amide,
3-Methylsulfanyl-2-(4-trifluoromethoxy-benzenesulfonylamino)-propionic acid cyanoethyl-amide,
3-Methylsulfanyl-2-(4-*tert*-butyl-benzenesulfonylamino)-propionic acid cyanoethyl-amide,
3-Methylsulfanyl-2-(4-chloro-benzenesulfonylamino)-propionic acid cyanoethyl-amide,
3-Methylsulfanyl-2-(4-methoxy-benzenesulfonylamino)-propionic acid cyanoethyl-amide,
3-Methylsulfanyl-2-(quinoline-6-sulfonylamino)-propionic acid cyanoethyl-amide,
3-Methylsulfanyl-2-benzenesulfonylamino-propionic acid cyanoethyl-amide,
1-Methyl-1H-indole-2-carboxylic acid [1-(cyanoethyl-carbamoyl)-2-methylsulfanyl-ethyl]-amide,
2-Propyl-pentanoic acid [1-(cyanoethyl-carbamoyl)-2-methylsulfanyl-ethyl]-amide,
1-Methyl-cyclopropanecarboxylic acid [1-(cyanoethyl-carbamoyl)-2-methylsulfanyl-ethyl]-amide,
Thiophene-2-carboxylic acid [1-(cyanoethyl-carbamoyl)-2-methylsulfanyl-ethyl]-amide,
*N*-[1-(Cyanoethyl-carbamoyl)-2-methylsulfanyl-ethyl]-3-trinuoromethyl-benzamide,
Biphenyl-2-carboxylic acid [1-(cyanoethyl-carbamoyl)-2-methylsulfanyl-ethyl]-amide,
4-Acetylamino-*N*-[1-(cyanoethyl-carbamoyl)-2-methylsulfanyl-ethyl]-benzamide,
3-Methylsulfanyl-2-(2-1*H*-indol-3-yl-acetylamino)-propionic acid cyanoethyl-amide,
3-Methylsulfanyl-2-(3-1*H*-indol-3-yl-propionylamino)-propionic acid cyanoethyl-amide,
*N*-[1-(Cyanoethyl-carbamoyl)-2-methylsulfanyl-ethyl)-4-(1*H*-indol-3-yl)-butyramide,
N-[1-(Cyanoethyl-carbamoyl)-2-methylsulfanyl-ethyl]-benzamide,
3-Chloromethyl-*N*-[1-(cyanoethyl-carbamoyl)-2-methylsulfanyl-ethyl]-benzamide,
4-Chloromethyl-*N*-[1-(cyanoethyl-carbamoyl)-2-methylsulfanyl-ethyl]-benzamide,
*N*-[1-(Cyanoethyl-carbamoyl)-2-methylsulfanyl-ethyl]-2-fluoro-benzamide,
*N*-[1-(Cyanoethyl-carbamoyl)-2-methylsulfanyl-ethyl]-2-nitro-benzamide,
*N*-[1-(Cyanoethyl-carbamoyl)-2-methylsulfanyl-ethyl]-acrylamide,
3-Methylsulfanyl-2,2-dimethyl-propionic acid cyanoethyl-amide,
3-Methylsulfanyl-2-(2-methoxy-acetylamino)-propionic acid cyanoethyl-amide,
*N*-[1-(Cyanoethyl-carbamoyl)-2-methylsulfanyl-ethyl]-oxalamic acid methyl ester,
*N*-[1-(Cyanoethyl-carbamoyl)-2-methylsulfanyl-ethyl]-phthalamic acid,
*N*-[1-(Cyanoethyl-carbamoyl)-2-methylsulfanyl-ethyl]-succinamic acid,
3-[1-(Cyanoethyl-carbamoyl)-2-methylsulfanyl-ethylcarbamoyl]-acrylic acid,

[1-(Cyanoethyl-carbamoyl)-2-methylsulfanyl-ethyl]-carbamic acid isobutyl ester,
[1-(Cyanoethyl-carbamoyl)-2-methylsulfanyl-ethyl]-carbamic acid butyl ester,
[1-(Cyanomethyl-carbamoyl)-2-methylsulfanyl-ethyl]-carbamic acid cyanomethyl ester,
[1-(Cyanoethyl-carbamoyl)-2-methylsulfanyl-ethyl]-carbamic acid cyanomethyl ester,
[1-(Cyanomethyl-carbamoyl)-2-methylsulfanyl-ethyl]-carbamic acid but-3-enyl ester,
[1-(Cyanoethyl-carbamoyl)-2-methylsulfanyl-ethyl]-carbamic acid but-3-enyl ester,
[1-(Cyanomethyl-carbamoyl)-2-methylsulfanyl-ethyl]-carbamic acid 2-isopropyl-5-methyl-cyclohexyl ester,
[1-(Cyanoethyl-carbamoyl)-2-methylsulfanyl-ethyl]-carbamic acid 2-isopropyl-5-methyl-cyclohexyl ester,
[1-(Cyanoethyl-carbamoyl)-2-methylsulfanyl-ethyl]-carbamic acid hexyl ester,
[1-(Cyanoethyl-carbamoyl)-2-methylsulfanyl-ethyl]-carbamic acid *tert*-butyl ester,
[1-(Cyanoethyl-carbamoyl)-2-methylsulfanyl-ethyl]-carbamic acid methyl ester,
[1-(Cyanoethyl-carbamoyl)-2-methylsulfanyl-ethyl]-carbamic acid ethyl ester,
[1-(Cyanoethyl-carbamoyl)-2-methylsulfanyl-ethyl]-carbamic acid 9*H*-fluoren-9-ylmethylester,
3-Methanesulfonyl-2-acetylamino-propionic acid cyanomethyl-amide,
3-Methanesulfonyl-2-[3-(4-trifluoromethyl-phenyl)-ureido]-propionic acid cyanomethyl-amide,
3-Methanesulfonyl-2-[3-(4-trifluoromethylsulfanyl-phenyl)-ureido]-propionic acid cyanomethyl-amide,
3-Methanesulfonyl-2-[3-(4-trifluoromethoxy-phenyl)-ureido]-propionic acid cyanomethyl-amide,
3-Methanesulfonyl-2-[3-(4-cyano-phenyl)-ureido]-propionic acid cyanomethyl-amide,
3-Methanesulfonyl-2-(3-benzyl-ureido)-propionic acid cyanomethyl-amide,
3-Methanesulfonyl-2-(3-o-tolyl-ureido)-propionic acid cyanomethyl-amide,
3-Methanesulfonyl-2-[3-(*S*)-(1-phenyl-ethyl)-ureido]-propionic acid cyanomethyl-amide,
3-Methanesulfonyl-2-[3-(2,6-dimethyl-phenyl)-ureido]-propionic acid cyanomethyl-amide,
3-Methanesulfonyl-2-[3-(3-methyl-benzyl)-ureido]-propionic acid cyanomethyl-amide,
3-Methanesulfonyl-2-[3-(1,1,3,3-tetramethyl-butyl)-ureido]-propionic acid cyanomethyl-amide,
3-Methanesulfonyl-2-(3-indan-5-yl-ureido)-propionic acid cyanomethyl-amide,
3-Methanesulfonyl-2-[3-(2-phenyl-cyclopropyl)-ureido]-propionic acid cyanomethyl-amide,
3-Methanesulfonyl-2-(3-adamantan-1-yl-ureido)-propionic acid cyanomethyl-amide,
3-Methanesulfonyl-2-(3-biphenyl-4-yl-ureido)-propionic acid cyanomethyl-amide,
3-Methanesulfonyl-2-[3-(4-phenoxy-phenyl)-ureido]-propionic acid cyanomethyl-amide,
3-Methanesulfonyl-2-[3-(4-nitro-phenyl)-ureido]-propionic acid cyanomethyl-amide,
3-Methanesulfonyl-2-(3-cyclohexyl-ureido)-propionic acid cyanomethyl-amide,
3-Methanesulfonyl-2-(3-benzo[1,3]dioxol-5-yl-ureido)-propionic acid cyanomethyl-amide,
3-Methanesulfonyl-2-[3-(2-fluoro-benzyl)-ureido]-propionic acid cyanomethyl-amide,
3-Methanesulfonyl-2-[3-(4-methyl-benzyl)-ureido]-propionic acid cyanomethyl-amide,
3-Methanesulfonyl-2-(3-phenethyl-ureido) -ureido]-propionic acid cyanomethyl-amide,
3-Methanesulfonyl-2-[3-(3,4,5-trimethoxy-phenyl)-ureido]-propionic acid cyanomethyl-amide,
3-Methanesulfonyl-2-(3-ethyl-thioureido)-propionic acid cyanomethyl-amide,
3-Methanesulfonyl-2-(3-isopropyl-thioureido)-propionic acid cyanomethyl-amide,
3-Methanesulfonyl-2-[3-(4-nitro-phenyl)-thioureido]-propionic acid cyanomethyl-amide,
3-Methanesulfonyl-2-(3-phenyl-thioureido)-propionic acid cyanomethyl-amide,
3-Methanesulfonyl-2-[3-(4-trifluoromethoxy-phenyl)-thioureido]-propionic acid cyanomethyl-amide,
3-Methanesulfonyl-2-{3-[4-(2,2,2-trifluoro-ethyl)-phenyl]-thioureido}-propionic acid cyanomethyl-amide,
3-Methanesulfonyl-2-[3-(4-methoxy-phenyl)-thioureido]-propionic acid cyanomethyl-amide,
3-Methanesulfonyl-2-(7,7-dimethyl-2-oxo-bicyclo[2.2.1]hept-1-ylmethanesulfonylamino)-propionic acid cyanomethyl-amide,
3-Methanesulfonyl-2-(thiophene-2-sulfonylamino)-propionic acid cyanomethyl-amide,
3-Methanesulfonyl-2-(4-trifluoromethoxy-benzenesulfonylamino)-propionic acid cyanomethyl-amide,
3-Methanesulfonyl-2-(4-*tert*-butyl-benzenesulfonylamino)-propionic acid cyanomethyl-amide,
3-Methanesulfonyl-2-(4-chloro-benzenesulfonylamino)-propionic acid cyanomethyl-amide,
3-Methanesulfonyl-2-(4-methoxy-benzenesulfonylamino)-propionic acid cyanomethyl-amide,
3-Methanesulfonyl-2-(quinoline-6-sulfonylamino)-propionic acid cyanomethyl-amide,
3-Methanesulfonyl-2-benzenesulfonylamino-propionic acid cyanomethyl-amide,
1-Methyl-1H-indole-2-carboxylic acid [1-(cyanomethyl-carbamoyl)-2-methanesulfonyl-ethyl]-amide,
2-Propyl-pentanoic acid [1-(cyanomethyl-carbamoyl)-2-methanesulfonyl-ethyl]-amide,
1-Methyl-cyclopropanecarboxylic acid [1-(cyanomethyl-carbamoyl)-2-methanesulfonyl-ethyl]-amide,
Thiophene-2-carboxylic acid [1-(cyanomethyl-carbamoyl)-2-methanesulfonyl-ethyl]-amide,
*N*-[1-(Cyanomethyl-carbamoyl)-2-methanesulfonyl-ethyl]-3-trifluoromethyl-benzamide,
Biphenyl-2-carboxylic acid [1-(cyanomethyl-carbamoyl)-2-methanesulfonyl-ethyl]-amide,

4-Acetylamino-*N*-[1-(cyanomethyl-carbamoyl)-2-methanesulfonyl-ethyl)-benzamide,
3-Methanesulfonyl-2-(2-1*H*-indol-3-yl-acetylamino)-propionic acid cyanomethyl-amide,
3-Methanesulfonyl-2-(3-1*H* indol-3-yl-propionylamino)-propionic acid cyanomethyl-amide,
*N*-[1-(Cyanomethyl-carbamoyl)-2-methanesulfonyl-ethyl]-4-(1*H*-indol-3-yl)-butyramide,
*N*-[ 1-(Cyanomethyl-carbamoyl)-2-methanesulfonyl-ethyl]-benzamide,
3-Chloromethyl-*N*-[1-(cyanomethyl-carbamoyl)-2-methanesulfonyl-ethyl]-benzamide,
4-Chloromethyl-*N*-[1-(cyanomethyl-carbamoyl)-2-methanesulfonyl-ethyl]-benzamide,
*N*-[1-(Cyanomethyl-carbamoyl)-2-methanesulfonyl-ethyl]-2-fluoro-benzamide,
*N*-[1-(Cyanomethyl-carbamoyl)-2-methanesulfonyl-ethyl]-2-nitro-benzamide,
*N*-[1-(Cyanomethyl-carbamoyl)-2-methanesulfonyl-ethyl]-acrylamide,
3-Methanesulfonyl-2,2-dimethyl-propionic acid cyanomethyl-amide,
3-Methanesulfonyl-2-(2-methoxy-acetylamino)-propionic acid cyanomethyl-amide,
*N*-[1-(Cyanomethyl-carbamoyl)-2-methanesulfonyl-ethyl]-oxalamic acid methyl ester,
*N*-[1-(Cyanomethyl-carbamoyl)-2-methanesulfonyl-ethyl]-phthalamic acid,
*N*-[1-(Cyanomethyl-carbamoyl)-2-methanesulfonyl-ethyl]-succinamic acid,
3-[1-(Cyanomethyl-carbamoyl)-2-methanesulfonyl-ethylcarbamoyl]-acrylic acid,
[1-(Cyanomethyl-carbamoyl)-2-methanesulfonyl-ethyl]-carbamic acid isobutyl ester,
[1-(Cyanomethyl-carbamoyl)-2-methanesulfonyl-ethyl]-carbamic acid butyl ester,
[1-(Cyanomethyl-carbamoyl)-2-methanesulfonyl-ethyl]-carbamic acid hexyl ester,
[1-(Cyanomethyl-carbamoyl)-2-methanesulfonyl-ethyl]-carbamic acid tert-butyl ester,
[1-(Cyanomethyl-carbamoyl)-2-methanesulfonyl-ethyl]-carbamic acid methyl ester,
[1-(Cyanomethyl-carbamoyl)-2-methanesulfonyl-ethyl]-carbamic acid ethyl ester,
[1-(Cyanomethyl-carbamoyl)-2-methanesulfonyl-ethyl]-carbamic acid 9*H*-fluoren-9-ylmethyl ester,
3-Methanesulfonyl-2-acetylamino-propionic acid cyanoethyl-amide,
3-Methanesulfonyl-2-[3-(4-trifluoromethyl-phenyl)-ureido]-propionic acid cyanoethyl-amide,
3-Methanesulfonyl-2-[3-(4-trifluoromethylsulfanyl-phenyl)-ureido]-propionic acid cyanoethyl-amide,
3-Methanesulfonyl-2-[3-(4-trifluoromethoxy-phenyl)-ureido]-propionic acid cyanoethyl-amide,
3-Methanesulfonyl-2-[3-(4-cyano-phenyl)-ureido]-propionic acid cyanoethyl-amide,
3-Methanesulfonyl-2-(3-benzyl-ureido)-propionic acid cyanoethyl-amide,
3-Methanesulfonyl-2-(3-o-tolyl-ureido)-propionic acid cyanoethyl-amide,
3-Methanesulfonyl-2-[3-(*S*)-(1-phenyl-ethyl)-ureido]-propionic acid cyanoethyl-amide,
3-Methanesulfonyl-2-[3-(2,6-dimethyl-phenyl)-ureido]-propionic acid cyanoethyl-amide,
3-Methanesulfonyl-2-[3-(3-methyl-benzyl)-ureido]-propionic acid cyanoethyl-amide,
3-Methanesulfonyl-2-[3-(1,1,3,3-tetramethyl-butyl)-ureido]-propionic acid cyanoethyl-amide,
3-Methanesulfonyl-2-(3-indan-5-yl-ureido)-propionic acid cyanoethyl-amide,
3-Methanesulfonyl-2-[3-(2-phenyl-cyclopropyl)-ureido]-propionic acid cyanoethyl-amide,
3-Methanesulfonyl-2-(3-adamantan-1-yl-ureido)-propionic acid cyanoethyl-amide,
3-Methanesulfonyl-2-(3-biphenyl-4-yl-ureido)-propionic acid cyanoethyl-amide,
3-Methanesulfonyl-2-[3-(4-phenoxy-phenyl)-ureido]-propionic acid cyanoethyl-amide,
3-Methanesulfonyl-2-[3-(4-nitro-phenyl)-ureido]-propionic acid cyanoethyl-amide,
3-Methanesulfonyl-2-(3-cyclohexyl-ureido)-propionic acid cyanoethyl-amide,
3-Methanesulfonyl-2-(3-benzo[1,3]dioxol-5-yl-ureido)-propionic acid cyanoethyl-amide,
3-Methanesulfonyl-2-[3-(2-fluoro-benzyl)-ureido]-propionic acid cyanoethyl-amide,
3-Methanesulfonyl-2-[3-(4-methyl-benzyl)-ureido]-propionic acid cyanoethyl-amide,
3-Methanesulfonyl-2-(3-phenethyl-ureido) -ureido]-propionic acid cyanoethyl-amide,
3-Methanesulfonyl-2-[3-(3,4,5-trimethoxy-phenyl)-ureido]-propionic acid cyanoethyl-amide,
3-Methanesulfonyl-2-(3-ethyl-thioureido)-propionic acid cyanoethyl-amide,
3-Methanesulfonyl-2-(3-isopropyl-thioureido)-propionic acid cyanoethyl-amide,
3-Methanesulfonyl-2-[3-(4-nitro-phenyl)-thioureido]-propionic acid cyanoethyl-amide,
3-Methanesulfonyl-2-(3-phenyl-thioureido)-propionic acid cyanoethyl-amide,
3-Methanesulfonyl-2-[3-(4-trifluoromethoxy-phenyl)-thioureido]-propionic acid cyanoethyl-amide,
3-Methanesulfonyl-2-{3-[4-(2,2,2-trifluoro-ethyl)-phonyl]-thioureido}-propionic acid cyanoethyl-amide,
3-Methanesulfonyl-2-[3-(4-methoxy-phenyl)-thioureido]-propionic acid cyanoethyl-amide,
3-Methanesulfonyl-2-(7,7-dimethyl-2-oxo-bicyclo[2.2. 1]hept-1-ylmethanesulfonylamino)-propionic acid cyanoethyl-amide,
3-Methanesulfonyl-2-(thiophene-2-sulfonylamino)-propionic acid cyanoethyl-amide,
3-Methanesulfonyl-2-(4-trifluoromethoxy-benzenesulfonylamino)-propionic acid cyanoethyl-amide,
3-Methanesulfonyl-2-(4-*tert*-butyl-benzenesulfonylamino)-propionic acid cyanoethyl-amide,

3-Methanesulfonyl-2-(4-chloro-benzenesulfonylamino)-propionic acid cyanoethyl-amide,
3-Methanesulfonyl-2-(4-methoxy-benzenesulfonylamino)-propionic acid cyanoethyl-amide,
3-Methanesulfonyl-2-(quinoline-6-sulfonylamino)-propionic acid cyanoethyl-amide,
3-Methanesulfonyl-2-benzenesulfonylamino-propionic acid cyanoethyl-amide,
1-Methyl-1H-indole-2-carboxylic acid [1-(cyanoethyl-carbamoyl)-2-methanesulfonyl-ethyl]-amide,
2-Propyl-pentanoic acid [1-(cyanoethyl-carbamoyl)-2-methanesulfonyl-ethyl]-amide,
1-Methyl-cyclopropanecarboxylic acid [1-(cyanoethyl-carbamoyl)-2-methanesulfonyl-ethyl]-amide,
Thiophene-2-carboxylic acid [1-(cyanoethyl-carbamoyl)-2-methanesulfonyl-ethyl]-amide,
*N*-[1-(Cyanoethyl-carbamoyl)-2-methanesulfonyl-ethyl]-3-trifluoromethyl-benzamide,
Biphenyl-2-carboxylic acid [1-(cyanoethyl-carbamoyl)-2-methanesulfonyl-ethyl]-amide,
4-Acetylamino-*N*-[1-(cyanoethyl-carbamoyl)-2-methanesulfonyl-ethyl]-benzamide,
3-Methanesulfonyl-2-(2-1*H*-indol-3-yl-acetylamino)-propionic acid cyanoethyl-amide,
3-Methanesulfonyl-2-(3-1*H*-indol-3-yl-propionylamino)-propionic acid cyanoethyl-amide,
*N*-[1-(Cyanoethyl-carbamoyl)-2-methanesulfonyl-ethyl]-4-(1*H*-indol-3-yl)-butyramide,
N-[1-(Cyanoethyl-carbamoyl)-2-methanesulfonyl-ethyl]-benzamide,
3-Chloromethyl-*N*-[1-(cyanoethyl-carbamoyl)-2-methanesulfonyl-ethyl]-benzamide,
4-Chloromethyl-*N*-[1-(cyanoethyl-carbamoyl)-2-methanesulfonyl-ethyl]-benzamide,
*N*-[1-(Cyanoethyl-carbamoyl)-2-methanesulfonyl-ethyl]-2-fluoro-benzamide,
*N*-[1-(Cyanoethyl-carbamoyl)-2-methanesulfonyl-ethyl]-2-nitro-benzamide,
*N*-[1-(Cyanoethyl-carbamoyl)-2-methanesulfonyl-ethyl]-acrylamide,
3-Methanesulfonyl-2,2-dimethyl-propionic acid cyanoethyl-amide,
3-Methanesulfonyl-2-(2-methoxy-acetylamino)-propionic acid cyanoethyl-amide,
*N*-[1-(Cyanoethyl-carbamoyl)-2-methanesulfonyl-ethyl]-oxalamic acid methyl ester,
*N*-[1-(Cyanoethyl-carbamoyl)-2-methanesulfonyl-ethyl]-phthalamic acid,
*N*-[1-(Cyanoethyl-carbamoyl)-2-methanesulfonyl-ethyl]-succinamic acid,
3-[1-(Cyanoethyl-carbamoyl)-2-methanesulfonyl-ethylcarbamoyl]-acrylic acid,
[1-(Cyanoethyl-carbamoyl)-2-methanesulfonyl-ethyl]-carbamic acid isobutyl ester,
[1-(Cyanoethyl-carbamoyl)-2-methanesulfonyl-ethyl]-carbamic acid butyl ester,
[1-(Cyanomethyl-carbamoyl)-2-methanesulfonyl-ethyl]-carbamic acid cyanomethyl ester,
[1-(Cyanoethyl-carbamoyl)-2-methanesulfonyl-ethyl]-carbamic acid cyanomethyl ester,
[1-(Cyanomethyl-carbamoyl)-2-methanesulfonyl-ethyl]-carbamic acid but-3-enyl ester,
[1-(Cyanoethyl-carbamoyl)-2-methanesulfonyl-ethyl]-carbamic acid but-3-enyl ester,
[1-(Cyanomethyl-carbamoyl)-2-methanesulfonyl-ethyl]-carbamic acid 2-isopropyl-5-methyl-cyclohexyl ester,
[1-(Cyanoethyl-carbamoyl)-2-methanesulfonyl-ethyl]-carbamic acid 2-isopropyl-5-methyl-cyclohexyl ester,
[1-(Cyanoethyl-carbamoyl)-2-methanesulfonyl-ethyl]-carbamic acid hexyl ester,
[1-(Cyanoethyl-carbamoyl)-2-methanesulfonyl-ethyl]-carbamic acid *tert*-butyl ester,
[1-(Cyanoethyl-carbamoyl)-2-methanesulfonyl-ethyl]-carbamic acid methyl ester,
[1-(Cyanoethyl-carbamoyl)-2-methanesulfonyl-ethyl]-carbamic acid ethyl ester,
[1-(Cyanoethyl-carbamoyl)-2-methanesulfonyl-ethyl]-carbamic acid 9*H*-fluoren-9-ylmethylester,
3-Naphthalen-2-yl-2-acetylamino-propionic acid cyanomethyl-amide,
3-Naphthalen-2-yl-2-[3-(4-trifluoromethyl-phenyl)-ureido]-propionic acid cyanomethyl-amide,
3-Naphthalen-2-yl-2-[3-(4-trifluoromethylsulfanyl-phenyl)-ureido]-propionic acid cyanomethyl-amide,
3-Naphthalen-2-yl-2-[3-(4-trifluoromethoxy-phenyl)-ureido]-propionic acid cyanomethyl-amide,
3-Naphthalen-2-yl-2-[3-(4-cyano-phenyl)-ureido]-propionic acid cyanomethyl-amide,
3-Naphthalen-2-yl-2-(3-benzyl-ureido)-propionic acid cyanomethyl-amide,
3-Naphthalen-2-yl-2-(3-o-tolyl-ureido)-propionic acid cyanomethyl-amide,
3-Naphthalen-2-yl-2-[3-(*S*)-(1-phenyl-ethyl)-ureido]-propionic acid cyanomethyl-amide,
3-Naphthalen-2-yl-2-[3-(2,6-dimethyl-phenyl)-ureido]-propionic acid cyanomethyl-amide,
3-Naphthalen-2-yl-2-[3-(3-methyl-benzyl)-ureido]-propionic acid cyanomethyl-amide,
3-Naphthalen-2-yl-2-[3-(1,1,3,3-tetramethyl-butyl)-ureido]-propionic acid cyanomethyl-amide,
3-Naphthalen-2-yl-2-(3-indan-5-yl-ureido)-propionic acid cyanomethyl-amide,
3-Naphthalen-2-yl-2-[3-(2-phenyl-cyclopropyl)-ureido]-propionic acid cyanomethyl-amide,
3-Naphthalen-2-yl-2-(3-adamantan-1-yl-ureido)-propionic acid cyanomethyl-amide,
3-Naphthalen-2-yl-2-(3-biphenyl-4-yl-ureido)-propionic acid cyanomethyl-amide,
3-Naphthalen-2-yl-2-[3-(4-phenoxy-phenyl)-ureido]-propionic acid cyanomethyl-amide,
3-Naphthalen-2-yl-2-[3-(4-nitro-phenyl)-ureido]-propionic acid cyanomethyl-amide,
3-Naphthalen-2-yl-2-(3-cyclohexyl-ureido)-propionic acid cyanomethyl-amide,
3-Naphthalen-2-yl-2-(3-benzo[1,3]dioxol-5-yl-ureido)-propionic acid cyanomethyl-amide,

3-Naphthalen-2-yl-2-[3-(2-fluoro-benzyl)-ureido]-propionic acid cyanomethyl-amide,
3-Naphthalen-2-yl-2-[3-(4-methyl-benzyl)-ureido]-propionic acid cyanomethyl-amide,
3-Naphthalen-2-yl-2-(3-phenethyl-ureido) -ureido]-propionic acid cyanomethyl-amide,
3-Naphthalen-2-yl-2-[3-(3,4,5-trimethoxy-phenyl)-ureido]-propionic acid cyanomethyl-amide,
3-Naphthalen-2-yl-2-(3-ethyl-thioureido)-propionic acid cyanomethyl-amide,
3-Naphthalen-2-yl-2-(3-isopropyl-thioureido)-propionic acid cyanomethyl-amide,
3-Naphthalen-2-yl-2-[3-(4-nitro-phenyl)-thioureido]-propionic acid cyanomethyl-amide,
3-Naphthalen-2-yl-2-(3-phenyl-thioureido)-propionic acid cyanomethyl-amide,
3-Naphthalen-2-yl-2-[3-(4-trifluoromethoxy-phenyl)-thioureido]-propionic acid cyanomethyl-amide,
3-Naphthalen-2-yl-2-{3-[4-(2,2,2-trifluoro-ethyl)-phenyl]-thioureido}-propionic acid cyanomethyl-amide,
3-Naphthalen-2-yl-2-[3-(4-methoxy-phenyl)-thioureido]-propionic acid cyanomethyl-amide,
3-Naphthalen-2-yl-2-(7,7-dimethyl-2-oxo-bicyclo[2.2.1]hept-1-ylmethanesulfonylamino)-propionic acid cyanomethyl-amide,
3-Naphthalen-2-yl-2-(thiophene-2-sulfonylamino)-propionic acid cyanomethyl-amide,
3-Naphthalen-2-yl-2-(4-trifluoromethoxy-benzenesulfonylamino)-propionic acid cyanomethyl-amide,
3-Naphthalen-2-yl-2-(4-*tert*-butyl-benzenesulfonylamino)-propionic acid cyanomethyl-amide,
3-Naphthalen-2-yl-2-(4-chloro-benzenesulfonylamino)-propionic acid cyanomethyl-amide,
3-Naphthalen-2-yl-2-(4-methoxy-benzenesulfonylamino)-propionic acid cyanomethyl-amide,
3-Naphthalen-2-yl-2-(quinoline-6-sulfonylamino)-propionic acid cyanomethyl-amide,
3-Naphthalen-2-yl-2-benzenesulfonylamino-propionic acid cyanomethyl-amide,
1-Methyl-1H-indole-2-carboxylic acid [1-(cyanomethyl-carbamoyl)-2-naphthalen-2-yl-ethyl]-amide,
2-Propyl-pentanoic acid [1-(cyanomethyl-carbamoyl)-2-naphthalen-2-yl-ethyl]-amide,
1-Methyl-cyclopropanecarboxylic acid [1-(cyanomethyl-carbamoyl)-2-naphthalen-2-yl-ethyl]-amide,
Thiophene-2-carboxylic acid [1-(cyanomethyl-carbamoyl)-2-naphthalen-2-yl-ethyl]-amide,
*N*-[1-(Cyanomethyl-carbamoyl)-2-naphthalen-2-yl-ethyl]-3-trifluoromethyl-benzamide,
Biphenyl-2-carboxylic acid [1-(cyanomethyl-carbamoyl)-2-naphthalen-2-yl-ethyl]-amide,
4-Acetylamino-*N*-[1-(cyanomethyl-carbamoyl)-2-naphthalen-2-yl-ethyl]-benzamide,
3-Naphthalen-2-yl-2-(2-1*H*-indol-3-yl-acetylamino)-propionic acid cyanomethyl-amide,
3-Naphthalen-2-yl-2-(3-1*H*-indol-3-yl-propionylamino)-propionic acid cyanomethyl-amide,
*N*-[1-(Cyanomethyl-carbamoyl)-2-naphthalen-2-yl-ethyl]-4-(1*H*-indol-3-yl)-butyramide,
N-[1-(Cyanomethyl-carbamoyl)-2-naphthalen-2-yl-ethyl]-benzamide,
3-Chloromethyl-*N*-[1-(cyanomethyl-carbamoyl)-2-naphthalen-2-yl-ethyl]-benzamide,
4-Chloromethyl-*N*-[1-(cyanomethyl-carbamoyl)-2-naphthalen-2-yl-ethyl]-benzamide,
*N*-[1-(Cyanomethyl-carbamoyl)-2-naphthalen-2-yl-ethyl]-2-fluoro-benzamide,
*N*-[1-(Cyanomethyl-carbamoyl)-2-naphthalen-2-yl-ethyl]-2-nitro-benzamide,
*N*-[1-(Cyanomethyl-carbamoyl)-2-naphthalen-2-yl-ethyl]-acrylamide,
3-Naphthalen-2-yl-2,2-dimethyl-propionic acid cyanomethyl-amide,
3-Naphthalen-2-yl-2-(2-methoxy-acetylamino)-propionic acid cyanomethyl-amide,
*N*-[1-(Cyanomethyl-carbamoyl)-2-naphthalen-2-yl-ethyl]-oxalamic acid methyl ester,
*N*-[1-(Cyanomethyl-carbamoyl)-2-naphthalen-2-yl-ethyl]-phthalamic acid,
*N*-[1-(Cyanomethyl-carbamoyl)-2-naphthalen-2-yl-ethyl]-succinamic acid,
3-[1-(Cyanomethyl-carbamoyl)-2-naphthalen-2-yl-ethylcarbamoyl]-acrylic acid,
[1-(Cyanomethyl-carbamoyl)-2-naphthalen-2-yl-ethyl]-carbamic acid isobutyl ester,
[1-(Cyanomethyl-carbamoyl)-2-naphthalen-2-yl-ethyl]-carbamic acid butyl ester,
[1-(Cyanomethyl-carbamoyl)-2-naphthalen-2-yl-ethyl]-carbamic acid hexyl ester,
[1-(Cyanomethyl-carbamoyl)-2-naphthalen-2-yl-ethyl]-carbamic acid *tert*-butyl ester,
[1-(Cyanomethyl-carbamoyl)-2-naphthalen-2-yl-ethyl]-carbamic acid methyl ester,
[1-(Cyanomethyl-carbamoyl)-2-naphthalen-2-yl-ethyl]-carbamic acid ethyl ester,
[1-(Cyanomethyl-carbamoyl)-2-naphthalen-2-yl-ethyl]-carbamic acid 9*H*-fluoren-9-ylmethyl ester,
3-Naphthalen-2-yl-2-acetylamino-propionic acid cyanoethyl-amide,
3-Naphthalen-2-yl-2-[3-(4-trifluoromethyl-phenyl)-ureido]-propionic acid cyanoethyl-amide,
3-Naphthalen-2-yl-2-[3-(4-trifluoromethylsulfanyl-phenyl)-ureido]-propionic acid cyanoethyl-amide,
3-Naphthalen-2-yl-2-[3-(4-trifluoromethoxy-phenyl)-ureido]-propionic acid cyanoethyl-amide,
3-Naphthalen-2-yl-2-[3-(4-cyano-phenyl)-ureido]-propionic acid cyanoethyl-amide,
3-Naphthalen-2-yl-2-(3-benzyl-ureido)-propionic acid cyanoethyl-amide,
3-Naphthalen-2-yl-2-(3-o-tolyl-ureido)-propionic acid cyanoethyl-amide,
3-Naphthalen-2-yl-2-[3-(*S*)-(1-phenyl-ethyl)-ureido]-propionic acid cyanoethyl-amide,
3-Naphthalen-2-yl-2-[3-(2,6-dimethyl-phenyl)-ureido]-propionic acid cyanoethyl-amide,

3-Naphthalen-2-yl-2-[3-(3-methyl-benzyl)-ureido]-propionic acid cyanoethyl-amide,
3-Naphthalen-2-yl-2-[3-(1,1,3,3-tetramethyl-butyl)-ureido]-propionic acid cyanoethyl-amide,
3-Naphthalen-2-yl-2-(3-indan-5-yl-ureido)-propionic acid cyanoethyl-amide,
3-Naphthalen-2-yl-2-[3-(2-phenyl-cyclopropyl)-ureido]-propionic acid cyanoethyl-amide,
3-Naphthalen-2-yl-2-(3-adamantan-1-yl-ureido)-propionic acid cyanoethyl-amide,
3-Naphthalen-2-yl-2-(3-biphenyl-4-yl-ureido)-propionic acid cyanoethyl-amide,
3-Naphthalen-2-yl-2-[3-(4-phenoxy-phenyl)-ureido]-propionic acid cyanoethyl-amide,
3-Naphthalen-2-yl-2-[3-(4-nitro-phenyl)-ureido]-propionic acid cyanoethyl-amide,
3-Naphthalen-2-yl-2-(3-cyclohexyl-ureido)-propionic acid cyanoethyl-amide,
3-Naphthalen-2-yl-2-(3-benzo[1,3]dioxol-5-yl-ureido)-propionic acid cyanoethyl-amide,
3-Naphthalen-2-yl-2-[3-(2-fluoro-benzyl)-ureido]-propionic acid cyanoethyl-amide,
3-Naphthalen-2-yl-2-[3-(4-methyl-benzyl)-ureido]-propionic acid cyanoethyl-amide,
3-Naphthalen-2-yl-2-(3-phenethyl-ureido) -ureido]-propionic acid cyanoethyl-amide,
3-Naphthalen-2-yl-2-[3-(3,4,5-trimethoxy-phenyl)-ureido]-propionic acid cyanoethyl-amide,
3-Naphthalen-2-yl-2-(3-ethyl-thioureido)-propionic acid cyanoethyl-amide,
3-Naphthalen-2-yl-2-(3-isopropyl-thioureido)-propionic acid cyanoethyl-amide,
3-Naphthalen-2-yl-2-[3-(4-nitro-phenyl)-thioureido]-propionic acid cyanoethyl-amide,
3-Naphthalen-2-yl-2-(3-phenyl-thioureido)-propionic acid cyanoethyl-amide,
3-Naphthalen-2-yl-2-[3-(4-trifluoromethoxy-phenyl)-thioureido]-propionic acid cyanoethyl-amide,
3-Naphthalen-2-yl-2-{3-[4-(2,2,2-trifluoro-ethyl)-phenyl]-thioureido}-propionic acid cyanoethyl-amide,
3-Naphthalen-2-yl-2-[3-(4-methoxy-phenyl)-thioureido]-propionic acid cyanoethyl-amide,
3-Naphthalen-2-yl-2-(7,7-dimethyl-2-oxo-bicyclo[2.2.1]hept-1-ylmethanesulfonylamino)-propionic acid cyanoethyl-amide,
3-Naphthalen-2-yl-2-(thiophene-2-sulfonylamino)-propionic acid cyanoethyl-amide,
3-Naphthalen-2-yl-2-(4-trifluoromethoxy-benzenesulfonylamino)-propionic acid cyanoethyl-amide,
3-Naphthalen-2-yl-2-(4-*tert*-butyl-benzenesulfonylamino)-propionic acid cyanoethyl-amide,
3-Naphthalen-2-yl-2-(4-chloro-benzenesulfonylamino)-propionic acid cyanoethyl-amide,
3-Naphthalen-2-yl-2-(4-methoxy-benzenesulfonylamino)-propionic acid cyanoethyl-amide,
3-Naphthalen-2-yl-2-(quinoline-6-sulfonylamino)-propionic acid cyanoethyl-amide,
3-Naphthalen-2-yl-2-benzenesulfonylamino-propionic acid cyanoethyl-amide,
1-Methyl-1H-indole-2-carboxylic acid [1-(cyanoethyl-carbamoyl)-2-naphthalen-2-yl-ethyl]-amide,
2-Propyl-pentanoic acid [1-(cyanoethyl-carbamoyl)-2-naphthalen-2-yl-ethyl]-amide,
1-Methyl-cyclopropanecarboxylic acid [1-(cyanoethyl-carbamoyl)-2-naphthalen-2-yl-ethyl]-amide,
Thiophene-2-carboxylic acid [1-(cyanoethyl-carbamoyl)-2-naphthalen-2-yl-ethyl]-amide,
*N*-[1-(Cyanoethyl-carbamoyl)-2-naphthalen-2-yl-ethyl]-3-trifluoromethyl-benzamide,
Biphenyl-2-carboxylic acid [1-(cyanoethyl-carbamoyl)-2-naphthalen-2-yl-ethyl]-amide,
4-Acetylamino-*N*-[1-(cyanoethyl-carbamoyl)-2-naphthalen-2-yl-ethyl]-benzamide,
3-Naphthalen-2-yl-2-(2-1*H*-indol-3-yl-acetylamino)-propionic acid cyanoethyl-amide,
3-Naphthalen-2-yl-2-(3-1*H*-indol-3-yl-propionylamino)-propionic acid cyanoethyl-amide,
*N*-[1-(Cyanoethyl-carbamoyl)-2-naphthalen-2-yl-ethyl]-4-(1*H*-indol-3-yl)-butyramide,
N-[1-(Cyanoethyl-carbamoyl)-2-naphthalen-2-yl-ethyl]-benzamide,
3-Chloromethyl-*N*-[1-(cyanoethyl-carbamoyl)-2-naphthalen-2-yl-ethyl]-benzamide,
4-Chloromethyl-*N*-[1-(cyanoethyl-carbamoyl)-2-naphthalen-2-yl-ethyl]-benzamide,
*N*-[1-(Cyanoethyl-carbamoyl)-2-naphthalen-2-yl-ethyl]-2-fluoro-benzamide,
*N*-[1-(Cyanoethyl-carbamoyl)-2-naphthalen-2-yl-ethyl]-2-nitro-benzamide,
*N*-[1-(Cyanoethyl-carbamoyl)-2-naphthalen-2-yl-ethyl]-acrylamide,
3-Naphthalen-2-yl-2,2-dimethyl-propionic acid cyanoethyl-amide,
3-Naphthalen-2-yl-2-(2-methoxy-acetylamino)-propionic acid cyanoethyl-amide,
*N*-[1-(Cyanoethyl-carbamoyl)-2-naphthalen-2-yl-ethyl]-oxalamic acid methyl ester,
*N*-[1-(Cyanoethyl-carbamoyl)-2-naphthalen-2-yl-ethyl]-phthalamic acid,
*N*-[1-(Cyanoethyl-carbamoyl)-2-naphthalen-2-yl-ethyl]-succinamic acid,
3-[1-(Cyanoethyl-carbamoyl)-2-naphthalen-2-yl-ethylcarbamoyl]-acrylic acid,
[1-(Cyanoethyl-carbamoyl)-2-naphthalen-2-yl-ethyl]-carbamic acid isobutyl ester,
[1-(Cyanoethyl-carbamoyl)-2-naphthalen-2-yl-ethyl]-carbamic acid butyl ester,
[1-(Cyanomethyl-carbamoyl)-2-naphthalen-2-yl-ethyl]-carbamic acid cyanomethyl ester,
[1-(Cyanoethyl-carbamoyl)-2-naphthalen-2-yl-ethyl]-carbamic acid cyanomethyl ester,
[1-(Cyanomethyl-carbamoyl)-2-naphthalen-2-yl-ethyl]-carbamic acid but-3-enyl ester,
[1-(Cyanoethyl-carbamoyl)-2-naphthalen-2-yl-ethyl]-carbamic acid but-3-enyl ester,

[1-(Cyanomethyl-carbamoyl)-2-naphthalen-2-yl-ethyl]-carbamic acid 2-isopropyl-5-methyl-cyclohexyl ester,
[1-(Cyanoethyl-carbamoyl)-2-naphthalen-2-yl-ethyl]-carbamic acid 2-isopropyl-5-methyl-cyclohexyl ester,
[1-(Cyanoethyl-carbamoyl)-2-naphthalen-2-yl-ethyl]-carbamic acid hexyl ester,
[1-(Cyanoethyl-carbamoyl)-2-naphthalen-2-yl-ethyl]-carbamic acid *tert*-butyl ester,
[1-(Cyanoethyl-carbamoyl)-2-naphthalen-2-yl-ethyl]-carbamic acid methyl ester,
[1-(Cyanoethyl-carbamoyl)-2-naphthalen-2-yl-ethyl]-carbamic acid ethyl ester,
[1-(Cyanoethyl-carbamoyl)-2-naphthalen-2-yl-ethyl]-carbamic acid 9*H*-fluoren-9-ylmethylester,
3-Benzo[b]thiophen-3-yl-2-acetylamino-propionic acid cyanomethyl-amide,
3-Benzo[b]thiophen-3-yl-2-[3-(4-trifluoromethyl-phenyl)-ureido]-propionic acid cyanomethyl-amide,
3-Benzo[b]thiophen-3-yl-2-[3-(4-trifluoromethylsulfanyl-phenyl)-ureido]-propionic acid cyanomethyl-amide,
3-Benzo[b]thiophen-3-yl-2-[3-(4-trifluoromethoxy-phenyl)-ureido]-propionic acid cyanomethyl-amide,
3-Benzo[b]thiophen-3-yl-2-[3-(4-cyano-phenyl)-ureido]-propionic acid cyanomethyl-amide,
3-Benzo[b]thiophen-3-yl-2-(3-benzyl-ureido)-propionic acid cyanomethyl-amide,
3-Benzo[b]thiophen-3-yl-2-(3-*o*-tolyl-ureido)-propionic acid cyanomethyl-amide,
3-Benzo[b]thiophen-3-yl-2-[3-(*S*)-(1-phenyl-ethyl)-ureido]-propionic acid cyanomethyl-amide,
3-Benzo[b]thiophen-3-yl-2-[3-(2,6-dimethyl-phenyl)-ureido]-propionic acid cyanomethyl-amide,
3-Benzo[b]thiophen-3-yl-2-[3-(3-methyl-benzyl)-ureido]-propionic acid cyanomethyl-amide,
3-Benzo[b]thiophen-3-yl-2-[3-(1,1,3,3-tetramethyl-butyl)-ureido]-propionic acid cyanomethyl-amide,
3-Benzo[b]thiophen-3-yl-2-(3-indan-5-yl-ureido)-propionic acid cyanomethyl-amide,
3-Benzo[b]thiophen-3-yl-2-[3-(2-phenyl-cyclopropyl)-ureido]-propionic acid cyanomethyl-amide,
3-Benzo[b]thiophen-3-yl-2-(3-adamantan-1-yl-ureido)-propionic acid cyanomethyl-amide,
3-Benzo[b]thiophen-3-yl-2-(3-biphenyl-4-yl-ureido)-propionic acid cyanomethyl-amide,
3-Benzo[b]thiophen-3-yl-2-[3-(4-phenoxy-phenyl)-ureido]-propionic acid cyanomethyl-amide,
3-Benzo[b]thiophen-3-yl-2-[3-(4-nitro-phenyl)-ureido]-propionic acid cyanomethyl-amide,
3-Benzo[b]thiophen-3-yl-2-(3-cyclohexyl-ureido)-propionic acid cyanomethyl-amide,
3-Benzo[b]thiophen-3-yl-2-(3-benzo[ 1,3]dioxol-5-yl-ureido)-propionic acid cyanomethyl-amide,
3-Benzo[b]thiophen-3-yl-2-[3-(2-fluoro-benzyl)-ureido]-propionic acid cyanomethyl-amide,
3-Benzo[b]thiophen-3-yl-2-[3-(4-methyl-benzyl)-ureido]-propionic acid cyanomethyl-amide,
3-Benzo[b]thiophen-3-yl-2-(3-phenethyl-ureido) -ureido]-propionic acid cyanomethyl-amide,
3-Benzo[b]thiophen-3-yl-2-[3-(3,4,5-trimethoxy-phenyl)-ureido]-propionic acid cyanomethyl-amide,
3-Benzo[b]thiophen-3-yl-2-(3-ethyl-thioureido)-propionic acid cyanomethyl-amide,
3-Benzo[b]thiophen-3-yl-2-(3-isopropyl-thioureido)-propionic acid cyanomethyl-amide,
3-Benzo[b]thiophen-3-yl-2-[3-(4-nitro-phenyl)-thioureido]-propionic acid cyanomethyl-amide,
3-Benzo[b]thiophen-3-yl-2-(3-phenyl-thioureido)-propionic acid cyanomethyl-amide,
3-Benzo[b]thiophen-3-yl-2-[3-(4-trifluoromethoxy-phenyl)-thioureido]-propionic acid cyanomethyl-amide,
3-Benzo[b]thiophen-3-yl-2-{3-[4-(2,2,2-trifluoro-ethyl)-phenyl]-thioureido}-propionic acid cyanomethyl-amide,
3-Benzo[b]thiophen-3-yl-2-[3-(4-methoxy-phenyl)-thioureido]-propionic acid cyanomethyl-amide,
3-Benzo[b]thiophen-3-yl-2-(7,7-dimethyl-2-oxo-bicyclo[2.2.1]hept-1-ylmethanesulfonylamino)-propionic acid cyanomethyl-amide,
3-Benzo[b]thiophen-3-yl-2-(thiophene-2-sulfonylamino)-propionic acid cyanomethyl-amide,
3-Benzo[b]thiophen-3-yl-2-(4-trifluoromethoxy-benzenesulfonylamino)-propionic acid cyanomethyl-amide,
3-Benzo[b]thiophen-3-yl-2-(4-tert-butyl-benzenesulfonylamino)-propionic acid cyanomethyl-amide,
3-Benzo[b]thiophen-3-yl-2-(4-chloro-benzenesulfonylamino)-propionic acid cyanomethyl-amide,
3-Benzo[b]thiophen-3-yl-2-(4-methoxy-benzenesulfonylamino)-propionic acid cyanomethyl-amide,
3-Benzo[b]thiophen-3-yl-2-(quinoline-6-sulfonylamino)-propionic acid cyanomethyl-amide,
3-Benzo[b]thiophen-3-yl-2-benzenesulfonylamino-propionic acid cyanomethyl-amide,
1-Methyl-1H-indole-2-carboxylic acid [2-benzo[b]thiophen-3-yl-1-(cyanomethyl-carbamoyl)-ethyl]-amide,
2-Propyl-pentanoic acid [2-benzo[b]thiophen-3-yl-1-(cyanomethyl-carbamoyl)-ethyl]-amide,
1-Methyl-cyclopropanecarboxylic acid [2-benzo[b]thiophen-3-yl-1-(cyanomethyl-carbamoyl)-ethyl]-amide,
Thiophene-2-carboxylic acid [2-benzo[b]thiophen-3-yl-1-(cyanomethyl-carbamoyl)-ethyl]-amide,
N-[2-Benzo[b]thiophen-3-yl-1-(cyanomethyl-carbamoyl)-ethyl]-3-trifluoromethyl-benzamide,
Biphenyl-2-carboxylic acid [2-benzo[b]thiophen-3-yl-1-(cyanomethyl-carbamoyl)-ethyl]-amide,
4-Acetylamino-*N*-[2-benzo[b]thiophen-3-yl-1-(cyanomethyl-carbamoyl)-ethyl]-benzamide,
3-Benzo[b]thiophen-3-yl-2-(2-1*H*-indol-3-yl-acetylamino)-propionic acid cyanomethyl-amide,
3-Benzo[b]thiophen-3-yl-2-(3-1*H*-indol-3-yl-propionylamino)-propionic acid cyanomethyl-amide,
*N*-[2-Benzo[b]thiophen-3-yl-1-(cyanomethyl-carbamoyl)-ethyl)-4-(1*H*-indol-3-yl)-butyramide,
*N*-[2-Benzo[b]thiophen-3-yl-1-(cyanomethyl-carbamoyl)-ethyl]-benzamide,
*N*-[2-Benzo[b]thiophen-3-yl-1-(cyanomethyl-carbamoyl)-ethyl]-acrylamide,

*N*-[2-Benzo[b]thiophen-3-yl-1-(cyanomethyl-carbamoyl)-ethyl]-acrylamide,
3-Benzo[b]thiophen-3-yl-2,2-dimethyl-propionic acid cyanomethyl-amide,
3-Benzo[b]thiophen-3-yl-2-(2-methoxy-acetylamino)-propionic acid cyanomethyl-amide,
*N*-[2-Benzo[b]thiophen-3-yl-1-(cyanomethyl-carbamoyl)-ethyl]-oxalamic acid methyl ester,
*N*-[2-Benzo[b]thiophen-3-yl-1-(cyanomethyl-carbamoyl)-ethyl]-phthalamic acid,
*N*-[2-Benzo[b]thiophen-3-yl-1-(cyanomethyl-carbamoyl)-ethyl]-succinamic acid,
3-[2-Benzo[b]thiophen-3-yl-1-(cyanomethyl-carbamoyl)-ethylcarbamoyl]-acrylic acid,
[2-Benzo[b]thiophen-3-yl-1-(cyanomethyl-carbamoyl)-ethyl]-carbamic acid isobutyl ester,
[2-Benzo[b]thiophen-3-yl-1-(cyanomethyl-carbamoyl)-ethyl]-carbamic acid butyl ester,
[2-Benzo[b]thiophen-3-yl-1-(cyanomethyl-carbamoyl)-ethyl]-carbamic acid cyanomethyl ester,
[2-Benzo[b]thiophen-3-yl-1-(cyanomethyl-carbamoyl)-ethyl]-carbamic acid but-3-enyl ester,
[2-Benzo[b]thiophen-3-yl-1-(cyanomethyl-carbamoyl)-ethyl]-carbamic acid 2-isopropyl-5-methyl-cyclohexyl ester,
[2-Benzo[b]thiophen-3-yl-1-(cyanomethyl-carbamoyl)-ethyl]-carbamic acid hexyl ester,
[2-Benzo[b]thiophen-3-yl-1-(cyanomethyl-carbamoyl)-ethyl]-carbamic acid tert-butyl ester,
[2-Benzo[b]thiophen-3-yl-1-(cyanomethyl-carbamoyl)-ethyl]-carbamic acid methyl ester,
[2-Benzo[b]thiophen-3-yl-1-(cyanomethyl-carbamoyl)--ethyl]-carbamic acid ethyl ester,
[2-Benzo[b]thiophen-3-yl-1-(cyanomethyl-carbamoyl)-ethyl]-carbamic acid 9*H*-fluoren-9-ylmethyl ester,
3-Benzo[b]thiophen-3-yl-2-acetylamino-propionic acid cyanoethyl-amide,
3-Benzo[b]thiophen-3-yl-2-[3-(4-trifluoromethyl-phenyl)-ureido]-propionic acid cyanoethyl-amide,
3-Benzo[b]thiophen-3-yl-2-[3-(4-trifluoromethylsulfanyl-phenyl)-ureido]-propionic acid cyanoethyl-amide,
3-Benzo[b]thiophen-3-yl-2-[3-(4-trifluoromethoxy-phenyl)-ureido]-propionic acid cyanoethyl-amide,
3-Benzo[b]thiophen-3-yl-2-[3-(4-cyano-phenyl)-ureido]-propionic acid cyanoethyl-amide,
3-Benzo[b]thiophen-3-yl-2-(3-benzyl-ureido)-propionic acid cyanoethyl-amide,
3-Benzo[b]thiophen-3-yl-2-(3-*o*-tolyl-ureido)-propionic acid cyanoethyl-amide,
3-Benzo[b]thiophen-3-yl-2-[3-(*S*)-(1-phenyl-ethyl)-ureido]-propionic acid cyanoethyl-amide,
3-Benzo[b]thiophen-3-yl-2-[3-(2,6-dimethyl-phenyl)-ureido]-propionic acid cyanoethyl-amide,
3-Benzo[b]thiophen-3-yl-2-[3-(3-methyl-benzyl)-ureido]-propionic acid cyanoethyl-amide,
3-Benzo[b]thiophen-3-yl-2-[3-(1,1,3,3-tetramethyl-butyl)-ureido]-propionic acid cyanoethyl-amide,
3-Benzo[b]thiophen-3-yl-2-(3-indan-5-yl-ureido)-propionic acid cyanoethyl-amide,
3-Benzo[b]thiophen-3-yl-2-[3-(2-phenyl-cyclopropyl)-ureido]-propionic acid cyanoethyl-amide,
3-Benzo[b]thiophen-3-yl-2-(3-adamantan-1-yl-ureido)-propionic acid cyanoethyl-amide,
3-Benzo[b]thiophen-3-yl-2-(3-biphenyl-4-yl-ureido)-propionic acid cyanoethyl-amide,
3-Benzo[b]thiophen-3-yl-2-[3-(4-phenoxy-phenyl)-ureido]-propionic acid cyanoethyl-amide,
3-Benzo[b]thiophen-3-yl-2-[3-(4-nitro-phenyl)-ureido]-propionic acid cyanoethyl-amide,
3-Benzo[b]thiophen-3-yl-2-(3-cyclohexyl-ureido)-propionic acid cyanoethyl-amide,
3-Benzo[b]thiophen-3-yl-2-(3-benzo[1,3]dioxol-5-yl-ureido)-propionic acid cyanoethyl-amide,
3-Benzo[b]thiophen-3-yl-2-[3-(2-fluoro-benzyl)-ureido]-propionic acid cyanoethyl-amide,
3-Benzo[b]thiophen-3-yl-2-[3-(4-methyl-benzyl)-ureido]-propionic acid cyanoethyl-amide,
3-Benzo[b]thiophen-3-yl-2-(3-phenethyl-ureido) -ureido]-propionic acid cyanoethyl-amide,
3-Benzo[b]thiophen-3-yl-2-[3-(3,4,5-trimethoxy-phenyl)-ureido]-propionic acid cyanoethyl-amide,
3-Benzo[b]thiophen-3-yl-2-(3-ethyl-thioureido)-propionic acid cyanoethyl-amide,
3-Benzo[b]thiophen-3-yl-2-(3-isopropyl-thioureido)-propionic acid cyanoethyl-amide,
3-Benzo[b]thiophen-3-yl-2-[3-(4-nitro-phenyl)-thioureido]-propionic acid cyanoethyl-amide,
3-Benzo[b]thiophen-3-yl-2-(3-phenyl-thioureido)-propionic acid cyanoethyl-amide,
3-Benzo[b]thiophen-3-yl-2-[3-(4-trifluoromethoxy-phenyl)-thioureido]-propionic acid cyanoethyl-amide,
3-Benzo[b]thiophen-3-yl-2-{3-[4-(2,2,2-trifluoro-ethyl)-phenyl]-thioureido}-propionic acid cyanoethyl-amide,
3-Benzo[b]thiophen-3-yl-2-[3-(4-methoxy-phenyl)-thioureido]-propionic acid cyanoethyl-amide,
3-Benzo[b]thiophen-3-yl-2-(7,7-dimethyl-2-oxo-bicyclo[2.2. 1]hept-1-ylmethanesulfonylamino)-propionic acid cyanoethyl-amide,
3-Benzo[b]thiophen-3-yl-2-(thiophene-2-sulfonylamino)-propionic acid cyanoethyl-amide,
3-Benzo[b]thiophen-3-yl-2-(4-trifluoromethoxy-benzenesulfonylamino)-propionic acid cyanoethyl-amide,
3-Benzo[b]thiophen-3-yl-2-(4-tert-butyl-benzenesulfonylamino)-propionic acid cyanoethyl-amide,
3-Benzo[b]thiophen-3-yl-2-(4-chloro-benzenesulfonylamino)-propionic acid cyanoethyl-amide,
3-Benzo[b]thiophen-3-yl-2-(4-methoxy-benzenesulfonylamino)-propionic acid cyanoethyl-amide,
3-Benzo[b]thiophen-3-yl-2-(quinoline-6-sulfonylamino)-propionic acid cyanoethyl-amide,
3-Benzo[b]thiophen-3-yl-2-benzenesulfonylamino-propionic acid cyanoethyl-amide,
1-Methyl-1H-indole-2-carboxylic acid [2-benzo[b]thiophen-3-yl-1-(cyanoethyl-carbamoyl)-ethyl]-amide,
2-Propyl-pentanoic acid [2-benzo[b]thiophen-3-yl-1-(cyanoethyl-carbamoyl)-ethyl]-amide,

1-Methyl-cyclopropanecarboxylic acid [2-benzo[b]thiophen-3-yl-1-(cyanoethyl-carbamoyl)-ethyl]-amide,
Thiophene-2-carboxylic acid [2-benzo[b]thiophen-3-yl-1-(cyanoethyl-carbamoyl)-ethyl]-amide,
*N*-[2-Benzo[b]thiophen-3-yl-1-(cyanoethyl-carbamoyl)-ethyl]-3-trifluoromethyl-benzamide,
Biphenyl-2-carboxylic acid [2-benzo[b]thiophen-3-yl-1-(cyanoethyl-carbamoyl)-ethyl]-amide,
4-Acetylamino-*N*-[2-benzo[b]thiophen-3-yl-1-(cyanoethyl-carbamoyl)-ethyl]-benzamide,
3-Benzo[b]thiophen-3-yl-2-(2-1*H*-indol-3-yl-acetylamino)-propionic acid cyanoethyl-amide,
3-Benzo[b]thiophen-3-yl-2-(3-1*H*-indol-3-yl-propionylamino)-propionic acid cyanoethyl-amide,
*N*-[2-Benzo[b]thiophen-3-yl-1-(cyanoethyl-carbamoyl)-ethyl]-4-(1*H*-indol-3-yl)-butyramide,
*N*-[2-Benzo[b]thiophen-3-yl-1-(cyanoethyl-carbamoyl)-ethyl]-benzamide,
3-Chloromethyl-*N*-[2-benzo[b]thiophen-3-yl-1-(cyanomethyl-carbamoyl)-ethyl]-benzamide,
4-Chloromethyl-*N*-[2-benzo[b]thiophen-3-yl-1-(cyanomethyl-carbamoyl)-ethyl]-benzamide,
*N*-[2-Benzo[b]thiophen-3-yl-1-(cyanomethyl-carbamoyl)-ethyl]-2-fluoro-benzamide,
*N*-[2-Benzo[b]thiophen-3-yl-1-(cyanomethyl-carbamoyl)-ethyl]-2-nitro-benzamide,
3-Chloromethyl-*N*-[2-benzo[b]thiophen-3-yl-1-(cyanoethyl-carbamoyl)-ethyl]-benzamide,
4-Chloromethyl-*N*-[2-benzo[b]thiophen-3-yl-1-(cyanoethyl-carbamoyl)-ethyl]-benzamide,
*N*-[2-Benzo[b]thiophen-3-yl-1-(cyanoethyl-carbamoyl)-ethyl]-2-fluoro-benzamide,
*N*-[2-Benzo[b]thiophen-3-yl-1-(cyanoethyl-carbamoyl)-ethyl]-2-nitro-benzamide,
3-Benzo[b]thiophen-3-yl-2,2-dimethyl-propionic acid cyanoethyl-amide,
3-Benzo[b]thiophen-3-yl-2-(2-methoxy-acetylamino)-propionic acid cyanoethyl-amide,
*N*-[2-Benzo[b]thiophen-3-yl-1-(cyanoethyl-carbamoyl)-ethyl]-oxalamic acid methyl ester,
*N*-[2-Benzo[b]thiophen-3-yl-1-(cyanoethyl-carbamoyl)-ethyl]-phthalamic acid,
*N*-[2-Benzo[b]thiophen-3-yl-1-(cyanoethyl-carbamoyl)-ethyl]-succinamic acid,
3-[2-Benzo[b]thiophen-3-yl-1-(cyanoethyl-carbamoyl)-ethylcarbamoyl]-acrylic acid,
[2-Benzo[b]thiophen-3-yl-1-(cyanoethyl-carbamoyl)-ethyl]-carbamic acid isobutyl ester,

1-Cyanomethyl-pyrrolidine-2-carboxylic acid carbamoylmethyl-amide
1-Cyanomethyl-pyrrolidine-2-carboxylic acid (1-carbamoyl-2-methyl-propyl)-amide
1-Cyanomethyl-pyrrolidine-2-carboxylic acid (1-carbamoyl-2-hydroxy-ethyl)-amide
1-Cyanomethyl-pyrrolidine-2-carboxylic acid (1-carbamoyl-4-guanidino-butyl)-amide
1-Cyanomethyl-pyrrolidine-2-carboxylic acid (1-carbamoyl-2-phenyl-ethyl)-amide
1-Cyanomethyl-pyrrolidine-2-carboxylic acid [1-carbamoyl-2-(1H-indol-3-yl)-ethyl]-amide
1-Cyanomethyl-pyrrolidine-2-carboxylic acid [1-carbamoyl-2-(4-hydroxy-phenyl)-ethyl]-amide
3-[(1-Cyanomethyl-pyrrolidine-2-carbonyl)-amino]-succinamic acid
D-1-Cyanomethyl-pyrrolidine-2-carboxylic acid (1-carbamoyl-3-methyl-butyl)-amide
1-Cyanomethyl-pyrrolidine-2-carboxylic acid (1-carbamoyl-cyclohexyl)-amide
1-Cyanomethyl-pyrrolidine-2-carboxylic acid [1-carbamoyl-2-(4-trifluoromethyl-benzylsulfanyl)-ethyl]-amide
1-Cyanomethyl-pyrrolidine-2-carboxylic acid [1-carbamoyl-2-(4-fluoro-benzylsulfanyl)-ethyl]-amide
1-Cyanomethyl-pyrrolidine-2-carboxylic acid [1-carbamoyl-2-(4-fluoro-phenylmethanesulfonyl)-ethyl]-amide
1-Cyanomethyl-pyrrolidine-2-carboxylic acid (3-carbamoyl-phenyl)-amide
1-Cyanomethyl-pyrrolidine-2-carboxylic acid [1-carbamoyl-2-(1-methyl-1H-imidazol-4-yl)-ethyl]-amide
1-Cyanomethyl-pyrrolidine-2-carboxylic acid (1-carbamoyl-2-pyridin-4-yl-ethyl)-amide
1-Cyanomethyl-pyrrolidine-2-carboxylic acid (1-carbamoyl-3-methyl-butyl)-amide
1-Cyanomethyl-pyrrolidine-2-carboxylic acid methyl ester
1-Cyanomethyl-pyrrolidine-2-carboxylic acid

N-(2-Cyanoethyl)-4-(4-dimethylamino-phenylazo)-benzenesulfonamide
N-(2-Cyanoethyl)-4-trifluoromethoxy-benzenesulfonamide
4-tert-Butyl-N-(2-cyanoethyl)-benzenesulfonamide
4-Bromo-N-(2-cyanoethyl)-benzenesulfonamide
4-Chloro-N-(2-cyanoethyl)-benzenesulfonamide
N-(2-Cyanoethyl)-4-methoxy-benzenesulfonamide
N-[4-(2-Cyanoethyl-sulfamoyl)-phenyl]-acetamide
N-(2-Cyanoethyl)-4-methyl-benzenesulfonamide
N-(2-Cyanoethyl)-benzenesulfonamide
N-(2-Cyanoethyl)-C-phenyl-methanesulfonamide
C,C,C-Trichloro-N-(2-cyanoethyl)-methanesulfonamide
Butane-1-sulfonic acid (2-cyanoethyl)-amide
Naphthalene-1-sulfonic acid (2-cyanoethyl)-amide

Octane-1-sulfonic acid (2-cyanoethyl)-amide
N-(2-Cyanoethyl)-2,4,6-triisopropyl-benzenesulfonamide
N-(2-Cyanoethyl)-2-trifluoromethyl-benzenesulfonamide
N-[5-(2-Cyanoethyl-sulfamoyl)-4-methyl-thiazol-2-yl]-acetamide
2-Bromo-N-(2-cyanoethyl)-benzenesulfonamide
N-(2-Cyanoethyl)-2,4,6-trimethyl-benzenesulfonamide
Thiophene-2-sulfonic acid (2-cyanoethyl)-amide
N-(2-Cyanoethyl)-3-nitro-benzenesulfonamide

1-(2-Cyanoethyl)-3-(4-nitro-phenyl)-thiourea
1-(2-Cyano 1-(2-Cyano-ethyl)-3-phenyl-thioureaethyl)-3-phenyl-thiourea
1-(2-Cyanoethyl)-3-(4-trifluoromethoxy-phenyl)-thiourea
1-(2-Cyanoethyl)-3-(4-methylsulfanyl-phenyl)-thiourea
1-(2-Cyanoethyl)-3-(3,4,5-trimethoxy-phenyl)-thiourea
1-(2-Cyanoethyl)-3-naphthalen-1-yl-thiourea
1-Benzyl-3-(2-cyanoethyl)-thiourea
1-Acetyl-3-(2-cyanoethyl)-thiourea
1-(4-Azido-phenyl)-3-(2-cyanoethyl)-thiourea
1-(2-Cyanoethyl)-3-(3-cyano-phenyl)-thiourea
1-(2-Cyano 1-(2-Cyano-ethyl)-3-(4-ethyl-phenyl)-thioureaethyl)-3-(4-ethyl-phenyl)-thiourea
1-(2-Cyanol-(2-Cyano-ethyl)-3-(4-cyano-phenyl)-thioureaethyl)-3-(4-cyano-phenyl)-thiourea
1-Carbamoylmethyl-pyrrolidine-2-carboxylic acid (1-carbamoyl-3-methyl-butyl)-amide 1-(2-Cyanoethyl)-3-pyridin-4-yl-thiourea
1-(2-Cyanoethyl)-3-(2,3,4-trifluoro-phenyl)-thiourea
1-(2-Cyanoethyl)-3-(2,6-difluoro-phenyl)-thiourea
1-(4-Bromo-phenyl)-3-(2-cyanoethyl)-thiourea
1-(2-Cyanoethyl)-3-(4-methoxy-phenyl)-thiourea
1-(2-Cyanoethyl)-3-m-tolyl-thiourea
1-(2-Cyanoethyl)-3-p-tolyl-thiourea
1-(2-Cyanoethyl)-3-cyclohexyl-urea
1-(2-Cyanoethyl)-3-o-tolyl-urea
1-(2-Cyanoethyl)-3-(2-methoxy-phenyl)-urea
[2-Benzo[b]thiophen-3-yl-1-(cyanoethyl-carbamoyl)-ethyl]-carbamic acid butyl ester,
[2-Benzo[b]thiophen-3-yl-1-(cyanoethyl-carbamoyl)-ethyl]-carbamic acid cyanomethyl ester,
[2-Benzo[b]thiophen-3-yl-1-(cyanoethyl-carbamoyl)-ethyl]-carbamic acid but-3-enyl ester,
[2-Benzo[b]thiophen-3-yl-1-(cyanoethyl-carbamoyl)-ethyl]-carbamic acid 2-isopropyl-5-methyl-cyclohexyl ester,
[2-Benzo[b]thiophen-3-yl-1-(cyanoethyl-carbamoyl)-ethyl]-carbamic acid hexyl ester,
[2-Benzo[b]thiophen-3-yl-1-(cyanoethyl-carbamoyl)-ethyl]-carbamic acid tert-butyl ester,
[2-Benzo[b]thiophen-3-yl-1-(cyanoethyl-carbamoyl)-ethyl]-carbamic acid methyl ester,
[2-Benzo[b]thiophen-3-yl-1-(cyanoethyl-carbamoyl)-ethyl]-carbamic acid ethyl ester,
[2-Benzo[b]thiophen-3-yl-1-(cyanoethyl-carbamoyl)-ethyl]-carbamic acid 9*H*-fluoren-9-ylmethylester,

or a pharmaceutically acceptable salt or prodrug thereof.

Even more preferred compounds according to the present invention are those mentioned in any of the tables herein and those further disclosed and/or characterized in the examples.

As used herein, each of the following terms, used alone or in conjunction with other terms, are defined as follows (except where noted to the contrary):

The term "alkyl" refers to a saturated aliphatic radical containing from one to ten carbon atoms or a mono- or polyunsaturated aliphatic hydrocarbon radical containing from two to twelve carbon atoms, containing at least one double and triple bound, respectively. "Alkyl" refers to both branched and unbranched alkyl groups. Preferred alkyl groups are straight chain alkyl groups containing from one to eight carbon atoms. More preferred alkyl groups are straight chain alkyl groups containing from one to six carbon atoms and branched alkyl groups containing from three to six carbon atoms. It should be understood that any combination term using an "alk" or "alkyl" prefix refers to analogs according to the above definition of "alkyl". For example, terms such as "alkoxy", "alkylthio" refer to alkyl group linked to a second group via an oxygen or sulfur atom. "Alkanoyl" refers to an alkyl group linked to a carbonyl group (C=O).

The term "cycloalkyl" refers to the cyclic analog of an alkyl group, as defined above, optionally unsaturated and/or substituted. Preferred cycloalkyl groups are saturated cycloalkyl groups, more particularly those containing from three to eight carbon atoms, and even more preferably three to six carbon atoms.

The term "aryl" refers to aromatic groups having in the range of 6 to 14 carbon atoms and "substituted aryl" refers to aryl groups further bearing one or more substituents.

Each of the above defined "alkyl", "cycloalkyl", and "aryl" shall be understood to include their halogenated analogs, whereby the halogenated analogs may comprise one or several halogen atoms. The halogenated analogs thus comprise any halogen radical as defined in the following.

The term "halo" refers to a halogen radical selected from the group comprising fluoro, chloro, bromo and iodo. Preferred halo groups are fluoro, chloro and bromo.

The term "heteroaryl" refers to a stable 5 to 8 membered, preferably 5 or 6 membered monocyclic or 8 to 11 membered bicyclic aromatic heterocycle radical. Each heterocycle consists of carbon atoms and from 1 to 4 heteroatoms selected from the group consisting of nitrogen, oxygen, sulfur. The heterocycle may be attached by any atom of the cycle which results in the creation of a stable structure. Preferred heteroaryl radicals as used herein include, for example, furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzthiazolyl, benzoxazolyl, purinyl, quinolizinyl, quinolinyl, isoquinolinyl, cinnolinyl, phthalazinyl, quinazolinyl, quinoxalinyl, naphthridinyl, pteridinyl, carbazolyl, acridinyl, phenazinyl, phenothiazinyl and phenoxazinyl.

The term "heterocyclyl" refers to a stable 5 to 8 membered, preferably 5 or 6 membered monocyclic or 8 to 11 membered bicyclic heterocycle radical which may be either saturated or unsaturated, and is non-aromatic. Each heterocycle consists of carbon atom(s) and from 1 to 4 heteroatoms selected from the group consisting of nitrogen, oxygen and sulfur. The heterocycle may be attached by any atom of the cycle, which results in the creation of a stable structure. Preferred heterocycle radicals as used herein include, for example, pyrrolinyl, pyrrolidinyl, pyrazolinyl, pyrazolidinyl, piperidinyl, morpholinyl, thiomorpholinyl, pyranyl, thiopyranyl, piperazinyl, indolinyl, azetidinyl, tetrahydropyranyl, tetrahydrothiopyranyl, tetrahydrofuranyl, hexahydropyrimidinyl, hexahydropyridazinyl, 1,4,5,6-tetrahydropyrimidin-2-ylamine, dihydro-oxazolyl, 1,2-thiazinanyl-1,1-dioxide, 1,2,6-thiadiazinanyl-1,1-dioxide, isothiazolidinyl-1,1-dioxide and imidazolidinyl-2,4-dione.

The terms "heterocyclyl", "heteroaryl" and "aryl", when associated with another moiety, unless otherwise specified, shall have the same meaning as given above. For example, "aroyl" refers to phenyl or naphthyl linked to a carbonyl group (C=O).

Each aryl or heteroaryl unless otherwise specified includes its partially or fully hydrogenated derivative. For example, quinolinyl may include decahydroquinolinyl and tetrahydroquinolinyl, naphthyl may include its hydrogenated derivatives such as tetrahydranaphthyl.

As used herein above and throughout this application, "nitrogen" or "N" and "sulfur" or "S" include any oxidized form of nitrogen such as nitrone, N-oxide and sulfur such as sulfoxide, sulfone and the quatemized form of any basic nitrogen such as HC1 or TFA salts.

As used herein a wording defining the limits of a range of length such as e. g. "1 to 5" means any integer from 1 to 5, i. e. 1, 2, 3, 4 and 5. In other words, any range defined by two integers explicitly mentioned is meant to comprise any integer defining said limits and any integer comprised in said range.

As used herein the term substituted shall mean that one or more H atom of the group or compound which is substituted, is replaced by a different atom, a group of atoms, a molecule or a molecule moiety. Such atom, group of atoms, molecule or molecule moiety is also referred to herein as substituent.

The substituent can be selected from the group comprising hydroxy, alkoxy, mercapto, cycloalkyl, heterocyclic, aryl, heteroaryl, aryloxy, halogen, trifluoromethyl, difluoromethyl, cyano, nitrone, amino, amido, -C(O)H, acyl, oxyacyl, carboxyl, carbamate, sulfonyl, sulphonamide and sulfuryl. Any of the substituents may be substituted itself by any of the aforementioned substituents. This applies preferably to cycloalkyl, heterocylic, aryl, heteroaryl and aryloxy. It is also preferred that alkoxy and mercapto are those of a lower alkyl group. It is to be acknowledged that any of the definition provided herein also applies to any substituent.

Any of the compounds according to the present invention may be subject to or result from a chemical transformation. Such transformation converts the compound or the respective precursor thereof. The chemical transformation is selected from the group comprising hydrolysis, oxidation and reduction. Preferably, such chemical transformation is an enzymatic transformation. More preferably, the transformation is carried out *in vitro* or *in vivo.* This kind of chemical transformation preferably happens to a prodrug which as such or the product thereof may be pharmaceutically active in the meaning of the present invention.

The compounds according to the present invention, the pharmaceutical salts thereof, products and any derivatives, may be modified such that the *in vivo* and/or *in vitro* enzymatic degradation, such as proteolytic degradation, of said compounds is reduced or prevented. Generally, this is done through the incorporation of synthetic amino acids, derivatives, or substituents into the respective compound. Preferably, only one non-naturally occurring amino acid or amino acid side chain is incorporated into the compound, such that the targeting of the inhibitor of the appropriate enzyme is not significantly affected. However, some embodiments that use longer compounds according to the present

invention containing a number of targeting residues may tolerate more than one synthetic derivative. In addition, non-naturally occurring amino acid substituents may be designed to mimic the binding of the naturally occurring side chain to the targeted enzyme, such that more than one synthetic substituent is tolerated. Alternatively, peptide isosteres are used to reduce or prevent the compound's degradation. The resistance of the thus modified compound may be tested against the variety of known commercially available enzymes *in vitro* to determine the stability against enzymatic, preferably proteolytic, stability. Promising candidates may then be routinely screened in animal models, for example using labeled compounds as described herein, to determine the *in vivo* stability and efficacy.

In this embodiment, the resistance of the modified nonproteolytic reactive enzyme inhibitors may be tested against a variety of known commercially available nonproteolytic reactive enzymes *in vitro* to determine their proteolytic stability. Promising candidates may then be routinely screened in animal models, for example using labelled inhibitors, to determine the *in vivo* stability and efficacy.

In a still further aspect the compounds according to the present invention have at least one amino acid side chain. Preferably, the amino acid side chain is in the (S) or L-configuration or in the (R) or D-configuration. Of said configurations the (S) or L-configuration is particularly preferred.

In a preferred embodiment, the compound or pharmaceutically acceptable salt or product of said compound according to the present invention comprises one or more non-naturally occurring amino acids or amino acid side chains. Alternatively or in addition thereto, said compounds may comprise a peptide isostere.

In a further aspect the present invention is related to the use of any of the aforementioned compounds according to the present invention as an inhibitor to rotamases. In connection with the characteristics of the compounds according to the present invention to be active as an inhibitor of (a) rotamase(s), it is sufficient that the respective compound is at least suitable to inhibit at least one rotamase. The compounds according to the present invention which may be used as inhibitors, are also referred to as rotamase inhibitors herein.

Rotamases as such are known in the art and, for example, described in the introductory part of this specification which is incorporated by reference. Rotamases as used herein shall preferably mean cyclophilins, FK-506 binding proteins and the rotamases of the Pin1/parvulin class. The Pin1/parvulin class includes Pins 1, PinL/parvulin, dodo, and Es1/Pft1. Suitable assays to determine whether a compound is suitable to inhibit a rotamase are known to the one skilled in the art and also described in the present examples. Basically, a rotamase is provided the activity of which or non-activity of which may be determined. A candidate inhibitor, i. e. a compound which is to be tested whether it is active as an inhibitor to rotamase, is added to the rotamase and tested whether upon the addition and/or influence of the candidate inhibitor the activity of the rotamase is changed relative to the activity without candidate rotamase inhibitor. If the rotamase activity is decreased by the candidate rotamase inhibitor, said candidate rotamase inhibitor is a rotamase inhibitor according to the present invention.

In another aspect of the present invention the compounds according to the present invention may be used in a method for inhibiting a rotamase. In such case a rotamase is provided and a candidate rotamase inhibitor is added thereto whereupon the activity of rotamase is decreased. Optionally, such decrease in rotamase activity is measured. The techniques used theretofore are basically the same as outlined in connection with the use of the compounds according to the present invention as rotamase inhibitors.

In another aspect of the present invention the compounds according to the present invention are used in a method for quantifying the amount of rotamase activity present in a sample and are for the same purposes used in assays and diagnostic kits for the quantification of rotamases in samples such as blood, lymph, saliva or other tissue samples, bacterial, fungal, plant, yeast, viral or mammalian cell culture. Thus in a preferred embodiment, the sample is assayed using a standard substrate for the appropriate rotamase. A known concentration of a specific inhibitor according to the present invention is added, and allowed to bind to a particular rotamase present. The assay is then rerun, and the loss of activity is correlated to rotamase activity using techniques well known to those skilled in the art. Thus, methods of inhibiting a rotamase are provided, wherein the rotamase inhibitors of the present invention may be added to a sample of rotamase or a sample where it is assumed that a rotamase activity is contained.

The compounds according to the present invention are preferably reversible rotamase inhibitors.

By "reversible" herein is meant that the inhibitor binds non-covalently to the enzyme, and is to be distinguished from irreversible inhibition. See Walsh, Enzymatic Reaction Mechanisms, Freeman & Co., N.Y., 1979. "Reversible" in this context is a term understood by those skilled in the art. Preferably the rotamase inhibitors according to the present invention are competitive inhibitors, that is, they compete with substrate in binding reversibly to the enzyme, with the binding of inhibitor and substrate being mutually exclusive.

In a preferred embodiment of the compounds according to the present invention being active as a rotamase inhibitor, the dissociation constant for inhibition of a rotamase with the inhibitor, generally referred to and characterized by those in the art as $K_{i,}$ is at most about 100 μM. By the term "binding constant" or "dissociation constant" or grammatical equivalents herein is meant the equilibrium dissociation constant for the reversible association of inhibitor with enzyme. The dissociation constants are defined and determined as described below. The determination of dissociation constants is known in the art. For example, for reversible inhibition reactions such as those of the present invention,

the reaction scheme is as follows:

$$E+I \underset{k_2}{\overset{k_1}{\rightleftharpoons}} E^*I \quad \text{(Equation 1)}$$

The enzyme (E) and the inhibitor (I) combine to give an enzyme-inhibitor complex (E*I). This step is assumed to be rapid and reversible, with no chemical changes taking place; the enzyme and the inhibitor are held together by non-covalent forces. In this reaction, $k_1$ is the second order rate constant for the formation of the E*I reversible complex. $k_2$ is the first order rate constant for the dissociation of the reversible E*I complex. In this reaction, $Ki = k_2/K_1$.

The measurement of the equilibrium constant $K_i$ proceeds according to techniques well known in the art. For example, assays generally use synthetic chromogenic or fluorogenic substrates. The respective $K_i$ values may be estimated using the Dixon plot as described by Irwin Segel in Enzyme Kinetics: Behavior and analysis of rapid equilibrium and steady-state enzyme systems, 1975, Wiley-Interscience Publication, John Wiley & Sons, New York, or for competitive binding inhibitors from the following calculation:

$$1-(\nu_i/\nu_o)=[I]/[I]+K_i\ (1+([S]/K_m))) \qquad \text{(Equation 2)}$$

wherein $\nu_o$ is the rate of substrate hydrolysis in the absence of inhibitor, and $\nu_i$ is the rate in the presence of competitive inhibitor.

It is to be understood that dissociation constants are a particularly useful way of quantifying the efficiency of an enzyme with a particular substrate or inhibitor, and are frequently used in the art as such. If an inhibitor exhibits a very low $K_i$ value, it is an efficient inhibitor. Accordingly, the rotamase inhibitors of the present invention have dissociation constants, $K_i$, of at most about 100 µM. Preferably, the rotamase inhibitors according to the present invention exhibit dissociation constants of at most about 10 µM, more preferably about 1 µM, most preferably of at most about 100 nM.

The rotamase inhibitors of the present invention may be easily screened for their inhibitory effect. The inhibitor is first tested against different classes of rotamases for which the targeting group of the inhibitor was chosen, as outlined above. The activity of rotamases is typically measured by using a protease coupled assay with chromogenic substrates and conformer specific proteases. Basically, upon the conformer specific protease activity the chromogenic substrate is converted into a compound which has an absorption characteristic which is different from the starting chromogenic substrate and may thus be selectively measured. This reaction is accelerated in the presence of the rotamase and decelerated in the presence of rotamase-inhibitors. Alternatively, many rotamases and their corresponding chromogenic substrates are commercially available. Thus, a variety of rotamases are routinely assayed with synthetic chromogenic substrates in the presence and absence of the rotamase inhibitor, to confirm the inhibitory action of the compound, using techniques well known in the art. The effective inhibitors are then subjected to kinetic analysis to calculate the $K_i$ values, and the dissociation constants determined.

If a compound inhibits at least one rotamase, it is a rotamase inhibitor for the purposes of the present invention. Preferred embodiments of the rotamase inhibitors according to the present invention are compounds and inhibitors, respectively, that exhibit the correct kinetic parameters Ki below 100 µM against the targeted rotamases.

In a further aspect of the present invention any of the compounds used as rotamase inhibitors or as a medicament may be labelled.

By a "labelled rotamase inhibitor" herein is meant a rotamase inhibitor that has at least one element, isotope or chemical compound attached to enable the detection of the rotamase inhibitor or the rotamase inhibitor bound to a rotamase. In general, labels as used herein, fall into three classes: a) isotopic labels, which may be radioactive or heavy isotopes; b) immune labels, which may be antibodies or antigens; and c) colored or fluorescent dyes. The labels may be incorporated into the rotamase inhibitor at any position. Examples of useful labels include $^{14}C$, $^{13}C$, $^{15}N$, $^{3}H$, biotin, and fluorescent labels as are well known in the art. Examples for fluorescent labels are fluorescein, 6-FAM, HEX, TET, CY-5, CY-3, CY-7 and Texas Red.

In a further aspect the compounds according to the present invention, particularly those having rotamase inhibitory activity, may be used for removing, identifying and/or inhibiting rotamases, preferably contaminating rotamases, in a sample. Preferably, the sample is a biological sample. Even more preferably such sample is selected from the group comprising blood, lymph, saliva, tissue samples and bacterial, fungal, plant, viral and mammalian cell cultures.

In an embodiment of the present invention the rotamase inhibitors of the present invention are, for example,

added to a sample where the catalytic activity by contaminating rotamases is undesirable. Alternatively, the rotamase inhibitors of the present invention may be bound to a chromatographic support, using techniques well known in the art, to form an affinity chromatography column. A sample containing an undesirable rotamase is run through the column to remove the rotamase. Alternatively, the same methods may be used to identify new rotamases. In doing so, a new rotamase contained in a sample may bind to the rotamase inhibitor bound to the chromatographic support and upon elution, preferably a specific elution, from said chromatographic support, characterized and compared to other rotamase activities with regard to, among others, specificities. The characterization of the rotamase as such is known to the one skilled in the art.

In a further aspect the present invention is related to the use of the compounds according to the present invention as a medicament and for the manufacture of a medicament, respectively.

This use of the compounds according to the present invention is based on the fact that the compounds according to the present invention are inhibitors of rotamases and rotamases in turn have been identified in both procaryotic and eucaryotic cells such as in bacteria, fungi, insect and mammalian cells. In this cellular environment rotamases are known to have an impact on cell proliferation and mitosis, respectively. Because of this, rotamase inhibitors may be used for the treatment of a wide variety of disorders involving cell cycle regulation, both procaryotic and eucaryotic cell cycle regulation. The term "treatment" as used herein comprises both treatment and prevention of a disease. It also comprises follow-up treatment of a disease. Follow-up treatment is realized upon a treatment of a disease using compounds preferably different from the one according to the present invention. For example, after stimulating the growth of a cell, tissue or the like by the application of a respective compound such as, e. g., erythropoietin, it might be necessary to stop an overshooting reaction of cell proliferation which may be obtained using the compounds according to the present invention.

As used herein, the term "disease" describes any disease, diseased condition or pathological condition. Such disease may also be defined as abnormal condition. Also, in case of a pathogen, disease means a condition where a pathogen or an unwanted organism is present or present in a concentration or compartment where it is undesired and thus subject to reduction in numbers, removal, elimination and/or destruction by using the compounds according to the present invention.

Cell proliferative disorders contemplated for treatment using the compounds according to the present invention and for the methods disclosed herein include disorders characterized by unwanted or undesired, inappropriate or uncontrolled cell growth. Preferably, the disease is selected from the group comprising neurodegenerative diseases, stroke, inflammatory diseases, immune based disorders, infectious diseases, heart diseases, fibrotic disorders, cardiovascular diseases and cell proliferative diseases. Rotamases comprise families of ubiquitous and highly conserved enzymes who have been reported to play important roles in biological processes like protein folding, proteolysis, protein dephosphorylation, peptide transport function, cell cycle regulation, protein synthesis. Furthermore various isomerases have been shown to have regulatory functions as stable or dynamic part of heterooligomeric complexes containing physiologically relevant proteins e.g. hormone receptors, ion channels, kinases, and growth factor receptors.

Preferably, the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral atrophy, prion diseases, and motor neuron diseases.

The compounds according to the present invention are additionally useful in inhibiting cell cycle (mitosis) or cell division in pathogenic organisms and are, therefore, useful for treating infectious diseases.

In a preferred embodiment the infectious is selected from the group comprising fungal, viral, bacterial and parasite infection.

Fungal infections contemplated for treatment using the compounds and methods according to the present invention include systemic fungal infections, dermatophytoses and fungal infections of the genito-urinary tract. Fungal infections, preferably systemic fungal infections, include those caused by *Histoplasma, Coccidioides, Cryptococcus, Blastomyces, Paracoccidioides, Aspergillus, Nocardia, Sporothrix, Rhizopus, Absidia, Mucor, Hormodendrum, Phialophora, Rhinosporidium,* and the like. Dermatophyte infections include those caused by *Microsporum, Trichophyton, Epidermophyton, Candida, Pityrosporum,* and the like. Fungal disorders of the genito-urinary tract include infections caused by *Candida, Cryptococcus, Aspergillus, Zygomycodoides,* and the like. Infection by such organisms causes a wide variety of disorders such as ringworm, thrush or candidiasis, San Joaquin fever or Valley fever or coccidiodomycosis, Gilchrist's disease or blastomycosis, aspergillosis, cryptococcosis, histioplasmosis, paracoccidiomycosis, zygomycosis, mycotic keratitis, nail hair and skin disease, Lobo's disease, lobomycosis, chromoblastomycosis, mycetoma, and the like. These infections can be particularly serious, and even fatal, in patients with a depressed immune system such as organ transplant recipients and persons with acquired immunodefficiency syndrome (AIDS). Insofar a patient group which can be treated using the inhibitors according to the present invention are persons with AIDS, particularly those suffering from any of the aforementioned infectious diseases.

In a further embodiment the bacterial infection is selected from the group comprising infections caused by

both Gram-positive and Gram-negative bacteria, including infections caused by *Staphylococcus, Clostridium, Streptococcus, Enterococcus, Diplococcus, Hemophilus, Neisseria, Erysipelothricosis, Listeria, Bacillus, Salmonella, Shigella, Escherichia, Klebsiella, Enterobacter, Serratia, Proteus, Morganella, Providencia, Yersinia, Camphylobacter, Mycobacteria, Helicobacter, Legionalla, Nocardia,* and the like.

In a preferred embodiment the bacterial infection causes a wide variety of diseases. Said disorders are selected, among others, from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

In another embodiment the disease is a viral infection, more particularly a viral infection caused by a virus selected from the group comprising retrovirus, HIV, Papilloma virus, Polio virus, Epstein-Barr, Herpes virus, Hepatitis virus, Papova virus, Influenza virus, Rabies, JC, encephalitis causing virus, hemorrhagic fever causing virus (such Ebola Virus and Marburg Virus).

In a further embodiment the parasite infection is selected from the group comprising infections caused by *Trypanosoma, Leishmania, Trichinella, Echinococcus, Nematodes, Classes Cestoda, Trematoda, Monogenea, Toxoplasma, Giardia, Balantidium, Paramecium, Plasmodium or Entamoeba.*

The disease may further be a cell proliferative disorder which preferably is selected from the group characterized by unwanted, inappropriate or uncontrolled cell growth. Particular examples include cancer, fibrotic disorders, non-neoplastic growths. The neoplastic cell proliferative disorder is preferably selected from the group comprising solid tumors, and hematopoeitic cancers such as lymphoma and leukemia.

More preferably, the solid tumor is selected from the group comprising carcinoma, sarcoma, osteoma, fibrosarcoma, and chondrosarcoma.

More preferably, the cell proliferative disorder is selected from the group comprising breast cancer, prostate cancer, colon cancer, brain cancer, lung cancer, pancreatic cancer, gastric cancer, bladder cancer, kidney cancer and head and neck cancer. Preferably, the lung cancer is non-small lung cancer and small lung cancer.

In case the disease is a non-proliferative cell proliferative disorder, it is preferably selected from the group comprising fibrotic disorder. Preferably, the fibrotic disorder is fibrosis.

The disease may also be a non-neoplastic cell proliferative disorder which is selected from the group comprising prostatic hypertrophy, preferably benign prostatic hypertrophy, endometriosis, psoriasis, tissue repair and wound healing.

Fibrotic disorders which may be treated using the compounds according to the present invention are generally characterized by inappropriate overproliferation of non-cancerous fibroblasts. Examples thereof include fibromyalgia, fibrosis (cystic, hepatic, idopathic pulmonary, pericardial and the like), cardiac fibromas, fibromuscular hyperplasia, restenosis, atherosclerosis, fibromyositis, and the like.

In another embodiment the immune based and/or inflammatory disease is an autoimmune disease or autoimmune disorder. In a further embodiment, the immune based and/or inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease, and dermatomyositis.

In a further preferred embodiment the immune based and/or inflammatory disease is an inflammatory condition.

In a still further embodiment the immune based and/or inflammatory disease is selected from the group comprising inflammation associated with bums, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

In a further embodiment of the various aspects of the present invention the rotamase is human Pin1 and the rotamase involved in the mechanism underlying the various diseases is human Pin1, respectively.

It is also within the present invention that the compounds according to the present invention may be used for the treatment of a patient suffering from a disease or disease condition as defined above. Such treatment comprises the administration of one or several of the compounds according to the present invention or a medicament or pharmaceutical composition described herein.

Toxicity and therapeutic efficacy of a compound can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Compounds which exhibit large therapeutic indices are preferred. The data obtained from these cell culture assays and animal studies can be used in formulating a range of dosages suitable for use in humans. The dosage may vary

within this range depending upon a variety of factors, e.g., the dosage form employed, the route of administration utilized, the condition of the subject, and the like

For any compound used according to the present invention, the therapeutically effective dose can be estimated initially from cell culture assays by determining an $IC_{50}$ (i.e., the concentration of the test substance which achieves a half-maximal inhibition of rotamase activity). A dose can then be formulated in animal models to achieve a circulating plasma concentration range that includes the $IC_{50}$ as determined in cell culture. Such information can be used to more accurately determine useful doses in humans. Levels in plasma may be measured, for example by HPLC.

It should be noted that the attending physician would know how to and when to terminate, interrupt, or adjust administration due to toxicity, to organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient. Typically, the dose will be between about 1-1000 mg/kg of body weight. About 1 mg to about 50 mg will preferably be administered to a child, and between 25 mg and about 1000 mg will preferably be administered to an adult.

A program comparable to that discussed above may be used in veterinary medicine. The exact dose will depend on the disorder to be treated and the amount of rotamases to be inhibited, and will be ascertainable by one skilled in the art using known techniques. For example, as outlined above, some disorders are associated with increased levels of rotamases.

Depending on the specific conditions being treated, such agents may be formulated and administrated systemically or locally. Techniques for formulation and administration may be found in "Remington's Pharmaceutical Sciences", 1990, 18th ed., Mack Publishing Co., Easton, PA. The administration of a compound according to the present invention can be done in a variety of ways, including, but not limited to, orally, subcutaneously, intravenously, intranasally, transdermally, intraperitoneally, intramuscularly, intrapulmonary, vaginally, rectally, or intraocularly, just to name a few. In some instances, for example, in the treatment of wounds and inflammation, the rotamase inhibitors may be directly applied as a solution or spray.

In a further aspect the present invention is related to a medicament or a pharmaceutical composition comprising at least one active compound and at least one pharmaceutically acceptable carrier, excipient or diluent. As used herein, the active compound is a compound according to the present invention, a pharmaceutically salt or base thereof or a prodrug thereof, if not indicated to the contrary. The active compound may also be a pharmaceutically acceptable derivative of any of the compounds of the present invention. A pharmaceutically acceptable derivative refers to any pharmaceutially acceptable salt or ester of a compound of the present invention, however, is not limited thereto, or any other compound which, upon administration to a patient, is capable of providing, either directly or indirectly, a compound of the present invention, a pharmacologically active metabolite or pharmacologically active residue thereof.

For injection, compounds of the invention may be formulated in aqueous solution, preferably in physiologically compatible buffers such as Hank's solution, Ringer's solution, or physiologically saline buffer. For transmucosal administration, penetrants appropriate to the barrier to be permeated are used in the formulation. Such penetrants are generally known in the art.

The use of pharmaceutical acceptable carriers to formulate the compounds according to the present invention into dosages or pharmaceutical compositions suitable for systemic administration is within the scope of the present invention. With proper choice of carrier and suitable manufacturing practice, the compositions of the present invention, in particular those formulated as solutions, may be administered parenterally, such as by intravenous injection. The compounds can be readily formulated using pharmaceutically acceptable carriers well known in the art into dosages suitable for oral administration. Such carriers enable the compounds according to the present invention to be formulated as tablets, pills, capsules, dragees, liquids, gels, syrups, slurries, suspensions and the like, for oral ingestion by a subject to be treated. Suitable pharmaceutical carriers include, but are not limited to, water, salt solutions, alcohols, polyethylene glycols, gelatine, carbohydrates, such as lactose, amylose or starch, magnesium stearate, talc, silicic acid, viscous paraffine, fatty acid esters, hydroxymethylcellulose, polyvinylpyrolidone and the like.

Compounds according to the present invention or medicaments comprising them intended to be administered intracellularly may be administered using techniques well known to those of ordinary skill in the art. For example, such agents may be encapsulated into liposomes, then administered as described above. Liposomes are spherical lipid bilayers with aqueous interiors. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Delivery systems involving liposomes are discussed in International Patent Publication No. WO 91/19501, as well as U.S. Patent No. 4,880,635 to Janoff et al. The publications and patents provide useful descriptions of techniques for liposome drug delivery and are incorporated by reference herein in their entirety.

Pharmaceutical compositions comprising a compound according to the present invention for parenteral administration include aqueous solutions of the active compound(s) in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injections suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions.

Pharmaceutical compositions comprising a compound according to the present invention for oral use can be obtained by combining the active compound(s) with solid excipient, optionally grinding the resulting mixture, and processing the mixture of granules, after adding suitable auxiliaries, if desired, to obtain tablets or dragee cores.

Suitable excipients are, in particular, fillers such as sugars, including lactose, sucrose, mannitol, sorbitol, and the like; cellulose preparations, such as, for example, maize starch, wheat starch, rice starch, potato starch, gelatin, gum tragacanth, methyl cellulose, hydroxypropylmethyl cellulose, sodium carboxymethyl cellulose, polyvinylpyrrolidone (PVP) and the like, as well as mixtures of any two or more thereof. If desired, disintegrating agents may be added, such as cross-linked polyvinyl pyrrolidone, agar, alginic acid or a salt thereof such as sodium alginate, and the like.

Dragee cores as a pharmaceutical composition comprising a compound according to the present invention are provided with suitable coatings. For this purpose, concentrated sugar solutions may be used, which may optionally contain gum arabic, talc, polyvinyl pyrrolidone, carbopol gel, polyethylene glycol, titanium dioxide, lacquer solutions, suitable organic solvents or solvent mixtures, and the like. Dyestuffs or pigments may be added to the tablets or dragee coatings for identification or to characterize different combinations of active compound doses.

Pharmaceutical preparations comprising a compound according to the present invention which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

A "patient" for the purposes of the present invention, i. e. to whom a compound according to the present invention or a pharmaceutical composition according to the present invention is administered, includes both humans and other animals and organisms. Thus the compounds, pharmaceutical compositions and methods are applicable to or in connection with both human therapy and veterinary applications. For example, the veterinary applications include, but are not limited to, canine, bovine, feline, porcine, caprine, equine, and ovine animals, as well as other domesticated animals including reptiles, such as iguanas, turtles and snakes, birds such as finches and members of the parrot family, lagomorphs such as rabbits, rodents such as rats, mice, guinea pigs and hamsters, amphibians, fish, and arthropods. Valuable non-domesticated animals, such as zoo animals, may also be treated. In the preferred embodiment the patient is a mammal, and in the most preferred embodiment the patient is human.

The pharmaceutical composition according to the present invention comprises at least one compound according to the present invention, preferably a rotamase inhibitor according to the present application, in a form suitable for administration to a patient. Preferably, a compound according to the present application is in a water soluble form, such as being present as a pharmaceutically acceptable salt, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The pharmaceutical compositions according to the present invention may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Additives are well known in the art, and are used in a variety of formulations.

The compounds according to the present invention are, in a further embodiment, administered to a subject either alone or in a pharmaceutical composition where the compound(s) is mixed with suitable carriers or excipient(s).

In treating a subject, a therapeutically effective dose of compound (i.e. active ingredient) is administered. A therapeutically effective dose refers to that amount of the active ingredient that produces amelioration of symptoms or a prolongation of survival of a subject which can be determined by the one skilled in the art doing routine testing.

On the other hand, the compounds according to the present invention which have a rotamase inhibitory activity may as such or contained in a pharmaceutical composition according to the present invention be used in drug potentiation applications.

For example, therapeutic agents such as antibiotics or antitumor drugs can be inactivated through the catalytic action of endogenous rotamases, thus rendering the administered drug less effective or inactive. Accordingly, the rotamase inhibitors of the invention may be administered to a patient in conjunction with a therapeutic agent in order to potentiate or increase the activity of the drug. This coadministration may be by simultaneous administration, such as a mixture of the rotamase inhibitor and the drug, or by separate simultaneous or sequential administration.

According to the present invention the compounds disclosed herein, referred to as compounds according to the present invention, may be used as a medicament or for the manufacture of medicament or in a method of treatment of a patient in need thereof. Insofar any of these compounds constitute a pharmaceutical compound. The use of this kind of compound also comprises the use of pharmaceutically acceptable derivatives of such compounds.

In addition, the compounds according to the present invention may be transformed upon application to an organism such as a patient, into the pharmaceutically active compound. Insofar the compounds according to the present invention may be prodrugs which, however, are nevertheless used for the manufacture of the medicaments as disclosed herein given the fact that at least in the organism they are changed in a form which allows the desired pharmaceutical effect.

It is to be understood that any of the pharmaceutical compositions according to the present invention may be used for any of the diseases or conditions described herein.

The pharmaceutical compositions according to the present invention may be manufactured in a manner that itself is known, e.g., by means of conventional mixing, dissolving, granulating, dragee-mixing, levigating, emulsifying, encapsulating, entrapping, lyophilizing, processes, or the like.

In a further aspect of the present invention the compounds of the present invention may be used as insecticides as they may prevent cell cycle mitosis in insect cells and thus can be used to control the growth and proliferation of a variety of insect pests. This aspect of the present invention has important applications in agriculture, such as in the field, in the storage of agricultural products and the like. Additionally, the compounds according to the present invention are useful for controlling insect populations, preferably in places inhabited by men, such as homes, offices and the like.

Any of the compounds according to the present invention containing one or more asymmetric carbon atoms may occur as racemates and racemic mixtures, single enantiomers, diastereomeric mixtures and individual diastereomers. All such isomeric forms of these compounds are expressly included in the present invention. Each stereogenic carbon may be in the R or S configuration, or a combination of configurations.

It shall be understood by one of ordinary skill in the art that all compounds of the invention are those which are chemically stable. This applies to any of the various uses of the compounds according to the present invention disclosed herein.

In determining the suitability of any of the compounds according to the present applications for the various uses, besides the particular profile to be met by such a compound, also it has to be checked whether it is stable to proteolytic degradation. The resistance of the compound used as rotamase inhibitor or pharmaceutical may be tested against a variety of non-commercially available rotamases *in vitro* to determine its proteolytic stability. Promising candidates may then be routinely screened in animal models, for example using labelled inhibitors, to determine the *in vivo* stability and efficacy. In any of the aforementioned uses the compound may be present in a crude or purified form. Methods for purifying the compounds according to the present invention are known to the one skilled in the art.

The invention is now further illustrated by reference to the following figure and examples from which further advantages, features and embodiments may be taken. It is understood that these examples are given for purpose of illustration only and not for purpose of limitation. All references cited herein are incorporated by reference.

Figs. 1 to 6b show various methods for the synthesis of the compounds according to the present invention which will be explained in more detail in the following examples.

EXAMPLE 1

**General synthetic methods**

Compounds of the invention may in principle be synthesized by methods described below. In addition, further methods for the synthesis of the known compounds used according to the present invention are described, for example, in WO 01/19816, WO 01/30772, US patent application 2001/0046207, US 4,927,809; WO 00/55126, WO 99/56765, WO 01/09110, WO 01/47886, WO 00/49008, WO 99/24460, WO 00/51998, WO 00/48992 and WO 01/49288. Standard

peptide coupling, protection and deprotection reactions (M. Bodansky, The Practice of Peptide Synthesis, Springer-Verlag, 1984) are employed in these syntheses.

As used herein, the following abbreviations are used:

Ar is argon;
Boc is tertiary butoxy carbamoyl;
Bth is benzo[b]thiophen-2-yl;
t-Bu is tertiary butyl;
DCM is dichloromethane;
DIC is diisopropyl carbodiimide;
DIPEA is *N,N*-diisopropylethylamine;
DMF is *N,N*-dimethylformamide;
DMSO is *N,N*-dimethylsulfoxide;
eq is equivalent;
$Et_3N$ is triethylamine;
EtOAc is ethyl acetate;
HBTU is 2-(1H-benzotriazole-1-yl)-1,1,3,3-tetramethyluronium hexafluorophosphate;
HPLC is high performance liquid chromatography;
h is hour;
MeOH is methanol;
$MgSO_4$ is magnesium sulfate;
NaCl is sodium chloride;
$NaHCO_3$ is sodium hydrogencarbonate;
Nal is naphthalene-2-yl;
PyBrOP is benzotriazole-1-yl-oxy-tris-pyrrolidino-phosphonium hexafluorophosphate;
TFA is trifluoroacetic acid

According to scheme 1 depicted in Fig. 1 suitable protected amino acid derivatives 1 bearing $R_5$, $R_6$, $R_7$ and 4 bearing $R_5$, $R_6$, $R_7$, $R_8$, $R_9$ are transformed to the corresponding primary amides 2 and 5 using ammonia or ammonium chloride under standard coupling conditions typically used in peptide synthesis. Suitable protecting groups ($R_5$) for the amino functions are the t-butoxycarbonyl (Boc), 9-fluorenylmethoxycarbonyl (Fmoc), and other groups. Examples of standard coupling conditions would be reacting the protected amino acid derivatives 1 and 4 in the presence of a coupling reagent such as benzotriazol-1-yloxytrispyrrolidinophosphonium hexafluorophosphate (PyBOP®), 1-(3-dimethylaminopropyl)-3-ethylcarbodiimide hydrochloride (EDCI), *O*-benzotriazol-1-yl-*N,N,N',N'*-tetramethyluronium hexafluorophosphate (HBTU), 1,3-dicyclohexylcarbodiimide (DCC), or the like, in a suitable solvent (*N*-methylpyrrolidinone, DMF, DCM, or the like). Additionally an appropriate catalyst (e.g., 1-hydroxybenzotriazole (HOBT), 1-hydroxy-7-azabenzotriazole (HOAt), or the like) and non-nucleophilic bases (e.g., *N*-methylmorpholine, triethylamine, *N, N*-diisopropylethylamine or the like, or any suitable combination thereof) may be added. The resulting amides are converted to the corresponding nitriles 3 and 6 by dehydration. Suitable dehydratation conditions can be cyanuric chloride in DMF, trifluoroacetic anhydride in DCM in the presence of pyridine (N. D. Hone, L. J: Payne, C. M. Tice, *Tetrahedron Lett.,* (2001) **42**, 1115-1118), benzoylsulfonyl chloride in pyridine (T. T. Van, E. Kojro, Z. Grzonka, *Tetrahedron* (1977) **33**, 2299).

According to scheme 2 depicted in Fig. 2 suitable protected building block derivatives 8 bearing $R_{5,}$ $R_{6,}$ $R_7$ and 10 bearing $R_{5,}$ $R_{6,}$ $R_{7,}$ $R_{8,}$ $R_9$ are reacted with activated amino acid derivatives 7 bearing $R_{2,}$ $R_{3,}$ $R_4$ under standard coupling conditions used in peptide synthesis.the resulting derivatives 9 and 11 are obtained after deprotection of the amine.

According to scheme 3a depicted in Fig. 3a a suitable protected amines 9 bearing $R_{2,}$ $R_{3,}$ $R_{4,}$ $R_{5,}$ $R_{6,}$ $R_7$ are allowed to react with different reagents. They can be reacted with acids, acyl chlorides or anhydrides to provide 9a. Acids can be condensed by standard peptide coupling conditions such as PyBrOP, DIPEA, 1-hydroxybenzotriazole-6-sulfonamidomethyl polystyrene in dry DMF (I. E. Pop, *J. Org. Chem.* (1997) **62**, 2594). Acyl chlorides or anhydrides can be reacted in dry DCM in the presence of a non nucleophilic base like DIPEA followed by sequestering any remaining acyl chlorides or anhydride by a polymer-supported quenching reagent like tris-(2-aminoethyl)-amine polystyrene (R. J. Booth, J. C. Hodges, *J. Am. Chem. Soc.* (1997) **119**, 4882; M. W. Creswell, G. L. Bolton, J. C. Hodges, M. Meppen, *Tetrahedron* (1998) **54**, 3983). Sulfonamide derivatives 9b are obtained after reaction with different sulfonyl chlorides in dry DCM in the presence of a non nucleophilic base like DIPEA followed by sequestering any remaining sulfonyl chloride by a polymer-supported quenching reagent like tris-(2-aminoethyl)-amine polystyrene (R. J. Booth, J. C. Hodges, *J. Am. Chem. Soc.* (1997) **119**, 4882).

Carbamates 9c (R. J. Booth, J. C. *Hodges, J. Am. Chem. Soc.* (1997) **119**, 4882) are obtained by reacting

chloroformates in dry DCM in the presence of a non nucleophilic base like DIPEA, followed by sequestering any remaining chloroformate by a polymer-supported quenching reagent like tris-(2-aminoethyl)-amine polystyrene. Thioureas 9d and ureas 9e were obtained after reaction with thio-isocyanates or isocyanates in DCM in the presence of a non nucleophilic base like DIPEA, followed by sequestering any remaining thio-isocyanate or isocyanate by a polymer-supported quenching reagent like tris-(2-aminoethyl)-amine polystyrene (R. J. Booth, J. C. Hodges, *J. Am. Chem. Soc.* (1997) **119**, 4882).

According to scheme 3b depicted in Fig. 3b suitable protected amines 11 bearing $R_2$, $R_3$, $R_4$, $R_5$, $R_6$, $R_7$, $R_8$, $R_9$ is allowed to react with different reagents as described for scheme 3 a.

According to scheme 4 depicted in Fig. 4 suitable protected dipeptides 1 bearing $R_5$, $R_6$, $R_7$ or 7 $R_5$, $R_6$, $R_7$, $R_8$, $R_9$ are coupled with a resin like Rink amide PEGA resin in the presence of a coupling reagent such as benzotriazol-1-yloxytrispyrrolidinophosphonium hexafluorophosphate (PyBOP®), 1-(3-dimethylaminopropyl)-3-ethylcarbodiimide hydrochloride (EDCI), *O*-benzotriazol-1-yl-*N,N,N',N'*-tetramethyluronium hexafluorophosphate (HBTU), 1,3-dicyclohexylcarbodiimide (DCC), or the like, in a suitable solvent (*N*-methylpyrrolidinone, DMF, DCM, or the like). Additionally an appropriate catalyst (e.g., 1-hydroxybenzotriazole (HOBT), 1-hydroxy-7-azabenzotriazole (HOAt), or the like) and non-nucleophilic bases (e.g., *N*-methylmorpholine, triethylamine, *N,N*-diisopropylethylamine or the like, or any suitable combination thereof) may be added. An example of a suitable protecting group for the amine function is the 9-fluorenylmethoxycarbonyl (Fmoc) group. This is followed by deprotection to give the free amine. An example of a suitable deprotection is piperidine in DMF. A suitable protected building block bearing $R_2$, $R_3$, $R_4$ is then coupled in the same conditions ad described before. Desired compounds 14 and 17 are obtained after deprotection of the amine function.

According to scheme 5a depicted in Fig. 5a suitable protected immobilized amines 14 bearing $R_2$, $R_3$, $R_4$, $R_5$, $R_6$, $R_7$ are allowed to react with different reagents. They can be reacted with acids, acyl chlorides or anhydrides to provide the corresponding amides 14a. An example of standard coupling conditions with acide would be to combine with HBTU and diisopropylethylamine in anhydrous DMF. Acyl chloride or anhydride would be reacted in dry DCM in the presence of a non nucleophilic base like DIPEA (B. Raju, T. P. Kogan, *Tetrahedron Lett.* (1997) **38**, 4965). Sulfonamide derivatives 14b are obtained after reaction with differents sulfonyl chlorides in dry DCM in the presence of DMAP (C. Gennari, B. Salom, D. Potenza, A. Williams, *Angew. Chem., Int. Ed. Engl.* (1994) **33** 2067). Carbamates 14c are obtained by reacting chloroformates in dry DCM in the presence of a non nucleophilic base like DIPEA (T. Fukuyama, L. Li, A. A. Laird, R. K. Frank, *J. Am. Chem. Soc.* (1987) **109**, 1587). Thioureas 14d and ureas 14e were obtained after reaction with thio-isocyanates or isocyanates in DCM with or without a non nucleophilic base like DIPEA (P. C. Kearney, M. Fernandez, J. A. Flygare, *J. Am. Chem. Soc.* (1998) **63**, 196).

According to scheme 5b depicted in Fig. 5b suitable protected immobilized amines 17 bearing $R_2$, $R_3$, $R_4$, $R_5$, $R_6$, $R_7$, $R_8$, $R_9$ are allowed to react with different reagents as described for scheme 5 a.

According to scheme 6a depicted in Fig. 6a suitable immobilized derivatives 14 a-e bearing $R_1$, $R_2$, $R_3$, $R_4$, $R_5$, $R_6$, $R_7$ are cleaved from the resin to give the amides 18 a-e that are converted to the corresponding nitriles 19 a-e by dehydration. An example of a suitable cleavage is TFA in DCM. The nitriles 19 a-e are also obtained directly from the immobilized derivatives 14 a-e using trifluoroacetic anhydride for the dehydration.

According to scheme 6b depicted in Fig. 6b suitable immobilized derivatives 17 a-e bearing $R_1$, $R_2$, $R_3$, $R_4$, $R_5$, $R_6$, $R_7$, $R_8$, $R_9$ are reacted to give the corresponding nitriles 21 a-e as described for scheme 6 a.

**Method A: Coupling of acyl chlorides with derivatized amines in solution.**

Amine salt (1 eq) was dissolved in a mixture of 10% dry DMSO in anhydrous DCM, acyl chloride (1.5 eq) and diisopropylethylamine (2 eq) were added under Ar, and stirred for 2 h at room temperature. Tris-(2-aminoethyl)-amine polystyrene (6 eq relative to excess acyl chloride) and (polystyrylmethyl)trimethylammonium bicarbonate (4 eq relative to expected amine salt) were added to the reaction mixture and agitated for 18 h at room temperature. The supernatant was separated from the resin by filtration and the polymeric beads were washed with DCM and a mixture of DCM/MeOH (1/1) (three times). After evaporation of the solvent, the residue was dissolved/suspended in water and lyophilized to give the crude product.

**Method B: Coupling of anhydrides with derivatized amines in solution.**

Amine salt (1 eq) was dissolved in a mixture of 10% dry DMSO in anhydrous DCM, anhydride (1.5 eq) and diisopropylethylamine (2 eq) were added under Ar, and stirred for 18 h at room temperature. Tris-(2-aminoethyl)-amine polystyrene (6 eq relative to excess anhydride) and (polystyrylmethyl)trimethylammonium bicarbonate (4 eq relative to expected amine salt) were added to the reaction mixture and agitated for 18 h at room temperature. The supernatant was separated from the resin by filtration and the polymeric beads were washed with DCM and a mixture of DCM/MeOH (1/1) (three times). After evaporation of the solvent, the residue was dissolved/suspended in water and lyophilized to give the crude product.

**Method C: Coupling of chloroformates with derivatized amines in solution.**

Amine salt (1 eq) was dissolved in a mixture of 10% dry DMSO in anhydrous DCM, chloroformate (1.5 eq) and diisopropylethylamine (2 eq) were added under Ar, and stirred for 18 h at room temperature. Tris-(2-aminoethyl)-amine polystyrene (6 eq relative to excess anhydride) and (polystyrylmethyl)trimethylammonium bicarbonate (4 eq relative to expected amine salt) were added to the reaction mixture and agitated for 18 h at room temperature. The supernatant was separated from the resin by filtration and the polymeric beads were washed with DCM and a mixture of DCM/MeOH (1/1) (three times). After evaporation of the solvent, the residue was dissolved/suspended in water and lyophilized to give the crude product.

**Method D: Coupling of acids with derivatized amines in solution.**

<u>Method D1: Coupling using 1-hydroxybenzotriazole-6-sulfonamidomethyl polystyrene</u>

To a solution of PyBrOP (2 eq), acid (2 eq) and diisopropylethylamine (4 eq) in anhydrous DMF was added 1-hydroxybenzotriazole-6-sulfonamidomethyl polystyrene (2 eq). The mixture was reacted at room temperature for 5 h. After the first activation step the resin was washed with DMF (three times). The second activation step was performed under the same conditions as the first one, and the resin was washed with DMF (five times).
The amine salt (1 eq) was added to a suspension of the resin in anhydrous DCM and diisopropylethylamine (2 eq). The polymer-bound activated ester was reacted with this mixture at room temperature. After 20 h, the supernatant was separated from the resin by filtration. The polymeric beads were washed with DCM and a mixture of DCM/MeOH (1/1) (three times), the solvent was removed under vacuum. The residue was dissolved/suspended in water, and lyophilized to give the crude product.

<u>Method D2: Coupling using *N*-cyclohexylcarbodiimide, *N*'-methyl polystyrene</u>

Amine salt (1 eq) and acid (1.5 eq) in a mixture of 10% dry DMSO in anhydrous DCM were stirred under Ar. After 10 min *N*-cyclohexylcarbodiimide, *N'*-methyl polystyrene (2 eq) was added. The reaction was stirred overnight at room temperature. The supernatant was separated from the resin by filtration. The polymeric beads were washed with DCM and a mixture of DCM/MeOH (1/1) (three times), the solvent was removed under vacuum. The residue was dissolved/suspended in water and lyophilized to give the crude product.

<u>Method D3: Coupling using standard conditions for peptides</u>

To a solution of amine salt (1 eq) in anhydrous DMF was added the acid (1 eq), HBTU (1 eq) and $Et_3N$ (2 eq). The reaction mixture was stirred overnight and then diluted with EtOAc. The organic phase was washed with saturated aqueous solution of $NaHCO_3$ and saturated aqueous solution of NaCl, dried over $MgSO_4$, filtered and evaporated. The residue was purified by flash-chromatography.

**Method E: Coupling of sulfonyl chlorides with derivatized amines in solution.**

Amine salt (1 eq) was dissolved in a mixture of 10% dry DMSO in anhydrous DCM, sulfonyl chloride (1.5 eq) and diisopropylethylamine (4 eq) were added under Ar, and stirred 2 h at room temperature. Tris-(2-aminoethyl)-amine polystyrene (3 eq relative to excess sulfonyl chloride) and (polystyrylmethyl)trimethylammonium bicarbonate (4 eq relative to expected amine salt) were added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was dissolved/suspended in water and lyophilized to give the crude product.

**Method F: Coupling of isocyanates with derivatized amines in solution.**

Amine salt (1 eq) was dissolved in a mixture of 10% dry DMSO in anhydrous DCM, isocyanate (1.5 eq) and diisopropylethylamine (2 eq) were added under Ar, and stirred for 2 h at room temperature. Tris-(2-aminoethyl)-amine polystyrene (3 eq relative to excess isocyanate) and (polystyrylmethyl)trimethylammonium bicarbonate (4 eq relative to expected amine salt) were added to the reaction mixture and agitated for 18 h at room temperature. The supernatant was separated from the resin by filtration, the polymeric beads were washed with DCM and a mixture of DCM/MeOH

(1/1) (three times). After evaporation of the solvent, the residue was dissolved/suspended in water, and lyophilized to give the crude product.

Method G: Coupling of thio-isocyanates with derivatized amines in solution. Amine salt (1 eq) was dissolved in a mixture of 10% dry DMSO in anhydrous DCM, thio-isocyanate (1.5 eq) and diisopropylethylamine (2 eq) were added under Ar, and stirred for 5 h at room temperature. Tris-(2-aminoethyl)-amine polystyrene (6 eq relative to excess thio-isocyanate) and (polystyrylmethyl)trimethylammonium bicarbonate (4 eq relative to expected amine salt) were added to the reaction mixture and agitated for 18 h at room temperature The supernatant was separated from the resin by filtration, the polymeric beads were washed with DCM and a mixture of DCM/MeOH (1/1) (three times). After evaporation of the solvent, the residue was dissolved/suspended in water, and lyophilized to give the crude product.

**Method H: Coupling of acyl chlorides with derivatized amines on solid-phase.**

The immobilized amine (1 eq) was swollen in anhydrous DCM, acyl chloride (5 eq) and diisopropylethylamine (5 eq) were added, the mixture was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude product.

**Method I: Coupling of anhydrides with derivatized amines on solid-phase.**

The immobilized amine (1 eq) was swollen in anhydrous DCM, anhydride (5 eq) and diisopropylethylamine (5 eq) were added, the mixture was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude products.

**Method J: Coupling of chloroformates with derivatized amines on solid-phase.**

The immobilized amine (1 eq) was swollen in anhydrous DCM, chloroformate (5 eq) and diisopropylethylamine (5 eq) were added, the mixture was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude products.

**Method K: Coupling of acids with derivatized amines on solid-phase.**

Acid (5 eq) was preactivated with HBTU (5 eq) and diisopropylethylamine (5 eq) in anhydrous DMF for 5 min, and added to the immobilized amine (1 eq). The mixture was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude product.

**Method L: Coupling of sulfonyl chlorides with derivatized amines on solid-phase.**

The immobilized amine (1 eq) was swollen in anhydrous DCM, sulfonyl chloride (5 eq) and DMAP (5 eq) were added, the mixture was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude product.

**Method M: Coupling of isocyanates with derivatized amines on solid-phase.**

The immobilized amine (1 eq) was swollen in anhydrous DCM, isocyanate (5 eq) was added and the mixture

was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude product.

**Method N: Coupling of thio-isocyanates with derivatized amines on solid-phase.**

The immobilized amine (1 eq) was swollen in anhydrous DCM, thio-isocyanate (5 eq) was added and the mixture was shaken 18 h at room temperature. The resin was filtered off and washed successively with DMF, methanol, and dichloromethane and dried. A solution of 50% trifluoroacetic acid in dichloromethane was added to the resin. The mixture was shaken 30 min at room temperature. After filtration, the resin was washed with a solution of 50% trifluoroacetic acid in dichloromethane. After evaporation of the solvent, the residue was dissolved/suspended in water, and lyophilized to give the crude product.

Compounds were either purified by HPLC or used directly for biological testings.

EXAMPLE 2

**2-Amino-3-naphthalen-2-yl-propionic acid cyanomethyl-amide trifluoroacetate**

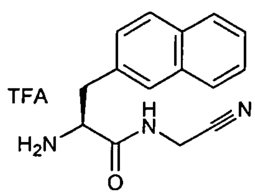

To a solution of 3-aminoacetonitrile hydrogensulfate (1.61 g, 1.25 eq) in anhydrous DMF (36 mL) was added Boc- -Nal-OH (3 g, 1 eq), HBTU (3.62 g, 1 eq) and $Et_3N$ (4 mL, 3.8 eq). The reaction mixture was stirred overnight and then diluted with EtOAc. The organic phase was washed with saturated aqueous solutions of $NaHCO_3$ and NaCl, dried over $MgSO_4$, filtered and evaporated. The residue was purified by flash-chromatography hexane/ethyl acetate 3/2 to give [1-(cyanomethyl-carbamoyl)-2-naphthalen-2-yl-ethyl]-carbamic acid tert-butyl ester. The ester was dissolved in a solution of 50% TFA in DCM (15 mL), stirred at room temperature for 15 min and then evaporated with toluene. The residue was purified by HPLC to give 2-amino-3-naphthalen-2-yl-propionic acid cyanomethyl-amide trifluoroacetate (2.5 g, 70%).

EXAMPLE 3

**[1-(Cyanoethyl-carbamoyl)-2-naphthalen-2-yl-ethyl]-carbamic acid tert-butyl ester**

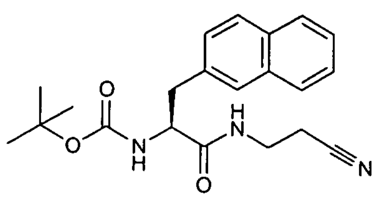

To a solution of 3-aminopropionitrile (146 µL, 1.25 eq) in anhydrous DMF (4.3 mL) was added Boc-β-Nal-OH (500 mg, 1 eq), HBTU (601.3 mg, 1 eq) and $Et_3N$ (331.5 µL, 1.5 eq). The reaction mixture was stirred overnight and then diluted with EtOAc. The organic phase was washed with saturated aqueous solutions of $NaHCO_3$ and NaCl, dried over $MgSO_4$, filtered and evaporated. The residue was purified by flash-chromatography hexane/ethyl acetate 3/2 to give [1-(cyanoethyl-carbamoyl)-2-naphthalen-2-yl-ethyl]-carbamic acid tert-butyl ester (559 mg, 96%).

NMR-$^1$H (DMSO-$d_6$) δ = 1.24 (s, 9 H), 2.61 (m, 2 H), 2.93 (dd, 1 H, *J* = 10.3, *J* = 13.7 Hz, 1 H), 3.13 (dd, 1 H, *J* = 4.3, *J* = 13.7 Hz, 1 H), 3 .3 7 (m, 2 H), 4.24 (m, 1 H), 6.99 (d, *J* = 8.6 Hz, 1 H), 7.43-7.50 (m, 3 H), 7.73-7.87 (m, 3 H), 8.33 (m, 1 H); MS: *m*/*z*: 367.8 [*M*+].

EXAMPLE 4

**3-Naphthalen-2-yl-2-[3-(4-trifluoromethylsulfanyl-phenyl)-ureido]-propionic acid cyanomethyl-amide**

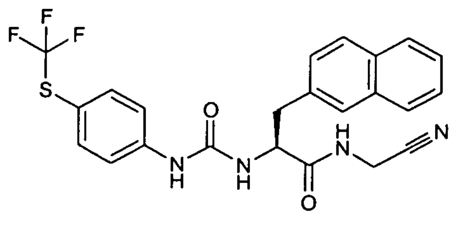

Amine salt (prepared as described in example 1) (10 mg) was dissolved in a mixture of 10% dry DMSO (100 µL) in anhydrous DCM (1 mL), 4-(trifluoromethylthio)-phenyl isocyanate (6.98 µL) and diisopropylethylamine (10 µL) were added under Ar, and stirred for 2 h at room temperature. Tris-(2-aminoethyl)-amine polystyrene (10 mg) and (polystyrylmethyl) trimethylammonium bicarbonate (10 mg) were added to the reaction mixture and agitated for 18 h at room temperature The supernatant was separated from the resin by filtration, the polymeric beads were washed with DCM and a mixture of DCM/MeOH (1/1) (three times). After evaporation of the solvent, the residue was suspended in water, and lyophilized to give 3-naphthalen-2-yl-2-[3-(4-trifluoromethylsulfanyl-phenyl)-ureido]-propionic acid cyanomethyl-amide (13.4 mg, 98%).
NMR-$^1$H (DMSO-$d_6$) δ = 3.06 (dd, 1 H, *J* = 7.7, *J* = 13.7 Hz), 3.19 (dd, 1 H, *J* = 5.8, *J* = 13.7 Hz), 4.18 (m, 2 H), 4.60 (m, 1 H), 7.38 (m, 1 H), 7.43-7.52 (m, 4 H), 7.63 (m, 2 H), 7.70 (s, 1 H), 7.82-7.88 (m, 3 H), 8.90 (m, 1 H); MS: *m/z:* 472.9 [*M*+].

EXAMPLE 5

**3-Naphthalen-2-yl-2-(3-phenyl-thioureido)-propionic acid cyanomethyl-amide**

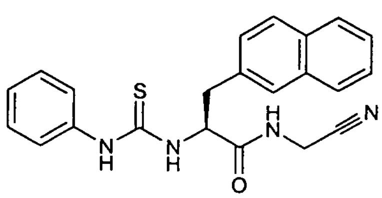

Amine salt (prepared as described in example 1) (10 mg) was dissolved in a mixture of 10% dry DMSO (100 µL) in anhydrous DCM (1 mL), phenyl isothiocyanate (5.20 µL) and diisopropylethylamine (10 µL) were added under Ar, and stirred for 5 h at room temperature. Tris-(2-aminoethyl)-amine polystyrene (10 mg) and (polystyrylmethyl)trimethylammonium bicarbonate (10 mg) were added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was suspended in water, and lyophilized to give 3-naphthalen-2-yl-2-(3-phenyl-thioureido)-propionic acid cyanomethyl-amide (10.5 mg, 93%).
NMR-$^1$H (DMSO-$d_6$) δ= 3.21 (m, 1 H), 3.29 (m, 1 H), 4.18 (m, 2 H), 5.26 (m, 1 H), 7.09 (m, 1 H), 7.26 (m, 2 H), 7.36 (m, 3 H), 7.49 (m, 2 H), 7.69 (s, 1 H), 7.82-7.90 (m, 3 H), 8.92 (m, 1 H), 9.82 (s, 1 H); MS: *m*/*z*: 388.9 [*M*+].

EXAMPLE 6

**3-Naphthalen-2-yl-2-(4-tert-butyl-benzenesulfonylamino)-propionic acid cyanomethyl-amide**

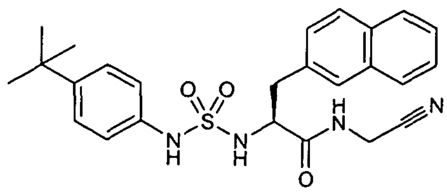

Amine salt (prepared as described in example 1) (10 mg) was dissolved in a mixture of 10% dry DMSO (100 μL) in anhydrous DCM (1 mL), 4-tert-butyl-benzene-sulfonyl chloride (10.1 mg) and diisopropylethylamine (20 μL) were added under Ar, and stirred 2 h at room temperature. Tris-(2-aminoethyl)-amine polystyrene (10 mg) and (polystyrylmethyl)trimethylammonium bicarbonate (10 mg) were added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was suspended in water, and lyophilized to give 3-naphthalen-2-yl-2-(4-tert-butyl-benzenesulfonylamino)-propionic acid cyanomethyl-amide (11.8 mg, 91%).
NMR-$^1$H (DMSO-$d_6$) δ= 1.19 (s, 9 H), 3.14 (m, 1 H), 3.62 (m, 3 H), 3.99 (m, 2 H), 7.13 (m, 1 H), 7.18-7.38 (m, 5 H), 7.46-7.52 (m, 3 H), 7.61 (m, 1 H), 7.71-7.88 (m, 3 H), 8.28 (m, 1 H), 8.78 (m, 1 H); MS: *m*/*z*: 450.2 [M$^+$].

EXAMPLE 7

**1-Methyl-cyclopropanecarboxylic acid [1-(cyanomethyl-carbamoyl)-2-naphthalen-2-yl-ethyl]-amide**

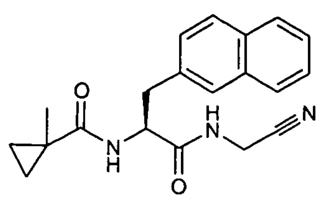

To a solution of PyBrOP (148.5 mg), 1-methylcyclopropane carboxylic acid (33.4 mg) and diisopropylethylamine (152 μL) in anhydrous DMF (1.1 mL) was added 1-hydroxybenzotriazole-6-sulfonamidomethyl polystyrene (113.8 mg). The mixture was reacted at room temperature for 5 h. After the first activation step the resin was washed with DMF (three times). The second activation step was performed under the same conditions as the first one, and the resin was washed with DMF (five times).
The amine salt (prepared as described in example 1) (30 mg) was added to a suspension of the resin in anhydrous DCM (1 mL) and diisopropylethylamine (152 μL). The polymer-bound activated ester was reacted with this mixture at room temperature. After 20 h, the supernatant was separated from the resin by filtration. The polymeric beads were washed with DCM and a mixture of DCM/MeOH (1/1) (three times). After evaporation of the solvent, the residue was suspended in water, and lyophilized to give 1-methyl-cyclopropanecarboxylic acid [1-(cyanomethyl-carbamoyl)-2-naphthlen-2-yl-ethyl]-amide (38.9 mg, 98%).
NMR-$^1$H (DMSO-$d_6$) δ= 0.42 (m, 2 H), 0.79 (m, 2 H), 1.20 (s, 3 H), 3.10 (dd, 1 H, *J* = 9.8, *J* = 13.6 Hz), 3.20 (dd, 1 H, *J* = 4.9, *J* = 13.6 Hz, 1 H), 4.16 (m, 2 H), 4.57 (m, 1 H), 7.40-7.56 (m, 3 H), 7.73 (s, 1 H), 7.73-7.88 (m, 3 H), 8.66 (m, 1 H); MS: *m*/*z*: 336.0 [*M*$^+$].

EXAMPLE 8

**4-Chloromethyl-N-[1-(cyanomethyl-carbamoyl)-2-naphthalen-2-yl-ethyl]-benzamide**

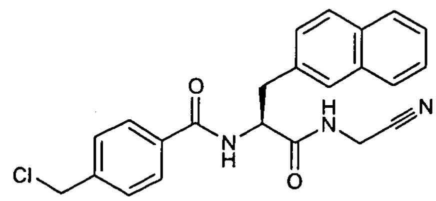

Amine salt (prepared as described in example 1) (30 mg) was dissolved in a mixture of 10% dry DMSO (100 μL) in anhydrous DCM (1 mL), 4-(chloromethyl)benzoyl chloride (27 mg) and diisopropylethylamine (103 μL) were added under Ar, and stirred for 2 h at room temperature. Tris-(2-aminoethyl)-amine polystyrene (40 mg) and (polystyrylmethyl)-trimethylammonium bicarbonate (40 mg) were added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was suspended in water, and lyophilized to give 4-chloromethyl-*N*-[1-(cyanomethyl-carbamoyl)-2-naphthalen-2-yl-ethyl]-benzamide (30.3 mg, 86%).
NMR-$^1$H (DMSO-$d_6$) δ= 3.18 (m, 1 H), 3.30 (m, 1 H), 4.18 (m, 2 H), 4.78 (s, 2 H), 4.83 (m, 1 H), 7.40-7.62 (m, 5 H), 7.78-7.85 (m, 6 H), 8.82 (m, 1 H); MS: *m*/*z*: 405.9 [$M^+$].

EXAMPLE 9

**N-[1-(Cyanomethyl-carbamoyl)-2-naphthalen-2-yl-ethyl]-oxalamic acid methyl ester**

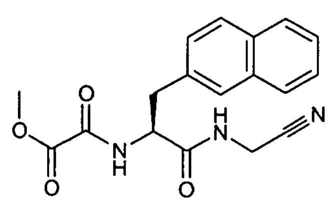

Amine salt (prepared as described in example 1) (30 mg) was dissolved in a mixture of 10% dry DMSO (100 μL) in anhydrous DCM (1 mL), mono-methyl oxalyl chloride (14 μL) and diisopropylethylamine (103 μL) were added under Ar, and stirred for 2 h at room temperature. Tris-(2-aminoethyl)-amine polystyrene (40 mg) and (polystyrylmethyl) trimethylammonium bicarbonate (40 mg) were added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was suspended in water, and lyophilized to give *N*-[1-(cyanomethyl-carbamoyl)-2-naphthalen-2-yl-ethyl]-oxalamic acid methyl ester (25.1 mg, 85%).
NMR-$^1$H (DMSO-$d_6$) δ = 3.14 (dd, 1 H, *J* = 9.6, *J* = 14.0 Hz, 1 H), 3.29 (dd, 1 H, *J* = 4.9, *J* = 14.0 Hz, 1 H), 3.72 (s, 3 H), 4.17 (m, 2 H), 4.63 (m, 1 H), 7.13 (m, 1 H), 7.40-7.49 (m, 3 H), 7.73-7.87 (m, 4 H), 8.84 (m, 1 H), 9.14 (m, 1 H); MS: *m*/*z*: 340.0 [$M^+$] .

EXAMPLE 10

**N-[1-(Cyanomethyl-carbamoyl)-2-naphthalen-2-yl-ethyl]-phthalamic acid**

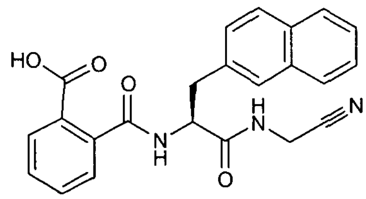

Amine salt (prepared as described in example 1) (30 mg) was dissolved in a mixture of 10% dry DMSO (100 µL) in anhydrous DCM (1 mL), phthalic anhydride (21 mg) and diisopropylethylamine (103 µL) were added under Ar, and stirred for 2 h at room temperature. Tris-(2-aminoethyl)-amine polystyrene (40 mg) and (polystyrylmethyl)-trimethylammonium bicarbonate (40 mg) were added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was suspended in water, and lyophilized to give *N*-[1-(cyanomethyl-carbamoyl)-2-naphthalen-2-yl-ethyl]-phthalamic acid (28.6 mg, 82%).
NMR-$^1$H (DMSO-$d_6$) δ= 3.09 (dd, 1 H, *J* = 9.8, *J* = 14.0 Hz, 1 H), 3.35 (dd, 1 H, *J* = 5.1, *J* = 14.0 Hz, 1 H), 4.19 (m, 2 H), 4.75 (m, 1 H), 7.13 (m, 1 H), 7.45-7.51 (m, 5 H), 7.72-7.87 (m, 5 H), 8.55 (m, 1 H), 8.79 (d, *J* = 7.7 Hz, 1 H), 13.12 (bs, 1 H).; MS: *m*/*z*: 401.9 [$M^+$].

EXAMPLE 11

**[1-(Cyanomethyl-carbamoyl)-2-naphthalen-2-yl-ethyl]-carbamic acid ethyl ester**

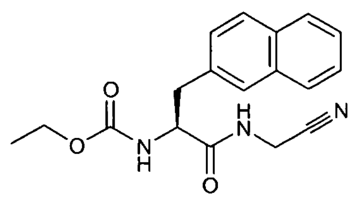

Amine salt (prepared as described in example 1) (30 mg) was dissolved in a mixture of 10% dry DMSO (100 µL) in anhydrous DCM (1 mL), ethyl chloroformate (14 µL) and diisopropylethylamine (103 µL) were added under Ar, and stirred for 2 h at room temperature. Tris-(2-aminoethyl)-amine polystyrene (40 mg) was added to the reaction mixture and agitated for 18 h at room temperature. The supernatant was separated from the resin by filtration, the polymeric beads were washed with DCM and a mixture of DCM/MeOH (1/1) (three times). After evaporation of the solvent, the residue was suspended in water, and lyophilized to give *N*-[1-(cyanomethyl-carbamoyl)-2-naphthalen-2-yl-ethyl]-phthalamic acid (25.5 mg, 90%).
NMR-$^1$H (DMSO-$d_6$) δ= 1.04 (dd, 3 *J* = 7.5, *J* = 6.6 Hz H), 2.93 (dd, 1 H, *J* = 10.3, *J* = 13.7 Hz, 1 H), 3.14 (dd, 1 H, *J* = 4.5, *J* = 13.7 Hz, 1 H), 3.86 (m, 3 H), 4.15 (m, 2 H), 4.30 (m, 1 H), 7.43-7.51 (m, 3 H), 7.75-7.88 (m, 4 H), 8.76 (m, 1 H); MS: *m*/*z*: 326.0 [$M^+$].

The following additional compounds of Formula I that can be prepared by the methods described in this application.

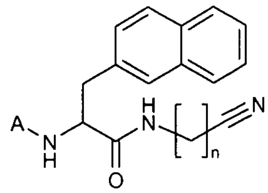

Column A lists the different A substituents:

| A | Name<br>$^1$H-NMR (DMSO-$d_6$, ppm) | Methods | MS Data |
|---|---|---|---|
| 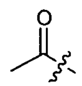 | **n** = 1; 3-Naphthalen-2-yl-2-acetylamino-propionic acid cyanomethyl-amide | A, B, H or I | *m/z*: 295.9 [$M^+$] |
| | **n** = 2; 3-Naphthalen-2-yl-2-acetylamino-propionic acid cyanoethyl-amide | A, B, H or I | *m/z*: 309.9 [$M^+$] |
| 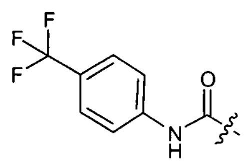 | **n** = **1**; 3-Naphthalen-2-yl-2-[3-(4-trifluoromethyl-phenyl)-ureido]-propionic acid cyanomethyl-amide | F or M | *m/z*: 440.9 [$M^+$] |
| | **n** = **2**; 3-Naphthalen-2-yl-2-[3-(4-trifluoromethyl-phenyl)-ureido]-propionic acid cyanoethyl-amide | F or M | *m/z*: 454.9 [$M^+$] |
| 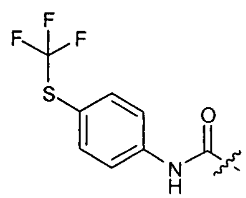 | **n** = **1**; 3-Naphthalen-2-yl-2-[3-(4-trifluoromethylsulfanyl-phenyl)-ureido]-propionic acid cyanomethyl-amide<br>$\delta$ = 3.06 (dd, 1 H, $J$ = 7.7, $J$ = 13.7 Hz), 3.19 (dd, 1 H, $J$ = 5.8, $J$ = 13.7 Hz), 4.18 (m, 2 H), 4.60 (m, 1 H), 7.38 (m, 1 H), 7.43-7.52 (m, 4 H), 7.63 (m, 2 H), 7.70 (s, 1 H), 7.82-7.88 (m, 3 H), 8.90 (m, 1 H). | F or M | *m/z*: 472.9 [$M^+$] |
| | **n** = **2**; 3-Naphthalen-2-yl-2-[3-(4-trifluoromethylsulfanyl-phenyl)-ureido]-propionic acid cyanoethyl-amide | F or M | *m/z*: 486.9 [$M^+$] |

| | | | |
|---|---|---|---|
| 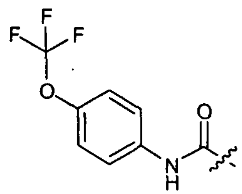 | **n** = **1**; 3-Naphthalen-2-yl-2-[3-(4-trifluoromethoxy-phenyl)-ureido]-propionic acid cyanomethyl-amide<br>$\delta$ = 3.08 (dd, 1 H, $J$ = 7.8, $J$ = 13.5 Hz), 3.16 (dd, 1 H, $J$ = 5.5, $J$ = 13.5 Hz), 4.17 (m, 2 H), 4.59 (m, 1 H), 6.48 (m, 1 H), 7.19 (m, 2 H), 7.28 (m, 2 H), 7.47-7.57 (m, 3 H), 7.70 (s, 1 H), 7.82-7.88 (m, 3 H), 8.87 (m, 1 H). | F or M | *m/z*: 457.0 [$M^+$] |
| | **n** = **2**; 3-Naphthalen-2-yl-2-[3-(4-trifluoromethoxy-phenyl)-ureido]-propionic acid cyanoethyl-amide | F or M | *m/z*: 471.0 [$M^+$] |
| 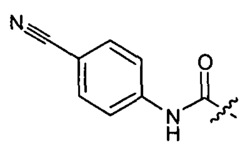 | **n** = **1**; 3-Naphthalen-2-yl-2-[3-(4-cyano-phenyl)-ureido]-propionic acid cyanomethyl-amide | F or M | *m/z*: 398.0 [$M^+$] |
| | **n** = **2**; 3-Naphthalen-2-yl-2-[3-(4-cyano-phenyl)-ureido]-propionic acid cyanoethyl-amide | F or M | *m/z*: 412.0 [$M^+$] |
| 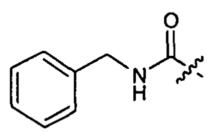 | **n** = **1**; 3-Naphthalen-2-yl-2-(3-benzyl-ureido)-propionic acid cyanomethyl-amide | F or M | *m/z*: 387.0 [$M^+$] |
| | **n** = **2**; 3-Naphthalen-2-yl-2-(3-benzyl-ureido)-propionic acid cyanoethyl-amide | F or M | *m/z*: 401.0 [$M^+$] |
| | **n** = **1**; 3-Naphthalen-2-yl-2-(3-o-tolyl-ureido)-propionic acid cyanomethyl-amide | F or M | *m/z*: 387.0 [$M^+$] |

| | | | |
|---|---|---|---|
| 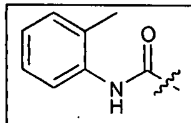 | **n** = **2**; 3-Naphthalen-2-yl-2-(3-o-tolyl-ureido)-propionic acid cyanoethyl-amide | F or M | *m/z*: 401.0 [$M^+$] |
| 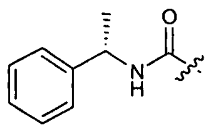 | **n** = **1**; 3-Naphthalen-2-yl-2-[3-(*S*)-(1-phenyl-ethyl)-ureido]-propionic acid cyanomethyl-amide | F or M | *m/z*: 400.9 [$M^+$] |
| | **n** = **2**; 3-Naphthalen-2-yl-2-[3-(*S*)-(1-phenyl-ethyl)-ureido]-propionic acid cyanoethyl-amide | F or M | *m/z*: 414.9 [$M^+$] |
| 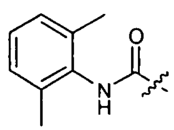 | **n** = **1**; 3-Naphthalen-2-yl-2-[3-(2,6-dimethyl-phenyl)-ureido]-propionic acid cyanomethyl-amide. $\delta$ = 2,00, 2,01 (2 s , 6H), 3.00 (dd, 1 H, *J* = 8.2, *J* = 13.3Hz), 3.17 (dd, 1 H, *J* = 5.0, *J* = 13.3 Hz, 1 H), 4.18 (m, 2 H), 4.60 (m, 1 H), 6,97 (s, 3 H), 7.37 (m, 1 H), 7.49 (m, 2 H), 7.63 (s, 1 H), 7.79-7.88 (m, 3 H), 8.84 (m, 1 H). | F or M | *m/z*: 401.0 [$M^+$] |
| | **n** = **2**; 3-Naphthalen-2-yl-2-[3-(2,6-dimethyl-phenyl)-ureido]-propionic acid cyanoethyl-amide | F or M | *m/z*: 414.9 [$M^+$] |
| 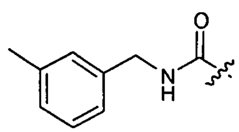 | **n** = **1**; 3-Naphthalen-2-yl-2-[3-(3-methyl-benzyl)-ureido]-propionic acid cyanomethyl-amide | F or M | *m/z*: 401.0 [$M^+$] |
| | **n** = **2**; 3-Naphthalen-2-yl-2-[3-(3-methyl-benzyl)-ureido]-propionic acid cyanoethyl-amide | F or M | *m/z*: 414.9 [$M^+$] |

| | | | |
|---|---|---|---|
| 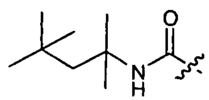 | **n** = 1; 3-Naphthalen-2-yl-2-[3-(1,1,3,3-tetramethyl-butyl)-ureido]-propionic acid cyanomethyl-amide | F or M | *m/z*: 408.9 [$M^+$] |
| | **n** = 2; 3-Naphthalen-2-yl-2-[3-(1,1,3,3-tetramethyl-butyl)-ureido]-propionic acid cyanoethyl-amide | F or M | *m/z*: 422.9 [$M^+$] |
| 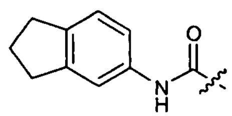 | **n** = 1; 3-Naphthalen-2-yl-2-(3-indan-5-yl-ureido)-propionic acid cyanomethyl-amide | F or M | *m/z*: 413.0 [$M^+$] |
| | **n** = 2; 3-Naphthalen-2-yl-2-(3-indan-5-yl-ureido)-propionic acid cyanoethyl-amide | F or M | *m/z*: 427.0 [$M^+$] |
| 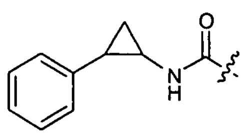 | **n** = 1; 3-Naphthalen-2-yl-2-[3-(2-phenyl-cyclopropyl)-ureido]-propionic acid cyanomethyl-amide | F or M | *m/z*: 413.1 [$M^+$] |
| | **n** = 2; 3-Naphthalen-2-yl-2-[3-(2-phenyl-cyclopropyl)-ureido]-propionic acid cyanoethyl-amide | E or K | *m/z*: 427.1 [$M^+$] |
| 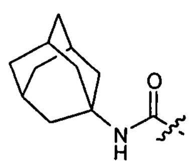 | **n** = 1; 3-Naphthalen-2-yl-2-(3-adamantan-1-yl-ureido)-propionic acid cyanomethyl-amide | F or M | *m/z*: 431.0 [$M^+$] |
| | **n** = 2; 3-Naphthalen-2-yl-2-(3-adamantan-1-yl-ureido)-propionic acid cyanoethyl-amide | F or M | *m/z*: 445.0 [$M^+$] |
| | **n** = 1; 3-Naphthalen-2-yl-2-(3-biphenyl-4-yl-ureido)-propionic acid cyanomethyl-amide | F or M | *m/z*: 449.1 [$M^+$] |

| | | | |
|---|---|---|---|
| 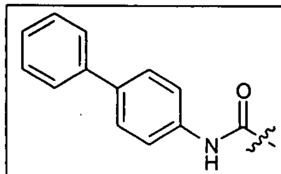 | **n** = **2**; 3-Naphthalen-2-yl-2-(3-biphenyl-4-yl-ureido)-propionic acid cyanoethyl-amide | F or M | *m/z*: 463.1 [$M^+$] |
| 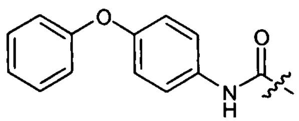 | **n** = 1; 3-Naphthalen-2-yl-2-[3-(4-phenoxy-phenyl)-ureido]-propionic acid cyanomethyl-amide | F or M | *m/z*: 465.0 [$M^+$] |
| | **n** = 2; 3-Naphthalen-2-yl-2-[3-(4-phenoxy-phenyl)-ureido]-propionic acid cyanoethyl-amide | F or M | *m/z*: 479.0 [$M^+$] |
| 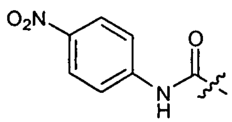 | **n** = 1; 3-Naphthalen-2-yl-2-[3-(4-nitro-phenyl)-ureido]-propionic acid cyanomethyl-amide | F or M | *m/z*: 418.0 [$M^+$] |
| | **n** = 2; 3-Naphthalen-2-yl-2-[3-(4-nitro-phenyl)-ureido]-propionic acid cyanoethyl-amide | F or M | *m/z*: 432.0 [$M^+$] |
| 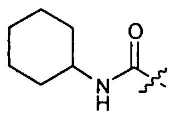 | **n** = 1; 3-Naphthalen-2-yl-2-(3-cyclohexyl-ureido)-propionic acid cyanomethyl-amide | F or M | *m/z*: 378.9 [$M^+$] |
| | **n** = 2; 3-Naphthalen-2-yl-2-(3-cyclohexyl-ureido)-propionic acid cyanoethyl-amide | F or M | *m/z*: 392.9 [$M^+$] |

| | | | |
|---|---|---|---|
| 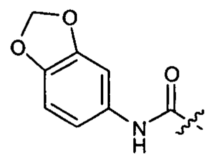 | **n = 1**; 3-Naphthalen-2-yl-2-(3-benzo[1,3]dioxol-5-yl-ureido)-propionic acid cyanomethyl-amide<br>$\delta$ = 3.03 (dd, 1 H, $J$ = 7.5, $J$ = 13.9 Hz, 1 H), 3.16 (dd, 1 H, $J$ = 5.5, $J$ = 13.9 Hz, 1 H), 4.16 (m, 2 H), 4.58 (m, 1 H), 5.91 (s, 2 H), 6.34 (d, 1 H, $J$ = 8.4 Hz, 1 H), 6.60 (dd, 1 H, $J$ = 2.0, $J$ = 8.4 Hz, 1 H), 6.74 (d, 1 H, $J$ = 8.4 Hz, 1 H), 7.09 (d, 1 H, $J$ = 2.0 Hz, 1 H), 7.37 (m, 1 H), 7.36-7.48 (m, 2 H), 7.70 (s, 1 H), 7.81-7.88 (m, 3 H), 8.55 (s, 1 H), 8.84 (m, 1 H). | F or M | *m/z*: 417.0 [$M^+$] |
| | **n = 2**; 3-Naphthalen-2-yl-2-(3-benzo[1,3]dioxol-5-yl-ureido)-propionic acid cyanoethyl-amide | F or M | *m/z*: 431.0 [$M^+$] |
| 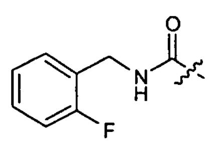 | **n = 1**; 3-Naphthalen-2-yl-2-[3-(2-fluoro-benzyl)-ureido]-propionic acid cyanomethyl-amide | F or M | *m/z*: 405.0 [$M^+$] |
| | **n = 2**; 3-Naphthalen-2-yl-2-[3-(2-fluoro-benzyl)-ureido]-propionic acid cyanoethyl-amide | F or M | *m/z*: 419.0 [$M^+$] |
| 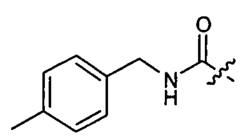 | **n = 1**; 3-Naphthalen-2-yl-2-[3-(4-methyl-benzyl)-ureido]-propionic acid cyanomethyl-amide | F or M | *m/z*: 401.0 [$M^+$] |
| | **n = 2**; 3-Naphthalen-2-yl-2-[3-(4-methyl-benzyl)-ureido]-propionic acid cyanoethyl-amide | F or M | *m/z*: 415.0 [$M^+$] |

| | | | |
|---|---|---|---|
| 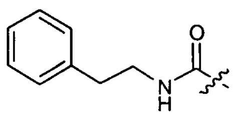 | **n** = **1**; 3-Naphthalen-2-yl-2-(3-phenethyl-ureido) -ureido]-propionic acid cyanomethyl-amide<br>$\delta$ = 2.58 (dd, 2 H, $J$ = 7.2, $J$ = 7.4 Hz), 2.93 (dd, 1 H, $J$ = 8.5, $J$ = 13.7 Hz), 3.07 (m, 1 H), 3.14 (m, 2 H), 4.13 (m, 1 H), 4.49 (m, 1 H), 6.07 (m, 1 H), 6.22 (m, 1 H), 7.11-7.26 (m, 5 H), 7.35 (m, 1 H), 7.47 (m, 2 H), 7.67 (s, 1 H), 7.80-7.88 (m, 3 H), 8.73 (m, 1 H). | F or M | *m/z*: 401.0 [$M^+$] |
| | **n** = **2**; 3-Naphthalen-2-yl-2-(3-phenethyl-ureido) -ureido]-propionic acid cyanoethyl-amide | F or M | *m/z*: 415.0 [$M^+$] |
| 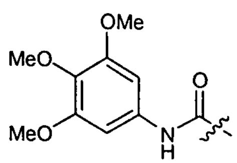 | **n** = **1**; 3-Naphthalen-2-yl-2-[3-(3,4,5-trimethoxy-phenyl)-ureido]-propionic acid cyanomethyl-amide | F or M | *m/z*: 463.0 [$M^+$] |
| | **n** = **2**; 3-Naphthalen-2-yl-2-[3-(3,4,5-trimethoxy-phenyl)-ureido]-propionic acid cyanoethyl-amide | F or M | *m/z*: 477.0 [$M^+$] |
| 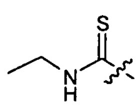 | **n** = **1**; 3-Naphthalen-2-yl-2-(3-ethyl-thioureido)-propionic acid cyanomethyl-amide | G or N | *m/z*: 340.9 [$M^+$] |
| | **n** = **2**; 3-Naphthalen-2-yl-2-(3-ethyl-thioureido)-propionic acid cyanoethyl-amide | G or N | *m/z*: 354.9 [$M^+$] |
| | **n** = **1**; 3-Naphthalen-2-yl-2-(3-isopropyl-thioureido)-propionic acid cyanomethyl-amide | G or N | *m/z*: 355.0 [$M^+$] |

| | | | |
|---|---|---|---|
| 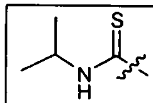 | **n** = **2**; 3-Naphthalen-2-yl-2-(3-isopropyl-thioureido)-propionic acid cyanoethyl-amide | G or N | *m/z*: 369.0 [$M^+$] |
| 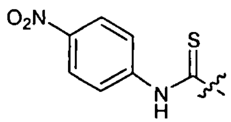 | **n** = **1**; 3-Naphthalen-2-yl-2-[3-(4-nitro-phenyl)-thioureido]-propionic acid cyanomethyl-amide | G or N | *m/z*: 433.9 [$M^+$] |
| | **n** = **2**; 3-Naphthalen-2-yl-2-[3-(4-nitro-phenyl)-thioureido]-propionic acid cyanoethyl-amide | G or N | *m/z*: 447.9 [$M^+$] |
| 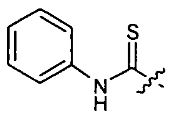 | **n** = **1**; 3-Naphthalen-2-yl-2-(3-phenyl-thioureido)-propionic acid cyanomethyl-amide<br>$\delta$ = 3.21 (m, 1 H), 3.29 (m, 1 H), 4.18 (m, 2 H), 5.26 (m, 1 H), 7.09 (m, 1 H), 7.26 (m, 2 H), 7.36 (m, 3 H), 7.49 (m, 2 H), 7.69 (s, 1 H), 7.82-7.90 (m, 3 H), 8.92 (m, 1 H), 9.82 (s, 1 H). | G or N | *m/z*: 388.9 [$M^+$] |
| | **n** = **2**; 3-Naphthalen-2-yl-2-(3-phenyl-thioureido)-propionic acid cyanoethyl-amide | G or N | *m/z*: 402.9 [$M^+$] |
| 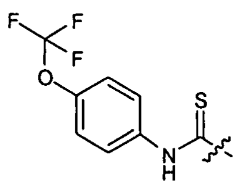 | **n** = **1**; 3-Naphthalen-2-yl-2-[3-(4-trifluoromethoxy-phenyl)-thioureido]-propionic acid cyanomethyl-amide<br>$\delta$ = 3.20 (m, 1 H), 3.29 (m, 1 H), 4.18 (m, 2 H), 5.25 (m, 1 H), 7.24 (m, 2 H), 7.38 (m, 1 H), 7.44-7.54 (m, 4 H), 7.70 (s, 1 H), 7.82-7.90 (m, 3 H), 8.93 (m, 1 H), 9.92 (s, 1 H). | G or N | *m/z*: 472.9 [$M^+$] |

| | | | |
|---|---|---|---|
| | **n** = **2**; 3-Naphthalen-2-yl-2-[3-(4-trifluoromethoxy-phenyl)-thioureido]-propionic acid cyanoethyl-amide | G or N | *m/z*: 486.9 [$M^+$] |
| 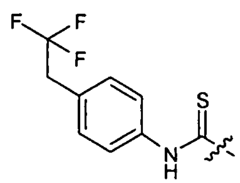 | **n** = **1**; 3-Naphthalen-2-yl-2-{3-[4-(2,2,2-trifluoro-ethyl)-phenyl]-thioureido}-propionic acid cyanomethyl-amide | G or N | *m/z*: 456.9 [$M^+$] |
| | **n** = **2**; 3-Naphthalen-2-yl-2-{3-[4-(2,2,2-trifluoro-ethyl)-phenyl]-thioureido}-propionic acid cyanoethyl-amide | G or N | *m/z*: 470.9 [$M^+$] |
| 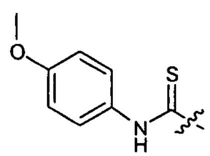 | **n** = **1**; 3-Naphthalen-2-yl-2-[3-(4-methoxy-phenyl)-thioureido]-propionic acid cyanomethyl-amide<br>$\delta$ = 3.21 (m, 1 H), 3.29 (m, 1 H), 3.72 (s, 3 H), 4.17 (m, 2 H), 5.25 (m, 1 H), 6.81 (m, 1 H), 7.16 (m, 2 H), 7.34 (m, 1 H), 7.49 (m, 2 H), 7.66 (s, 1 H), 7.82-7.90 (m, 3 H), 8.86 (m, 1 H), 9.64 (s, 1 H). | G or N | *m/z*: 419.0 [$M^+$] |
| | **n** = **2**; 3-Naphthalen-2-yl-2-[3-(4-methoxy-phenyl)-thioureido]-propionic acid cyanoethyl-amide | G or N | *m/z*: 433.0 [$M^+$] |
| | **n** = **1**; 3-Naphthalen-2-yl-2-(7,7-dimethyl-2-oxo-bicyclo[2.2.1]hept-1-ylmethanesulfonylamino)-propionic acid cyanomethyl-amide | E or L | *m/z*: 468.2 [$M^+$] |

| | | | |
|---|---|---|---|
| 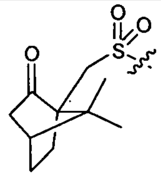 | **n** = **2**; 3-Naphthalen-2-yl-2-(7,7-dimethyl-2-oxo-bicyclo[2.2.1]hept-1-ylmethanesulfonylamino)-propionic acid cyanoethyl-amide | E or L | *m/z*: 482.2 [$M^+$] |
| 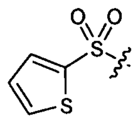 | **n** = **1**; 3-Naphthalen-2-yl-2-(thiophene-2-sulfonylamino)-propionic acid cyanomethyl-amide | E or L | *m/z*: 400.0 [$M^+$] |
| | **n** = **2**; 3-Naphthalen-2-yl-2-(thiophene-2-sulfonylamino)-propionic acid cyanoethyl-amide | E or L | *m/z*: 414.0 [$M^+$] |
| 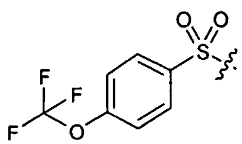 | **n** = **1**; 3-Naphthalen-2-yl-2-(4-trifluoromethoxy-benzenesulfonylamino)-propionic acid cyanomethyl-amide | E or L | *m/z*: 478.1 [$M^+$] |
| | **n** = **2**; 3-Naphthalen-2-yl-2-(4-trifluoromethoxy-benzenesulfonylamino)-propionic acid cyanoethyl-amide | E or L | *m/z*: 492.1 [$M^+$] |
| 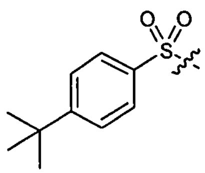 | **n** = **1**; 3-Naphthalen-2-yl-2-(4-*tert*-butyl-benzenesulfonylamino)-propionic acid cyanomethyl-amide<br>$\delta$ = 1.19 (s, 9 H), 3.14 (m, 1 H), 3.62 (m, 3 H), 3.99 (m, 2 H), 7.13 (m, 1 H), 7.18-7.38 (m, 5 H), 7.46-7.52 (m, 3 H), 7.61 (m, 1 H), 7.71-7.88 (m, 3 H), 8.28 (m, 1 H), 8.78 (m, 1 H). | E or L | *m/z*: 450.2 [$M^+$] |
| | **n** = **2**; 3-Naphthalen-2-yl-2-(4-*tert*-butyl-benzenesulfonylamino)-propionic acid cyanoethyl-amide | E or L | *m/z*: 464.2 [$M^+$] |

| | | | |
|---|---|---|---|
| 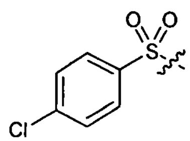 | **n** = **1**; 3-Naphthalen-2-yl-2-(4-chloro-benzenesulfonylamino)-propionic acid cyanomethyl-amide | E or L | *m/z*: 428.1 [$M^+$] |
| | **n** = **2**; 3-Naphthalen-2-yl-2-(4-chloro-benzenesulfonylamino)-propionic acid cyanoethyl-amide | E or L | *m/z*: 442.1 [$M^+$] |
| 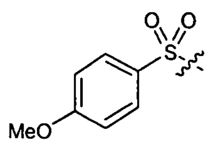 | **n** = **1**; 3-Naphthalen-2-yl-2-(4-methoxy-benzenesulfonylamino)-propionic acid cyanomethyl-amide | E or L | *m/z*: 424.1 [$M^+$] |
| | **n** = **2**; 3-Naphthalen-2-yl-2-(4-methoxy-benzenesulfonylamino)-propionic acid cyanoethyl-amide | E or L | *m/z*: 438.1 [$M^+$] |
| 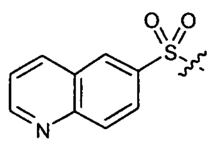 | **n** = **1**; 3-Naphthalen-2-yl-2-(quinoline-6-sulfonylamino)-propionic acid cyanomethyl-amide | E or L | *m/z*: 445.2 [$M^+$] |
| | **n** = **2**; 3-Naphthalen-2-yl-2-(quinoline-6-sulfonylamino)-propionic acid cyanoethyl-amide | E or L | *m/z*: 459.2 [$M^+$] |
| 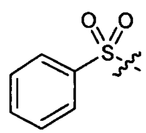 | **n** = **1**; 3-Naphthalen-2-yl-2-benzenesulfonylamino-propionic acid cyanomethyl-amide $\delta$ = 2.80 (dd, 1 H, $J$ = 9.2, $J$ = 13.5Hz), 3.02 (dd, 1 H, $J$ = 5.7, $J$ = 13.5 Hz, 1 H), 3.97 (m, 2 H), 4.03 (m, 1 H), 7.23 (m, 3 H), 7.37 (m, 1 H), 7.45-7.51 (m, 4 H), 7.58 (s, 1 H), 7.70-7.85 (m, 3 H), 8.34 (m, 1 H). | E or L | *m/z*: 394.1 [$M^+$] |

| | | | |
|---|---|---|---|
| | **n** = **2**; 3-Naphthalen-2-yl-2-benzenesulfonylamino-propionic acid cyanoethyl-amide | E or L | *m/z*: 408.1 [$M^+$] |
| 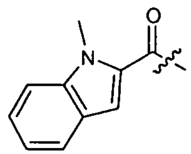 | **n** = **1**; 1-Methyl-1H-indole-2-carboxylic acid [1-(cyanomethyl-carbamoyl)-2-naphthalen-2-yl-ethyl]-amide | D or K | *m/z*: 411.1 [$M^+$] |
| | **n** = **2**; 1-Methyl-1H-indole-2-carboxylic acid [1-(cyanoethyl-carbamoyl)-2-naphthalen-2-yl-ethyl]-amide | D or K | *m/z*: 425.0 [$M^+$] |
| 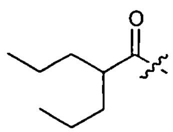 | **n** = **1**; 2-Propyl-pentanoic acid [1-(cyanomethyl-carbamoyl)-2-naphthalen-2-yl-ethyl]-amide | D or K | *m/z*: 380.1 [$M^+$] |
| | **n** = **2**; 2-Propyl-pentanoic acid [1-(cyanoethyl-carbamoyl)-2-naphthalen-2-yl-ethyl]-amide | D or K | *m/z*: 394.0 [$M^+$] |
| 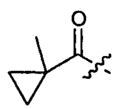 | **n** = **1**; 1-Methyl-cyclopropanecarboxylic acid [1-(cyanomethyl-carbamoyl)-2-naphthalen-2-yl-ethyl]-amide $\delta$ = 0.42 (m, 2 H), 0.79 (m, 2 H), 1.20 (s, 3 H), 3.10 (dd, 1 H, $J$ = 9.8, $J$ = 13.6 Hz), 3.20 (dd, 1 H, $J$ = 4.9, $J$ = 13.6 Hz, 1 H), 4.16 (m, 2 H), 4.57 (m, 1 H), 7.40-7.56 (m, 3 H), 7.73 (s, 1 H), 7.73-7.88 (m, 3 H), 8.66 (m, 1 H). | D or K | *m/z*: 336.0 [$M^+$] |
| | **n** = **2**; 1-Methyl-cyclopropanecarboxylic acid [1-(cyanoethyl-carbamoyl)-2-naphthalen-2-yl-ethyl]-amide | D or K | *m/z*: 350.0 [$M^+$] |

| | | | |
|---|---|---|---|
| 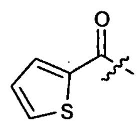 | **n** = **1**; Thiophene-2-carboxylic acid [1-(cyanomethyl-carbamoyl)-2-naphthalen-2-yl-ethyl]-amide | D or K | *m/z*: 363.9 [$M^+$] |
| | **n** = **2**; Thiophene-2-carboxylic acid [1-(cyanoethyl-carbamoyl)-2-naphthalen-2-yl-ethyl]-amide | D or K | *m/z*: 377.9 [$M^+$] |
| 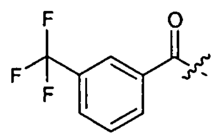 | **n** = **1**; *N*-[1-(Cyanomethyl-carbamoyl)-2-naphthalen-2-yl-ethyl]-3-trifluoromethyl-benzamide | D or K | *m/z*: 425.9 [$M^+$] |
| | **n** = **2**; *N*-[1-(Cyanoethyl-carbamoyl)-2-naphthalen-2-yl-ethyl]-3-trifluoromethyl-benzamide | D or K | *m/z*: 439.9 [$M^+$] |
| 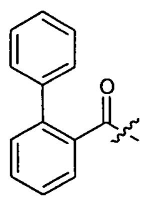 | **n** = **1**; Biphenyl-2-carboxylic acid [1-(cyanomethyl-carbamoyl)-2-naphthalen-2-yl-ethyl]-amide | D or K | *m/z*: 434.0 [$M^+$] |
| | **n** = **2**; Biphenyl-2-carboxylic acid [1-(cyanoethyl-carbamoyl)-2-naphthalen-2-yl-ethyl]-amide | D or K | *m/z*: 448.0 [$M^+$] |
| 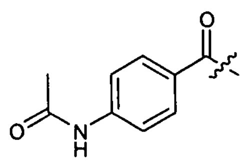 | **n** = **1**; 4-Acetylamino-*N*-[1-(cyanomethyl-carbamoyl)-2-naphthalen-2-yl-ethyl]-benzamide | D or K | *m/z*: 415.0 [$M^+$] |
| | **n** = **2**; 4-Acetylamino-*N*-[1-(cyanoethyl-carbamoyl)-2-naphthalen-2-yl-ethyl]-benzamide | D or K | *m/z*: 429.0 [$M^+$] |

| | | | |
|---|---|---|---|
| 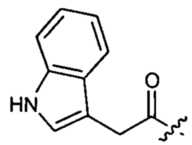 | **n** = 1; 3-Naphthalen-2-yl-2-(2-1*H*-indol-3-yl-acetylamino)-propionic acid cyanomethyl-amide<br>$\delta$ = 2.99 (dd, 1 H, $J$ = 8.9, $J$ = 13.5 Hz), 3.15 (dd, 1 H, $J$ = 5.1, $J$ = 13.5 Hz), 3.51 (s, 2 H), 4.14 (m, 2 H), 4.59 (m, 1 H), 6.69 (m, 1 H), 6.98 (m, 1 H), 7.07 (s, 1 H), 7.25 (m, 2 H), 7.35 (m, 1 H), 7.43-7.48 (m, 2 H), 7.68 (s, 1 H), 7.75 (m, 2 H), 7.85 (m, 1 H), 8.29 (m, 1 H), 8.79 (m, 1 H). | D or K | *m/z*: 411.1 [$M^+$] |
| | **n** = 2; 3-Naphthalen-2-yl-2-(2-1*H*-indol-3-yl-acetylamino)-propionic acid cyanoethyl-amide | D or K | *m/z*: 426.0 [$M^+$] |
| 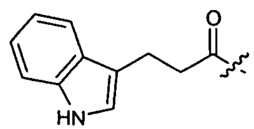 | **n** = 1; 3-Naphthalen-2-yl-2-(3-1*H*-indol-3-yl-propionylamino)-propionic acid cyanomethyl-amide<br>$\delta$ = 2.42 (m, 2 H), 2.74 (m, 2 H), 2.94 (dd, 1 H, $J$ = 9.3, $J$ = 13.7 Hz), 3.15 (m, 1 H), 4.14 (m, 2 H), 4.64 (m, 1 H), 6.94 (m, 1 H), 7.01 (m, 2 H), 7.10 (m, 1 H), 7.31 (m, 1 H), 7.38-7.49 (m, 3 H), 7.71 (s, 1 H), 7.79 (m, 2 H), 7.86 (m, 1 H), 8.30 (m, 1 H), 8.75 (m, 1 H). | D or K | *m/z*: 425.0 [$M^+$] |
| | **n** = 2; 3-Naphthalen-2-yl-2-(3-1*H*-indol-3-yl-propionylamino)-propionic acid cyanoethyl-amide | D or K | *m/z*: 439.0 [$M^+$] |

| | | | |
|---|---|---|---|
| 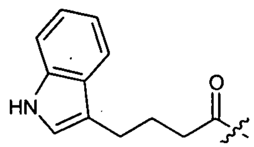 | **n = 1**; *N*-[1-(Cyanomethyl-carbamoyl)-2-naphthalen-2-yl-ethyl]-4-(1*H*-indol-3-yl)-butyramide<br>$\delta$ = 1.70 (m, 2 H), 2.11 (m, 2 H), 2.47 (m, 2 H), 2.94 (dd, 1 H, *J* = 9.3, *J* = 13.7 Hz), 3.15 (m, 1 H), 4.14 (m, 2 H), 4.64 (m, 1 H), 6.92 (m, 2 H), 7.03 (m, 2 H), 7.31 (m, 1 H), 7.37-7.49 (m, 3 H), 7.72 (s, 1 H), 7.75-7.85 (m, 4 H), 8.22 (m, 1 H), 8.75 (m, 1 H). | D or K | *m/z*: 439.0 [$M^+$] |
| | **n = 2**; *N*-[1-(Cyanoethyl-carbamoyl)-2-naphthalen-2-yl-ethyl]-4-(1*H*-indol-3-yl)-butyramide | D or K | *m/z*: 453.0 [$M^+$] |
| 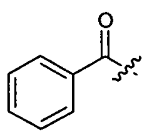 | **n = 1**; N-[1-(Cyanomethyl-carbamoyl)-2-naphthalen-2-yl-ethyl]-benzamide | D or K | *m/z*: 358.0 [$M^+$] |
| | **n = 2**; N-[1-(Cyanoethyl-carbamoyl)-2-naphthalen-2-yl-ethyl]-benzamide | D or K | *m/z*: 372.0 [$M^+$] |
| 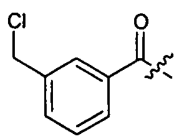 | **n = 1**; 3-Chloromethyl-*N*-[1-(cyanomethyl-carbamoyl)-2-naphthalen-2-yl-ethyl]-benzamide | D or K | *m/z*: 406.0 [$M^+$] |
| | **n = 2**; 3-Chloromethyl-*N*-[1-(cyanoethyl-carbamoyl)-2-naphthalen-2-yl-ethyl]-benzamide | D or K | *m/z*: 420.0 [$M^+$] |

| | | | |
|---|---|---|---|
| 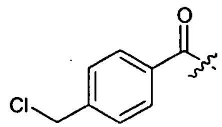 | **n** = **1**; 4-Chloromethyl-*N*-[1-(cyanomethyl-carbamoyl)-2-naphthalen-2-yl-ethyl]-benzamide $\delta$ = 3.18 (m, 1 H), 3.30 (m, 1 H), 4.18 (m, 2 H), 4.78 (s, 2 H), 4.83 (m, 1 H), 7.40-7.62 (m, 5 H), 7.78-7.85 (m, 6 H), 8.82 (m, 1 H). | D or K | *m/z*: 405.9 [$M^+$] |
| | **n** = **2**; 4-Chloromethyl-*N*-[1-(cyanoethyl-carbamoyl)-2-naphthalen-2-yl-ethyl]-benzamide | D or K | *m/z*: 420.0 [$M^+$] |
| 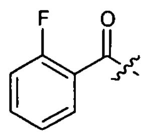 | **n** = **1**; *N*-[1-(Cyanomethyl-carbamoyl)-2-naphthalen-2-yl-ethyl]-2-fluoro-benzamide | D or K | *m/z*: 375.9 [$M^+$] |
| | **n** = **2**; *N*-[1-(Cyanoethyl-carbamoyl)-2-naphthalen-2-yl-ethyl]-2-fluoro-benzamide | D or K | *m/z*: 389.9 [$M^+$] |
| 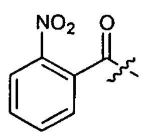 | **n** = **1**; *N*-[1-(Cyanomethyl-carbamoyl)-2-naphthalen-2-yl-ethyl]-2-nitro-benzamide | D or K | *m/z*: 402.9 [$M^+$] |
| | **n** = **2**; *N*-[1-(Cyanoethyl-carbamoyl)-2-naphthalen-2-yl-ethyl]-2-nitro-benzamide | D or K | *m/z*: 416.9 [$M^+$] |
| 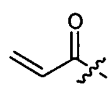 | **n** = **1**; *N*-[1-(Cyanomethyl-carbamoyl)-2-naphthalen-2-yl-ethyl]-acrylamide | D or K | *m/z*: 307.9 [$M^+$] |
| | **n** = **2**; *N*-[1-(Cyanomethyl-carbamoyl)-2-naphthalen-2-yl-ethyl]-acrylamide | D or K | *m/z*: 321.9 [$M^+$] |

| | | | |
|---|---|---|---|
| 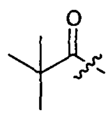 | **n** = **1**; 3-Naphthalen-2-yl-2,2-dimethyl-propionic acid cyanomethyl-amide | D or K | *m/z*: 338.0 [$M^+$] |
| | **n** = **2**; 3-Naphthalen-2-yl-2,2-dimethyl-propionic acid cyanoethyl-amide | D or K | *m/z*: 352.0 [$M^+$] |
| 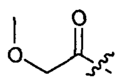 | **n** = **1**; 3-Naphthalen-2-yl-2-(2-methoxy-acetylamino)-propionic acid cyanomethyl-amide | D or K | *m/z*: 326.0 [$M^+$] |
| | **n** = **2**; 3-Naphthalen-2-yl-2-(2-methoxy-acetylamino)-propionic acid cyanoethyl-amide | D or K | *m/z*: 340.0 [$M^+$] |
| 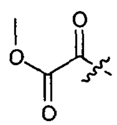 | **n** = **1**; *N*-[1-(Cyanomethyl-carbamoyl)-2-naphthalen-2-yl-ethyl]-oxalamic acid methyl ester $\delta$ = 3.14 (dd, 1 H, $J$ = 9.6, $J$ = 14.0 Hz), 3.29 (dd, 1 H, $J$ = 4.9, $J$ = 14.0 Hz), 3.72 (s, 3 H), 4.17 (m, 2 H), 4.63 (m, 1 H), 7.13 (m, 1 H), 7.40-7.49 (m, 3 H), 7.73-7.87 (m, 4 H), 8.84 (m, 1 H), 9.14 (m, 1 H). | | *m/z*: 340.0 [$M^+$] |
| | **n** = **2**; *N*-[1-(Cyanoethyl-carbamoyl)-2-naphthalen-2-yl-ethyl]-oxalamic acid methyl ester | | *m/z*: 354.0 [$M^+$] |

| | | | |
|---|---|---|---|
| 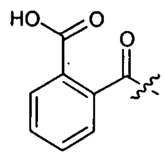 | **n** = **1**; *N*-[1-(Cyanomethyl-carbamoyl)-2-naphthalen-2-yl-ethyl]-phthalamic acid<br>$\delta$ = 3.09 (dd, 1 H, $J$ = 9.8, $J$ = 14.0 Hz), 3.35 (dd, 1 H, $J$ = 5.1, $J$ = 14.0 Hz), 4.19 (m, 2 H), 4.75 (m, 1 H), 7.13 (m, 1 H), 7.45-7.51 (m, 5 H), 7.72-7.87 (m, 5 H), 8.55 (m, 1 H), 8.79 (d, $J$ = 7.7 Hz, 1 H), 13.12 (bs, 1 H). | B or I | *m/z*: 401.9 [$M^+$] |
| | **n** = **2**; *N*-[1-(Cyanoethyl-carbamoyl)-2-naphthalen-2-yl-ethyl]-phthalamic acid | B or I | *m/z*: 415.9 [$M^+$] |
| 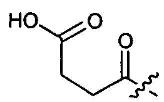 | **n** = **1**; *N*-[1-(Cyanomethyl-carbamoyl)-2-naphthalen-2-yl-ethyl]-succinamic acid | B or I | *m/z*: 353.9 [$M^+$] |
| | **n** = **2**; *N*-[1-(Cyanoethyl-carbamoyl)-2-naphthalen-2-yl-ethyl]-succinamic acid | B or I | *m/z*: 367.9 [$M^+$] |
| 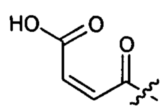 | **n** = **1**; 3-[1-(Cyanomethyl-carbamoyl)-2-naphthalen-2-yl-ethylcarbamoyl]-acrylic acid | B or I | *m/z*: 352.0 [$M^+$] |
| | **n** = **2**; 3-[1-(Cyanoethyl-carbamoyl)-2-naphthalen-2-yl-ethylcarbamoyl]-acrylic acid | B or I | *m/z*: 366.0 [$M^+$] |
| | **n** = **1**; [1-(Cyanomethyl-carbamoyl)-2-naphthalen-2-yl-ethyl]-carbamic acid isobutyl ester | C or J | *m/z*: 354.0 [$M^+$] |

| | | | |
|---|---|---|---|
| 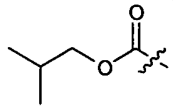 | **n** = 2; [1-(Cyanoethyl-carbamoyl)-2-naphthalen-2-yl-ethyl]-carbamic acid isobutyl ester | C or J | *m/z*: 368.0 [$M^+$] |
| 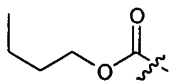 | **n** = 1; [1-(Cyanomethyl-carbamoyl)-2-naphthalen-2-yl-ethyl]-carbamic acid butyl ester | C or J | *m/z*: 354.0 [$M^+$] |
| | **n** = 1; [1-(Cyanoethyl-carbamoyl)-2-naphthalen-2-yl-ethyl]-carbamic acid butyl ester | C or J | *m/z*: 368.0 [$M^+$] |
| 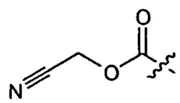 | **n** = 1; [1-(Cyanomethyl-carbamoyl)-2-naphthalen-2-yl-ethyl]-carbamic acid cyanomethyl ester | C or J | *m/z*: 335.9 [$M^+$] |
| | **n** = 2; [1-(Cyanoethyl-carbamoyl)-2-naphthalen-2-yl-ethyl]-carbamic acid cyanomethyl ester | C or J | *m/z*: 349.9 [$M^+$] |
| 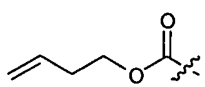 | **n** = 1; [1-(Cyanomethyl-carbamoyl)-2-naphthalen-2-yl-ethyl]-carbamic acid but-3-enyl ester | C or J | *m/z*: 352.0 [$M^+$] |
| | **n** = 2; [1-(Cyanoethyl-carbamoyl)-2-naphthalen-2-yl-ethyl]-carbamic acid but-3-enyl ester | C or J | *m/z*: 366.0 [$M^+$] |
| 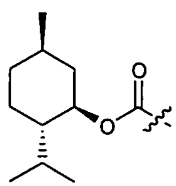 | **n** = 1; [1-(Cyanomethyl-carbamoyl)-2-naphthalen-2-yl-ethyl]-carbamic acid 2-isopropyl-5-methyl-cyclohexyl ester | C or J | *m/z*: 436.0 [$M^+$] |
| | **n** = 2; [1-(Cyanoethyl-carbamoyl)-2-naphthalen-2-yl-ethyl]-carbamic acid 2-isopropyl-5-methyl-cyclohexyl ester | C or J | *m/z*: 450.0 [$M^+$] |

| | | | |
|---|---|---|---|
| 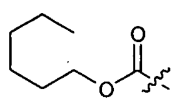 | **n = 1**; [1-(Cyanomethyl-carbamoyl)-2-naphthalen-2-yl-ethyl]-carbamic acid hexyl ester | C or J | *m/z*: 382.0 [$M^+$] |
| | **n = 2**; [1-(Cyanoethyl-carbamoyl)-2-naphthalen-2-yl-ethyl]-carbamic acid hexyl ester | C or J | *m/z*: 396.0 [$M^+$] |
| 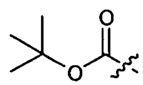 | **n = 1**; [1-(Cyanomethyl-carbamoyl)-2-naphthalen-2-yl-ethyl]-carbamic acid *tert*-butyl ester $\delta$ = 1.24 (s, 9 H), 2.93 (dd, 1 H, $J$ = 10.2, $J$ = 13.7 Hz), 3.14 (dd, 1 H, $J$ = 4.6, $J$ = 13.7 Hz), 4.14 (m, 2 H), 4.27 (m, 1 H), 7.13 (d, 1 H, $J$ = 8.3 Hz), 7.42-7.50 (m, 3 H), 7.72-7.87 (m, 3 H), 8.68 (m, 1 H). | | *m/z*: 353.9 [$M^+$] |
| | **n = 2**; [1-(Cyanoethyl-carbamoyl)-2-naphthalen-2-yl-ethyl]-carbamic acid *tert*-butyl ester $\delta$ = 1.24 (s, 9 H), 2.61 (m, 2 H), 2.93 (dd, 1 H, $J$ = 10.3, $J$ = 13.7 Hz), 3.13 (dd, 1 H, $J$ = 4.3, $J$ = 13.7 Hz), 3.37 (m, 2 H), 4.24 (m, 1 H), 6.99 (d, 1 H, $J$ = 8.6 Hz), 7.43-7.50 (m, 3 H), 7.73-7.87 (m, 3 H), 8.33 (m, 1 H). | | *m/z*: 367.8 [$M^+$] |
| 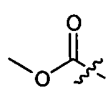 | **n = 1**; [1-(Cyanomethyl-carbamoyl)-2-naphthalen-2-yl-ethyl]-carbamic acid methyl ester | C or J | *m/z*: 312.0 [$M^+$] |
| | **n = 2**; [1-(Cyanoethyl-carbamoyl)-2-naphthalen-2-yl-ethyl]-carbamic acid methyl ester | C or J | *m/z*: 326.0 [$M^+$] |

| | | | |
|---|---|---|---|
| 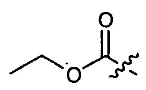 | **n** = **1**; [1-(Cyanomethyl-carbamoyl)-2-naphthalen-2-yl-ethyl]-carbamic acid ethyl ester<br>$\delta$ = 1.04 (dd, 3 H, $J$ = 7.5, $J$ = 6.6 Hz), 2.93 (dd, 1 H, $J$ = 10.3, $J$ = 13.7 Hz), 3.14 (dd, 1 H, $J$ = 4.5, $J$ = 13.7 Hz), 3.86 (m, 3 H), 4.15 (m, 2 H), 4.30 (m, 1 H), 7.43-7.51 (m, 3 H), 7.75-7.88 (m, 4 H), 8.76 (m, 1 H). | C or J | *m/z*: 326.0 [$M^+$] |
| | **n** = **2**; [1-(Cyanoethyl-carbamoyl)-2-naphthalen-2-yl-ethyl]-carbamic acid ethyl ester | C or J | *m/z*: 340.0 [$M^+$] |
| 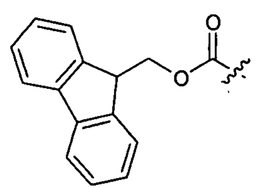 | **n** = **1**; [1-(Cyanomethyl-carbamoyl)-2-naphthalen-2-yl-ethyl]-carbamic acid 9*H*-fluoren-9-ylmethyl ester | C or J | *m/z*: 475.9 [$M^+$] |
| | **n** = **2**; [1-(Cyanoethyl-carbamoyl)-2-naphthalen-2-yl-ethyl]-carbamic acid 9*H*-fluoren-9-ylmethylester | C or J | *m/z*: 489.9 [$M^+$] |

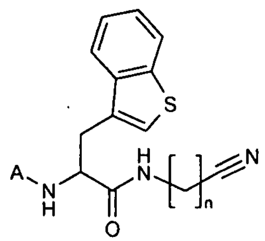

Column A lists the different A substituents:

| A | Name<br>$^{1}$H-NMR (DMSO-$d_6$, ppm) | Methods | MS Data |
|---|---|---|---|
| 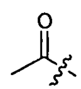 | **n** = 1; 3-Benzo[b]thiophen-3-yl-2-acetylamino-propionic acid cyanomethyl-amide | A, B, H or I | *m/z*: 301.9 [$M^+$] |
| | **n** = 2; 3-Benzo[b]thiophen-3-yl-2-acetylamino-propionic acid cyanoethyl-amide<br>$\delta$ = 1.78 (s, 3 H), 2.60 (m, 2 H), 3.03 (dd, 1 H, $J$ = 9.3, $J$ = 14.7 Hz), 3.22 (m, 1 H), 3.28 (m, 2 H), 4.58 (m, 1 H), 7.34-7.44 (m, 3 H), 7.89-7.98 (m, 2 H), 8.22 (m, 1 H), 8.44 (m, 1 H). | A, B, H or I | *m/z*: 315.9 [$M^+$] |
| | **n** = 1; 3-Benzo[b]thiophen-3-yl-2-[3-(4-trifluoromethyl-phenyl)-ureido]-propionic acid cyanomethyl-amide | F or M | *m/z*: 446.9 [$M^+$] |

| | | | |
|---|---|---|---|
| 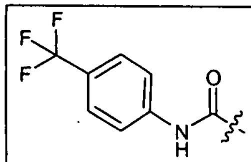 | **n** = **2**; 3-Benzo[b]thiophen-3-yl-2-[3-(4-trifluoromethyl-phenyl)-ureido]-propionic acid cyanoethyl-amide | F or M | *m/z*: 460.9 [$M^+$] |
| 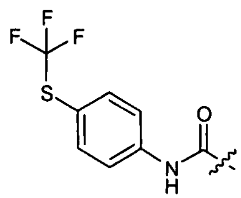 | **n** = **1**; 3-Benzo[b]thiophen-3-yl-2-[3-(4-trifluoromethylsulfanyl-phenyl)-ureido]-propionic acid cyanomethyl-amide | F or M | *m/z*: 478.9 [$M^+$] |
| | **n** = **2**; 3-Benzo[b]thiophen-3-yl-2-[3-(4-trifluoromethylsulfanyl-phenyl)-ureido]-propionic acid cyanoethyl-amide | F or M | *m/z*: 492.9 [$M^+$] |
| 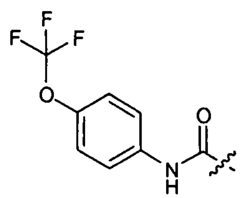 | **n** = **1**; 3-Benzo[b]thiophen-3-yl-2-[3-(4-trifluoromethoxy-phenyl)-ureido]-propionic acid cyanomethyl-amide | F or M | *m/z*: 463.0 [$M^+$] |
| | **n** = **2**; 3-Benzo[b]thiophen-3-yl-2-[3-(4-trifluoromethoxy-phenyl)-ureido]-propionic acid cyanoethyl-amide | F or M | *m/z*: 477.0 [$M^+$] |
| 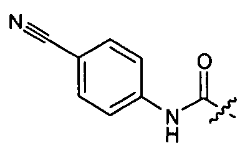 | **n** = **1**; 3-Benzo[b]thiophen-3-yl-2-[3-(4-cyano-phenyl)-ureido]-propionic acid cyanomethyl-amide | F or M | *m/z*: 404.0 [$M^+$] |
| | **n** = **2**; 3-Benzo[b]thiophen-3-yl-2-[3-(4-cyano-phenyl)-ureido]-propionic acid cyanoethyl-amide | F or M | *m/z*: 418.0 [$M^+$] |
| | **n** = **1**; 3-Benzo[b]thiophen-3-yl-2-(3-benzyl-ureido)-propionic acid cyanomethyl-amide | F or M | *m/z*: 393.0 [$M^+$] |

| | | | |
|---|---|---|---|
| 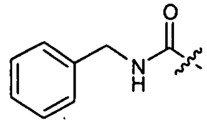 | **n** = **2**; 3-Benzo[b]thiophen-3-yl-2-(3-benzyl-ureido)-propionic acid cyanoethyl-amide | F or M | *m/z*: 407.0 [$M^+$] |
| 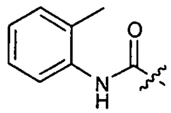 | **n** = **1**; 3-Benzo[b]thiophen-3-yl-2-(3-*o*-tolyl-ureido)-propionic acid cyanomethyl-amide | F or M | *m/z*: 393.0 [$M^+$] |
| | **n** = **2**; 3-Benzo[b]thiophen-3-yl-2-(3-*o*-tolyl-ureido)-propionic acid cyanoethyl-amide | F or M | *m/z*: 407.0 [$M^+$] |
| 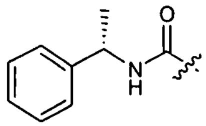 | **n** = **1**; 3-Benzo[b]thiophen-3-yl-2-[3-(*S*)-(1-phenyl-ethyl)-ureido]-propionic acid cyanomethyl-amide | F or M | *m/z*: 406.9 [$M^+$] |
| | **n** = **2**; 3-Benzo[b]thiophen-3-yl-2-[3-(*S*)-(1-phenyl-ethyl)-ureido]-propionic acid cyanoethyl-amide | F or M | *m/z*: 420.9 [$M^+$] |
| 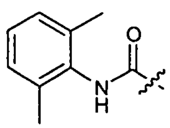 | **n** = **1**; 3-Benzo[b]thiophen-3-yl-2-[3-(2,6-dimethyl-phenyl)-ureido]-propionic acid cyanomethyl-amide. | F or M | *m/z*: 407.0 [$M^+$] |
| | **n** = **2**; 3-Benzo[b]thiophen-3-yl-2-[3-(2,6-dimethyl-phenyl)-ureido]-propionic acid cyanoethyl-amide | F or M | *m/z*: 420.9 [$M^+$] |
| 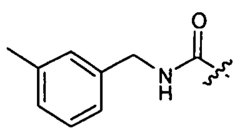 | **n** = **1**; 3-Benzo[b]thiophen-3-yl-2-[3-(3-methyl-benzyl)-ureido]-propionic acid cyanomethyl-amide | F or M | *m/z*: 407.0 [$M^+$] |
| | **n** = **2**; 3-Benzo[b]thiophen-3-yl-2-[3-(3-methyl-benzyl)-ureido]-propionic acid cyanoethyl-amide | F or M | *m/z*: 420.9 [$M^+$] |

| | | | |
|---|---|---|---|
| 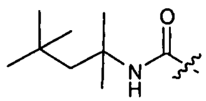 | **n** = **1**; 3-Benzo[b]thiophen-3-yl-2-[3-(1,1,3,3-tetramethyl-butyl)-ureido]-propionic acid cyanomethyl-amide | F or M | *m/z*: 414.9 [$M^+$] |
| | **n** = **2**; 3-Benzo[b]thiophen-3-yl-2-[3-(1,1,3,3-tetramethyl-butyl)-ureido]-propionic acid cyanoethyl-amide | F or M | *m/z*: 428.9 [$M^+$] |
| 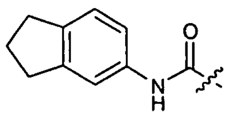 | **n** = **1**; 3-Benzo[b]thiophen-3-yl-2-(3-indan-5-yl-ureido)-propionic acid cyanomethyl-amide | F or M | *m/z*: 419.0 [$M^+$] |
| | **n** = **2**; 3-Benzo[b]thiophen-3-yl-2-(3-indan-5-yl-ureido)-propionic acid cyanoethyl-amide | F or M | *m/z*: 433.0 [$M^+$] |
| 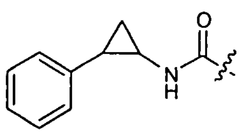 | **n** = **1**; 3-Benzo[b]thiophen-3-yl-2-[3-(2-phenyl-cyclopropyl)-ureido]-propionic acid cyanomethyl-amide | F or M | *m/z*: 419.1 [$M^+$] |
| | **n** = **2**; 3-Benzo[b]thiophen-3-yl-2-[3-(2-phenyl-cyclopropyl)-ureido]-propionic acid cyanoethyl-amide | E or K | *m/z*: 433.1 [$M^+$] |
| 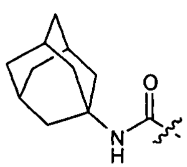 | **n** = **1**; 3-Benzo[b]thiophen-3-yl-2-(3-adamantan-1-yl-ureido)-propionic acid cyanomethyl-amide | F or M | *m/z*: 437.0 [$M^+$] |
| | **n** = **2**; 3-Benzo[b]thiophen-3-yl-2-(3-adamantan-1-yl-ureido)-propionic acid cyanoethyl-amide | F or M | *m/z*: 451.0 [$M^+$] |
| | **n** = **1**; 3-Benzo[b]thiophen-3-yl-2-(3-biphenyl-4-yl-ureido)-propionic acid cyanomethyl-amide | F or M | *m/z*: 455.1 [$M^+$] |

| | | | |
|---|---|---|---|
| 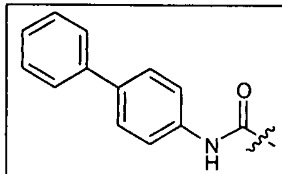 | **n** = **2**; 3-Benzo[b]thiophen-3-yl-2-(3-biphenyl-4-yl-ureido)-propionic acid cyanoethyl-amide | F or M | *m/z*: 469.1 [$M^+$] |
| 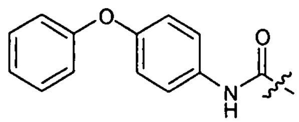 | **n** = 1; 3-Benzo[b]thiophen-3-yl-2-[3-(4-phenoxy-phenyl)-ureido]-propionic acid cyanomethyl-amide | F or M | *m/z*: 471.0 [$M^+$] |
| | **n** = **2**; 3-Benzo[b]thiophen-3-yl-2-[3-(4-phenoxy-phenyl)-ureido]-propionic acid cyanoethyl-amide | F or M | *m/z*: 485.0 [$M^+$] |
| 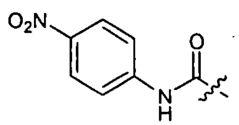 | **n** = 1; 3-Benzo[b]thiophen-3-yl-2-[3-(4-nitro-phenyl)-ureido]-propionic acid cyanomethyl-amide | F or M | *m/z*: 424.0 [$M^+$] |
| | **n** = 2; 3-Benzo[b]thiophen-3-yl-2-[3-(4-nitro-phenyl)-ureido]-propionic acid cyanoethyl-amide | F or M | *m/z*: 438.0 [$M^+$] |
| 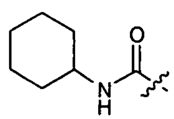 | **n** = **1**; 3-Benzo[b]thiophen-3-yl-2-(3-cyclohexyl-ureido)-propionic acid cyanomethyl-amide | F or M | *m/z*: 384.9 [$M^+$] |
| | **n** = **2**; 3-Benzo[b]thiophen-3-yl-2-(3-cyclohexyl-ureido)-propionic acid cyanoethyl-amide | F or M | *m/z*: 398.9 [$M^+$] |
| | **n** = 1; 3-Benzo[b]thiophen-3-yl-2-(3-benzo[1,3]dioxol-5-yl-ureido)-propionic acid cyanomethyl-amide | F or M | *m/z*: 423.0 [$M^+$] |

| | | | |
|---|---|---|---|
| 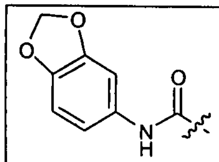 | **n** = 2; 3-Benzo[b]thiophen-3-yl-2-(3-benzo[1,3]dioxol-5-yl-ureido)-propionic acid cyanoethyl-amide | F or M | *m/z*: 437.0 [$M^+$] |
| 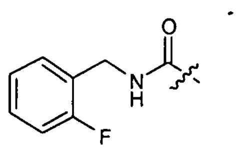 | **n** = 1; 3-Benzo[b]thiophen-3-yl-2-[3-(2-fluoro-benzyl)-ureido]-propionic acid cyanomethyl-amide | F or M | *m/z*: 411.0 [$M^+$] |
| | **n** = 2; 3-Benzo[b]thiophen-3-yl-2-[3-(2-fluoro-benzyl)-ureido]-propionic acid cyanoethyl-amide | F or M | *m/z*: 425.0 [$M^+$] |
| 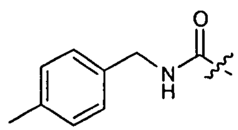 | **n** = 1; 3-Benzo[b]thiophen-3-yl-2-[3-(4-methyl-benzyl)-ureido]-propionic acid cyanomethyl-amide | F or M | *m/z*: 407.0 [$M^+$] |
| | **n** = 2; 3-Benzo[b]thiophen-3-yl-2-[3-(4-methyl-benzyl)-ureido]-propionic acid cyanoethyl-amide | F or M | *m/z*: 421.0 [$M^+$] |
| 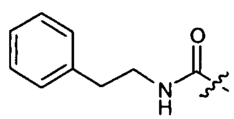 | **n** = 1; 3-Benzo[b]thiophen-3-yl-2-(3-phenethyl-ureido) -ureido]-propionic acid cyanomethyl-amide | F or M | *m/z*: 407.0 [$M^+$] |
| | **n** = 2; 3-Benzo[b]thiophen-3-yl-2-(3-phenethyl-ureido) -ureido]-propionic acid cyanoethyl-amide | F or M | *m/z*: 421.0 [$M^+$] |
| 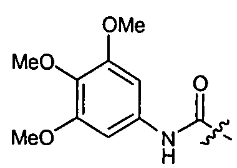 | **n** = 1; 3-Benzo[b]thiophen-3-yl-2-[3-(3,4,5-trimethoxy-phenyl)-ureido]-propionic acid cyanomethyl-amide | F or M | *m/z*: 469.0 [$M^+$] |
| | **n** = 2; 3-Benzo[b]thiophen-3-yl-2-[3-(3,4,5-trimethoxy-phenyl)-ureido]-propionic acid cyanoethyl-amide | F or M | *m/z*: 483.0 [$M^+$] |

| | | | |
|---|---|---|---|
| 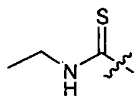 | **n = 1**; 3-Benzo[b]thiophen-3-yl-2-(3-ethyl-thioureido)-propionic acid cyanomethyl-amide | G or N | *m/z*: 346.9 [$M^+$] |
| | **n = 2**; 3-Benzo[b]thiophen-3-yl-2-(3-ethyl-thioureido)-propionic acid cyanoethyl-amide | G or N | *m/z*: 360.9 [$M^+$] |
| 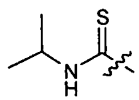 | **n = 1**; 3-Benzo[b]thiophen-3-yl-2-(3-isopropyl-thioureido)-propionic acid cyanomethyl-amide | G or N | *m/z*: 361.0 [$M^+$] |
| | **n = 2**; 3-Benzo[b]thiophen-3-yl-2-(3-isopropyl-thioureido)-propionic acid cyanoethyl-amide | G or N | *m/z*: 375.0 [$M^+$] |
| 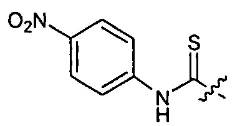 | **n = 1**; 3-Benzo[b]thiophen-3-yl-2-[3-(4-nitro-phenyl)-thioureido]-propionic acid cyanomethyl-amide | G or N | *m/z*: 439.9 [$M^+$] |
| | **n = 2**; 3-Benzo[b]thiophen-3-yl-2-[3-(4-nitro-phenyl)-thioureido]-propionic acid cyanoethyl-amide | G or N | *m/z*: 453.9 [$M^+$] |
| 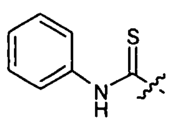 | **n = 1**; 3-Benzo[b]thiophen-3-yl-2-(3-phenyl-thioureido)-propionic acid cyanomethyl-amide | G or N | *m/z*: 394.9 [$M^+$] |
| | **n = 2**; 3-Benzo[b]thiophen-3-yl-2-(3-phenyl-thioureido)-propionic acid cyanoethyl-amide | G or N | *m/z*: 408.9 [$M^+$] |
| | **n = 1**; 3-Benzo[b]thiophen-3-yl-2-[3-(4-trifluoromethoxy-phenyl)-thioureido]-propionic acid cyanomethyl-amide | G or N | *m/z*: 478.9 [$M^+$] |

| | | | |
|---|---|---|---|
| 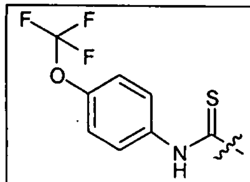 | **n** = **2**; 3-Benzo[b]thiophen-3-yl-2-[3-(4-trifluoromethoxy-phenyl)-thioureido]-propionic acid cyanoethyl-amide | G or N | *m/z*: 492.9 [$M^+$] |
| 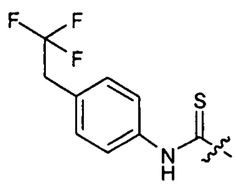 | **n** = 1; 3-Benzo[b]thiophen-3-yl-2-{3-[4-(2,2,2-trifluoro-ethyl)-phenyl]-thioureido}-propionic acid cyanomethyl-amide | G or N | *m/z*: 462.9 [$M^+$] |
| | **n** = **2**; 3-Benzo[b]thiophen-3-yl-2-{3-[4-(2,2,2-trifluoro-ethyl)-phenyl]-thioureido}-propionic acid cyanoethyl-amide | G or N | *m/z*: 476.9 [$M^+$] |
| 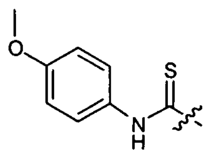 | **n** = 1; 3-Benzo[b]thiophen-3-yl-2-[3-(4-methoxy-phenyl)-thioureido]-propionic acid cyanomethyl-amide | G or N | *m/z*: 425.0 [$M^+$] |
| | **n** = **2**; 3-Benzo[b]thiophen-3-yl-2-[3-(4-methoxy-phenyl)-thioureido]-propionic acid cyanoethyl-amide | G or N | *m/z*: 439.0 [$M^+$] |
| 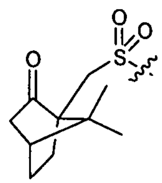 | **n** = **1**; 3-Benzo[b]thiophen-3-yl-2-(7,7-dimethyl-2-oxo-bicyclo[2.2.1]hept-1-ylmethanesulfonylamino)-propionic acid cyanomethyl-amide | E or L | *m/z*: 474.2 [$M^+$] |
| | **n** = **2**; 3-Benzo[b]thiophen-3-yl-2-(7,7-dimethyl-2-oxo-bicyclo[2.2.1]hept-1-ylmethanesulfonylamino)-propionic acid cyanoethyl-amide | E or L | *m/z*: 488.2 [$M^+$] |

| | | | |
|---|---|---|---|
| 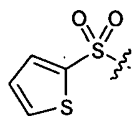 | **n** = **1**; 3-Benzo[b]thiophen-3-yl-2-(thiophene-2-sulfonylamino)-propionic acid cyanomethyl-amide | E or L | *m/z*: 406.0 [$M^+$] |
| | **n** = **2**; 3-Benzo[b]thiophen-3-yl-2-(thiophene-2-sulfonylamino)-propionic acid cyanoethyl-amide | E or L | *m/z*: 420.0 [$M^+$] |
| 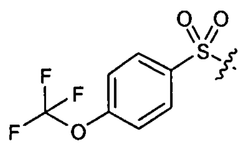 | **n** = 1; 3-Benzo[b]thiophen-3-yl-2-(4-trifluoromethoxy-benzenesulfonylamino)-propionic acid cyanomethyl-amide | E or L | *m/z*: 484.1 [$M^+$] |
| | **n** = 2; 3-Benzo[b]thiophen-3-yl-2-(4-trifluoromethoxy-benzenesulfonylamino)-propionic acid cyanoethyl-amide | E or L | *m/z*: 498.1 [$M^+$] |
| 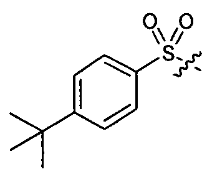 | **n** = 1; 3-Benzo[b]thiophen-3-yl-2-(4-*tert*-butyl-benzenesulfonylamino)-propionic acid cyanomethyl-amide | E or L | *m/z*: 456.2 [$M^+$] |
| | **n** = 2; 3-Benzo[b]thiophen-3-yl-2-(4-*tert*-butyl-benzenesulfonylamino)-propionic acid cyanoethyl-amide | E or L | *m/z*: 470.2 [$M^+$] |
| 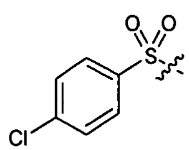 | **n** = 1; 3-Benzo[b]thiophen-3-yl-2-(4-chloro-benzenesulfonylamino)-propionic acid cyanomethyl-amide | E or L | *m/z*: 434.1 [$M^+$] |
| | **n** = 2; 3-Benzo[b]thiophen-3-yl-2-(4-chloro-benzenesulfonylamino)-propionic acid cyanoethyl-amide | E or L | *m/z*: 448.1 [$M^+$] |
| | **n** = 1; 3-Benzo[b]thiophen-3-yl-2-(4-methoxy-benzenesulfonylamino)-propionic acid cyanomethyl-amide | E or L | *m/z*: 430.1 [$M^+$] |

| | | | |
|---|---|---|---|
| 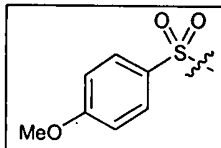 | **n** = **2**; 3-Benzo[b]thiophen-3-yl-2-(4-methoxy-benzenesulfonylamino)-propionic acid cyanoethyl-amide | E or L | *m/z*: 444.1 [$M^+$] |
| 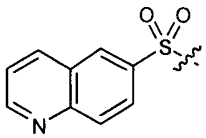 | **n** = **1**; 3-Benzo[b]thiophen-3-yl-2-(quinoline-6-sulfonylamino)-propionic acid cyanomethyl-amide | E or L | *m/z*: 451.2 [$M^+$] |
| | **n** = **2**; 3-Benzo[b]thiophen-3-yl-2-(quinoline-6-sulfonylamino)-propionic acid cyanoethyl-amide | E or L | *m/z*: 465.2 [$M^+$] |
| 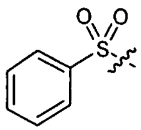 | **n** = **1**; 3-Benzo[b]thiophen-3-yl-2-benzenesulfonylamino-propionic acid cyanomethyl-amide | E or L | *m/z*: 400.1 [$M^+$] |
| | **n** = **2**; 3-Benzo[b]thiophen-3-yl-2-benzenesulfonylamino-propionic acid cyanoethyl-amide | E or L | *m/z*: 414.1 [$M^+$] |
| 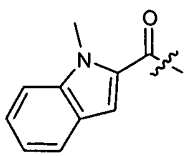 | **n** = **1**; 1-Methyl-1H-indole-2-carboxylic acid [2-benzo[b]thiophen-3-yl-1-(cyanomethyl-carbamoyl)-ethyl]-amide | D or K | *m/z*: 417.1 [$M^+$] |
| | **n** = **2**; 1-Methyl-1H-indole-2-carboxylic acid [2-benzo[b]thiophen-3-yl-1-(cyanoethyl-carbamoyl)-ethyl]-amide | D or K | *m/z*: 431.0 [$M^+$] |
| 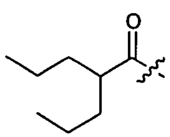 | **n** = **1**; 2-Propyl-pentanoic acid [2-benzo[b]thiophen-3-yl-1-(cyanomethyl-carbamoyl)-ethyl]-amide | D or K | *m/z*: 386.1 [$M^+$] |
| | **n** = **2**; 2-Propyl-pentanoic acid [2-benzo[b]thiophen-3-yl-1-(cyanoethyl-carbamoyl)-ethyl]-amide | D or K | *m/z*: 400.0 [$M^+$] |

| | | | |
|---|---|---|---|
| 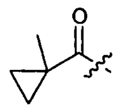 | **n** = 1; 1-Methyl-cyclopropanecarboxylic acid [2-benzo[b]thiophen-3-yl-1-(cyanomethyl-carbamoyl)-ethyl]-amide | D or K | *m/z*: 342.0 [$M^+$] |
| | **n** = 2; 1-Methyl-cyclopropanecarboxylic acid [2-benzo[b]thiophen-3-yl-1-(cyanoethyl-carbamoyl)-ethyl]-amide | D or K | *m/z*: 356.0 [$M^+$] |
| 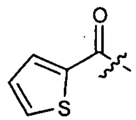 | n = 1; Thiophene-2-carboxylic acid [2-benzo[b]thiophen-3-yl-1-(cyanomethyl-carbamoyl)-ethyl]-amide | D or K | *m/z*: 369.9 [$M^+$] |
| | **n** = 2; Thiophene-2-carboxylic acid [2-benzo[b]thiophen-3-yl-1-(cyanoethyl-carbamoyl)-ethyl]-amide | D or K | *m/z*: 383.9 [$M^+$] |
| 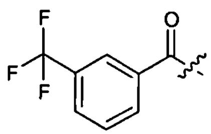 | **n** = 1; *N*-[2-Benzo[b]thiophen-3-yl-1-(cyanomethyl-carbamoyl)-ethyl]-3-trifluoromethyl-benzamide | D or K | *m/z*: 431.9 [$M^+$] |
| | **n** = 2; *N*-[2-Benzo[b]thiophen-3-yl-1-(cyanoethyl-carbamoyl)-ethyl]-3-trifluoromethyl-benzamide | D or K | *m/z*: 445.9 [$M^+$] |
| 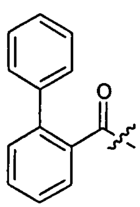 | **n** = 1; Biphenyl-2-carboxylic acid [2-benzo[b]thiophen-3-yl-1-(cyanomethyl-carbamoyl)-ethyl]-amide | D or K | *m/z*: 440.0 [$M^+$] |
| | **n** = 2; Biphenyl-2-carboxylic acid [2-benzo[b]thiophen-3-yl-1-(cyanoethyl-carbamoyl)-ethyl]-amide | D or K | *m/z*: 454.0 [$M^+$] |
| | **n** = 1; 4-Acetylamino-*N*-[2-benzo[b]thiophen-3-yl-1-(cyanomethyl-carbamoyl)-ethyl]-benzamide | D or K | *m/z*: 421.0 [$M^+$] |

| | | | |
|---|---|---|---|
| 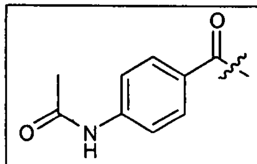 | **n** = **2**; 4-Acetylamino-*N*-[2-benzo[b]thiophen-3-yl-1-(cyanoethyl-carbamoyl)-ethyl]-benzamide | D or K | *m/z*: 435.0 [$M^+$] |
| 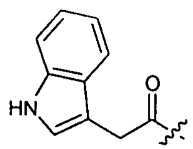 | **n** = 1; 3-Benzo[b]thiophen-3-yl-2-(2-1*H*-indol-3-yl-acetylamino)-propionic acid cyanomethyl-amide | D or K | *m/z*: 417.1 [$M^+$] |
| | **n** = 2; 3-Benzo[b]thiophen-3-yl-2-(2-1*H*-indol-3-yl-acetylamino)-propionic acid cyanoethyl-amide | D or K | *m/z*: 431.0 [$M^+$] |
| 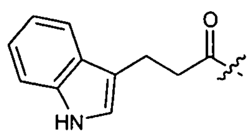 | **n** = 1; 3-Benzo[b]thiophen-3-yl-2-(3-1*H*-indol-3-yl-propionylamino)-propionic acid cyanomethyl-amide | D or K | *m/z*: 431.0 [$M^+$] |
| | **n** = 2; 3-Benzo[b]thiophen-3-yl-2-(3-1*H*-indol-3-yl-propionylamino)-propionic acid cyanoethyl-amide | D or K | *m/z*: 445.0 [$M^+$] |
| 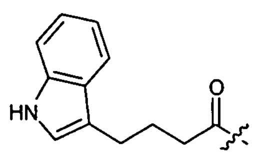 | **n** = 1; *N*-[2-Benzo[b]thiophen-3-yl-1-(cyanomethyl-carbamoyl)-ethyl]-4-(1*H*-indol-3-yl)-butyramide | D or K | *m/z*: 445.0 [$M^+$] |
| | **n** = 2; *N*-[2-Benzo[b]thiophen-3-yl-1-(cyanoethyl-carbamoyl)-ethyl]-4-(1*H*-indol-3-yl)-butyramide | D or K | *m/z*: 459.0 [$M^+$] |
| 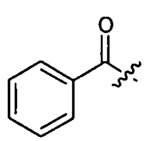 | **n** = 1; *N*-[2-Benzo[b]thiophen-3-yl-1-(cyanomethyl-carbamoyl)-ethyl]-benzamide | D or K | *m/z*: 364.0 [$M^+$] |
| | **n** = 2; *N*-[2-Benzo[b]thiophen-3-yl-1-(cyanoethyl-carbamoyl)-ethyl]-benzamide | D or K | *m/z*: 378.0 [$M^+$] |

| | | | |
|---|---|---|---|
| 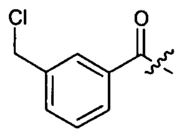 | **n** = 1; 3-Chloromethyl-*N*-[2-benzo[b]thiophen-3-yl-1-(cyanomethyl-carbamoyl)-ethyl]-benzamide | D or K | *m/z*: 412.0 [$M^+$] |
| | **n** = 2; 3-Chloromethyl-*N*-[2-benzo[b]thiophen-3-yl-1-(cyanoethyl-carbamoyl)-ethyl]-benzamide | D or K | *m/z*: 426.0 [$M^+$] |
| 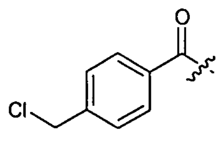 | n = 1; 4-Chloromethyl-*N*-[2-benzo[b]thiophen-3-yl-1-(cyanomethyl-carbamoyl)-ethyl]-benzamide | D or K | *m/z*: 411.9 [$M^+$] |
| | **n** = 2; 4-Chloromethyl-*N*-[2-benzo[b]thiophen-3-yl-1-(cyanoethyl-carbamoyl)-ethyl]-benzamide | D or K | *m/z*: 426.0 [$M^+$] |
| 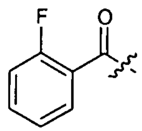 | **n** = 1; *N*-[2-Benzo[b]thiophen-3-yl-1-(cyanomethyl-carbamoyl)-ethyl]-2-fluoro-benzamide | D or K | *m/z*: 381.9 [$M^+$] |
| | **n** = 2; *N*-[2-Benzo[b]thiophen-3-yl-1-(cyanoethyl-carbamoyl)-ethyl]-2-fluoro-benzamide | D or K | *m/z*: 395.9 [$M^+$] |
| 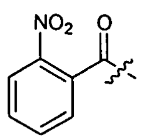 | **n** = 1; *N*-[2-Benzo[b]thiophen-3-yl-1-(cyanomethyl-carbamoyl)-ethyl]-2-nitro-benzamide | D or K | *m/z*: 408.9 [$M^+$] |
| | **n** = 2; *N*-[2-Benzo[b]thiophen-3-yl-1-(cyanoethyl-carbamoyl)-ethyl]-2-nitro-benzamide | D or K | *m/z*: 422.9 [$M^+$] |
| | **n** = 1; *N*-[2-Benzo[b]thiophen-3-yl-1-(cyanomethyl-carbamoyl)-ethyl]-acrylamide | D or K | *m/z*: 313.9 [$M^+$] |

| | | | |
|---|---|---|---|
| 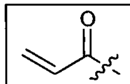 | **n** = **2**; *N*-[2-Benzo[b]thiophen-3-yl-1-(cyanomethyl-carbamoyl)-ethyl]-acrylamide | D or K | *m/z*: 327.9 [$M^+$] |
| 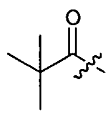 | **n** = **1**; 3-Benzo[b]thiophen-3-yl-2,2-dimethyl-propionic acid cyanomethyl-amide | D or K | *m/z*: 344.0 [$M^+$] |
| | **n** = **2**; 3-Benzo[b]thiophen-3-yl-2,2-dimethyl-propionic acid cyanoethyl-amide | D or K | *m/z*: 358.0 [$M^+$] |
| 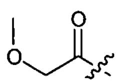 | **n** = **1**; 3-Benzo[b]thiophen-3-yl-2-(2-methoxy-acetylamino)-propionic acid cyanomethyl-amide | D or K | *m/z*: 332.0 [$M^+$] |
| | **n** = **2**; 3-Benzo[b]thiophen-3-yl-2-(2-methoxy-acetylamino)-propionic acid cyanoethyl-amide | D or K | *m/z*: 346.0 [$M^+$] |
| 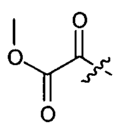 | **n** = **1**; *N*-[2-Benzo[b]thiophen-3-yl-1-(cyanomethyl-carbamoyl)-ethyl]-oxalamic acid methyl ester | | *m/z*: 346.0 [$M^+$] |
| | **n** = **2**; *N*-[2-Benzo[b]thiophen-3-yl-1-(cyanoethyl-carbamoyl)-ethyl]-oxalamic acid methyl ester | | *m/z*: 360.0 [$M^+$] |
| 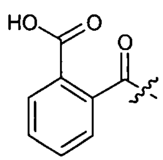 | **n** = **1**; *N*-[2-Benzo[b]thiophen-3-yl-1-(cyanomethyl-carbamoyl)-ethyl]-phthalamic acid | B or I | *m/z*: 407.9 [$M^+$] |
| | **n** = **2**; *N*-[2-Benzo[b]thiophen-3-yl-1-(cyanoethyl-carbamoyl)-ethyl]-phthalamic acid | B or I | *m/z*: 421.9 [$M^+$] |

| | | | |
|---|---|---|---|
| 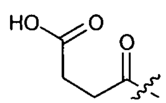 | **n** = **1**; *N*-[2-Benzo[b]thiophen-3-yl-1-(cyanomethyl-carbamoyl)-ethyl]-succinamic acid | B or I | *m/z*: 359.9 [$M^+$] |
| | **n** = **2**; *N*-[2-Benzo[b]thiophen-3-yl-1-(cyanoethyl-carbamoyl)-ethyl]-succinamic acid | B or I | *m/z*: 373.9 [$M^+$] |
| 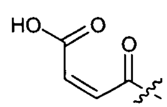 | **n** = **1**; 3-[2-Benzo[b]thiophen-3-yl-1-(cyanomethyl-carbamoyl)-ethylcarbamoyl]-acrylic acid | B or I | *m/z*: 358.0 [$M^+$] |
| | **n** = **2**; 3-[2-Benzo[b]thiophen-3-yl-1-(cyanoethyl-carbamoyl)-ethylcarbamoyl]-acrylic acid | B or I | *m/z*: 372.0 [$M^+$] |
| 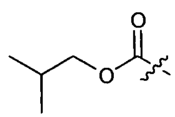 | **n** = **1**; [2-Benzo[b]thiophen-3-yl-1-(cyanomethyl-carbamoyl)-ethyl]-carbamic acid isobutyl ester | C or J | *m/z*: 360.0 [$M^+$] |
| | **n** = **2**; [2-Benzo[b]thiophen-3-yl-1-(cyanoethyl-carbamoyl)-ethyl]-carbamic acid isobutyl ester | C or J | *m/z*: 374.0 [$M^+$] |
| 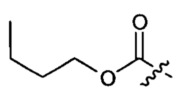 | **n** = **1**; [2-Benzo[b]thiophen-3-yl-1-(cyanomethyl-carbamoyl)-ethyl]-carbamic acid butyl ester | C or J | *m/z*: 360.0 [$M^+$] |
| | **n** = **1**; [2-Benzo[b]thiophen-3-yl-1-(cyanoethyl-carbamoyl)-ethyl]-carbamic acid butyl ester | C or J | *m/z*: 374.0 [$M^+$] |
| | **n** = **1**; [2-Benzo[b]thiophen-3-yl-1-(cyanomethyl-carbamoyl)-ethyl]-carbamic acid cyanomethyl ester | C or J | *m/z*: 341.9 [$M^+$] |

| | | | |
|---|---|---|---|
| 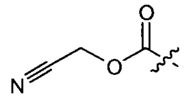 | **n = 2**; [2-Benzo[b]thiophen-3-yl-1-(cyanoethyl-carbamoyl)-ethyl]-carbamic acid cyanomethyl ester | C or J | *m/z*: 355.9 [$M^+$] |
| 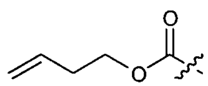 | **n** = 1; [2-Benzo[b]thiophen-3-yl-1-(cyanomethyl-carbamoyl)-ethyl]-carbamic acid but-3-enyl ester | C or J | *m/z*: 358.0 [$M^+$] |
| | **n = 2**; [2-Benzo[b]thiophen-3-yl-1-(cyanoethyl-carbamoyl)-ethyl]-carbamic acid but-3-enyl ester | C or J | *m/z*: 372.0 [$M^+$] |
| 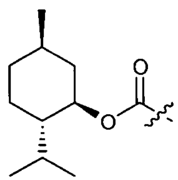 | **n** = 1; [2-Benzo[b]thiophen-3-yl-1-(cyanomethyl-carbamoyl)-ethyl]-carbamic acid 2-isopropyl-5-methyl-cyclohexyl ester | C or J | *m/z*: 442.0 [$M^+$] |
| | **n = 2**; [2-Benzo[b]thiophen-3-yl-1-(cyanoethyl-carbamoyl)-ethyl]-carbamic acid 2-isopropyl-5-methyl-cyclohexyl ester | C or J | *m/z*: 456.0 [$M^+$] |
| 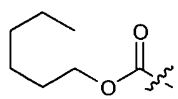 | **n** = 1; [2-Benzo[b]thiophen-3-yl-1-(cyanomethyl-carbamoyl)-ethyl]-carbamic acid hexyl ester | C or J | *m/z*: 388.0 [$M^+$] |
| | **n = 2**; [2-Benzo[b]thiophen-3-yl-1-(cyanoethyl-carbamoyl)-ethyl]-carbamic acid hexyl ester | C or J | *m/z*: 402.0 [$M^+$] |

| | | | |
|---|---|---|---|
| 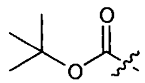 | **n** = **1**; [2-Benzo[b]thiophen-3-yl-1-(cyanomethyl-carbamoyl)-ethyl]-carbamic acid tert-butyl ester<br>$\delta$ = 1.28 (s, 9 H), 3.05 (dd, 1 H, $J$ = 9.9, $J$ = 14.2 Hz), 3.22 (dd, 1 H, $J$ = 4.6, $J$ = 14.2 Hz), 4.15 (m, 2 H), 4.32 (m, 1 H), 7.15 (d, 1 H, $J$ = 8.4 Hz), 7.35-7.48 (m, 3 H), 7.89-7.98 (m, 2 H), 8.74 (m, 1 H). | | *m/z*: 359.9 [$M^+$] |
| | **n** = **2**; [2-Benzo[b]thiophen-3-yl-1-(cyanoethyl-carbamoyl)-ethyl]-carbamic acid tert-butyl ester<br>$\delta$ = 1.29 (s, 9 H), 2.61 (m, 2 H), 3.04 (m, 2 H), 3.22 (m, 2 H), 3.27-3.40 (m, 4 H), 4.28 (m, 1 H), 7.01 (d, 1 H, $J$ = 8.2 Hz), 7.40 (m, 2 H), 7.90 (d, 1 H, $J$ = 7.3 Hz), 7.96 (d, 1 H, $J$ = 7.9 Hz), 8.38 (m, 1 H). | | *m/z*: 373.8 [$M^+$] |
| 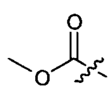 | **n** = **1**; [2-Benzo[b]thiophen-3-yl-1-(cyanomethyl-carbamoyl)-ethyl]-carbamic acid methyl ester | C or J | *m/z*: 318.0 [$M^+$] |
| | **n** = **2**; [2-Benzo[b]thiophen-3-yl-1-(cyanoethyl-carbamoyl)-ethyl]-carbamic acid methyl ester | C or J | *m/z*: 332.0 [$M^+$] |
| 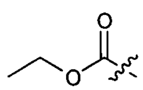 | **n** = **1**; [2-Benzo[b]thiophen-3-yl-1-(cyanomethyl-carbamoyl)--ethyl]-carbamic acid ethyl ester | C or J | *m/z*: 332.0 [$M^+$] |
| | **n** = **2**; [2-Benzo[b]thiophen-3-yl-1-(cyanoethyl-carbamoyl)-ethyl]-carbamic acid ethyl ester | C or J | *m/z*: 346.0 [$M^+$] |

| | | | |
|---|---|---|---|
| 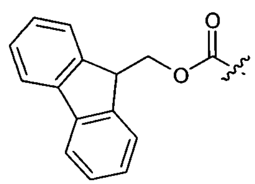 | **n** = **1**; [2-Benzo[b]thiophen-3-yl-1-(cyanomethyl-carbamoyl)-ethyl]-carbamic acid 9*H*-fluoren-9-ylmethyl ester | C or J | *m/z*: 481.9 [$M^+$] |
| | **n** = **2**; [2-Benzo[b]thiophen-3-yl-1-(cyanoethyl-carbamoyl)-ethyl]-carbamic acid 9*H*-fluoren-9-ylmethylester | C or J | *m/z*: 495.9 [$M^+$] |

EXAMPLE 12

**Specificity of inhibition of certain enzymes by compounds according to the present invention**

In order to characterize the specificity of various compounds the following assays were performed. PPIase activity of hpin1, hCyp 18, LpCyp 18, hFKBP 12 and EcParvulin was measured using the protease-coupled PPIase assay according to Fischer et al. (Fischer, G.; Bang, H.; Mech, C. Determination of enzymatic catalysis fort he cis-trans-isomerization of peptide binding in proline-containing peptides. [German] Biomed. Biochem. Acta 1984, 43, 1101-1111; Hennig et al., Selective Inactivation of Parvulin-like peptidyl-prolyl cis/trans isomerases by Juglon, Biochemistry. 1998, 37(17):5953-5960). For hPin1 measurements Ac-Ala-Ala-Ser($PO_3H_2$) -Pro-Arg-pNA was used as a substrate and trypsin (final concentration 190 μg/ml) as an isomer-specific protease. Activity measurements of other PPIases were made with the substrate peptide Suc-Ala-Phe-Pro-Phe-pNA and the protease α-chymotrypsin (final concentration 470 μg/ml). The assays were performed in a final reaction volume of 150 μl at final concentrations of 6 nM hPin1, 10 nM hCyp18, 5 nM LpCyp18, 20 nM EcParvulin and 20 nM hFKBP12, respectively, and 120 μM substrate peptide in 35 mM HEPES (pH 7.8). For inhibition experiments 100-0.01 μM of effector freshly diluted from a DMSO stock solution were added. The amount of solvent was kept constant within each experiment, usually below 0.3% (v/v). All reactions were started by addition of protease. The test was performed by observing the released 4-nitroaniline at 390 nm with a MR5000 UV/Vis spectrophotometer (Dynex) at 6°C. Data were evaluated by calculation of pseudo-first-order rate constants $k_{obs}$ in presence of PPIase and PPIase/effector, respectively, and corrected for the contribution of the non-catalyzed reaction ($k_0$). Inhibition constants $IC_{50}$ were calculated using SigmaPlot 8.0 (SPSS).

The following target enzymes which are all rotamases belonging to different classes of rotamases were used:

T-1: Protein interacting with NIMA (-kinase), hPin1
T-2: First described human Rapamycin receptor, hFKBP12
T-3: Human Cyclosporin A receptor with 18 kDa molecular weight, hCyp18
T-4: Leishmonia pneumophila virulence Cyclosporin A receptor with 18 kDa molecular weight, LpCyp18
T-5: Bacterial Juglon sensitive non proteolytic enzyme, EcParv

These rotamases are known in the art. Their production and characteristics may be taken from the following references.

Review about all PPIase families

Gothel, S. F.; Marahiel, M. A. TI Peptidyl-prolyl cis-trans isomerases, a superfamily of ubiquitous folding catalysts [Review]. Cell. Molec. Life Sci. 1999, 55, 423-436

Pin1

Lu, K. P.; Hanes, S. D.; Hunter, T. (1996) A human peptidyl-prolyl isomerase essential for regulation of mitosis. *Nature* **1996,** *380*, 544-547

Yaffe, M. B.; Schutkowski, M.; Shen, M. H.; Zhou, X. Z.; Stukenberg, P. T.; Rahfeld, J. U.; Xu, J.; Kuang, J.; Kirschner, M. W.; Fischer, G.; Cantley, L. C.; Lu K. P. SEQUENCE-SPECIFIC AND PHOSPHORYLATION-DEPENDENT PROLINE ISOMERIZATION - A POTENTIAL MITOTIC REGULATORY MECHANISM.
Science **1997**, *278*, 1957-1960
Shen, M.; Stukenberg, P. T.; Kirschner, M. W.; Lu, K. P. The essential mitotic peptidyl-prolyl isomerase Pin1 binds and regulates mitosis-specific phosphoproteins. *Genes Developm.* **1998,** *12*, 706-720.

EcParvulin

Rahfeld JU. Schierhom A. Mann K. Fischer G. A novel peptidyl-prolyl cis/trans isomerase from Escherichia coli. *FEBS Letters.* **1994,** *343,* 65-69

Rahfeld JU. Rucknagel KP. Schelbert B. Ludwig B. Hacker J. Mann K. Fischer G. Confirmation of the existence of a third family among peptidyl-prolyl cis/trans isomerases. Amino acid sequence and recombinant production of parvulin. *FEBS Letters.* **1994,** *352*, 180-184

FKBPs (including FKBP12) and Cyclophilins (including Cyp18)

For recent reviews on cyclophilins and FKBPs and their effectors, see: (a) Fischer, G. Peptidyl-prolyl *cis/trans*

isomerases and their effectors. *Angew. Chem., Int. Ed. Engl.* **1994,** *33,* 1415-1436. (b) Galat, A.; Metcalfe, S. M. Peptidylproline *cis*/*trans* isomerases. *Prog. Biophys. Molec. Biol.* **1995,** *63*, 67-118.

LpCyp18

Schmidt B. Tradler T. Rahfeld JU. Ludwig B. Jain B. Mann K. Rucknagel KP. Janowski B. Schierhom A. Kullertz G. Hacker J. Fischer G. A cyclophilin-like peptidyl-prolyl cis/trans isomerase from Legionella pneumophila--characterization, molecular cloning and overexpression. *Mol. Microbiol.* **1996,** *21*,1147-1160

In order to cluster the various rotamase inhibitors the following classes were defined with "A" indicating the most potent rotamase inhibitor.

A: IC50 < 5 μM
B: 5 μM < IC50 < 10 μM
C: 10 μM < IC50 < 50 μM
D: 50 μM < IC50 < 100 μM
E: IC50 > 100 μM

Table

Specificity of the inhibition with rotamases

| Compound | N° | Target | | | | |
|---|---|---|---|---|---|---|
| | | T-1 | T-2 | T-3 | T-4 | T-5 |
| 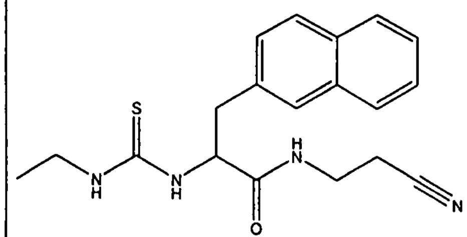 | 1 | A | E | E | E | E |
| 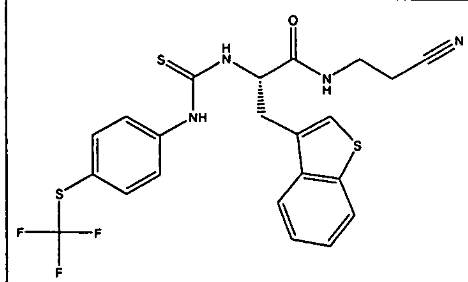 | 2 | B | E | E | E | D |
| 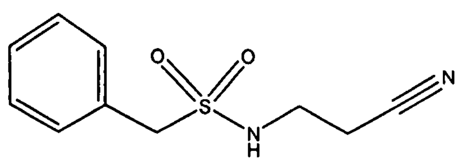 | 3 | B | E | E | E | E |

| Compound | N° | Target | | | | |
|---|---|---|---|---|---|---|
| | | T-1 | T-2 | T-3 | T-4 | T-5 |
| 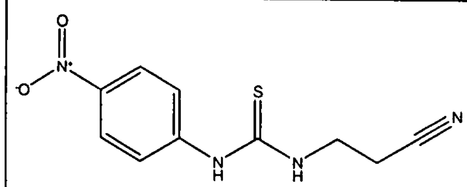 | 4 | A | E | E | E | C |
| 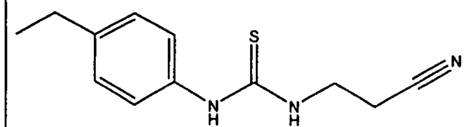 | 5 | A | E | E | E | |
| 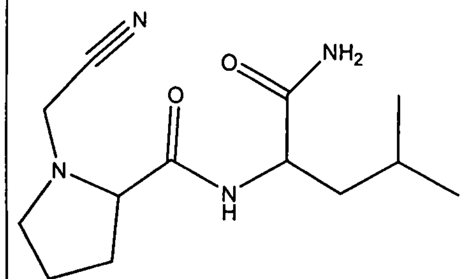 | 6 | B | E | E | E | |
| 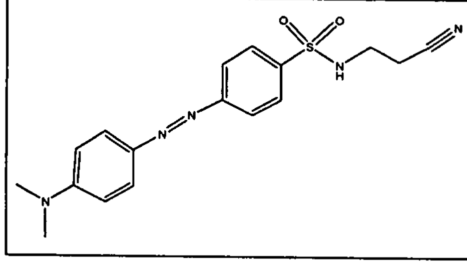 | 7 | B | E | E | E | C |

| Compound | N° | Target | | | | |
|---|---|---|---|---|---|---|
| | | T-1 | T-2 | T-3 | T-4 | T-5 |
| 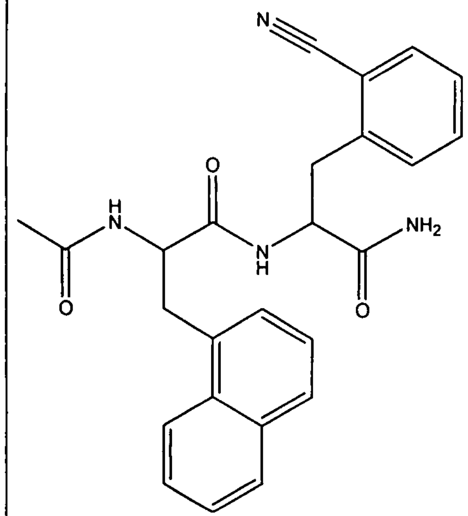 | 8 | A | E | E | E | B |

| Compound | N° | Target | | | | |
|---|---|---|---|---|---|---|
| | | T-1 | T-2 | T-3 | T-4 | T-5 |
| 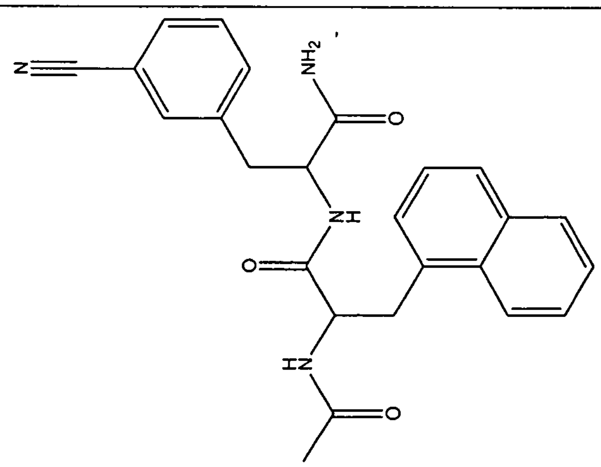 | 9 | B | E | E | E | B |

As may be taken from the above table the following compounds 1, 5, 8, are of class A and are thus extremely specific for hPin1.

The features of the present invention disclosed in the specification, the claims and/or the drawings may both separately and in any combination thereof be material for realizing the invention in various forms thereof.

## Claims

1. Use of a compound as an inhibitor to a rotamase, whereby the compound has the structure of formula (I):

A-B-C-D **(I)**

wherein:

A is selected from the group comprising

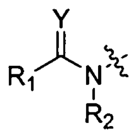 ; 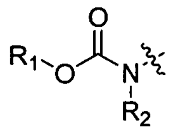 ; 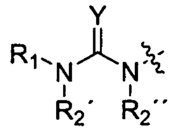

; 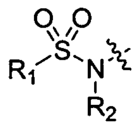 ; 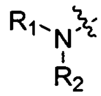 ; 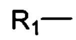

and

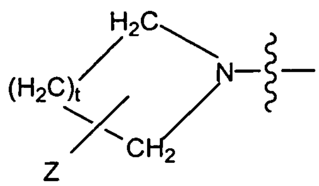

B is either present or absent, but if B is present B is

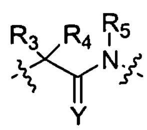

C is

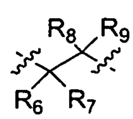 or 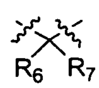

D is selected from the group comprising $-(CH_2)_rC(O)H$, $-(CH_2)_rC \equiv N$, $-(CH_2)_rNHNHC(O)NR_{A'}R_{A''}$, $-C(CH_2)_rC(O)(CH_2)_{r'}C(O)OR_{A'}$, $-(CH_2)_rC(O)(CH_2)_{r'}C(O)NR_{A'}R_{A''}$, $-(CH_2)_rCH(OH)(CH_2)_{r'}C(O)U$, $-(CH_2)_rC(O)W$ $-(CH_2)_rC(O)CH_2W$, $-(CH_2)_rC(O)$haloalkyl, and $-(CH_2)_rC(O)(CH_2)_{r'}$,$CHN_2$;

whereby

U is $-OR_{A'}$ or $-NR_{A'}R_{A''}$; and

W is $-OR_{A'}$, $-SR_{A'}$, $NR_{A'}R_{A''}$, or a heterocyclic moiety;

whereby r and r' are any integers from 0 to 5 and any r and r'mentioned are independently selected from any other r and r' mentioned or present and whereby $R_{A'}$ and $R_{A''}$ are selected independently from the group comprising H, alkyl, cycloalkyl, aryl, heteroaryl and heterocyclyl and derivatives of any of these groups.

Y is O, S, or $NR_a$ wherein $R_a$ is selected from the group comprising H, alkyl, cycloalkyl, aryl, heteroaryl and heterocyclyl and derivatives of any of these groups;

t is 1, 2 or 3;

Z is

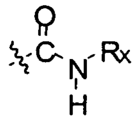

, whereby $R_x$ is selected from the group comprising amino acids, peptides and alkyl;

$R_1$, $R_3$, $R_4$, $R_6$, $R_7$, $R_8$, and $R_9$ are each individually and independently selected from the group comprising H, halogen, alkyl, cycloalkyl, aryl, heterocyclyl, heteroaryl and derivatives of any of these groups;

$R_2$, $R_2$', $R_2$", $R_5$ are each independently selected from the group consisting of H, alkyl, cycloalkyl, aryl, heteroaryl and heterocyclyl and derivatives of any of these groups or is a pharmaceutically acceptable salt or prodrug thereof.

**2.** Use of a compound for the manufacture of a medicament for the treatment of a disease, whereby the disease involves an abnormal cell proliferation, an undesired cell proliferation, an abnormal mitosis and/or an undesired mitosis.
wherein the compound has the structure of formula (I):

A-B-C-D **(I)**

wherein:

A is selected from the group comprising

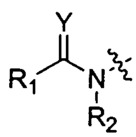 ; 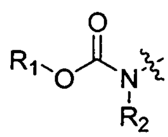 ; 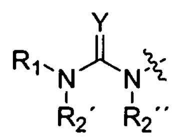

; 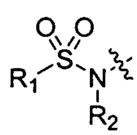 ; 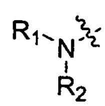 ; 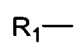

and

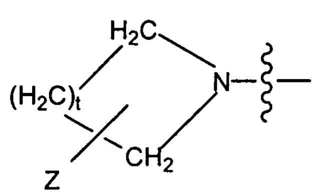

B is either present or absent, but if B is present B is

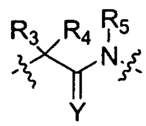

C is

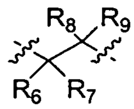 or 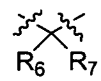

D is selected from the group comprising $-(CH_2)_r C(O)H$, $-(CH_2)_r C \equiv N$, $-(CH_2)_r NHNHC(O)NR_{A'} R_{A''}$, $-C(CH_2)_r C(O)(CH_2)_r C(O)OR_{A'}$ $-(CH_2)_r C(O)(CH_2)_r C(O)NR_{A'} R_{A''}$, $-(CH_2)_r CH(OH)(CH_2)_r C(O)U$, $-(CH_2)_r C(O)W$ $-(CH_2)_r C(O)CH_2W$, $-(CH_2)_r C(O)$haloalkyl, and $-(CH_2)_r C(O)(CH_2)_r CHN_2$;

whereby

U is $-OR_{A'}$ or $-NR_{A'} R_{A''}$, and

W is $-OR_{A'}$, $-SR_{A''}$, $-NR_{A'}$, $R_{A''}$, or a heterocyclic moiety;

whereby r and r' are any integers from 0 to 5 and any r and r' mentioned are independently selected from any other r and r' mentioned or present; and whereby $R_{A'}$ and $R_{A''}$ are selected independently from the group comprising H, alkyl, cycloalkyl, aryl, heteroaryl and heterocyclyl and derivatives of any of these groups.

Y is O, S, or $NR_a$ wherein $R_a$ is selected from the group comprising H, alkyl, cycloalkyl, aryl, heteroaryl and heterocyclyl and derivatives of any of these groups;

t is 1,2 or 3;

Z is

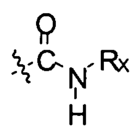

, whereby $R_x$ is selected from the group comprising amino acids, peptides and alkyl;

$R_1$, $R_3$, $R_4$, $R_6$, $R_7$, $R_8$ and $R_9$ are each individually and independently selected from the group comprising H, halogen, alkyl, cycloalkyl, aryl, heterocyclyl, heteroaryl and derivatives of any of these groups;

$R_2$, $R_2'$, $R_2''$, $R_5$ are each independently selected from the group consisting of H, alkyl, cycloalkyl, aryl, heteroaryl and heterocyclyl and derivatives of any of these groups or is a pharmaceutically acceptable salt or prodrug thereof.

**3.** The use according to claim 1 or 2, wherein each $R_1$, $R_3$, $R_4$, $R_6$, $R_7$, $R_8$ and $R_9$ is individually and independently a derivative of any of alkyl, cycloalkyl, aryl, heterocyclyl, or heteroaryl,

whereby any of these groups is individually and independently substituted by one or more groups of the formula $R_b$,

whereby $R_b$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, carbamoyl derivative, alkanoylamino, aroylamino, alkylthio, alkylthio derivatives, arylthio, arylthio derivatives, ureido, ureido derivatives, alkoxycarbonylamino, aryloxycarbonylamino, alkylcarbamoyloxy, arylcarbamoyloxy, alkylsulfonylamino, arylsulfonylamino, alkylaminosulfonyl, arylaminosulfonyl, amino, amino derivatives

and preferably $R_b$ is further substituted by one or more $R_c$,

whereby $R_c$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

**4.** The use according to any of claims 1 to 3, wherein the carbamoyl group is derivatized, preferably the nitrogen atom may be independently mono- or di-substituted by alkyl, aryl, heterocyclyl or heteroaryl; and/or

the alkylthio group is derivatized, preferably the sulfur atom is oxidized to a sulfoxide or sulfone; and/or
the arylthio group is derivatized, preferably the sulfur atom is oxidized to a sulfoxide or sulfone; and/or
the ureido group is derivatized, preferably either the nitrogen atom is independently mono- or di-substituted by a group which is selected from the group comprising alkyl, aryl, heterocyclyl or heteroaryl, alkoxycarbonylamino, aryloxycarbonylamino, alkylcarbamoyloxy, arylcarbamoyloxy, alkylsulfonylamino, arylsulfonylamino, alkylaminosulfonyl, arylaminosulfonyl; and/or
the amino group is derivatized, preferably the nitrogen atom is independently mono- or di-substituted by alkyl, aryl, heterocyclyl or heteroaryl, halogen, hydroxy, oxo, carboxy, cyano, nitro, amidino and guanidino.

**5.** The use according to any of claims 1 to 4, wherein
B is present and has the meaning as defined in any of the preceding claims,
C is

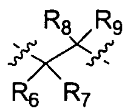

with $R_6$ to $R_9$ having the meaning as defined in any of the preceding claims.

**6.** The use according to any of claims 1 to 5, wherein D is selected from the group comprising $-(CH_2)_rC(O)H$, $-(CH_2)_rC\equiv N$, $-(CH_2)_rNHNHC(O)NR_{A'}R_{A''}$, $-C(CH_2)_rC(O)(CH_2)_{r'}C(O)OR_{A'}$, $-CH_2)_rC(O)(CH_2)_{r'}C(O)NR_{A'}R_{A''}$, $-(CH_2)_rCH(OH)(CH_2)_{r'}C(O)U$, $-(CH_2)_rC(O)W$,
whereby U is $-OR_{A'}$ or $-NR_{A'}R_{A''}$; W is $-OR_{A'}$, $-SR_{A'}$, $-NR_{A'}R_{A''}$, or a heterocyclic moiety; r and r' are independently any integer from 0 to 5; and $R_{A'}$ and $R_{A''}$ are selected independently from the group comprising H, alkyl, phenyl, benzyl, and phenethyl.

**7.** The use according to claim 6, wherein D is $-(CH_2)_rC\equiv N$ and r is any integer from 0 to 3.

**8.** The use according to any of claims 1 to 7, wherein
B is

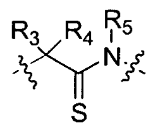

**9.** The use according to claim 8, wherein D is selected from the group comprising $-(CH_2)_rC(O)H$,$-(CH_2)_rC\equiv N$, $-(CH_2)_rNHNHC(O)NR_{A'}R_{A''}$, $-C(CH_2)_rC(O)(CH_2)_{r'}C(O)OR_{A'}$, $-(CH_2)_rC(O)(CH_2)_{r'}C(O)NR_{A'}R_{A''}$, $-(CH_2)_rCH(OH)(CH_2)_{r'}C(O)U$, $-(CH_2)_rC(O)W$, whereby U is $-OR_{A'}$ or $-NR_{A'}R_{A''}$, W is $-OR_{A'}$, $-SR_{A'}$, $-NR_{A'}R_{A''}$, or a heterocyclic moiety; r and r' are independently any integer from 0 to 5; and $R_{A'}$ and $R_{A''}$ are selected independently from the group comprising H, alkyl, phenyl, benzyl, and phenethyl.

**10.** The use according to claim 9, wherein D is $-(CH_2)_rC\equiv N$ and r is any integer from 0 to 3.

**11.** The use according to any of claims 1 to 10, wherein
A is

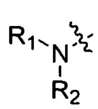 ; 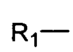 ; 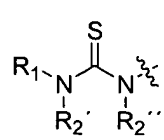

or a pharmaceutically acceptable salt or prodrug thereof.

**12.** The use according to claim 11, wherein D is selected from the group comprising $-(CH_2)_rC(O)H$,$-(CH_2)_{r'}C \equiv N$, $-(CH_2)_rNHNHC(O)NR_{A'}$ $R_{A''}$, $-C(CH_2)_rC(O)(CH_2)_{r'}C(O)OR_{A'}$, $-(CH_2)_rC(O)(CH_2)_{r'}C(O)NR_{A'}R_{A''}$, $-(CH_2)_rCH(OH)(CH_2)_{r'}C(O)U$, $-(CH_2)_rC(O)W$; whereby U is $-OR_{A'}$ or $-NR_{A'}R_{A''}$, W is $-OR_{A'}$, $-SR_{A'}$, $-NR_{A'}R_{A''}$, or a heterocyclic moiety; $R_{A'}$ and $R_{A''}$ are independently selected from H, alkyl, phenyl, benzyl, and phenethyl; and r and r' are independently any integer from 0 to 5.

**13.** The use according to claim 12, wherein D is $-(CH_2)_rC \equiv N$ and r is any integer from 0 to 3.

**14.** The use according to any of claims 1 to 13, wherein B is absent and wherein the other residues have the same meaning as given in any of the preceding claims.

**15.** The use according to claim 14, wherein D is selected from the group comprising $-(CH_2)_rC(O)H$,$—(CH_2)_{r'}C \equiv N$, $—(CH_2)_rNHNHC(O)NR_{A'}R_{A''}$, $-C(CH_2)_rC(O)(CH_2)_{r'}C(O)OR_{A'}$, $-(CH_2)_rC(O)(CH_2)_{r'}C(O)NR_{A'}R_{A''}$, $-(CH_2)_rCH(OH)(CH_2)_{r'}C(O)U$, $—(CH_2)_rC(O)W$;
whereby U is $—OR_{A'}$ or $—NR_{A'}R_{A''}$, W is $—OR_{A'}$, $-SR_{A'}$, $-NR_{A'}R_{A''}$, or a heterocyclic moiety; $R_{A'}$ and $R_{A''}$ are independently selected from H, alkyl, phenyl, benzyl, and phenethyl; and r and r' are independently any integer from 0 to 5.

**16.** The use according to claim 15, wherein D is $—(CH_2)_rC \equiv N$ and r is any integer from 0 to 3.

**17.** The use according to any of the claims 1-13, whereby the compound is selected from the group comprising:

3-(1*H*-Indol-3-yl)-2-acetylamino-propionic acid cyanomethyl-amide,
3-(1*H*-Indol-3-yl)-2-[3-(4-trifluoromethyl-phenyl)-ureido]-propionic acid cyanomethyl-amide,
3-(1*H*-Indol-3-yl)-2-[3-(4-trifluoromethylsulfanyl-phenyl)-ureido]-propionic acid cyanomethyl-amide,
3-(1*H*-Indol-3-yl)-2-[3-(4-trifluoromethoxy-phenyl)-ureido]-propionic acid cyanomethyl-amide,
3-(1*H*-Indol-3-yl)-2-[3-(4-cyano-phenyl)-ureido]-propionic acid cyanomethyl-amide,
3-(1*H*-Indol-3-yl)-2-(3-benzyl-ureido)-propionic acid cyanomethyl-amide,
3-(1*H*-Indol-3-yl)-2-(3-*o*-tolyl-ureido)-propionic acid cyanomethyl-amide,
3-(1*H*-Indol-3-yl)-2-[3-(*S*)-(1-phenyl-ethyl)-ureido]-propionic acid cyanomethyl-amide,
3-(1*H*-Indol-3-yl)-2-[3-(2,6-dimethyl-phenyl)-ureido]-propionic acid cyanomethyl-amide,
3-(1*H*-Indol-3-yl)-2-[3-(3-methyl-benzyl)-ureido]-propionic acid cyanomethyl-amide,
3-(1*H*-Indol-3-yl)-2-[3-(1,1,3,3-tetramethyl-butyl)-ureido]-propionic acid cyanomethyl-amide,
3-(1*H*-Indol-3-yl)-2-(3-indan-5-yl-ureido)-propionic acid cyanomethyl-amide,
3-(1*H*-Indol-3-yl)-2-[3-(2-phenyl-cyclopropyl)-ureido]-propionic acid cyanomethyl-amide,
3-(1*H*-Indol-3-yl)-2-(3-adamantan-1-yl-ureido)-propionic acid cyanomethyl-amide,
3-(1*H*-Indol-3-yl)-2-(3-biphenyl-4-yl-ureido)-propionic acid cyanomethyl-amide,
3-(1*H*-Indol-3-yl)-2-[3-(4-phenoxy-phenyl)-ureido]-propionic acid cyanomethyl-amide,
3-(1*H*-Indol-3-yl)-2-[3-(4-nitro-phenyl)-ureido]-propionic acid cyanomethyl-amide,
3-(1*H*-Indol-3-yl)-2-(3-cyclohexyl-ureido)-propionic acid cyanomethyl-amide,
3-(1*H*-Indol-3-yl)-2-(3-benzo[1,3]dioxol-5-yl-ureido)-propionic acid cyanomethyl-amide,
3-(1*H*-Indol-3-yl)-2-[3-(2-fluoro-benzyl)-ureido]-propionic acid cyanomethyl-amide,
3-(1*H*-Indol-3-yl)-2-[3-(4-methyl-benzyl)-ureido]-propionic acid cyanomethyl-amide,
3-(1*H*-Indol-3-yl)-2-(3-phenethyl-ureido) -ureido]-propionic acid cyanomethyl-amide,
3-(1*H*-Indol-3-yl)-2-[3-(3,4,5-trimethoxy-phenyl)-ureido]-propionic acid cyanomethyl-amide,
3-(1*H*-Indol-3-yl)-2-(3-ethyl-thioureido)-propionic acid cyanomethyl-amide,
3-(1*H*-Indol-3-yl)-2-(3-isopropyl-thioureido)-propionic acid cyanomethyl-amide,
3-(1*H*-Indol-3-yl)-2-[3-(4-nitro-phenyl)-thioureido]-propionic acid cyanomethyl-amide,
3-(1*H*-Indol-3-yl)-2-(3-phenyl-thioureido)-propionic acid cyanomethyl-amide,
3-(1*H*-Indol-3-yl)-2-[3-(4-trifluoromethoxy-phenyl)-thioureido]-propionic acid cyanomethyl-amide,
3-(1*H*-Indol-3-yl)-2-{3-[4-(2,2,2-trifluoro-ethyl)-phenyl]-thioureido}-propionic acid cyanomethyl-amide,
3-(1*H*-Indol-3-yl)-2-[3-(4-methoxy-phenyl)-thioureido]-propionic acid cyanomethyl-amide,
3-(1*H*-Indol-3-yl)-2-(7,7-dimethyl-2-oxo-bicyclo[2.2.1]hept-1-ylmethanesulfonylamino)-propionic acid cyanomethyl-amide,
3-(1*H*-Indol-3-yl)-2-(thiophene-2-sulfonylamino)-propionic acid cyanomethyl-amide,
3-(1*H*-Indol-3-yl)-2-(4-trifluoromethoxy-benzenesulfonylamino)-propionic acid cyanomethyl-amide,

3-(1*H*-Indol-3-yl)-2-(4-*tert*-butyl-benzenesulfonylamino)-propionic acid cyanomethyl-amide,
3-(1*H*-Indol-3-yl)-2-(4-chloro-benzenesulfonylamino)-propionic acid cyanomethyl-amide,
3-(1*H*-Indol-3-yl)-2-(4-methoxy-benzenesulfonylamino)-propionic acid cyanomethyl-amide,
3-(1*H*-Indol-3-yl)-2-(quinoline-6-sulfonylamino)-propionic acid cyanomethyl-amide,
3-(1*H*-Indol-3-yl)-2-benzenesulfonylamino-propionic acid cyanomethyl-amide,
1-Methyl-1*H* indole-2-carboxylic acid [1-(cyanomethyl-carbamoyl)-2-(1*H*-indol-3-yl)-ethyl]-amide,
2-Propyl-pentanoic acid [1-(cyanomethyl-carbamoyl)-2-(1*H*-indol-3-yl)-ethyl]-amide,
1-Methyl-cyclopropanecarboxylic acid [1-(cyanomethyl-carbamoyl)-2-(1*H*-indol-3-yl)-ethyl]-amide,
Thiophene-2-carboxylic acid [1-(cyanomethyl-carbamoyl)-2-(1*H*-indol-3-yl)-ethyl]-amide,
*N*-[1-(Cyanomethyl-carbamoyl)-2-(1*H*-indol-3-yl)-ethyl]-3-trifluoromethyl-benzamide,
Biphenyl-2-carboxylic acid [1-(cyanomethyl-carbamoyl)-2-(1*H*-indol-3-yl)-ethyl]-amide,
4-Acetylamino-*N*-[1-(cyanomethyl-carbamoyl)-2-(1*H*-indol-3-yl)-ethyl]-benzamide,
3-(1*H* Indol-3-yl)-2-(2-1*H*-indol-3-yl-acetylamino)-propionic acid cyanomethyl-amide,
3-(1*H*-Indol-3-yl)-2-(3-1*H*-indol-3-yl-propionylamino)-propionic acid cyanomethyl-amide,
*N*-[1-(Cyanomethyl-carbamoyl)-2-(1*H*-indol-3-yl)-ethyl]-4-(1*H*-indol-3-yl)-butyramide,
*N*-[1-(Cyanomethyl-carbamoyl)-2-(1*H*-indol-3-yl)-ethyl]-benzamide,
3-Chloromethyl-*N*-[1-(cyanomethyl-carbamoyl)-2-(1*H*-indol-3-yl)-ethyl]-benzamide,
4-Chloromethyl-*N*-[1-(cyanomethyl-carbamoyl)-2-(1*H*-indol-3-yl)-ethyl]-benzamide,
*N*-[[1-(Cyanomethyl-carbamoyl)-2-(1*H*-indol-3-yl)-ethyl]-2-fluoro-benzamide,
*N*-[1-(Cyanomethyl-carbamoyl)-2-(1*H*-indol-3-yl)-ethyl]-2-nitro-benzamide,
*N*-[1-(Cyanomethyl-carbamoyl)-2-(1*H*-indol-3-yl)-ethyl]-acrylamide,
3-(1*H*-Indol-3-yl)-2,2-dimethyl-propionic acid cyanomethyl-amide,
3-(1*H*-Indol-3-yl)-2-(2-methoxy-acetylamino)-propionic acid cyanomethyl-amide,
*N*-[1-(Cyanomethyl-carbamoyl)-2-(1*H*-indol-3-yl)-ethyl]-oxalamic acid methyl ester,
*N*-[1-(Cyanomethyl-carbamoyl)-2-(1*H*-indol-3-yl)-ethyl]-phthalamic acid,
*N*-[1-(Cyanomethyl-carbamoyl)-2-(1*H*-indol-3-yl)-ethyl]-succinamic acid,
3-[1-(Cyanomethyl-carbamoyl)-2-(1*H*-indol-3-yl)-ethylcarbamoyl]-acrylic acid,
[1-(Cyanomethyl-carbamoyl)-2-(1*H*-indol-3-yl)-ethyl]-carbamic acid isobutyl ester,
[1-(Cyanomethyl-carbamoyl)-2-(1*H*-indol-3-yl)-ethyl]-carbamic acid butyl ester,
[1-(Cyanomethyl-carbamoyl)-2-(1*H*-indol-3-yl)-ethyl]-carbamic acid hexyl ester,
[1-(Cyanomethyl-carbamoyl)-2-(1*H*-indol-3-yl)-ethyl]-carbamic acid tert-butyl ester,
[1-(Cyanomethyl-carbamoyl)-2-(1*H*-indol-3-yl)-ethyl]-carbamic acid methyl ester,
[1-(Cyanomethyl-carbamoyl)-2-(1*H*-indol-3-yl)-ethyl]-carbamic acid ethyl ester,
[1-(Cyanomethyl-carbamoyl)-2-(1*H*-indol-3-yl)-ethyl]-carbamic acid 9*H*-fluoren-9-ylmethyl ester,
3-(1*H*-Indol-3-yl)-2-acetylamino-propionic acid cyanoethyl-amide,
3-(1*H*-Indol-3-yl)-2-[3-(4-trifluoromethyl-phenyl)-ureido]-propionic acid cyanoethyl-amide,
3-(1*H*-Indol-3-yl)-2-[3-(4-trifluoromethylsulfanyl-phenyl)-ureido]-propionic acid cyanoethyl-amide,
3-(1*H*-Indol-3-yl)-2-[3-(4-trifluoromethoxy-phenyl)-ureido]-propionic acid cyanoethyl-amide,
3-(1*H*-Indol-3-yl)-2-[3-(4-cyano-phenyl)-ureido]-propionic acid cyanoethyl-amide,
3-(1*H*-Indol-3-yl)-2-(3-benzyl-ureido)-propionic acid cyanoethyl-amide,
3-(1*H*-Indol-3-yl)-2-(3-o-tolyl-ureido)-propionic acid cyanoethyl-amide,
3-(1*H*-Indol-3-yl)-2-[3-(*S*)-(1-phenyl-ethyl)-ureido]-propionic acid cyanoethyl-amide,
3-(1*H*-Indol-3-yl)-2-[3-(2,6-dimethyl-phenyl)-ureido]-propionic acid cyanoethyl-amide,
3-(1*H*-Indol-3-yl)-2-[3-(3-methyl-benzyl)-ureido]-propionic acid cyanoethyl-amide,
3-(1*H*-Indol-3-yl)-2-[3-(1,1,3,3-tetramethyl-butyl)-ureido]-propionic acid cyanoethyl-amide,
3-(1*H*-Indol-3-yl)-2-(3-indan-5-yl-ureido)-propionic acid cyanoethyl-amide,
3-(1*H*-Indol-3-yl)-2-[3-(2-phenyl-cyclopropyl)-ureido]-propionic acid cyanoethyl-amide,
3-(1*H*-Indol-3-yl)-2-(3-adamantan-1-yl-ureido)-propionic acid cyanoethyl-amide,
3-(1*H*-Indol-3-yl)-2-(3-biphenyl-4-yl-ureido)-propionic acid cyanoethyl-amide,
3-(1*H*-Indol-3-yl)-2-[3-(4-phenoxy-phenyl)-ureido]-propionic acid cyanoethyl-amide,
3-(1*H*-Indol-3-yl)-2-[3-(4-nitro-phenyl)-ureido]-propionic acid cyanoethyl-amide,
3-(1*H*-Indol-3-yl)-2-(3-cyclohexyl-ureido)-propionic acid cyanoethyl-amide,
3-(1*H*-Indol-3-yl)-2-(3-benzo[1,3]dioxol-5-yl-ureido)-propionic acid cyanoethyl-amide,
3-(1*H*-Indol-3-yl)-2-[3-(2-fluoro-benzyl)-ureido]-propionic acid cyanoethyl-amide,
3-(1*H*-Indol-3-yl)-2-[3-(4-methyl-benzyl)-ureido]-propionic acid cyanoethyl-amide,
3-(1*H*-Indol-3-yl)-2-(3-phenethyl-ureido)-ureido]-propionic acid cyanoethyl-amide,
3-(1*H*-Indol-3-yl)-2-[3-(3,4,5-trimethoxy-phenyl)-ureido]-propionic acid cyanoethyl-amide,
3-(1*H*-Indol-3-yl)-2-(3-ethyl-thioureido)-propionic acid cyanoethyl-amide,

3-(1*H*-Indol-3-yl)-2-(3-isopropyl-thioureido)-propionic acid cyanoethyl-amide,
3-(1*H*-Indol-3-yl)-2-[3-(4-nitro-phenyl)-thioureido]-propionic acid cyanoethyl-amide,
3-(1*H*-Indol-3-yl)-2-(3-phenyl-thioureido)-propionic acid cyanoethyl-amide,
3-(1*H*-Indol-3-yl)-2-[3-(4-trifluoromethoxy-phenyl)-thioureido]-propionic acid cyanoethyl-amide,
3-(1*H*-Indol-3-yl)-2-{3-[4-(2,2,2-trifluoro-ethyl)-phenyl]-thioureido}-propionic acid cyanoethyl-amide,
3-(1*H*-Indol-3-yl)-2-[3-(4-methoxy-phenyl)-thioureido]-propionic acid cyanoethyl-amide,
3-(1*H*-Indol-3-yl)-2-(7,7-dimethyl-2-oxo-bicyclo[2.2.1]hept-1-ylmethanesulfonylamino)-propionic acid cyanoethyl-amide,
3-(1*H*-Indol-3-yl)-2-(thiophene-2-sulfonylamino)-propionic acid cyanoethyl-amide,
3-(1*H*-Indol-3-yl)-2-(4-trifluoromethoxy-benzenesulfonylamino)-propionic acid cyanoethyl-amide,
3-(1*H*-Indol-3-yl)-2-(4-tert-butyl-benzenesulfonylamino)-propionic acid cyanoethyl-amide,
3-(1*H*-Indol-3-yl)-2-(4-chloro-benzenesulfonylamino)-propionic acid cyanoethyl-amide,
3-(1*H*-Indol-3-yl)-2-(4-methoxy-benzenesulfonylamino)-propionic acid cyanoethyl-amide,
3-(1*H*-Indol-3-yl)-2-(quinoline-6-sulfonylamino)-propionic acid cyanoethyl-amide,
3-(1*H*-Indol-3-yl)-2-benzenesulfonylamino-propionic acid cyanoethyl-amide,
1-Methyl-1*H*-indole-2-carboxylic acid [1-(cyanoethyl-carbamoyl)-2-(1*H*-indol-3-yl)-ethyl]-amide
2-Propyl-pentanoic acid [1-(cyanoethyl-carbamoyl)-2-(1*H*-indol-3-yl)-ethyl]-amide,
1-Methyl-cyclopropanecarboxylic acid [1-(cyanoethyl-carbamoyl)-2-(1*H*-indol-3-yl)-ethyl]-amide,
Thiophene-2-carboxylic acid [1-(cyanoethyl-carbamoyl)-2-(1*H*-indol-3-yl)-ethyl]-amide,
*N*-[1-(Cyanoethyl-carbamoyl)-2-(1*H*-indol-3-yl)-ethyl]-3-trifluoromethyl-benzamide,
Biphenyl-2-carboxylic acid [1-(cyanoethyl-carbamoyl)-2-(1*H*-indol-3-yl)-ethyl]-amide,
4-Acetylamino-*N*-[1-(cyanoethyl-carbamoyl)-2-(1*H*-indol-3-yl)-ethyl]-benzamide,
3-(1*H*-indol-3-yl)-2-(2-1*H*-indol-3-yl-acetylamino)-propionic acid cyanoethyl-amide,
3-(1*H*-Indol-3-yl)-2-(3-1*H*-indol-3-yl-propionylamino)-propionic acid cyanoethyl-amide,
*N*-[1-(Cyanoethyl-carbamoyl)-2-(1*H*-indol-3-yl)-ethyl]-4-(1*H*-indol-3-yl)-butyramide,
*N*-[1-(Cyanoethyl-carbamoyl)-2-(1*H*-indol-3-yl)-ethyl]-benzamide,
3-Chloromethyl-*N*-[1-(cyanoethyl-carbamoyl)-2-(1*H*-indol-3-yl)-ethyl]-benzamide,
4-Chloromethyl-*N*-[1-(cyanoethyl-carbamoyl)-2-(1*H*-indol-3-yl)-ethyl]-benzamide,
*N*-[1-(Cyanoethyl-carbamoyl)-2-(1*H*-indol-3-yl)-ethyl]-2-fluoro-benzamide,
*N*-[1-(Cyanoethyl-carbamoyl)-2-(1*H*-indol-3-yl)-ethyl]-2-nitro-benzamide,
*N*-[1-(Cyanoethyl-carbamoyl)-2-(1*H*-indol-3-yl)-ethyl]-acrylamide,
3-(1*H*-Indol-3-yl)-2,2-dimethyl-propionic acid cyanoethyl-amide,
3-(1*H*-Indol-3-yl)-2-(2-methoxy-acetylamino)-propionic acid cyanoethyl-amide,
*N*-[1-(Cyanoethyl-carbamoyl)-2-(1*H*-indol-3-yl)-ethyl]-oxalamic acid methyl ester,
*N*-[1-(Cyanoethyl-carbamoyl)-2-(1*H*-indol-3-yl)-ethyl]-phthalamic acid,
*N*-[1-(Cyanoethyl-carbamoyl)-2-(1*H*-indol-3-yl)-ethyl]-succinamic acid,
3-[1-(Cyanoethyl-carbamoyl)-2-(1*H*-indol-3-yl)-ethylcarbamoyl]-acrylic acid,
[1-(Cyanoethyl-carbamoyl)-2-(1*H*-indol-3-yl)-ethyl]-carbamic acid isobutyl ester,
[1-(Cyanoethyl-carbamoyl)-2-(1*H*-indol-3-yl)-ethyl]-carbamic acid butyl ester,
[1-(Cyanomethyl-carbamoyl)-2-(1*H*-indol-3-yl)-ethyl]-carbamic acid cyanomethyl ester,
[1-(Cyanoethyl-carbamoyl)-2-(1*H*-indol-3-yl)-ethyl]-carbamic acid cyanomethyl ester,
[1-(Cyanomethyl-carbamoyl)-2-(1*H*-indol-3-yl)-ethyl]-carbamic acid but-3-enyl ester,
[1-(Cyanoethyl-carbamoyl)-2-(1*H*-indol-3-yl)-ethyl]-carbamic acid but-3-enyl ester,
[1-(Cyanomethyl-carbamoyl)-2-(1*H*-indol-3-yl)-ethyl]-carbamic acid 2-isopropyl-5-methyl-cyclohexyl ester,
[1-(Cyanoethyl-carbamoyl)-2-(1*H*-indol-3-yl)-ethyl]-carbamic acid 2-isopropyl-5-methyl-cyclohexyl ester,
[1-(Cyanoethyl-carbamoyl)-2-(1*H*-indol-3-yl)-ethyl]-carbamic acid hexyl ester,
[1-(Cyanoethyl-carbamoyl)-2-(1*H*-indol-3-yl)-ethyl]-carbamic acid *tert*-butyl ester,
[1-(Cyanoethyl-carbamoyl)-2-(1*H*-indol-3-yl)-ethyl]-carbamic acid methyl ester,
[1-(Cyanoethyl-carbamoyl)-2-(1*H*-indol-3-yl)-ethyl]-carbamic acid ethyl ester,
[1-(Cyanoethyl-carbamoyl)-2-(1*H*-indol-3-yl)-ethyl]-carbamic acid 9*H*-fluoren-9-ylmethylester,
3-(4-Hydroxy-phenyl)-2-acetylamino-propionic acid cyanomethyl-amide,
3-(4-Hydroxy-phenyl)-2-[3-(4-trifluoromethyl-phenyl)-ureido]-propionic acid cyanomethyl-amide,
3-(4-Hydroxy-phenyl)-2-[3-(4-trifluoromethylsulfanyl-phenyl)-ureido]-propionic acid cyanomethyl-amide,
3-(4-Hydroxy-phenyl)-2-[3-(4-trifluoromethoxy-phenyl)-ureido]-propionic acid cyanomethyl-amide,
3-(4-Hydroxy-phenyl)-2-[3-(4-cyano-phenyl)-ureido]-propionic acid cyanomethyl-amide,
3-(4-Hydroxy-phenyl)-2-(3-benzyl-ureido)-propionic acid cyanomethyl-amide,
3-(4-Hydroxy-phenyl)-2-(3-o-tolyl-ureido)-propionic acid cyanomethyl-amide,
3-(4-Hydroxy-phenyl)-2-[3-(*S*)-(1-phenyl-ethyl)-ureido]-propionic acid cyanomethyl-amide,

3-(4-Hydroxy-phenyl)-2-[3-(2,6-dimethyl-phenyl)-ureido]-propionic acid cyanomethyl-amide,
3-(4-Hydroxy-phenyl)-2-[3-(3-methyl-benzyl)-ureido]-propionic acid cyanomethyl-amide,
3-(4-Hydroxy-phenyl)-2-[3-(1,1,3,3-tetramethyl-butyl)-ureido]-propionic acid cyanomethyl-amide,
3-(4-Hydroxy-phenyl)-2-(3-indan-5-yl-ureido)-propionic acid cyanomethyl-amide,
3-(4-Hydroxy-phenyl)-2-[3-(2-phenyl-cyclopropyl)-ureido]-propionic acid cyanomethyl-amide,
3-(4-Hydroxy-phenyl)-2-(3-adamantan-1-yl-ureido)-propionic acid cyanomethyl-amide,
3-(4-Hydroxy-phenyl)-2-(3-biphenyl-4-yl-ureido)-propionic acid cyanomethyl-amide,
3-(4-Hydroxy-phenyl)-2-[3-(4-phenoxy-phenyl)-ureido]-propionic acid cyanomethyl-amide,
3-(4-Hydroxy-phenyl)-2-[3-(4-nitro-phenyl)-ureido]-propionic acid cyanomethyl-amide,
3-(4-Hydroxy-phenyl)-2-(3-cyclohexyl-ureido)-propionic acid cyanomethyl-amide,
3-(4-Hydroxy-phenyl)-2-(3-benzo[1,3]dioxol-5-yl-ureido)-propionic acid cyanomethyl-amide,
3-(4-Hydroxy-phenyl)-2-[3-(2-fluoro-benzyl)-ureido]-propionic acid cyanomethyl-amide,
3-(4-Hydroxy-phenyl)-2-[3-(4-methyl-benzyl)-ureido]-propionic acid cyanomethyl-amide,
3-(4-Hydroxy-phenyl)-2-(3-phenethyl-ureido) -ureido]-propionic acid cyanomethyl-amide,
3-(4-Hydroxy-phenyl)-2-[3-(3,4,5-trimethoxy-phenyl)-ureido]-propionic acid cyanomethyl-amide,
3-(4-Hydroxy-phenyl)-2-(3-ethyl-thioureido)-propionic acid cyanomethyl-amide,
3-(4-Hydroxy-phenyl)-2-(3-isopropyl-thioureido)-propionic acid cyanomethyl-amide,
3-(4-Hydroxy-phenyl)-2-[3-(4-nitro-phenyl)-thioureido]-propionic acid cyanomethyl-amide,
3-(4-Hydroxy-phenyl)-2-(3-phenyl-thioureido)-propionic acid cyanomethyl-amide,
3-(4-Hydroxy-phenyl)-2-[3-(4-trifluoromethoxy-phenyl)-thioureido]-propionic acid cyanomethyl-amide,
3-(4-Hydroxy-phenyl)-2-{3-[4-(2,2,2-trif[uoro-ethyl)-phenyl]-thioureido}-propionic acid cyanomethyl-amide,
3-(4-Hydroxy-phenyl)-2-[3-(4-methoxy-phenyl)-thioureido]-propionic acid cyanomethyl-amide,
3-(4-Hydroxy-phenyl)-2-(7,7-dimethyl-2-oxo-bicyclo[2.2.1]hept-1-ylmethanesulfonylamino)-propionic acid cyanomethyl-amide,
3-(4-Hydroxy-phenyl)-2-(thiophene-2-sulfonylamino)-propionic acid cyanomethyl-amide,
3-(4-Hydroxy-phenyl)-2-(4-trifluoromethoxy-benzenesulfonylamino)-propionic acid cyanomethyl-amide,
3-(4-Hydroxy-phenyl)-2-(4-*tert*-butyl-benzenesulfonylamino)-propionic acid cyanomethyl-amide,
3-(4-Hydroxy-phenyl)-2-(4-chloro-benzenesulfonylamino)-propionic acid cyanomethyl-amide,
3-(4-Hydroxy-phenyl)-2-(4-methoxy-benzenesulfonylamino)-propionic acid cyanomethyl-amide,
3-(4-Hydroxy-phenyl)-2-(quinoline-6-sulfonylamino)-propionic acid cyanomethyl-amide,
3-(4-Hydroxy-phenyl)-2-benzenesulfonylamino-propionic acid cyanomethyl-amide,
1-Methyl-1H-indole-2-carboxylic acid [1-(cyanomethyl-carbamoyl)-2-(4-hydroxy-phenyl)-ethyl]-amide,
2-Propyl-pentanoic acid [1-(cyanomethyl-carbamoyl)-2-(4-hydroxy-phenyl)-ethyl]-amide,
1-Methyl-cyclopropanecarboxylic acid [1-(cyanomethyl-carbamoyl)-2-(4-hydroxy-phenyl)-ethyl]-amide,
Thiophene-2-carboxylic acid [1-(cyanomethyl-carbamoyl)-2-(4-hydroxy-phenyl)-ethyl]-amide,
*N*-[1-(Cyanomethyl-carbamoyl)-2-(4-hydroxy-phenyl)-ethyl]-3-trifluoromethyl-benzamide,
Biphenyl-2-carboxylic acid [1-(cyanomethyl-carbamoyl)-2-(4-hydroxy-phenyl)-ethyl]-amide,
4-Acetylamino-*N*-[1-(cyanomethyl-carbamoyl)-2-(4-hydroxy-phenyl)-ethyl]-benzamide,
3-(4-Hydroxy-phenyl)-2-(2-1*H*-indol-3-yl-acetylamino)-propionic acid cyanomethyl-amide,
3-(4-Hydroxy-phenyl)-2-(3-1*H*-indol-3-yl-propionylamino)-propionic acid cyanomethyl-amide,
*N*-[1-(Cyanomethyl-carbamoyl)-2-(4-hydroxy-phenyl)-ethyl]-4-(1*H*-indol-3-yl)-butyramide,
*N*-[1-(Cyanomethyl-carbamoyl)-2-(4-hydroxy-phenyl)-ethyl]-benzamide,
3-Chloromethyl-*N*-[1-(cyanomethyl-carbamoyl)-2-(4-hydroxy-phenyl)-ethyl]-benzamide,
4-Chloromethyl-*N*-[1-(cyanomethyl-carbamoyl)-2-(4-hydroxy-phenyl)-ethyl]-benzamide,
*N*-[1-(Cyanomethyl-carbamoyl)-2-(4-hydroxy-phenyl)-ethyl]-2-fluoro-benzamide,
*N*-[1-(Cyanomethyl-carbamoyl)-2-(4-hydroxy-phenyl)-ethyl]-2-nitro-benzamide,
*N*-[1-(Cyanomethyl-carbamoyl)-2-(4-hydroxy-phenyl)-ethyl]-acrylamide,
3-(4-Hydroxy-phenyl)-2,2-dimethyl-propionic acid cyanomethyl-amide,
3-(4-Hydroxy-phenyl)-2-(2-methoxy-acetylamino)-propionic acid cyanomethyl-amide,
*N*-[1-(Cyanomethyl-carbamoyl)-2-(4-hydroxy-phenyl)-ethyl]-oxalamic acid methyl ester,
*N*-[1-(Cyanomethyl-carbamoyl)-2-(4-hydroxy-phenyl)-ethyl]-phthalamic acid,
*N*-[1-(Cyanomethyl-carbamoyl)-2-(4-hydroxy-phenyl)-ethyl]-succinamic acid,
3-[1-(Cyanomethyl-carbamoyl)-2-(4-hydroxy-phenyl)-ethylcarbamoyl]-acrylic acid,
[1-(Cyanomethyl-carbamoyl)-2-(4-hydroxy-phenyl)-ethyl]-carbamic acid isobutyl ester,
[1-(Cyanomethyl-carbamoyl)-2-(4-hydroxy-phenyl)-ethyl]-carbamic acid butyl ester,
[1-(Cyanomethyl-carbamoyl)-2-( 4-hydroxy-phenyl)-ethyl]-carbamic acid hexyl ester,
[1-(Cyanomethyl-carbamoyl)-2-(4-hydroxy-phenyl)-ethyl]-carbamic acid *tert*-butyl ester,
[1-(Cyanomethyl-carbamoyl)-2-(4-hydroxy-phenyl)-ethyl]-carbamic acid methyl ester,

[1-(Cyanomethyl-carbamoyl)-2-(4-hydroxy-phenyl)-ethyl]-carbamic acid ethyl ester,
[1-(Cyanomethyl-carbamoyl)-2-(4-hydroxy-phenyl)-ethyl]-carbamic acid 9*H*-fluoren-9-ylmethyl ester,
3-(4-Hydroxy-phenyl)-2-acetylamino-propionic acid cyanoethyl-amide,
3-(4-Hydroxy-phenyl)-2-[3-(4-trifluoromethyl-phenyl)-ureido]-propionic acid cyanoethyl-amide,
3-(4-Hydroxy-phenyl)-2-[3-(4-trifluoromethylsulfanyl-phenyl)-ureido]-propionic acid cyanoethyl-amide,
3-(4-Hydroxy-phenyl)-2-[3-(4-trifluoromethoxy-phenyl)-ureido]-propionic acid cyanoethyl-amide,
3-(4-Hydroxy-phenyl)-2-[3-(4-cyano-phenyl)-ureido]-propionic acid cyanoethyl-amide,
3-(4-Hydroxy-phenyl)-2-(3-benzyl-ureido)-propionic acid cyanoethyl-amide,
3-(4-Hydroxy-phenyl)-2-(3-o-tolyl-ureido)-propionic acid cyanoethyl-amide,
3-(4-Hydroxy-phenyl)-2-[3-(*S*)-(1-phenyl-ethyl)-ureido]-propionic acid cyanoethyl-amide,
3-(4-Hydroxy-phenyl)-2-[3-(2,6-dimethyl-phenyl)-ureido]-propionic acid cyanoethyl-amide,
3-(4-Hydroxy-phenyl)-2-[3-(3-methyl-benzyl)-ureido]-propionic acid cyanoethyl-amide,
3-(4-Hydroxy-phenyl)-2-[3-(1,1,3,3-tetramethyl-butyl)-ureido]-propionic acid cyanoethyl-amide,
3-(4-Hydroxy-phenyl)-2-(3-indan-5-yl-ureido)-propionic acid cyanoethyl-amide,
3-(4-Hydroxy-phenyl)-2-[3-(2-phenyl-cyclopropyl)-ureido]-propionic acid cyanoethyl-amide,
3-(4-Hydroxy-phenyl)-2-(3-adamantan-1-yl-ureido)-propionic acid cyanoethyl-amide,
3-(4-Hydroxy-phenyl)-2-(3-biphenyl-4-yl-ureido)-propionic acid cyanoethyl-amide,
3-(4-Hydroxy-phenyl)-2-[3-(4-phenoxy-phenyl)-ureido]-propionic acid cyanoethyl-amide,
3-(4-Hydroxy-phenyl)-2-[3-(4-nitro-phenyl)-ureido]-propionic acid cyanoethyl-amide,
3-(4-Hydroxy-phenyl)-2-(3-cyclohexyl-ureido)-propionic acid cyanoethyl-amide,
3-(4-Hydroxy-phenyl)-2-(3-benzo[1,3]dioxol-5-yl-ureido)-propionic acid cyanoethyl-amide,
3-(4-Hydroxy-phenyl)-2-[3-(2-fluoro-benzyl)-ureido]-propionic acid cyanoethyl-amide,
3-(4-Hydroxy-phenyl)-2-[3-(4-methyl-benzyl)-ureido]-propionic acid cyanoethyl-amide,
3-(4-Hydroxy-phenyl)-2-(3-phenethyl-ureido) -ureido]-propionic acid cyanoethyl-amide,
3-(4-Hydroxy-phenyl)-2-[3-(3,4,5-trimethoxy-phenyl)-ureido]-propionic acid cyanoethyl-amide,
3-(4-Hydroxy-phenyl)-2-(3-ethyl-thioureido)-propionic acid cyanoethyl-amide,
3-(4-Hydroxy-phenyl)-2-(3-isopropyl-thioureido)-propionic acid cyanoethyl-amide,
3-(4-Hydroxy-phenyl)-2-[3-(4-nitro-phenyl)-thioureido]-propionic acid cyanoethyl-amide,
3-(4-Hydroxy-phenyl)-2-(3-phenyl-thioureido)-propionic acid cyanoethyl-amide,
3-(4-Hydroxy-phenyl)-2-[3-(4-trifluoromethoxy-phenyl)-thioureido]-propionic acid cyanoethyl-amide,
3-(4-Hydroxy-phenyl)-2-{3-[4-(2,2,2-trifluoro-ethyl)-phenyl]-thioureido}-propionic acid cyanoethyl-amide,
3-(4-Hydroxy-phenyl)-2-[3-(4-methoxy-phenyl)-thioureido]-propionic acid cyanoethyl-amide,
3-(4-Hydroxy-phenyl)-2-(7,7-dimethyl-2-oxo-bicyclo[2.2.1]hept-1-ylmethanesulfonylamino)-propionic acid cyanoethyl-amide,
3-(4-Hydroxy-phenyl)-2-(thiophene-2-sulfonylamino)-propionic acid cyanoethyl-amide,
3-(4-Hydroxy-phenyl)-2-(4-trifluoromethoxy-benzenesulfonylamino)-propionic acid cyanoethyl-amide,
3-(4-Hydroxy-phenyl)-2-(4-tert-butyl-benzenesulfonylamino)-propionic acid cyanoethyl-amide,
3-(4-Hydroxy-phenyl)-2-(4-chloro-benzenesulfonylamino)-propionic acid cyanoethyl-amide,
3-(4-Hydroxy-phenyl)-2-(4-methoxy-benzenesulfonylamino)-propionic acid cyanoethyl-amide,
3-(4-Hydroxy-phenyl)-2-(quinoline-6-sulfonylamino)-propionic acid cyanoethyl-amide,
3-(4-Hydroxy-phenyl)-2-benzenesulfonylamino-propionic acid cyanoethyl-amide,
1-Methyl-1H-indole-2-carboxylic acid [1-(cyanoethyl-carbamoyl)-2-(4-hydroxy-phenyl)-ethyl]-amide,
2-Propyl-pentanoic acid [1-(cyanoethyl-carbamoyl)-2-(4-hydroxy-phenyl)-ethyl]-amide,
1-Methyl-cyclopropanecarboxylic acid [1-(cyanoethyl-carbamoyl)-2-(4-hydroxy-phenyl)-ethyl]-amide,
Thiophene-2-carboxylic acid [1-(cyanoethyl-carbamoyl)-2-(4-hydroxy-phenyl)-ethyl]-amide,
*N*-[1-(Cyanoethyl-carbamoyl)-2-(4-hydroxy-phenyl)-ethyl]-3-trifluoromethyl-benzamide,
Biphenyl-2-carboxylic acid [1-(cyanoethyl-carbamoyl)-2-(4-hydroxy-phenyl)-ethyl]-amide,
4-Acetylamino-*N*-[1-(cyanoethyl-carbamoyl)-2-(4-hydroxy-phenyl)-ethyl]-benzamide,
3-(4-hydroxy-phenyl)-2-(2-1*H*-indol-3-yl-acetylamino)-propionic acid cyanoethyl-amide,
3-(4-Hydroxy-phenyl)-2-(3-1*H*-indol-3-yl-propionylamino)-propionic acid cyanoethyl-amide,
*N*-[1-(Cyanoethyl-carbamoyl)-2-(4-hydroxy-phenyl)-ethyl]-4-(1*H*-indol-3-yl)-butyramide,
N-[1-(Cyanoethyl-carbamoyl)-2-(4-hydroxy-phenyl)-ethyl]-benzamide,
3-Chloromethyl-*N*-[1-(cyanoethyl-carbamoyl)-2-(4-hydroxy-phenyl)-ethyl]-benzamide,
4-Chloromethyl-*N*-[1-(cyanoethyl-carbamoyl)-2-(4-hydroxy-phenyl)-ethyl]-benzamide,
*N*-[1-(Cyanoethyl-carbamoyl)-2-(4-hydroxy-phenyl)-ethyl]-2-fluoro-benzamide,
*N*-[1-(Cyanoethyl-carbamoyl)-2-(4-hydroxy-phenyl)-ethyl]-2-nitro-benzamide,
*N*-[1-(Cyanoethyl-carbamoyl)-2-(4-hydroxy-phenyl)-ethyl]-acrylamide,
3-(4-Hydroxy-phenyl)-2,2-dimethyl-propionic acid cyanoethyl-amide,

3-(4-Hydroxy-phenyl)-2-(2-methoxy-acetylamino)-propionic acid cyanoethyl-amide,
*N*-[1-(Cyanoethyl-carbamoyl)-2-(4-hydroxy-phenyl)-ethyl]-oxalamic acid methyl ester,
*N*-[1-(Cyanoethyl-carbamoyl)-2-(4-hydroxy-phenyl)-ethyl]-phthalamic acid,
*N*-[1-(Cyanoethyl-carbamoyl)-2-(4-hydroxy-phenyl)-ethyl]-succinamic acid,
3-[1-(Cyanoethyl-carbamoyl)-2-(4-hydroxy-phenyl)-ethylcarbamoyl]-acrylic acid,
[1-(Cyanoethyl-carbamoyl)-2-(4-hydroxy-phenyl)-ethyl]-carbamic acid isobutyl ester,
[1-(Cyanoethyl-carbamoyl)-2-(4-hydroxy-phenyl)-ethyl]-carbamic acid butyl ester,
[1-(Cyanomethyl-carbamoyl)-2-(4-hydroxy-phenyl)-ethyl]-carbamic acid cyanomethyl ester,
[1-(Cyanoethyl-carbamoyl)-2-(4-hydroxy-phenyl)-ethyl]-carbamic acid cyanomethyl ester,
[1-(Cyanomethyl-carbamoyl)-2-(4-hydroxy-phenyl)-ethyl]-carbamic acid but-3-enyl ester,
[1-(Cyanoethyl-carbamoyl)-2-(4-hydroxy-phenyl)-ethyl]-carbamic acid but-3-enyl ester,
[1-(Cyanomethyl-carbamoyl)-2-( 4-hydroxy-phenyl)-ethyl]-carbamic acid 2-isopropyl-5-methyl-cyclohexyl ester,
[1-(Cyanoethyl-carbamoyl)-2-(4-hydroxy-phenyl)-ethyl)-carbamic acid 2-isopropyl-5-methyl-cyclohexyl ester,
[1-(Cyanoethyl-carbamoyl)-2-(4-hydroxy-phenyl)-ethyl]-carbamic acid hexyl ester,
[1-(Cyanoethyl-carbamoyl)-2-(4-hydroxy-phenyl)-ethyl]-carbamic acid *tert*-butyl ester,
[1-(Cyanoethyl-carbamoyl)-2-(4-hydroxy-phenyl)-ethyl]-carbamic acid methyl ester,
[1-(Cyanoethyl-carbamoyl)-2-(4-hydroxy-phenyl)-ethyl]-carbamic acid ethyl ester,
[1-(Cyanoethyl-carbamoyl)-2-(4-hydroxy-phenyl)-ethyl]-carbamic acid 9*H*-fluoren-9-ylmethylester,
3-Phenyl-2-acetylamino-propionic acid cyanomethyl-amide,
3-Phenyl-2-[3-(4-trifluoromethyl-phenyl)-ureido]-propionic acid cyanomethyl-amide,
3-Phenyl-2-[3-(4-trifluoromethylsulfanyl-phenyl)-ureido]-propionic acid cyanomethyl-amide,
3-Phenyl-2-[3-(4-trifluoromethoxy-phenyl)-ureido]-propionic acid cyanomethyl-amide,
3-Phenyl-2-[3-(4-cyano-phenyl)-ureido]-propionic acid cyanomethyl-amide,
3-Phenyl-2-(3-benzyl-ureido)-propionic acid cyanomethyl-amide,
3-Phenyl-2-(3-o-tolyl-ureido)-propionic acid cyanomethyl-amide,
3-Phenyl-2-[3-(*S*)-(1-phenyl-ethyl)-ureido]-propionic acid cyanomethyl-amide,
3-Phenyl-2-[3-(2,6-dimethyl-phenyl)-ureido]-propionic acid cyanomethyl-amide,
3-Phenyl-2-(3-(3-methyl-benzyl)-ureido]-propionic acid cyanomethyl-amide,
3-Phenyl-2-[3-(1,1,3,3-tetramethyl-butyl)-ureido]-propionic acid cyanomethyl-amide,
3-Phenyl-2-(3-indan-5-yl-ureido)-propionic acid cyanomethyl-amide,
3-Phenyl-2-[3-(2-phenyl-cyclopropyl)-ureido]-propionic acid cyanomethyl-amide,
3-Phenyl-2-(3-adamantan-1-yl-ureido)-propionic acid cyanomethyl-amide,
3-Phenyl-2-(3-biphenyl-4-yl-ureido)-propionic acid cyanomethyl-amide,
3-Phenyl-2-[3-(4-phenoxy-phenyl)-ureido]-propionic acid cyanomethyl-amide,
3-Phenyl-2-[3-(4-nitro-phenyl)-ureido]-propionic acid cyanomethyl-amide,
3-Phenyl-2-(3-cyclohexyl-ureido)-propionic acid cyanomethyl-amide,
3-Phenyl-2-(3-benzo[1,3]dioxol-5-yl-ureido)-propionic acid cyanomethyl-amide,
3-Phenyl-2-[3-(2-fluoro-benzyl)-ureido]-propionic acid cyanomethyl-amide,
3-Phenyl-2-[3-(4-methyl-benzyl)-ureido]-propionic acid cyanomethyl-amide,
3-Phenyl-2-(3-phenethyl-ureido) -ureido]-propionic acid cyanomethyl-amide,
3-Phenyl-2-[3-(3,4,5-trimethoxy-phenyl)-ureido]-propionic acid cyanomethyl-amide,
3-Phenyl-2-(3-ethyl-thioureido)-propionic acid cyanomethyl-amide,
3-Phenyl-2-(3-isopropyl-thioureido)-propionic acid cyanomethyl-amide,
3-Phenyl-2-[3-(4-nitro-phenyl)-thioureido]-propionic acid cyanomethyl-amide,
3-Phenyl-2-(3-phenyl-thioureido)-propionic acid cyanomethyl-amide,
3-Phenyl-2-[3-(4-trifluoromethoxy-phenyl)-thioureido]-propionic acid cyanomethyl-amide,
3-Phenyl-2-{3-[4-(2,2,2-trifluoro-ethyl)-phenyl]-thioureido}-propionic acid cyanomethyl-amide,
3-Phenyl-2-[3-(4-methoxy-phenyl)-thioureido]-propionic acid cyanomethyl-amide,
3-Phenyl-2-(7,7-dimethyl-2-oxo-bicyclo[2.2.1]hept-1-ylmethanesulfonylamino)-propionic acid cyanomethyl-amide,
3-Phenyl-2-(thiophene-2-sulfonylamino)-propionic acid cyanomethyl-amide,
3-Phenyl-2-(4-trifluoromethoxy-benzenesulfonylamino)-propionic acid cyanomethyl-amide,
3-Phenyl-2-(4-*tert*-butyl-benzenesulfonylamino)-propionic acid cyanomethyl-amide,
3-Phenyl-2-(4-chloro-benzenesulfonylamino)-propionic acid cyanomethyl-amide,
3-Phenyl-2-(4-methoxy-benzenesulfonylamino)-propionic acid cyanomethyl-amide,
3-Phenyl-2-(quinoline-6-sulfonylamino)-propionic acid cyanomethyl-amide,
3-Phenyl-2-benzenesulfonylamino-propionic acid cyanomethyl-amide,

1-Methyl-1H-indole-2-carboxylic acid [1-(cyanomethyl-carbamoyl)-2-phenyl-ethyl]-amide,
2-Propyl-pentanoic acid [1-(cyanomethyl-carbamoyl)-2-phenyl-ethyl]-amide,
1-Methyl-cyclopropanecarboxylic acid [1-(cyanomethyl-carbamoyl)-2-phenyl-ethyl]-amide,
Thiophene-2-carboxylic acid [1-(cyanomethyl-carbamoyl)-2-phenyl-ethyl]-amide,
*N*-[1-(Cyanomethyl-carbamoyl)-2-phenyl-ethyl]-3-trifluoromethyl-benzamide,
Biphenyl-2-carboxylic acid [1-(cyanomethyl-carbamoyl)-2-phenyl-ethyl]-amide,
4-Acetylamino-*N*-[1-(cyanomethyl-carbamoyl)-2-phenyl-ethyl]-benzamide,
3-Phenyl-2-(2-1*H*-indol-3-yl-acetylamino)-propionic acid cyanomethyl-amide,
3-Phenyl-2-(3-1*H*-indol-3-yl-propionylamino)-propionic acid cyanomethyl-amide,
*N*-[1-(Cyanomethyl-carbamoyl)-2-phenyl-ethyl]-4-(1*H*-indol-3-yl)-butyramide,
N-[ 1-(Cyanomethyl-carbamoyl)-2-phenyl-ethyl]-benzamide,
3-Chloromethyl-*N*-[1-(cyanomethyl-carbamoyl)-2-phenyl-ethyl]-benzamide,
4-Chloromethyl-*N*-[1-(cyanomethyl-carbamoyl)-2-phenyl-ethyl]-benzamide,
*N*-[1-(Cyanomethyl-carbamoyl)-2-phenyl-ethyl]-2-fluoro-benzamide,
*N*-[1-(Cyanomethyl-carbamoyl)-2-phenyl-ethyl]-2-nitro-benzamide,
*N*-[1-(Cyanomethyl-carbamoyl)-2-phenyl-ethyl]-acrylamide,
3-Phenyl-2,2-dimethyl-propionic acid cyanomethyl-amide,
3-Phenyl-2-(2-methoxy-acetylamino)-propionic acid cyanomethyl-amide,
*N*-[1-(Cyanomethyl-carbamoyl)-2-phenyl-ethyl]-oxalamic acid methyl ester,
*N*-[1-(Cyanomethyl-carbamoyl)-2-phenyl-ethyl]-phthalamic acid,
*N*-[1-(Cyanomethyl-carbamoyl)-2-phenyl-ethyl]-succinamic acid,
3-[1-(Cyanomethyl-carbamoyl)-2-phenyl-ethylcarbamoyl]-acrylic acid,
[1-(Cyanomethyl-carbamoyl)-2-phenyl-ethyl]-carbamic acid isobutyl ester,
[1-(Cyanomethyl-carbamoyl)-2-phenyl-ethyl]-carbamic acid butyl ester,
[1-(Cyanomethyl-carbamoyl)-2-phenyl-ethyl]-carbamic acid hexyl ester,
[1-(Cyanomethyl-carbamoyl)-2-phenyl-ethyl]-carbamic acid *tert*-butyl ester,
[1-(Cyanomethyl-carbamoyl)-2-phenyl-ethyl]-carbamic acid methyl ester,
[1-(Cyanomethyl-carbamoyl)-2-phenyl-ethyl]-carbamic acid ethyl ester,
[1-(Cyanomethyl-carbamoyl)-2-phenyl-ethyl]-carbamic acid 9*H*-fluoren-9-ylmethyl ester,
3-Phenyl-2-acetylamino-propionic acid cyanoethyl-amide,
3-Phenyl-2-[3-(4-trifluoromethyl-phenyl)-ureido]-propionic acid cyanoethyl-amide
3-Phenyl-2-[3-(4-trifluoromethylsulfanyl-phenyl)-ureido]-propionic acid cyanoethyl-amide,
3-Phenyl-2-[3-(4-trifluoromethoxy-phenyl)-ureido]-propionic acid cyanoethyl-amide,
3-Phenyl-2-[3-(4-cyano-phenyl)-ureido]-propionic acid cyanoethyl-amide,
3-Phenyl-2-(3-benzyl-ureido)-propionic acid cyanoethyl-amide,
3-Phenyl-2-(3-o-tolyl-ureido)-propionic acid cyanoethyl-amide,
3-Phenyl-2-[3-(*S*)-(1-phenyl-ethyl)-ureido]-propionic acid cyanoethyl-amide,
3-Phenyl-2-[3-(2,6-dimethyl-phenyl)-ureido]-propionic acid cyanoethyl-amide,
3-Phenyl-2-[3-(3-methyl-benzyl)-ureido]-propionic acid cyanoethyl-amide,
3-Phenyl-2-[3-(1,1,3,3-tetramethyl-butyl)-ureido]-propionic acid cyanoethyl-amide,
3-Phenyl-2-(3-indan-5-yl-ureido)-propionic acid cyanoethyl-amide,
3-Phenyl-2-[3-(2-phenyl-cyclopropyl)-ureido]-propionic acid cyanoethyl-amide,
3-Phenyl-2-(3-adamantan-1-yl-ureido)-propionic acid cyanoethyl-amide,
3-Phenyl-2-(3-biphenyl-4-yl-ureido)-propionic acid cyanoethyl-amide,
3-Phenyl-2-[3-(4-phenoxy-phenyl)-ureido]-propionic acid cyanoethyl-amide,
3-Phenyl-2-[3-(4-nitro-phenyl)-ureido]-propionic acid cyanoethyl-amide,
3-Phenyl-2-(3-cyclohexyl-ureido)-propionic acid cyanoethyl-amide,
3-Phenyl-2-(3-benzo[1,3]dioxol-5-yl-ureido)-propionic acid cyanoethyl-amide,
3-Phenyl-2-[3-(2-fluoro-benzyl)-ureido]-propionic acid cyanoethyl-amide,
3-Phenyl-2-[3-(4-methyl-benzyl)-ureido]-propionic acid cyanoethyl-amide,
3-Phenyl-2-(3-phenethyl-ureido) -ureido]-propionic acid cyanoethyl-amide,
3-Phenyl-2-[3-(3,4,5-trimethoxy-phenyl)-ureido]-propionic acid cyanoethyl-amide,
3-Phenyl-2-(3-ethyl-thioureido)-propionic acid cyanoethyl-amide,
3-Phenyl-2-(3-isopropyl-thioureido)-propionic acid cyanoethyl-amide,
3-Phenyl-2-[3-(4-nitro-phenyl)-thioureido]-propionic acid cyanoethyl-amide,
3-Phenyl-2-(3-phenyl-thioureido)-propionic acid cyanoethyl-amide,
3-Phenyl-2-[3-(4-trifluoromethoxy-phenyl)-thioureido]-propionic acid cyanoethyl-amide,
3-Phenyl-2-{3-[4-(2,2,2-trifluoro-ethyl)-phenyl]-thioureido}-propionic acid cyanoethyl-amide,

3-Phenyl-2-[3-(4-methoxy-phenyl)-thioureido]-propionic acid cyanoethyl-amide,
3-Phenyl-2-(7,7-dimethyl-2-oxo-bicyclo[2.2.1]hept-1-ylmethanesulfonylamino)-propionic acid cyanoethyl-amide,
3-Phenyl-2-(thiophene-2-sulfonylamino)-propionic acid cyanoethyl-amide,
3-Phenyl-2-(4-trifluoromethoxy-benzenesulfonylamino)-propionic acid cyanoethyl-amide,
3-Phenyl-2-(4-*tert*-butyl-benzenesulfonylamino)-propionic acid cyanoethyl-amide,
3-Phenyl-2-(4-chloro-benzenesulfonylamino)-propionic acid cyanoethyl-amide,
3-Phenyl-2-(4-methoxy-benzenesulfonylamino)-propionic acid cyanoethyl-amide,
3-Phenyl-2-(quinoline-6-sulfonylamino)-propionic acid cyanoethyl-amide,
3-Phenyl-2-benzenesulfonylamino-propionic acid cyanoethyl-amide,
1-Methyl-1H-indole-2-carboxylic acid [1-(cyanoethyl-carbamoyl)-2-phenyl-ethyl]-amide,
2-Propyl-pentanoic acid [1-(cyanoethyl-carbamoyl)-2-phenyl-ethyl]-amide,
1-Methyl-cyclopropanecarboxylic acid [1-(cyanoethyl-carbamoyl)-2-phenyl-ethyl]-amide,
Thiophene-2-carboxylic acid [1-(cyanoethyl-carbamoyl)-2-phenyl-ethyl]-amide,
*N*-[1-(Cyanoethyl-carbamoyl)-2-phenyl-ethyl]-3-trifluoromethyl-benzamide,
Biphenyl-2-carboxylic acid [1-(cyanoethyl-carbamoyl)-2-phenyl-ethyl]-amide,
4-Acetylamino-*N*-[1-(cyanoethyl-carbamoyl)-2-phenyl-ethyl]-benzamide,
3-Phenyl-2-(2-1*H*-indol-3-yl-acetylamino)-propionic acid cyanoethyl-amide,
3-Phenyl-2-(3-1*H*-indol-3-yl-propionylamino)-propionic acid cyanoethyl-amide,
*N*-[1-(Cyanoethyl-carbamoyl)-2-phenyl-ethyl]-4-(1*H*-indol-3-yl)-butyramide,
N-[1-(Cyanoethyl-carbamoyl)-2-phenyl-ethyl]-benzamide,
3-Chloromethyl-*N*-[1-(cyanoethyl-carbamoyl)-2-phenyl-ethyl]-benzamide,
4-Chloromethyl-*N*-[1-(cyanoethyl-carbamoyl)-2-phenyl-ethyl]-benzamide,
*N*-[1-(Cyanoethyl-carbamoyl)-2-phenyl-ethyl]-2-fluoro-benzamide,
*N*-[1-(Cyanoethyl-carbamoyl)-2-phenyl-ethyl]-2-nitro-benzamide,
*N*-[1-(Cyanoethyl-carbamoyl)-2-phenyl-ethyl]-acrylamide,
3-Phenyl-2,2-dimethyl-propionic acid cyanoethyl-amide,
3-Phenyl-2-(2-methoxy-acetylamino)-propionic acid cyanoethyl-amide,
*N*-[1-(Cyanoethyl-carbamoyl)-2-phenyl-ethyl]-oxalamic acid methyl ester,
*N*-[1-(Cyanoethyl-carbamoyl)-2-phenyl-ethyl]-phthalamic acid,
*N*-[1-(Cyanoethyl-carbamoyl)-2-phenyl-ethyl]-succinamic acid,
3-[1-(Cyanoethyl-carbamoyl)-2-phenyl-ethylcarbamoyl]-acrylic acid,
[1-(Cyanoethyl-carbamoyl)-2-phenyl-ethyl]-carbamic acid isobutyl ester,
[1-(Cyanoethyl-carbamoyl)-2-phenyl-ethyl]-carbamic acid butyl ester,
[1-(Cyanomethyl-carbamoyl)-2-phenyl-ethyl)-carbamic acid cyanomethyl ester,
[1-(Cyanoethyl-carbamoyl)-2-phenyl-ethyl]-carbamic acid cyanomethyl ester,
[1-(Cyanomethyl-carbamoyl)-2-phenyl-ethyl]-carbamic acid but-3-enyl ester,
[1-(Cyanoethyl-carbamoyl)-2-phenyl-ethyl]-carbamic acid but-3-enyl ester,
[1-(Cyanomethyl-carbamoyl)-2-phenyl-ethyl]-carbamic acid 2-isopropyl-5-methyl-cyclohexyl ester,
[1-(Cyanoethyl-carbamoyl)-2-phenyl-ethyl]-carbamic acid 2-isopropyl-5-methyl-cyclohexyl ester,
[1-(Cyanoethyl-carbamoyl)-2-phenyl-ethyl]-carbamic acid hexyl ester,
[1-(Cyanoethyl-carbamoyl)-2-phenyl-ethyl]-carbamic acid tert-butyl ester,
[1-(Cyanoethyl-carbamoyl)-2-phenyl-ethyl]-carbamic acid methyl ester,
[1-(Cyanoethyl-carbamoyl)-2-phenyl-ethyl]-carbamic acid ethyl ester,
[1-(Cyanoethyl-carbamoyl)-2-phenyl-ethyl]-carbamic acid 9H-fluoren-9-ylmethylester,
3-Methylsulfanyl-2-acetylamino-propionic acid cyanomethyl-amide,
3-Methylsulfanyl-2-[3-(4-trifluoromethyl-phenyl)-ureido]-propionic acid cyanomethyl-amide,
3-Methylsulfanyl-2-[3-(4-trifluoromethylsulfanyl-phenyl)-ureido]-propionic acid cyanomethyl-amide,
3-Methylsulfanyl-2-[3-(4-trifluoromethoxy-phenyl)-ureido]-propionic acid cyanomethyl-amide,
3-Methylsulfanyl-2-[3-(4-cyano-phenyl)-ureido]-propionic acid cyanomethyl-amide,
3-Methylsulfanyl-2-(3-benzyl-ureido)-propionic acid cyanomethyl-amide,
3-Methylsulfanyl-2-(3-o-tolyl-ureido)-propionic acid cyanomethyl-amide,
3-Methylsulfanyl-2-[3-(*S*)-(1-phenyl-ethyl)-ureido]-propionic acid cyanomethyl-amide,
3-Methylsulfanyl-2-[3-(2,6-dimethyl-phenyl)-ureido]-propionic acid cyanomethyl-amide,
3-Methylsulfanyl-2-[3-(3-methyl-benzyl)-ureido]-propionic acid cyanomethyl-amide,
3-Methylsulfanyl-2-[3-(1,1,3,3-tetramethyl-butyl)-ureido]-propionic acid cyanomethyl-amide,
3-Methylsulfanyl-2-(3-indan-5-yl-ureido)-propionic acid cyanomethyl-amide,
3-Methylsulfanyl-2-[3-(2-phenyl-cyclopropyl)-ureido]-propionic acid cyanomethyl-amide,

3-Methylsulfanyl-2-(3-adamantan-1-yl-ureido)-propionic acid cyanomethyl-amide,
3-Methylsulfanyl-2-(3-biphenyl-4-yl-ureido)-propionic acid cyanomethyl-amide,
3-Methylsulfanyl-2-[3-(4-phenoxy-phenyl)-ureido]-propionic acid cyanomethyl-amide,
3-Methylsulfanyl-2-[3-(4-nitro-phenyl)-ureido]-propionic acid cyanomethyl-amide,
3-Methylsulfanyl-2-(3-cyclohexyl-ureido)-propionic acid cyanomethyl-amide,
3-Methylsulfanyl-2-(3-benzo[1,3]dioxol-5-yl-ureido)-propionic acid cyanomethyl-amide,
3-Methylsulfanyl-2-[3-(2-fluoro-benzyl)-ureido]-propionic acid cyanomethyl-amide,
3-Methylsulfanyl-2-[3-(4-methyl-benzyl)-ureido]-propionic acid cyanomethyl-amide,
3-Methylsulfanyl-2-(3-phenethyl-ureido) -ureido]-propionic acid cyanomethyl-amide,
3-Methylsulfanyl-2-[3-(3,4,5-trimethoxy-phenyl)-ureido]-propionic acid cyanomethyl-amide,
3-Methylsulfanyl-2-(3-ethyl-thioureido)-propionic acid cyanomethyl-amide,
3-Methylsulfanyl-2-(3-isopropyl-thioureido)-propionic acid cyanomethyl-amide,
3-Methylsulfanyl-2-[3-(4-nitro-phenyl)-thioureido]-propionic acid cyanomethyl-amide,
3-Methylsulfanyl-2-(3-phenyl-thioureido)-propionic acid cyanomethyl-amide,
3-Methylsulfanyl-2-[3-(4-trifluoromethoxy-phenyl)-thioureido]-propionic acid cyanomethyl-amide,
3-Methylsulfanyl-2-{3-[4-(2,2,2-trifluoro-ethyl)-phenyl]-thioureido}-propionic acid cyanomethyl-amide,
3-Methylsulfanyl-2-[3-(4-methoxy-phenyl)-thioureido]-propionic acid cyanomethyl-amide,
3-Methylsulfanyl-2-(7,7-dimethyl-2-oxo-bicyclo[2.2.1]hept-1-ylmethanesulfonylamino)-propionic acid cyanomethyl-amide,
3-Methylsulfanyl-2-(thiophene-2-sulfonylamino)-propionic acid cyanomethyl-amide,
3-Methylsulfanyl-2-(4-trifluoromethoxy-benzenesulfonylamino)-propionic acid cyanomethyl-amide,
3-Methylsulfanyl-2-(4-tert-butyl-benzenesulfonylamino)-propionic acid cyanomethyl-amide,
3-Methylsulfanyl-2-(4-chloro-benzenesulfonylamino)-propionic acid cyanomethyl-amide,
3-Methylsulfanyl-2-(4-methoxy-benzenesulfonylamino)-propionic acid cyanomethyl-amide,
3-Methylsulfanyl-2-(quinoline-6-sulfonylamino)-propionic acid cyanomethyl-amide,
3-Methylsulfanyl-2-benzenesulfonylamino-propionic acid cyanomethyl-amide,
1-Methyl-1H-indole-2-carboxylic acid [1-(cyanomethyl-carbamoyl)-2-methylsulfanyl-ethyl]-amide,
2-Propyl-pentanoic acid [1-(cyanomethyl-carbamoyl)-2-methylsulfanyl-ethyl]-amide,
1-Methyl-cyclopropanecarboxylic acid [1-(cyanomethyl-carbamoyl)-2-methylsulfanyl-ethyl]-amide,
Thiophene-2-carboxylic acid [1-(cyanomethyl-carbamoyl)-2-methylsulfanyl-ethyl]-amide,
*N*-[1-(Cyanomethyl-carbamoyl)-2-methylsulfanyl-ethyl]-3-trifluoromethyl-benzamide,
Biphenyl-2-carboxylic acid [1-(cyanomethyl-carbamoyl)-2-methylsulfanyl-ethyl]-amide,
4-Acetylamino-*N*-[1-(cyanomethyl-carbamoyl)-2-methylsulfanyl-ethyl]-benzamide,
3-Methylsulfanyl-2-(2-1*H*-indol-3-yl-acetylamino)-propionic acid cyanomethyl-amide,
3-Methylsulfanyl-2-(3-1*H*-indol-3-yl-propionylamino)-propionic acid cyanomethyl-amide,
*N*-[1-(Cyanomethyl-carbamoyl)-2-methylsulfanyl-ethyl]-4-(1*H*-indol-3-yl)-butyramide,
N-[1-(Cyanomethyl-carbamoyl)-2-methylsulfanyl-ethyl]-benzamide,
3-Chloromethyl-*N*-[1-(cyanomethyl-carbamoyl)-2-methylsulfanyl-ethyl]-benzamide,
4-Chloromethyl-*N*-[1-(cyanomethyl-carbamoyl)-2-methylsulfanyl-ethyl]-benzamide,
*N*-[1-(Cyanomethyl-carbamoyl)-2-methylsulfanyl-ethyl]-2-fluoro-benzamide,
*N*-[1-(Cyanomethyl-carbamoyl)-2-methylsulfanyl-ethyl]-2-nitro-benzamide,
*N*-[1-(Cyanomethyl-carbamoyl)-2-methylsulfanyl-ethyl]-acrylamide,
3-Methylsulfanyl-2,2-dimethyl-propionic acid cyanomethyl-amide,
3-Methylsulfanyl-2-(2-methoxy-acetylamino)-propionic acid cyanomethyl-amide,
*N*-[1-(Cyanomethyl-carbamoyl)-2-methylsulfanyl-ethyl]-oxalamic acid methyl ester,
*N*-[1-(Cyanomethyl-carbamoyl)-2-methylsulfanyl-ethyl]-phthalamic acid,
*N*-[1-(Cyanomethyl-carbamoyl)-2-methylsulfanyl-ethyl]-succinamic acid,
3-[1-(Cyanomethyl-carbamoyl)-2-methylsulfanyl-ethylcarbamoyl]-acrylic acid,
[1-(Cyanomethyl-carbamoyl)-2-methylsulfanyl-ethyl]-carbamic acid isobutyl ester,
[1-(Cyanomethyl-carbamoyl)-2-methylsulfanyl-ethyl]-carbamic acid butyl ester,
[1-(Cyanomethyl-carbamoyl)-2-methylsulfanyl-ethyl]-carbamic acid hexyl ester,
[1-(Cyanomethyl-carbamoyl)-2-methylsulfanyl-ethyl]-carbamic acid *tert*-butyl ester,
[1-(Cyanomethyl-carbamoyl)-2-methylsulfanyl-ethyl]-carbamic acid methyl ester,
[1-(Cyanomethyl-carbamoyl)-2-methylsulfanyl-ethyl]-carbamic acid ethyl ester,
[1-(Cyanomethyl-carbamoyl)-2-methylsulfanyl-ethyl]-carbamic acid 9*H*-fluoren-9-ylmethyl ester,
3-Methylsulfanyl-2-acetylamino-propionic acid cyanoethyl-amide,
3-Methylsulfanyl-2-[3-(4-trifluoromethyl-phenyl)-ureido]-propionic acid cyanoethyl-amide,
3-Methylsulfanyl-2-[3-(4-trifluoromethylsulfanyl-phenyl)-ureido]-propionic acid cyanoethyl-amide,

3-Methylsulfanyl-2-[3-(4-trifluoromethoxy-phenyl)-ureido]-propionic acid cyanoethyl-amide,
3-Methylsulfanyl-2-[3-(4-cyano-phenyl)-ureido]-propionic acid cyanoethyl-amide,
3-Methylsulfanyl-2-(3-benzyl-ureido)-propionic acid cyanoethyl-amide,
3-Methylsulfanyl-2-(3-o-tolyl-ureido)-propionic acid cyanoethyl-amide,
3-Methylsulfanyl-2-[3-(*S*)-(1-phenyl-ethyl)-ureido]-propionic acid cyanoethyl-amide,
3-Methylsulfanyl-2-[3-(2,6-dimethyl-phenyl)-ureido]-propionic acid cyanoethyl-amide,
3-Methylsulfanyl-2-[3-(3-methyl-benzyl)-ureido]-propionic acid cyanoethyl-amide,
3-Methylsulfanyl-2-[3-(1,1,3,3-tetramethyl-butyl)-ureido]-propionic acid cyanoethyl-amide,
3-Methylsulfanyl-2-(3-indan-5-yl-ureido)-propionic acid cyanoethyl-amide,
3-Methylsulfanyl-2-[3-(2-phenyl-cyclopropyl)-ureido]-propionic acid cyanoethyl-amide,
3-Methylsulfanyl-2-(3-adamantan-1-yl-ureido)-propionic acid cyanoethyl-amide,
3-Methylsulfanyl-2-(3-biphenyl-4-yl-ureido)-propionic acid cyanoethyl-amide,
3-Methylsulfanyl-2-[3-(4-phenoxy-phenyl)-ureido]-propionic acid cyanoethyl-amide,
3-Methylsulfanyl-2-[3-(4-nitro-phenyl)-ureido]-propionic acid cyanoethyl-amide,
3-Methylsulfanyl-2-(3-cyclohexyl-ureido)-propionic acid cyanoethyl-amide,
3-Methylsulfanyl-2-(3-benzo[1,3]dioxol-5-yl-ureido)-propionic acid cyanoethyl-amide,
3-Methylsulfanyl-2-[3-(2-fluoro-benzyl)-ureido]-propionic acid cyanoethyl-amide,
3-Methylsulfanyl-2-[3-(4-methyl-benzyl)-ureido]-propionic acid cyanoethyl-amide,
3-Methylsulfanyl-2-(3-phenethyl-ureido) -ureido]-propionic acid cyanoethyl-amide,
3-Methylsulfanyl-2-[3-(3,4,5-trimethoxy-phenyl)-ureido]-propionic acid cyanoethyl-amide,
3-Methylsulfanyl-2-(3-ethyl-thioureido)-propionic acid cyanoethyl-amide,
3-Methylsulfanyl-2-(3-isopropyl-thioureido)-propionic acid cyanoethyl-amide,
3-Methylsulfanyl-2-[3-(4-nitro-phenyl)-thioureido]-propionic acid cyanoethyl-amide,
3-Methylsulfanyl-2-(3-phenyl-thioureido)-propionic acid cyanoethyl-amide,
3-Methylsulfanyl-2-[3-(4-trifluoromethoxy-phenyl)-thioureido]-propionic acid cyanoethyl-amide,
3-Methylsulfanyl-2-{3-[4-(2,2,2-trifluoro-ethyl)-phenyl]-thioureido}-propionic acid cyanoethyl-amide,
3-Methylsulfanyl-2-[3-(4-methoxy-phenyl)-thioureido]-propionic acid cyanoethyl-amide,
3-Methylsulfanyl-2-(7,7-dimethyl-2-oxo-bicyclo[2.2.1]hept-1-ylmethanesulfonylamino)-propionic acid cyanoethyl-amide,
3-Methylsulfanyl-2-(thiophene-2-sulfonylamino)-propionic acid cyanoethyl-amide,
3-Methylsulfanyl-2-(4-trifluoromethoxy-benzenesulfonylamino)-propionic acid cyanoethyl-amide,
3-Methylsulfanyl-2-(4-tert-butyl-benzenesulfonylamino)-propionic acid cyanoethyl-amide,
3-Methylsulfanyl-2-(4-chloro-benzenesulfonylamino)-propionic acid cyanoethyl-amide,
3-Methylsulfanyl-2-(4-methoxy-benzenesulfonylamino)-propionic acid cyanoethyl-amide,
3-Methylsulfanyl-2-(quinoline-6-sulfonylamino)-propionic acid cyanoethyl-amide,
3-Methylsulfanyl-2-benzenesulfonylamino-propionic acid cyanoethyl-amide,
1-Methyl-1H-indole-2-carboxylic acid [1-(cyanoethyl-carbamoyl)-2-methylsulfanyl-ethyl]-amide,
2-Propyl-pentanoic acid [1-(cyanoethyl-carbamoyl)-2-methylsulfanyl-ethyl]-amide,
1-Methyl-cyclopropanecarboxylic acid [1-(cyanoethyl-carbamoyl)-2-methylsulfanyl-ethyl]-amide,
Thiophene-2-carboxylic acid [1-(cyanoethyl-carbamoyl)-2-methylsulfanyl-ethyl]-amide,
*N*-[1-(Cyanoethyl-carbamoyl)-2-methylsulfanyl-ethyl]-3-trifluoromethyl-benzamide,
Biphenyl-2-carboxylic acid [1-(cyanoethyl-carbamoyl)-2-methylsulfanyl-ethyl]-amide,
4-Acetylamino-*N*-[1-(cyanoethyl-carbamoyl)-2-methylsulfanyl-ethyl]-benzamide,
3-Methylsulfanyl-2-(2-1*H*-indol-3-yl-acetylamino)-propionic acid cyanoethyl-amide,
3-Methylsulfanyl-2-(3-1*H*-indol-3-yl-propionylamino)-propionic acid cyanoethyl-amide,
*N*-[1-(Cyanoethyl-carbamoyl)-2-methylsulfanyl-ethyl]-4-(1*H*-indol-3-yl)-butyramide,
*N*-[1-(Cyanoethyl-carbamoyl)-2-methylsulfanyl-ethyl]-benzamide,
3-Chloromethyl-*N*-[1-(cyanoethyl-carbamoyl)-2-methylsulfanyl-ethyl]-benzamide,
4-Chloromethyl-*N*-[1-(cyanoethyl-carbamoyl)-2-methylsulfanyl-ethyl]-benzamide,
*N*-[1-(Cyanoethyl-carbamoyl)-2-methylsulfanyl-ethyl]-2-fluoro-benzamide,
*N*-[1-(Cyanoethyl-carbamoyl)-2-methylsulfanyl-ethyl]-2-nitro-benzamide,
*N*-[1-(Cyanoethyl-carbamoyl)-2-methylsulfanyl-ethyl]-acrylamide,
3-Methylsulfanyl-2,2-dimethyl-propionic acid cyanoethyl-amide,
3-Methylsulfanyl-2-(2-methoxy-acetylamino)-propionic acid cyanoethyl-amide,
*N*-[1-(Cyanoethyl-carbamoyl)-2-methylsulfanyl-ethyl]-oxalamic acid methyl ester,
*N*-[1-(Cyanoethyl-carbamoyl)-2-methylsulfanyl-ethyl]-phthalamic acid,
*N*-[1-(Cyanoethyl-carbamoyl)-2-methylsulfanyl-ethyl]-succinamic acid,
3-[1-(Cyanoethyl-carbamoyl)-2-methylsulfanyl-ethylcarbamoyl]-acrylic acid,

[1-(Cyanoethyl-carbamoyl)-2-methylsulfanyl-ethyl]-carbamic acid isobutyl ester,
[1-(Cyanoethyl-carbamoyl)-2-methylsulfanyl-ethyl]-carbamic acid butyl ester,
[1-(Cyanomethyl-carbamoyl)-2-methylsulfanyl-ethyl]-carbamic acid cyanomethyl ester,
[1-(Cyanoethyl-carbamoyl)-2-methylsulfanyl-ethyl]-carbamic acid cyanomethyl ester,
[1-(Cyanomethyl-carbamoyl)-2-methylsulfanyl-ethyl]-carbamic acid but-3-enyl ester,
[1-(Cyanoethyl-carbamoyl)-2-methylsulfanyl-ethyl]-carbamic acid but-3-enyl ester,
[1-(Cyanomethyl-carbamoyl)-2-methylsulfanyl-ethyl]-carbamic acid 2-isopropyl-5-methyl-cyclohexyl ester,
[1-(Cyanoethyl-carbamoyl)-2-methylsulfanyl-ethyl]-carbamic acid 2-isopropyl-5-methyl-cyclohexyl ester,
[1-(Cyanoethyl-carbamoyl)-2-methylsulfanyl-ethyl]-carbamic acid hexyl ester,
[1-(Cyanoethyl-carbamoyl)-2-methylsulfanyl-ethyl]-carbamic acid *tert*-butyl ester,
[1-(Cyanoethyl-carbamoyl)-2-methylsulfanyl-ethyl]-carbamic acid methyl ester,
[1-(Cyanoethyl-carbamoyl)-2-methylsulfanyl-ethyl]-carbamic acid ethyl ester,
[1-(Cyanoethyl-carbamoyl)-2-methylsulfanyl-ethyl]-carbamic acid 9*H*-fluoren-9-ylmethylester,
3-Methanesulfonyl-2-acetylamino-propionic acid cyanomethyl-amide,
3-Methanesulfonyl-2-[3-(4-trifluoromethyl-phenyl)-ureido]-propionic acid cyanomethyl-amide,
3-Methanesulfonyl-2-[3-(4-trifluoromethylsulfanyl-phenyl)-ureido]-propionic acid cyanomethyl-amide,
3-Methanesulfonyl-2-[3-(4-trifluoromethoxy-phenyl)-ureido]-propionic acid cyanomethyl-amide,
3-Methanesulfonyl-2-[3-(4-cyano-phenyl)-ureido]-propionic acid cyanomethyl-amide,
3-Methanesulfonyl-2-(3-benzyl-ureido)-propionic acid cyanomethyl-amide,
3-Methanesulfonyl-2-(3-o-tolyl-ureido)-propionic acid cyanomethyl-amide,
3-Methanesulfonyl-2-[3-(*S*)-(1-phenyl-ethyl)-ureido]-propionic acid cyanomethyl-amide,
3-Methanesulfonyl-2-[3-(2,6-dimethyl-phenyl)-ureido]-propionic acid cyanomethyl-amide,
3-Methanesulfonyl-2-[3-(3-methyl-benzyl)-ureido]-propionic acid cyanomethyl-amide,
3-Methanesulfonyl-2-[3-(1,1,3,3-tetrainethyl-butyl)-ureido]-propionic acid cyanomethyl-amide,
3-Methanesulfonyl-2-(3-indan-5-yl-ureido)-propionic acid cyanomethyl-amide,
3-Methanesulfonyl-2-[3-(2-phenyl-cyclopropyl)-ureido]-propionic acid cyanomethyl-amide,
3-Methanesulfonyl-2-(3-adamantan-1-yl-ureido)-propionic acid cyanomethyl-amide,
3-Methanesulfonyl-2-(3-biphenyl-4-yl-ureido)-propionic acid cyanomethyl-amide,
3-Methanesulfonyl-2-[3-(4-phenoxy-phenyl)-ureido]-propionic acid cyanomethyl-amide,
3-Methanesulfonyl-2-[3-(4-nitro-phenyl)-ureido]-propionic acid cyanomethyl-amide,
3-Methanesulfonyl-2-(3-cyclohexyl-ureido)-propionic acid cyanomethyl-amide,
3-Methanesulfonyl-2-(3-benzo[1,3]dioxol-5-yl-ureido)-propionic acid cyanomethyl-amide,
3-Methanesulfonyl-2-[3-(2-fluoro-benzyl)-ureido]-propionic acid cyanomethyl-amide,
3-Methanesulfonyl-2-[3-(4-methyl-benzyl)-ureido]-propionic acid cyanomethyl-amide,
3-Methanesulfonyl-2-(3-phenethyl-ureido) -ureido]-propionic acid cyanomethyl-amide,
3-Methanesulfonyl-2-[3-(3,4,5-trimethoxy-phenyl)-ureido]-propionic acid cyanomethyl-amide,
3-Methanesulfonyl-2-(3-ethyl-thioureido)-propionic acid cyanomethyl-amide,
3-Methanesulfonyl-2-(3-isopropyl-thioureido)-propionic acid cyanomethyl-amide,
3-Methanesulfonyl-2-[3-(4-nitro-phenyl)-thioureido]-propionic acid cyanomethyl-amide,
3-Methanesulfonyl-2-(3-phenyl-thioureido)-propionic acid cyanomethyl-amide,
3-Methanesulfonyl-2-[3-(4-trifluoromethoxy-phenyl)-thioureido]-propionic acid cyanomethyl-amide,
3-Methanesulfonyl-2-{3-[4-(2,2,2-trifluoro-ethyl)-phenyl]-thioureido}-propionic acid cyanomethyl-amide,
3-Methanesulfonyl-2-[3-(4-methoxy-phenyl)-thioureido]-propionic acid cyanomethyl-amide,
3-Methanesulfonyl-2-(7,7-dimethyl-2-oxo-bicyclo[2.2.1]hept-1-ylmethanesulfonylamino)-propionic acid cyanomethyl-amide,
3-Methanesulfonyl-2-(thiophene-2-sulfonylamino)-propionic acid cyanomethyl-amide,
3-Methanesulfonyl-2-(4-trifluoromethoxy-benzenesulfonylamino)-propionic acid cyanomethyl-amide,
3-Methanesulfonyl-2-(4-*tert*-butyl-benzenesulfonylamino)-propionic acid cyanomethyl-amide,
3-Methanesulfonyl-2-(4-chloro-benzenesulfonylamino)-propionic acid cyanomethyl-amide,
3-Methanesulfonyl-2-(4-methoxy-benzenesulfonylamino)-propionic acid cyanomethyl-amide, 3-Methanesulfonyl-2-(quinoline-6-sulfonylamino)-propionic acid cyanomethyl-amide,
3-Methanesulfonyl-2-benzenesulfonylamino-propionic acid cyanomethyl-amide,
1-Methyl-1H-indole-2-carboxylic acid [1-(cyanomethyl-carbamoyl)-2-methanesulfonyl-ethyl]-amide,
2-Propyl-pentanoic acid [1-(cyanomethyl-carbamoyl)-2-methanesulfonyl-ethyl]-amide,
1-Methyl-cyclopropanecarboxylic acid [1-(cyanomethyl-carbamoyl)-2-methanesulfonyl-ethyl]-amide,
Thiophene-2-carboxylic acid [1-(cyanomethyl-carbamoyl)-2-methanesulfonyl-ethyl]-amide,
*N*-[1-(Cyanomethyl-carbamoyl)-2-methanesulfonyl-ethyl]-3-trifluoromethyl-benzamide,
Biphenyl-2-carboxylic acid [1-(cyanomethyl-carbamoyl)-2-methanesulfonyl-ethyl]-amide,

4-Acetylamino-*N*-[1-(cyanomethyl-carbamoyl)-2-methanesulfonyl-ethyl]-benzamide,
3-Methanesulfonyl-2-(2-1*H*-indol-3-yl-acetylamino)-propionic acid cyanomethyl-amide,
3-Methanesulfonyl-2-(3-1*H*-indol-3-yl-propionylamino)-propionic acid cyanomethyl-amide,
*N*-[1-(Cyanomethyl-carbamoyl)-2-methanesulfonyl-ethyl]-4-(1*H*-indol-3-yl)-butyramide,
N-[1-(Cyanomethyl-carbamoyl)-2-methanesulfonyl-ethyl]-benzamide,
3-Chloromethyl-*N*-[1-(cyanomethyl-carbamoyl)-2-methanesulfonyl-ethyl]-benzamide,
4-Chloromethyl-*N*-[1-(cyanomethyl-carbamoyl)-2-methanesulfonyl-ethyl]-benzamide,
*N*-[1-(Cyanomethyl-carbamoyl)-2-methanesulfonyl-ethyl]-2-fluoro-benzamide,
*N*-[1-(Cyanomethyl-carbamoyl)-2-methanesulfonyl-ethyl]-2-nitro-benzamide,
*N*-[1-(Cyanomethyl-carbamoyl)-2-methanesulfonyl-ethyl]-acrylamide,
3-Methanesulfonyl-2,2-dimethyl-propionic acid cyanomethyl-amide,
3-Methanesulfonyl-2-(2-methoxy-acetylamino)-propionic acid cyanomethyl-amide,
*N*-[1-(Cyanomethyl-carbamoyl)-2-methanesulfonyl-ethyl]-oxalamic acid methyl ester,
*N*-[1-(Cyanomethyl-carbamoyl)-2-methanesulfonyl-ethyl]-phthalamic acid,
*N*-[1-(Cyanomethyl-carbamoyl)-2-methanesulfonyl-ethyl]-succinamic acid,
3-[1-(Cyanomethyl-carbamoyl)-2-methanesulfonyl-ethylcarbamoyl]-acrylic acid,
[1-(Cyanomethyl-carbamoyl)-2-methanesulfonyl-ethyl]-carbamic acid isobutyl ester,
[1-(Cyanomethyl-carbamoyl)-2-methanesulfonyl-ethyl]-carbamic acid butyl ester,
[1-(Cyanomethyl-carbamoyl)-2-methanesulfonyl-ethyl]-carbamic acid hexyl ester,
[1-(Cyanomethyl-carbamoyl)-2-methanesulfonyl-ethyl]-carbamic acid tert-butyl ester,
[1-(Cyanomethyl-carbamoyl)-2-methanesulfonyl-ethyl]-carbamic acid methyl ester,
[1-(Cyanomethyl-carbamoyl)-2-methanesulfonyl-ethyl]-carbamic acid ethyl ester,
[1-(Cyanomethyl-carbamoyl)-2-methanesulfonyl-ethyl]-carbamic acid 9H-fluoren-9-ylmethyl ester,
3-Methanesulfonyl-2-acetylamino-propionic acid cyanoethyl-amide,
3-Methanesulfonyl-2-[3-(4-trifluoromethyl-phenyl)-ureido]-propionic acid cyanoethyl-amide,
3-Methanesulfonyl-2-[3-(4-trifluoromethylsulfanyl-phenyl)-ureido]-propionic acid cyanoethyl-amide,
3-Methanesulfonyl-2-[3-(4-trifluoromethoxy-phenyl)-ureido]-propionic acid cyanoethyl-amide,
3-Methanesulfonyl-2-[3-(4-cyano-phenyl)-ureido]-propionic acid cyanoethyl-amide,
3-Methanesulfonyl-2-(3-benzyl-ureido)-propionic acid cyanoethyl-amide,
3-Methanesulfonyl-2-(3-o-tolyl-ureido)-propionic acid cyanoethyl-amide,
3-Methanesulfonyl-2-[3-(*S*)-(1-phenyl-ethyl)-ureido]-propionic acid cyanoethyl-amide,
3-Methanesulfonyl-2-[3-(2,6-dimethyl-phenyl)-ureido]-propionic acid cyanoethyl-amide,
3-Methanesulfonyl-2-[3-(3-methyl-benzyl)-ureido]-propionic acid cyanoethyl-amide,
3-Methanesulfonyl-2-[3-(1,1,3,3-tetramethyl-butyl)-ureido]-propionic acid cyanoethyl-amide,
3-Methanesulfonyl-2-(3-indan-5-yl-ureido)-propionic acid cyanoethyl-amide,
3-Methanesulfonyl-2-[3-(2-phenyl-cyclopropyl)-ureido]-propionic acid cyanoethyl-amide,
3-Methanesulfonyl-2-(3-adamantan-1-yl-ureido)-propionic acid cyanoethyl-amide,
3-Methanesulfonyl-2-(3-biphenyl-4-yl-ureido)-propionic acid cyanoethyl-amide,
3-Methanesulfonyl-2-[3-(4-phenoxy-phenyl)-ureido]-propionic acid cyanoethyl-amide,
3-Methanesulfonyl-2-[3-(4-nitro-phenyl)-ureido]-propionic acid cyanoethyl-amide,
3-Methanesulfonyl-2-(3-cyclohexyl-ureido)-propionic acid cyanoethyl-amide,
3-Methanesulfonyl-2-(3-benzo[1,3]dioxol-5-yl-ureido)-propionic acid cyanoethyl-amide,
3-Methanesulfonyl-2-[3-(2-fluoro-benzyl)-ureido]-propionic acid cyanoethyl-amide,
3-Methanesulfonyl-2-[3-(4-methyl-benzyl)-ureido]-propionic acid cyanoethyl-amide,
3-Methanesulfonyl-2-(3-phenethyl-ureido) -ureido]-propionic acid cyanoethyl-amide,
3-Methanesulfonyl-2-[3-(3,4,5-trimethoxy-phenyl)-ureido)-propionic acid cyanoethyl-amide,
3-Methanesulfonyl-2-(3-ethyl-thioureido)-propionic acid cyanoethyl-amide,
3-Methanesulfonyl-2-(3-isopropyl-thioureido)-propionic acid cyanoethyl-amide,
3-Methanesulfonyl-2-[3-(4-nitro-phenyl)-thioureido]-propionic acid cyanoethyl-amide,
3-Methanesulfonyl-2-(3-phenyl-thioureido)-propionic acid cyanoethyl-amide,
3-Methanesulfonyl-2-[3-(4-trifluoromethoxy-phenyl)-thioureido]-propionic acid cyanoethyl-amide,
3-Methanesulfonyl-2-{3-[4-(2,2,2-trifluoro-ethyl)-phenyl]-thioureido} -propionic acid cyanoethyl-amide,
3-Methanesulfonyl-2-[3-(4-methoxy-phenyl)-thioureido]-propionic acid cyanoethyl-amide,
3-Methanesulfonyl-2-(7,7-dimethyl-2-oxo-bicyclo[2.2.11]hept-1-ylmethanesulfonylamino)-propionic acid cyanoethyl-amide,
3-Methanesulfonyl-2-(thiophene-2-sulfonylamino)-propionic acid cyanoethyl-amide,
3-Methanesulfonyl-2-(4-trifluoromethoxy-benzenesulfonylamino)-propionic acid cyanoethyl-amide,
3-Methanesulfonyl-2-(4-*tert*-butyl-benzenesulfonylamino)-propionic acid cyanoethyl-amide,

3-Methanesulfonyl-2-(4-chloro-benzenesulfonylamino)-propionic acid cyanoethyl-amide,
3-Methanesulfonyl-2-(4-methoxy-benzenesulfonylamino)-propionic acid cyanoethyl-amide,
3-Methanesulfonyl-2-(quinoline-6-sulfonylamino)-propionic acid cyanoethyl-amide,
3-Methanesulfonyl-2-benzenesulfonylamino-propionic acid cyanoethyl-amide,
1-Methyl-1H-indole-2-carboxylic acid [1-(cyanoethyl-carbamoyl)-2-methanesulfonyl-ethyl]-amide,
2-Propyl-pentanoic acid [1-(cyanoethyl-carbamoyl)-2-methanesulfonyl-ethyl]-amide,
1-Methyl-cyclopropanecarboxylic acid [1-(cyanoethyl-carbamoyl)-2-methanesulfonyl-ethyl]-amide,
Thiophene-2-carboxylic acid [1-(cyanoethyl-carbamoyl)-2-methanesulfonyl-ethyl]-amide,
*N*-[1-(Cyanoethyl-carbamoyl)-2-methanesulfonyl-ethyl]-3-trifluoromethyl-benzamide,
Biphenyl-2-carboxylic acid [1-(cyanoethyl-carbamoyl)-2-methanesulfonyl-ethyl]-amide,
4-Acetylamino-*N*-[1-(cyanoethyl-carbamoyl)-2-methanesulfonyl-ethyl]-benzamide,
3-Methanesulfonyl-2-(2-1*H*-indol-3-yl-acetylamino)-propionic acid cyanoethyl-amide,
3-Methanesulfonyl-2-(3-1*H*-indol-3-yl-propionylamino)-propionic acid cyanoethyl-amide,
*N*-[1-(Cyanoethyl-carbamoyl)-2-methanesulfonyl-ethyl]-4-(1*H*-indol-3-yl)-butyramide,
N-[1-(Cyanoethyl-carbamoyl)-2-methanesulfonyl-ethyl]-benzamide,
3-Chloromethyl-*N*-[1-(cyanoethyl-carbamoyl)-2-methanesulfonyl-ethyl]-benzamide,
4-Chloromethyl-*N*-[1-(cyanoethyl-carbamoyl)-2-methanesulfonyl-ethyl]-benzamide,
*N*-[1-(Cyanoethyl-carbamoyl)-2-methanesulfonyl-ethyl]-2-fluoro-benzamide,
*N*-[1-(Cyanoethyl-carbamoyl)-2-methanesulfonyl-ethyl]-2-nitro-benzamide,
*N*-[1-(Cyanoethyl-carbamoyl)-2-methanesulfonyl-ethyl]-acrylamide,
3-Methanesulfonyl-2,2-dimethyl-propionic acid cyanoethyl-amide,
3-Methanesulfonyl-2-(2-methoxy-acetylamino)-propionic acid cyanoethyl-amide,
*N*-[1-(Cyanoethyl-carbamoyl)-2-methanesulfonyl-ethyl]-oxalamic acid methyl ester,
*N*-[1-(Cyanoethyl-carbamoyl)-2-methanesulfonyl-ethyl]-phthalamic acid,
*N*-[1-(Cyanoethyl-carbamoyl)-2-methanesulfonyl-ethyl]-succinamic acid,
3-[1-(Cyanoethyl-carbamoyl)-2-methanesulfonyl-ethylcarbamoyl]-acrylic acid,
[1-(Cyanoethyl-carbamoyl)-2-methanesulfonyl-ethyl]-carbamic acid isobutyl ester,
[1-(Cyanoethyl-carbamoyl)-2-methanesulfonyl-ethyl]-carbamic acid butyl ester,
[1-(Cyanomethyl-carbamoyl)-2-methanesulfonyl-ethyl]-carbamic acid cyanomethyl ester,
[1-(Cyanoethyl-carbamoyl)-2-methanesulfonyl-ethyl]-carbamic acid cyanomethyl ester,
[1-(Cyanomethyl-carbamoyl)-2-methanesulfonyl-ethyl]-carbamic acid but-3-enyl ester,
[1-(Cyanoethyl-carbamoyl)-2-methanesulfonyl-ethyl]-carbamic acid but-3-enyl ester,
[1-(Cyanomethyl-carbamoyl)-2-methanesulfonyl-ethyl]-carbamic acid 2-isopropyl-5-methyl-cyclohexyl ester,
[1-(Cyanoethyl-carbamoyl)-2-methanesulfonyl-ethyl]-carbamic acid 2-isopropyl-5-methyl-cyclohexyl ester,
[1-(Cyanoethyl-carbamoyl)-2-methanesulfonyl-ethyl]-carbamic acid hexyl ester,
[1-(Cyanoethyl-carbamoyl)-2-methanesulfonyl-ethyl]-carbamic acid *tert*-butyl ester,
[1-(Cyanoethyl-carbamoyl)-2-methanesulfonyl-ethyl]-carbamic acid methyl ester,
[1-(Cyanoethyl-carbamoyl)-2-methanesulfonyl-ethyl]-carbamic acid ethyl ester,
[1-(Cyanoethyl-carbamoyl)-2-methanesulfonyl-ethyl]-carbamic acid 9*H*-fluoren-9-ylmethylester,
3-Naphthalen-2-yl-2-acetylamino-propionic acid cyanomethyl-amide,
3-Naphthalen-2-yl-2-[3-(4-trifluoromethyl-phenyl)-ureido]-propionic acid cyanomethyl-amide,
3-Naphthalen-2-yl-2-[3-(4-trifluoromethylsulfanyl-phenyl)-ureido]-propionic acid cyanomethyl-amide,
3-Naphthalen-2-yl-2-[3-(4-trifluoromethoxy-phenyl)-ureido]-propionic acid cyanomethyl-amide,
3-Naphthalen-2-yl-2-[3-(4-cyano-phenyl)-ureido]-propionic acid cyanomethyl-amide,
3-Naphthalen-2-yl-2-(3-benzyl-ureido)-propionic acid cyanomethyl-amide,
3-Naphthalen-2-yl-2-(3-o-tolyl-ureido)-propionic acid cyanomethyl-amide,
3-Naphthalen-2-yl-2-[3-(*S*)-(1-phenyl-ethyl)-ureido]-propionic acid cyanomethyl-amide,
3-Naphthalen-2-yl-2-[3-(2,6-dimethyl-phenyl)-ureido]-propionic acid cyanomethyl-amide,
3-Naphthalen-2-yl-2-[3-(3-methyl-benzyl)-ureido]-propionic acid cyanomethyl-amide,
3-Naphthalen-2-yl-2-[3-(1,1,3,3-tetramethyl-butyl)-ureido]-propionic acid cyanomethyl-amide,
3-Naphthalen-2-yl-2-(3-indan-5-yl-ureido)-propionic acid cyanomethyl-amide,
3-Naphthalen-2-yl-2-[3-(2-phenyl-cyclopropyl)-ureido]-propionic acid cyanomethyl-amide,
3-Naphthalen-2-yl-2-(3-adamantan-1-yl-ureido)-propionic acid cyanomethyl-amide,
3-Naphthalen-2-yl-2-(3-biphenyl-4-yl-ureido)-propionic acid cyanomethyl-amide,
3-Naphthalen-2-yl-2-[3-(4-phenoxy-phenyl)-ureido]-propionic acid cyanomethyl-amide,
3-Naphthalen-2-yl-2-[3-(4-nitro-phenyl)-ureido]-propionic acid cyanomethyl-amide,
3-Naphthalen-2-yl-2-(3-cyclohexyl-ureido)-propionic acid cyanomethyl-amide,
3-Naphthalen-2-yl-2-(3-benzo[1,3]dioxol-5-yl-ureido)-propionic acid cyanomethyl-amide,

3-Naphthalen-2-yl-2-[3-(2-fluoro-benzyl)-ureido]-propionic acid cyanomethyl-amide,
3-Naphthalen-2-yl-2-[3-(4-methyl-benzyl)-ureido]-propionic acid cyanomethyl-amide,
3-Naphthalen-2-yl-2-(3-phenethyl-ureido) -ureido]-propionic acid cyanomethyl-amide,
3-Naphthalen-2-yl-2-[3-(3,4,5-trimethoxy-phenyl)-ureido]-propionic acid cyanomethyl-amide,
3-Naphthalen-2-yl-2-(3-ethyl-thioureido)-propionic acid cyanomethyl-amide,
3-Naphthalen-2-yl-2-(3-isopropyl-thioureido)-propionic acid cyanomethyl-amide,
3-Naphthalen-2-yl-2-[3-(4-nitro-phenyl)-thioureido]-propionic acid cyanomethyl-amide,
3-Naphthalen-2-yl-2-(3-phenyl-thioureido)-propionic acid cyanomethyl-amide,
3-Naphthalen-2-yl-2-[3-(4-trifluoromethoxy-phenyl)-thioureido]-propionic acid cyanomethyl-amide,
3-Naphthalen-2-yl-2-{3-[4-(2,2,2-trifluoro-ethyl)-phenyl]-thioureido}-propionic acid cyanomethyl-amide,
3-Naphthalen-2-yl-2-[3-(4-methoxy-phenyl)-thioureido]-propionic acid cyanomethyl-amide,
3-Naphthalen-2-yl-2-(7,7-dimethyl-2-oxo-bicyclo[2.2.1]hept-1-ylmethanesulfonylamino)-propionic acid cyanomethyl-amide,
3-Naphthalen-2-yl-2-(thiophene-2-sulfonylamino)-propionic acid cyanomethyl-amide,
3-Naphthalen-2-yl-2-(4-trifluoromethoxy-benzenesulfonylamino)-propionic acid cyanomethyl-amide,
3-Naphthalen-2-yl-2-(4-*tert*-butyl-benzenesulfonylamino)-propionic acid cyanomethyl-amide,
3-Naphthalen-2-yl-2-(4-chloro-benzenesulfonylamino)-propionic acid cyanomethyl-amide,
3-Naphthalen-2-yl-2-(4-methoxy-benzenesulfonylamino)-propionic acid cyanomethyl-amide,
3-Naphthalen-2-yl-2-(quinoline-6-sulfonylamino)-propionic acid cyanomethyl-amide,
3-Naphthalen-2-yl-2-benzenesulfonylamino-propionic acid cyanomethyl-amide,
1-Methyl-1H-indole-2-carboxylic acid [1-(cyanomethyl-carbamoyl)-2-naphthalen-2-yl-ethyl]-amide,
2-Propyl-pentanoic acid [1-(cyanomethyl-carbamoyl)-2-naphthalen-2-yl-ethyl]-amide,
1-Methyl-cyclopropanecarboxylic acid [1-(cyanomethyl-carbamoyl)-2-naphthalen-2-yl-ethyl]-amide,
Thiophene-2-carboxylic acid [1-(cyanomethyl-carbamoyl)-2-naphthalen-2-yl-ethyl]-amide,
*N*-[1-(Cyanomethyl-carbamoyl)-2-naphthalen-2-yl-ethyl]-3-trifluoromethyl-benzamide,
Biphenyl-2-carboxylic acid [1-(cyanomethyl-carbamoyl)-2-naphthalen-2-yl-ethyl]-amide,
4-Acetylamino-*N*-[1-(cyanomethyl-carbamoyl)-2-naphthalen-2-yl-ethyl]-benzamide,
3-Naphthalen-2-yl-2-(2-1*H*-indol-3-yl-acetylamino)-propionic acid cyanomethyl-amide,
3-Naphthalen-2-yl-2-(3-1*H*-indol-3-yl-propionylamino)-propionic acid cyanomethyl-amide,
*N*-[1-(Cyanomethyl-carbamoyl)-2-naphthalen-2-yl-ethyl]-4-(1*H*-indol-3-yl)-butyramide,
N-[1-(Cyanomethyl-carbamoyl)-2-naphthalen-2-yl-ethyl]-benzamide,
3-Chloromethyl-*N*-[1-(cyanomethyl-carbamoyl)-2-naphthalen-2-yl-ethyl]-benzamide,
4-Chloromethyl-*N*-[1-(cyanomethyl-carbamoyl)-2-naphthalen-2-yl-ethyl]-benzamide,
*N*-[1-(Cyanomethyl-carbamoyl)-2-naphthalen-2-yl-ethyl]-2-fluoro-benzamide,
*N*-[1-(Cyanomethyl-carbamoyl)-2-naphthalen-2-yl-ethyl]-2-nitro-benzamide,
*N*-[1-(Cyanomethyl-carbamoyl)-2-naphthalen-2-yl-ethyl]-acrylamide,
3-Naphthalen-2-yl-2,2-dimethyl-propionic acid cyanomethyl-amide,
3-Naphthalen-2-yl-2-(2-methoxy-acetylamino)-propionic acid cyanomethyl-amide,
*N*-[1-(Cyanomethyl-carbamoyl)-2-naphthalen-2-yl-ethyl]-oxalamic acid methyl ester,
*N*-[1-(Cyanomethyl-carbamoyl)-2-naphthalen-2-yl-ethyl]-phthalamic acid,
*N*-[1-(Cyanomethyl-carbamoyl)-2-naphthalen-2-yl-ethyl]-succinamic acid,
3-[1-(Cyanomethyl-carbamoyl)-2-naphthalen-2-yl-ethylcarbamoyl]-acrylic acid,
[1-(Cyanomethyl-carbamoyl)-2-naphthalen-2-yl-ethyl]-carbamic acid isobutyl ester,
[1-(Cyanomethyl-carbamoyl)-2-naphthalen-2-yl-ethyl]-carbamic acid butyl ester,
[1-(Cyanomethyl-carbamoyl)-2-naphthalen-2-yl-ethyl]-carbamic acid hexyl ester,
[1-(Cyanomethyl-carbamoyl)-2-naphthalen-2-yl-ethyl]-carbamic acid *tert*-butyl ester,
[1-(Cyanomethyl-carbamoyl)-2-naphthalen-2-yl-ethyl]-carbamic acid methyl ester,
[1-(Cyanomethyl-carbamoyl)-2-naphthalen-2-yl-ethyl]-carbamic acid ethyl ester,
[1-(Cyanomethyl-carbamoyl)-2-naphthalen-2-yl-ethyl]-carbamic acid 9*H*-fluoren-9-ylmethyl ester,
3-Naphthalen-2-yl-2-acetylamino-propionic acid cyanoethyl-amide,
3-Naphthalen-2-yl-2-[3-(4-trifluoromethyl-phenyl)-ureido]-propionic acid cyanoethyl-amide,
3-Naphthalen-2-yl-2-[3-(4-trifluoromethylsulfanyl-phenyl)-ureido]-propionic acid cyanoethyl-amide,
3-Naphthalen-2-yl-2-[3-(4-trifluoromethoxy-phenyl)-ureido]-propionic acid cyanoethyl-amide,
3-Naphthalen-2-yl-2-[3-(4-cyano-phenyl)-ureido]-propionic acid cyanoethyl-amide,
3-Naphthalen-2-yl-2-(3-benzyl-ureido)-propionic acid cyanoethyl-amide,
3-Naphthalen-2-yl-2-(3-o-tolyl-ureido)-propionic acid cyanoethyl-amide,
3-Naphthalen-2-yl-2-[3-(*S*)-(1-phenyl-ethyl)-ureido]-propionic acid cyanoethyl-amide,
3-Naphthalen-2-yl-2-[3-(2,6-dimethyl-phenyl)-ureido]-propionic acid cyanoethyl-amide,

3-Naphthalen-2-yl-2-[3-(3-methyl-benzyl)-ureido]-propionic acid cyanoethyl-amide,
3-Naphthalen-2-yl-2-[3-(1,1,3,3-tetramethyl-butyl)-ureido]-propionic acid cyanoethyl-amide,
3-Naphthalen-2-yl-2-(3-indan-5-yl-ureido)-propionic acid cyanoethyl-amide,
3-Naphthalen-2-yl-2-[3-(2-phenyl-cyclopropyl)-ureido]-propionic acid cyanoethyl-amide,
3-Naphthalen-2-yl-2-(3-adamantan-1-yl-ureido)-propionic acid cyanoethyl-amide,
3-Naphthalen-2-yl-2-(3-biphenyl-4-yl-ureido)-propionic acid cyanoethyl-amide,
3-Naphthalen-2-yl-2-[3-(4-phenoxy-phenyl)-ureido]-propionic acid cyanoethyl-amide,
3-Naphthalen-2-yl-2-[3-(4-nitro-phenyl)-ureido]-propionic acid cyanoethyl-amide,
3-Naphthalen-2-yl-2-(3-cyclohexyl-ureido)-propionic acid cyanoethyl-amide,
3-Naphthalen-2-yl-2-(3-benzo[1,3]dioxol-5-yl-ureido)-propionic acid cyanoethyl-amide,
3-Naphthalen-2-yl-2-[3-(2-fluoro-benzyl)-ureido]-propionic acid cyanoethyl-amide,
3-Naphthalen-2-yl-2-[3-(4-methyl-benzyl)-ureido]-propionic acid cyanoethyl-amide,
3-Naphthalen-2-yl-2-(3-phenethyl-ureido) -ureido]-propionic acid cyanoethyl-amide,
3-Naphthalen-2-yl-2-[3-(3,4,5-trimethoxy-phenyl)-ureido]-propionic acid cyanoethyl-amide,
3-Naphthalen-2-yl-2-(3-ethyl-thioureido)-propionic acid cyanoethyl-amide,
3-Naphthalen-2-yl-2-(3-isopropyl-thioureido)-propionic acid cyanoethyl-amide,
3-Naphthalen-2-yl-2-[3-(4-nitro-phenyl)-thioureido]-propionic acid cyanoethyl-amide,
3-Naphthalen-2-yl-2-(3-phenyl-thioureido)-propionic acid cyanoethyl-amide,
3-Naphthalen-2-yl-2-[3-(4-trifluoromethoxy-phenyl)-thioureido]-propionic acid cyanoethyl-amide,
3-Naphthalen-2-yl-2-{3-[4-(2,2,2-trifluoro-ethyl)-phenyl]-thioureido}-propionic acid cyanoethyl-amide,
3-Naphthalen-2-yl-2-[3-(4-methoxy-phenyl)-thioureido]-propionic acid cyanoethyl-amide,
3-Naphthalen-2-yl-2-(7,7-dimethyl-2-oxo-bicyclo[2.2.1]hept-1-ylmethanesulfonylamino)-propionic acid cyanoethyl-amide,
3-Naphthalen-2-yl-2-(thiophene-2-sulfonylamino)-propionic acid cyanoethyl-amide,
3-Naphthalen-2-yl-2-(4-trifluoromethoxy-benzenesulfonylamino)-propionic acid cyanoethyl-amide,
3-Naphthalen-2-yl-2-(4-*tert*-butyl-benzenesulfonylamino)-propionic acid cyanoethyl-amide,
3-Naphthalen-2-yl-2-(4-chloro-benzenesulfonylamino)-propionic acid cyanoethyl-amide,
3-Naphthalen-2-yl-2-(4-methoxy-benzenesulfonylamino)-propionic acid cyanoethyl-amide,
3-Naphthalen-2-yl-2-(quinoline-6-sulfonylamino)-propionic acid cyanoethyl-amide,
3-Naphthalen-2-yl-2-benzenesulfonylamino-propionic acid cyanoethyl-amide,
1-Methyl-1H-indole-2-carboxylic acid [1-(cyanoethyl-carbamoyl)-2-naphthalen-2-yl-ethyl]-amide,
2-Propyl-pentanoic acid [1-(cyanoethyl-carbamoyl)-2-naphthalen-2-yl-ethyl]-amide,
1-Methyl-cyclopropanecarboxylic acid [1-(cyanoethyl-carbamoyl)-2-naphthalen-2-yl-ethyl]-amide,
Thiophene-2-carboxylic acid [1-(cyanoethyl-carbamoyl)-2-naphthalen-2-yl-ethyl]-amide,
*N*-[1-(Cyanoethyl-carbamoyl)-2-naphthalen-2-yl-ethyl]-3-trifluoromethyl-benzamide,
Biphenyl-2-carboxylic acid [1-(cyanoethyl-carbamoyl)-2-naphthalen-2-yl-ethyl]-amide,
4-Acetylamino-*N*-[1-(cyanoethyl-carbamoyl)-2-naphthalen-2-yl-ethyl]-benzamide,
3-Naphthalen-2-yl-2-(2-1*H*-indol-3-yl-acetylamino)-propionic acid cyanoethyl-amide,
3-Naphthalen-2-yl-2-(3-1*H*-indol-3-yl-propionylamino)-propionic acid cyanoethyl-amide,
*N*-[1-(Cyanoethyl-carbamoyl)-2-naphthalen-2-yl-ethyl]-4-(1*H*-indol-3-yl)-butyramide,
N-[1-(Cyanoethyl-carbamoyl)-2-naphthalen-2-yl-ethyl]-benzamide,
3-Chloromethyl-*N*-[1-(cyanoethyl-carbamoyl)-2-naphthalen-2-yl-ethyl]-benzamide,
4-Chloromethyl-*N*-[1-(cyanoethyl-carbamoyl)-2-naphthalen-2-yl-ethyl]-benzamide,
*N*-[1-(Cyanoethyl-carbamoyl)-2-naphthalen-2-yl-ethyl]-2-fluoro-benzamide,
*N*-[1-(Cyanoethyl-carbamoyl)-2-naphthalen-2-yl-ethyl]-2-nitro-benzamide,
*N*-[1-(Cyanoethyl-carbamoyl)-2-naphthalen-2-yl-ethyl]-acrylamide,
3-Naphthalen-2-yl-2,2-dimethyl-propionic acid cyanoethyl-amide,
3-Naphthalen-2-yl-2-(2-methoxy-acetylamino)-propionic acid cyanoethyl-amide,
*N*-[1-(Cyanoethyl-carbamoyl)-2-naphthalen-2-yl-ethyl]-oxalamic acid methyl ester,
*N*-[1-(Cyanoethyl-carbamoyl)-2-naphthalen-2-yl-ethyl]-phthalamic acid,
*N*-[1-(Cyanoethyl-carbamoyl)-2-naphthalen-2-yl-ethyl]-succinamic acid,
3-[1-(Cyanoethyl-carbamoyl)-2-naphthalen-2-yl-ethylcarbamoyl]-acrylic acid,
[1-(Cyanoethyl-carbamoyl)-2-naphthalen-2-yl-ethyl]-carbamic acid isobutyl ester,
[1-(Cyanoethyl-carbamoyl)-2-naphthalen-2-yl-ethyl]-carbamic acid butyl ester,
[1-(Cyanomethyl-carbamoyl)-2-naphthalen-2-yl-ethyl]-carbamic acid cyanomethyl ester,
[1-(Cyanoethyl-carbamoyl)-2-naphthalen-2-yl-ethyl]-carbamic acid cyanomethyl ester,
[1-(Cyanomethyl-carbamoyl)-2-naphthalen-2-yl-ethyl]-carbamic acid but-3-enyl ester,
[1-(Cyanoethyl-carbamoyl)-2-naphthalen-2-yl-ethyl]-carbamic acid but-3-enyl ester,

[1-(Cyanomethyl-carbamoyl)-2-naphthalen-2-yl-ethyl]-carbarnic acid 2-isopropyl-5-methyl-cyclohexyl ester,
[1-(Cyanoethyl-carbamoyl)-2-naphthalen-2-yl-ethyl]-carbamic acid 2-isopropyl-5-methyl-cyclohexyl ester,
[1-(Cyanoethyl-carbamoyl)-2-naphthalen-2-yl-ethyl]-carbamic acid hexyl ester,
[1-(Cyanoethyl-carbamoyl)-2-naphthalen-2-yl-ethyl]-carbamic acid tert-butyl ester,
[1-(Cyanoethyl-carbamoyl)-2-naphthalen-2-yl-ethyl]-carbamic acid methyl ester,
[1-(Cyanoethyl-carbamoyl)-2-naphthalen-2-yl-ethyl]-carbamic acid ethyl ester,
[1-(Cyanoethyl-carbamoyl)-2-naphthalen-2-yl-ethyl]-carbamic acid 9H-fluoren-9-ylmethylester,
3-Benzo[b]thiophen-3-yl-2-acetylamino-propionic acid cyanomethyl-amide,
3-Benzo[b]thiophen-3-yl-2-[3-(4-trifluoromethyl-phenyl)-ureido]-propionic acid cyanomethyl-amide,
3-Benzo[b]thiophen-3-yl-2-[3-(4-trifluoromethylsulfanyl-phenyl)-ureido]-propionic acid cyanomethyl-amide,
3-Benzo[b]thiophen-3-yl-2-[3-(4-trifluoromethoxy-phenyl)-ureido]-propionic acid cyanomethyl-amide,
3-Benzo[b]thiophen-3-yl-2-[3-(4-cyano-phenyl)-ureido]-propionic acid cyanomethyl-amide,
3-Benzo[b]thiophen-3-yl-2-(3-benzyl-ureido)-propionic acid cyanomethyl-amide,
3-Benzo[b]thiophen-3-yl-2-(3-*o*-tolyl-ureido)-propionic acid cyanomethyl-amide,
3-Benzo[b]thiophen-3-yl-2-[3-(*S*)-(1-phenyl-ethyl)-ureido]-propionic acid cyanomethyl-amide,
3-Benzo[b]thiophen-3-yl-2-[3-(2,6-dimethyl-phenyl)-ureido]-propionic acid cyanomethyl-amide,
3-Benzo[b]thiophen-3-yl-2-[3-(3-methyl-benzyl)-ureido]-propionic acid cyanomethyl-amide,
3-Benzo[b]thiophen-3-yl-2-[3-(1,1,3,3-tetramethyl-butyl)-ureido]-propionic acid cyanomethyl-amide,
3-Benzo[b]thiophen-3-yl-2-(3-indan-5-yl-ureido)-propionic acid cyanomethyl-amide,
3-Benzo[b]thiophen-3-yl-2-[3-(2-phenyl-cyclopropyl)-ureido]-propionic acid cyanomethyl-amide,
3-Benzo[b]thiophen-3-yl-2-(3-adamantan-1-yl-ureido)-propionic acid cyanomethyl-amide,
3-Benzo[b]thiophen-3-yl-2-(3-biphenyl-4-yl-ureido)-propionic acid cyanomethyl-amide,
3-Benzo[b]thiophen-3-yl-2-[3-(4-phenoxy-phenyl)-ureido]-propionic acid cyanomethyl-amide,
3-Benzo[b]thiophen-3-yl-2-[3-(4-nitro-phenyl)-ureido]-propionic acid cyanomethyl-amide,
3-Benzo[b]thiophen-3-yl-2-(3-cyclohexyl-ureido)-propionic acid cyanomethyl-amide,
3-Benzo[b]thiophen-3-yl-2-(3-benzo[1,3]dioxol-5-yl-ureido)-propionic acid cyanomethyl-amide,
3-Benzo[b]thiophen-3-yl-2-[3-(2-fluoro-benzyl)-ureido]-propionic acid cyanomethyl-amide,
3-Benzo[b]thiophen-3-yl-2-[3-(4-methyl-benzyl)-ureido]-propionic acid cyanomethyl-amide,
3-Benzo[b]thiophen-3-yl-2-(3-phenethyl-ureido) -ureido]-propionic acid cyanomethyl-amide,
3-Benzo[b]thiophen-3-yl-2-[3-(3,4,5-trimethoxy-phenyl)-ureido]-propionic acid cyanomethyl-amide,
3-Benzo[b]thiophen-3-yl-2-(3-ethyl-thioureido)-propionic acid cyanomethyl-amide,
3-Benzo[b]thiophen-3-yl-2-(3-isopropyl-thioureido)-propionic acid cyanomethyl-amide,
3-Benzo[b]thiophen-3-yl-2-[3-(4-nitro-phenyl)-thioureido]-propionic acid cyanomethyl-amide,
3-Benzo[b]thiophen-3-yl-2-(3-phenyl-thioureido)-propionic acid cyanomethyl-amide,
3-Benzo[b]thiophen-3-yl-2-[3-(4-trifluoromethoxy-phenyl)-thioureido]-propionic acid cyanomethyl-amide,
3-Benzo[b]thiophen-3-yl-2-{3-[4-(2,2,2-trifluoro-ethyl)-phenyl]-thioureido}-propionic acid cyanomethyl-amide,
3-Benzo[b]thiophen-3-yl-2-[3-(4-methoxy-phenyl)-thioureido]-propionic acid cyanomethyl-amide,
3-Benzo[b]thiophen-3-yl-2-(7,7-dimethyl-2-oxo-bicyclo[2.2.1]hept-1-ylmethanesulfonylamino)-propionic acid cyanomethyl-amide,
3-Benzo[b]thiophen-3-yl-2-(thiophene-2-sulfonylamino)-propionic acid cyanomethyl-amide,
3-Benzo[b]thiophen-3-yl-2-(4-trifluoromethoxy-benzenesulfonylamino)-propionic acid cyanomethyl-amide,
3-Benzo[b]thiophen-3-yl-2-(4-*tert*-butyl-benzenesulfonylamino)-propionic acid cyanomethyl-amide,
3-Benzo[b]thiophen-3-yl-2-(4-chloro-benzenesulfonylamino)-propionic acid cyanomethyl-amide,
3-Benzo[b]thiophen-3-yl-2-(4-methoxy-benzenesulfonylamino)-propionic acid cyanomethyl-amide,
3-Benzo[b]thiophen-3-yl-2-(quinoline-6-sulfonylamino)-propionic acid cyanomethyl-amide,
3-Benzo[b]thiophen-3-yl-2-benzenesulfonylamino-propionic acid cyanomethyl-amide,
1-Methyl-1H-indole-2-carboxylic acid [2-benzo[b]thiophen-3-yl-1-(cyanomethyl-carbamoyl)-ethyl]-amide,
2-Propyl-pentanoic acid [2-benzo[b]thiophen-3-yl-1-(cyanomethyl-carbamoyl)-ethyl]-amide,
1-Methyl-cyclopropanecarboxylic acid [2-benzo[b]thiophen-3-yl-1-(cyanomethyl-carbamoyl)-ethyl]-amide,
Thiophene-2-carboxylic acid [2-benzo[b]thiophen-3-yl-1-(cyanomethyl-carbamoyl)-ethyl]-amide,
*N*-[2-Benzo[b]thiophen-3-yl-1-(cyanomethyl-carbamoyl)-ethyl)-3-trifluoromethyl-benzamide, Biphenyl-2-carboxylic acid [2-benzo[b]thiophen-3-yl-1-(cyanomethyl-carbamoyl)-ethyl]-amide,
4-Acetylamino-*N*-[2-benzo[b]thiophen-3-yl-1-(cyanomethyl-carbamoyl)-ethyl)-benzamide,
3-Benzo[b]thiophen-3-yl-2-(2-1*H*-indol-3-yl-acetylamino)-propionic acid cyanomethyl-amide,
3-Benzo[b]thiophen-3-yl-2-(3-1*H*-indol-3-yl-propionylamino)-propionic acid cyanomethyl-amide,
*N*-[2-Benzo[b]thiophen-3-yl-1-(cyanomethyl-carbamoyl)-ethyl]-4-(1*H*-indol-3-yl)-butyramide,
*N*-[2-Benzo[b]thiophen-3-yl-1-(cyanomethyl-carbarnoyl)-ethyl]-benzamide,
*N*-[2-Benzo[b]thiophen-3-yl-1-(cyanomethyl-carbamoyl)-ethyl]-acrylamide,

*N*-[2-Benzo[b]thiophen-3-yl-1-(cyanomethyl-carbamoyl)-ethyl]-acrylamide,
3-Benzo[b]thiophen-3-yl-2,2-dimethyl-propionic acid cyanomethyl-amide,
3-Benzo[b]thiophen-3-yl-2-(2-methoxy-acetylamino)-propionic acid cyanomethyl-amide,
*N*-[2-Benzo[b]thiophen-3-yl-1-(cyanomethyl-carbamoyl)-ethyl]-oxalamic acid methyl ester,
*N*-[2-Benzo[b]thiophen-3-yl-1-(cyanomethyl-carbamoyl)-ethyl]-phthalamic acid,
*N*-[2-Benzo[b]thiophen-3-yl-1-(cyanomethyl-carbamoyl)-ethyl)-succinamic acid,
3-[2-Benzo[b]thiophen-3-yl-1-(cyanomethyl-carbamoyl)-ethylcarbamoyl]-acrylic acid,
[2-Benzo[b]thiophen-3-yl-1-(cyanomethyl-carbamoyl)-ethyl]-carbamic acid isobutyl ester,
[2-Benzo[b]thiophen-3-yl-1-(cyanomethyl-carbamoyl)-ethyl]-carbamic acid butyl ester,
[2-Benzo[b]thiophen-3-yl-1-(cyanomethyl-carbamoyl)-ethyl]-carbamic acid cyanomethyl ester,
[2-Benzo[b]thiophen-3-yl-1-(cyanomethyl-carbamoyl)-ethyl]-carbamic acid but-3-enyl ester,
(2-Benzo[b]thiophen-3-yl-1-(cyanomethyl-carbamoyl)-ethyl]-carbamic acid 2-isopropyl-5-methyl-cyclohexyl ester,
[2-Benzo[b]thiophen-3-yl-1-(cyanomethyl-carbamoyl)-ethyl]-carbamic acid hexyl ester,
[2-Benzo[b]thiophen-3-yl-1-(cyanomethyl-carbamoyl)-ethyl]-carbamic acid tert-butyl ester,
[2-Benzo[b]thiophen-3-yl-1-(cyanomethyl-carbamoyl)-ethyl]-carbamic acid methyl ester,
[2-Benzo[b]thiophen-3-yl-1-(cyanomethyl-carbamoyl)--ethyl]-carbamic acid ethyl ester,
[2-Benzo[b]thiophen-3-yl-1-(cyanomethyl-carbamoyl)-ethyl]-carbamic acid 9*H*-fluoren-9-ylmethyl ester,
3-Benzo[b]thiophen-3-yl-2-acetylamino-propionic acid cyanoethyl-amide,
3-Benzo[b]thiophen-3-yl-2-[3-(4-trifluoromethyl-phenyl)-ureido]-propionic acid cyanoethyl-amide,
3-Benzo[b]thiophen-3-yl-2-[3-(4-trifluoromethylsulfanyl-phenyl)-ureido]-propionic acid cyanoethyl-amide,
3-Benzo[b]thiophen-3-yl-2-[3-(4-trifluoromethoxy-phenyl)-ureido]-propionic acid cyanoethyl-amide,
3-Benzo[b]thiophen-3-yl-2-[3-(4-cyano-phenyl)-ureido]-propionic acid cyanoethyl-amide,
3-Benzo[b]thiophen-3-yl-2-(3-benzyl-ureido)-propionic acid cyanoethyl-amide,
3-Benzo[b]thiophen-3-yl-2-(3-*o*-tolyl-ureido)-propionic acid cyanoethyl-amide,
3-Benzo[b]thiophen-3-yl-2-[3-(*S*)-(1-phenyl-ethyl)-ureido]-propionic acid cyanoethyl-amide,
3-Benzo[b]thiophen-3-yl-2-[3-(2,6-dimethyl-phenyl)-ureido]-propionic acid cyanoethyl-amide,
3-Benzo[b]thiophen-3-yl-2-[3-(3-methyl-benzyl)-ureido]-propionic acid cyanoethyl-amide,
3-Benzo[b]thiophen-3-yl-2-[3-(1,1,3,3-tetramethyl-butyl)-ureido]-propionic acid cyanoethyl-amide,
3-Benzo[b]thiophen-3-yl-2-(3-indan-5-yl-ureido)-propionic acid cyanoethyl-amide,
3-Benzo[b]thiophen-3-yl-2-[3-(2-phenyl-cyclopropyl)-ureido]-propionic acid cyanoethyl-amide,
3-Benzo[b]thiophen-3-yl-2-(3-adamantan-1-yl-ureido)-propionic acid cyanoethyl-amide,
3-Benzo[b]thiophen-3-yl-2-(3-biphenyl-4-yl-ureido)-propionic acid cyanoethyl-amide,
3-Benzo[b]thiophen-3-yl-2-[3-(4-phenoxy-phenyl)-ureido]-propionic acid cyanoethyl-amide,
3-Benzo[b]thiophen-3-yl-2-[3-(4-nitro-phenyl)-ureido]-propionic acid cyanoethyl-amide,
3-Benzo[b]thiophen-3-yl-2-(3-cyclohexyl-ureido)-propionic acid cyanoethyl-amide,
3-Benzo[b]thiophen-3-yl-2-(3-benzo[1,3]dioxol-5-yl-ureido)-propionic acid cyanoethyl-amide,
3-Benzo[b]thiophen-3-yl-2-[3-(2-fluoro-benzyl)-ureido]-propionic acid cyanoethyl-amide,
3-Benzo[b]thiophen-3-yl-2-[3-(4-methyl-benzyl)-ureido]-propionic acid cyanoethyl-amide,
3-Benzo[b]thiophen-3-yl-2-(3-phenethyl-ureido) -ureido]-propionic acid cyanoethyl-amide,
3-Benzo[b]thiophen-3-yl-2-[3-(3,4,5-trimethoxy-phenyl)-ureido]-propionic acid cyanoethyl-amide,
3-Benzo[b]thiophen-3-yl-2-(3-ethyl-thioureido)-propionic acid cyanoethyl-amide,
3-Benzo[b]thiophen-3-yl-2-(3-isopropyl-thioureido)-propionic acid cyanoethyl-amide,
3-Benzo[b]thiophen-3-yl-2-[3-(4-nitro-phenyl)-thioureido]-propionic acid cyanoethyl-amide,
3-Benzo[b]thiophen-3-yl-2-(3-phenyl-thioureido)-propionic acid cyanoethyl-amide,
3-Benzo[b]thiophen-3-yl-2-[3-(4-trifluoromethoxy-phenyl)-thioureido]-propionic acid cyanoethyl-amide,
3-Benzo[b]thiophen-3-yl-2-{3-[4-(2,2,2-trifluoro-ethyl)-phenyl]-thioureido}-propionic acid cyanoethyl-amide,
3-Benzo[b]thiophen-3-yl-2-[3-(4-methoxy-phenyl)-thioureido]-propionic acid cyanoethyl-amide,
3-Benzo[b]thiophen-3-y1-2-(7,7-dimethyl-2-oxo-bicyclo[2.2.1]hept-1-ylmethanesulfonylamino)-propionic acid cyanoethyl-amide,
3-Benzo[b]thiophen-3-yl-2-(thiophene-2-sulfonylamino)-propionic acid cyanoethyl-amide,
3-Benzo[b]thiophen-3-yl-2-(4-trifluoromethoxy-benzenesulfonylamino)-propionic acid cyanoethyl-amide,
3-Benzo[b]thiophen-3-yl-2-(4-*tert*-butyl-benzenesulfonylamino)-propionic acid cyanoethyl-amide,
3-Benzo[b]thiophen-3-yl-2-(4-chloro-benzenesulfonylamino)-propionic acid cyanoethyl-amide,
3-Benzo[b]thiophen-3-yl-2-(4-methoxy-benzenesulfonylamino)-propionic acid cyanoethyl-amide,
3-Benzo[b]thiophen-3-yl-2-(quinoline-6-sulfonylamino)-propionic acid cyanoethyl-amide,
3-Benzo[b]thiophen-3-yl-2-benzenesulfonylamino-propionic acid cyanoethyl-amide,
1-Methyl-1H-indole-2-carboxylic acid [2-benzo[b]thiophen-3-yl-1-(cyanoethyl-carbamoyl)-ethyl]-amide,

2-Propyl-pentanoic acid [2-benzo[b]thiophen-3-yl-1-(cyanoethyl-carbamoyl)-ethyl]-amide,
1-Methyl-cyclopropanecarboxylic acid [2-benzo[b]thiophen-3-yl-1-(cyanoethyl-carbamoyl)-ethyl]-amide,
Thiophene-2-carboxylic acid [2-benzo[b]thiophen-3-yl-1-(cyanoethyl-carbamoyl)-ethyl]-amide,
*N*-[2-Benzo[b]thiophen-3-yl-1-(cyanoethyl-carbamoyl)-ethyl]-3-trifluoromethyl-benzamide,
Biphenyl-2-carboxylic acid [2-benzo[b]thiophen-3-yl-1-(cyanoethyl-carbamoyl)-ethyl]-amide,
4-Acetylamino-*N*-[2-benzo[b]thiophen-3-yl-1-(cyanoethyl-carbamoyl)-ethyl]-benzamide,
3-Benzo[b]thiophen-3-yl-2-(2-1*H*-indol-3-yl-acetylamino)-propionic acid cyanoethyl-amide,
3-Benzo[b]thiophen-3-yl-2-(3-1*H*-indol-3-yl-propionylamino)-propionic acid cyanoethyl-amide,
*N*-[2-Benzo[b]thiophen-3-yl-1-(cyanoethyl-carbamoyl)-ethyl]-4-(1*H*-indol-3-yl)-butyramide,
*N*-[2-Benzo[b]thiophen-3-yl-1-(cyanoethyl-carbamoyl)-ethyl]-benzamide,
3-Chloromethyl-*N*-[2-benzo[b]thiophen-3-yl-1-(cyanomethyl-carbamoyl)-ethyl]-benzamide,
4-Chloromethyl-*N*-[2-benzo[b]thiophen-3-yl-1-(cyanomethyl-carbamoyl)-ethyl]-benzamide,
*N*-[2-Benzo[b]thiophen-3-yl-1-(cyanomethyl-carbamoyl)-ethyl]-2-fluoro-benzamide,
*N*-[2-Benzo[b]thiophen-3-yl-1-(cyanomethyl-carbamoyl)-ethyl)-2-nitro-benzamide,
3-Chloromethyl-*N*-[2-benzo[b]thiophen-3-yl-1-(cyanoethyl-carbamoyl)-ethyl]-benzamide,
4-Chloromethyl-*N*-[2-benzo[b]thiophen-3-yl-1-(cyanoethyl-carbamoyl)-ethyl]-benzamide,
*N*-[2-Benzo[b]thiophen-3-yl-1-(cyanoethyl-carbamoyl)-ethyl]-2-fluoro-benzamide,
*N*-[2-Benzo[b]thiophen-3-yl-1-(cyanoethyl-carbamoyl)-ethyl]-2-nitro-benzamide,
3-Benzo[b]thiophen-3-yl-2,2-dimethyl-propionic acid cyanoethyl-amide,
3-Benzo[b]thiophen-3-yl-2-(2-methoxy-acetylamino)-propionic acid cyanoethyl-amide,
*N*-[2-Benzo[b]thiophen-3-yl-1-(cyanoethyl-carbamoyl)-ethyl]-oxalamic acid methyl ester,
*N*-[2-Benzo[b]thiophen-3-yl-1-(cyanoethyl-carbamoyl)-ethyl]-phthalamic acid,
*N*-[2-Benzo[b]thiophen-3-yl-1-(cyanoethyl-carbamoyl)-ethyl]-succinamic acid,
3-[2-Benzo[b]thiophen-3-yl-1-(cyanoethyl-carbamoyl)-ethylcarbamoyl]-acrylic acid,
[2-Benzo[b]thiophen-3-yl-1-(cyanoethyl-carbamoyl)-ethyl]-carbamic acid isobutyl ester,

1-Cyanomethyl-pyrrolidine-2-carboxylic acid carbamoylmethyl-amide
1-Cyanomethyl-pyrrolidine-2-carboxylic acid (1-carbamoyl-2-methyl-propyl)-amide
1-Cyanomethyl-pyrrolidine-2-carboxylic acid (1-carbamoyl-2-hydroxy-ethyl)-amide
1-Cyanomethyl-pyrrolidine-2-carboxylic acid (1-carbamoyl-4-guanidino-butyl)-amide
1-Cyanomethyl-pyrrolidine-2-carboxylic acid (1-carbamoyl-2-phenyl-ethyl)-amide
1-Cyanomethyl-pyrrolidine-2-carboxylic acid [1-carbamoyl-2-(1H-indol-3-yl)-ethyl)-amide
1-Cyanomethyl-pyrrolidine-2-carboxylic acid [1-carbamoyl-2-(4-hydroxy-phenyl)-ethyl]-amide
3-[(1-Cyanomethyl-pyrrolidine-2-carbonyl)-amino]-succinamic acid
D-1-Cyanomethyl-pyrrolidine-2-carboxylic acid (1-carbamoyl-3-methyl-butyl)-amide
1-Cyanomethyl-pyrrolidine-2-carboxylic acid (1-carbamoyl-cyclohexyl)-amide
1-Cyanomethyl-pyrrolidine-2-carboxylic acid [1-carbamoyl-2-(4-trifluoromethyl-benzylsulfanyl)-ethyl]-amide
1-Cyanomethyl-pyrrolidine-2-carboxylic acid [1-carbamoyl-2-(4-fluoro-benzylsulfanyl)-ethyl]-amide
1-Cyanomethyl-pyrrolidine-2-carboxylic acid [1-carbamoyl-2-(4-fluoro-phenylmethanesulfonyl)-ethyl]-amide
1-Cyanomethyl-pyrrolidine-2-carboxylic acid (3-carbamoyl-phenyl)-amide
1-Cyanomethyl-pyrrolidine-2-carboxylic acid [1-carbamoyl-2-(1-methyl-1H-imidazol-4-yl)-ethyl]-amide
1-Cyanomethyl-pyrrolidine-2-carboxylic acid (1-carbamoyl-2-pyridin-4-yl-ethyl)-amide
1-Cyanomethyl-pyrrolidine-2-carboxylic acid (1-carbamoyl-3-methyl-butyl)-amide
1-Cyanomethyl-pyrrolidine-2-carboxylic acid methyl ester
1-Cyanomethyl-pyrrolidine-2-carboxylic acid

N-(2-Cyanoethyl)-4-(4-dimethylamino-phenylazo)-benzenesulfonamide
N-(2-Cyanoethyl)-4-trifluoromethoxy-benzenesulfonamide
4-tert-Butyl-N-(2-cyanoethyl)-benzenesulfonamide
4-Bromo-N-(2-cyanoethyl)-benzenesulfonamide
4-Chloro-N-(2-cyanoethyl)-benzenesulfonamide
N-(2-Cyanoethyl)-4-methoxy-benzenesulfonamide
N-[4-(2-Cyanoethyl-sulfamoyl)-phenyl]-acetamide
N-(2-Cyanoethyl)-4-methyl-benzenesulfonamide
N-(2-Cyanoethyl)-benzenesulfonamide
N-(2-Cyanoethyl)-C-phenyl-methanesulfonamide
C,C,C-Trichloro-N-(2-cyanoethyl)-methanesulfonamide
Butane-1-sulfonic acid (2-cyanoethyl)-amide

Naphthalene-1-sulfonic acid (2-cyanoethyl)-amide
Octane-1-sulfonic acid (2-cyanoethyl)-amide
N-(2-Cyanoethyl)-2,4,6-triisopropyl-benzenesulfonamide
N-(2-Cyanoethyl)-2-trifluoromethyl-benzenesulfonamide
N-[5-(2-Cyanoethyl-sulfamoyl)-4-methyl-thiazol-2-yl]-acetamide
2-Bromo-N-(2-cyanoethyl)-benzenesulfonamide
N-(2-Cyanoethyl)-2,4,6-trimethyl-benzenesulfonamide
Thiophene-2-sulfonic acid (2-cyanoethyl)-amide
N-(2-Cyanoethyl)-3-nitro-benzenesulfonamide

1-(2-Cyanoethyl)-3-(4-nitro-phenyl)-thiourea
1-(2-Cyano 1-(2-Cyano-ethyl)-3-phenyl-thioureaethyl)-3-phenyl-thiourea
1-(2-Cyanoethyl)-3-(4-trifluoromethoxy-phenyl)-thiourea
1-(2-Cyanoethyl)-3-(4-methylsulfanyl-phenyl)-thiourea
1-(2-Cyanoethyl)-3-(3,4,5-trimethoxy-phenyl)-thiourea
1-(2-Cyanoethyl)-3-naphthalen-1-yl-thiourea
1-Benzyl-3-(2-cyanoethyl)-thiourea
1-Acetyl-3-(2-cyanoethyl)-thiourea
1-(4-Azido-phenyl)-3-(2-cyanoethyl)-thiourea
1-(2-Cyanoethyl)-3-(3-cyano-phenyl)-thiourea
1-(2-Cyanol-(2-Cyano-ethyl)-3-(4-ethyl-phenyl)-thioureaethyl)-3-(4-ethyl-phenyl)-thiourea
1-(2-Cyanol-(2-Cyano-ethyl)-3-(4-cyano-phenyl)-thioureaethyl)-3-(4-cyano-phenyl)-thiourea
1-Carbamoylmethyl-pyrrolidine-2-carboxylic acid (1-carbamoyl-3-methyl-butyl)-amide 1-(2-Cyanoethyl)-3-pyridin-4-yl-thiourea
1-(2-Cyanoethyl)-3-(2,3,4-trifluoro-phenyl)-thiourea
1-(2-Cyanoethyl)-3-(2,6-difluoro-phenyl)-thiourea
1-(4-Bromo-phenyl)-3-(2-cyanoethyl)-thiourea
1-(2-Cyanoethyl)-3-(4-methoxy-phenyl)-thiourea
1-(2-Cyanoethyl)-3-m-tolyl-thiourea
1-(2-Cyanoethyl)-3-p-tolyl-thiourea
1-(2-Cyanoethyl)-3-cyclohexyl-urea
1-(2-Cyanoethyl)-3-o-tolyl-urea
1-(2-Cyanoethyl)-3-(2-methoxy-phenyl)-urea
[2-Benzo[b]thiophen-3-yl-1-(cyanoethyl-carbamoyl)-ethyl]-carbamic acid butyl ester,
[2-Benzo[b]thiophen-3-yl-1-(cyanoethyl-carbamoyl)-ethyl]-carbamic acid cyanomethyl ester,
[2-Benzo[b]thiophen-3-yl-1-(cyanoethyl-carbamoyl)-ethyl]-carbamic acid but-3-enyl ester,
[2-Benzo[b]thiophen-3-yl-1-(cyanoethyl-carbamoyl)-ethyl]-carbamic acid 2-isopropyl-5-methyl-cyclohexyl ester,
[2-Benzo[b]thiophen-3-yl-1-(cyanoethyl-carbamoyl)-ethyl)-carbamic acid hexyl ester,
[2-Benzo[b]thiophen-3-yl-1-(cyanoethyl-carbamoyl)-ethyl]-carbamic acid tert-butyl ester,
[2-Benzo[b]thiophen-3-yl-1-(cyanoethyl-carbamoyl)-ethyl]-carbamic acid methyl ester,
[2-Benzo[b]thiophen-3-yl-1-(cyanoethyl-carbamoyl)-ethyl]-carbamic acid ethyl ester,
[2-Benzo[b]thiophen-3-yl-1-(cyanoethyl-carbamoyl)-ethyl]-carbamic acid 9*H*-fluoren-9-ylmethylester,

or a pharmaceutically acceptable salt or prodrug thereof.

**18.** Use of a derivative or of a precursor of a compound of formula (I) according to any of claims 1-17, which upon chemical transformation is converted into a compound of formula (I) according to any of the preceding claims, as inhibitor of or to a rotamase.

**19.** The use according to claim 18, whereby the chemical transformation is selected from the group comprising hydrolysis, oxidation and reduction, whereby such transformation is preferably carried out in vivo or in vitro.

**20.** The use according to any of claims 1-19, whereby the compound or a pharmaceutically acceptable salt or prodrug thereof is chiral.

**21.** The use according to claim 20, whereby the compound or a pharmaceutically acceptable salt or prodrug thereof, has at least one amino acid side chain, whereby the amino acid side chain is in the (S) or L-configuration or in the

(R) or D-configuration.

**22.** The use according to any of the claims 1 to 21, whereby the compound or a pharmaceutically acceptable salt or prodrug thereof comprises a label.

**23.** The use according to claim 22, whereby the compound or a pharmaceutically acceptable salt or prodrug thereof comprises at least one label, whereby the label is selected from the group comprising radioactive isotopes, heavy isotopes, immune labels, coloured labels and fluorescent labels.

**24.** The use according to claim 22 and 23, whereby the compound or a pharmaceutically acceptable salt or prodrug thereof, comprises at least one label, whereby the label is selected from the group comprising $^{14}C$, $^{13}C$, $^{15}N$, and $^{3}H$.

**25.** The use according to claim 22 and 23, whereby the compound or a pharmaceutically acceptable salt or prodrug thereof, comprises at least one immune label, whereby the label is selected from the group comprising antibodies and antigens.

**26.** The use according to claim 22 and 23, whereby the compound or a pharmaceutically acceptable salt or prodrug thereof, comprises at least one colored and/or fluorescent label, whereby the fluorescent label is selected from the group comprising fluorescein, 6-FAM, HEX, TET, CY-5, CY-3, CY-7, Texas Red.

**27.** Use of a compound for the manufacture of a kit for the inhibition of a rotamase and/or for quantification of the amount of rotamase in a sample, whereby the compound is a compound as described in any of the preceding claims, a pharmaceutically acceptable salt and/or a prodrug thereof, whereby the kit preferably comprises a standardized amount of or solution of said compound or a pharmaceutically acceptable salt or prodrug thereof.

**28.** Use of a compound for removing a rotamase from a sample or for identifying a rotamase in a sample, whereby the compound is a compound as described in any of claims 1 to 26, or a pharmaceutically acceptable salt or a prodrug thereof.

**29.** Use of a compound for the manufacture of or in an affinity device, whereby the compound is a compound as described in any of claims 1 to 26, or a pharmaceutically acceptable salt or a prodrug thereof, whereby preferably the affinity device is a chromatographic column.

**30.** The use according to claim 29, whereby the compound is bound to a chromatographic support.

**31.** The use according to claim 29 or 30, whereby the device is for removal and/or identifying of a rotamase from/in a sample.

**32.** Use according to any of claims 27 to 31, wherein the sample is a biological sample, preferably a sample which is selected from the group comprising blood, lymph, saliva, tissue samples and bacterial, fungal, plant, viral and mammalian cell cultures.

**33.** The use of a compound for the manufacture of a medicament for the treatment of a disease whereby the disease involves an abnormal cell proliferation, an undesired cell proliferation, an abnormal mitosis and/or an undesired mitosis, whereby the compound is a compound as desscribed in any of claims 1 to 26 or a pharmaceutically acceptable salt or a prodrug thereof.

**34.** The use according to any of claims 1 to 33, wherein the rotamase regulates a part of the cell cycle, preferably the part of the cell cycle is mitosis.

**35.** The use according to any of claims 1 to 34, wherein the rotamase is a mammalian rotamase, preferably a human rotamase, more preferably hPin1.

**36.** The use according to any of the preceeding claims, wherein the disease is selected from the group comprising neurodegenerative diseases, stroke, inflammatory diseases, immune based disorders, infectious diseases, heart diseases, cardiovascular diseases and cell proliferative diseases.

**37.** The use according to claim 36, wherein the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral sclerosis, prion diseases and motor neuron diseases.

**38.** The use according to claim 36, wherein the infectious disease is selected from the group comprising fungal, viral, bacterial and parasite infection.

**39.** The use according to claim 38, wherein the fungal infection is selected from the group comprising gynaecological and dermatological infection.

**40.** The use according to claim 38, wherein the fungal infection is caused by *Histoplasma, Coccidioides, Cryptococcus, Blastomyces, Paracoccidioides, Aspergillus, Sporothrix, Rhizopus, Absidia, Mucor, Hormodendrum, Phialophora Microsporum, Epidermophyton, Rhinosporidum* or by a yeast, preferably *Candida or Cryptococcus.*

**41.** The use according to claim 38, wherein the fungal infection causes a disorder selected from the group comprising ringworm, candidiasis, coccidioidomycosis, blastomycosis, aspergillosis, cryptococcosis, histioplasmosis, paracoccidiomycosis, zygomycosis, sporotrichiosis, mycotic keratitis, nail hair and skin disease, lobomycosis, chromoblastomycosis and mycetoma.

**42.** The use according to claim 38, wherein the bacterial infection is selected from the group comprising infections caused by Gram-positive and by Gram-negative bacteria.

**43.** The use according to claim 42, wherein the bacterial infection is caused by *Staphylococcus, Clostridium, Streptococcus, Listeria, Salmonella, Bacillus, Escherichia, Mycobacteria, Serratia, Enterobacter, Enterococcus, Nocardia, Hemophilus, Neisseria, Proteus, Yersinia, Helicobacter or Legionella.*

**44.** The use according to claim 38, wherein the bacterial infection causes a disorder selected from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

**45.** The use according to claim 38, wherein the viral infection is selected from the group comprising infections caused by retrovirus (HIV), Papilloma virus, Polio virus, Epstein-Barr, Herpes virus, Hepatitis virus, Papova virus, Influenza virus, Rabies, JC, encephalitis causing virus or hemorrhagic fever causing virus.

**46.** The use according to claim 38, wherein the parasite infection is selected from the group comprising infections caused by *Trypanosoma, Leishmania, Trichinella, Echinococcus, Nematodes, Classes Cestoda Trematoda, Monogenea, Toxoplasma, Giardia, Balantidium, Paramecium, Plasmodium, or Entamoeba.*

**47.** The use according to claim 36, wherein the cell proliferative disorder is selected from the group comprising neoplastic and non-neoplastic disorders.

**48.** The use according to claim 47, wherein the neoplastic cell proliferative disorder is selected from the group comprising solid tumor, lymphoma and leukemia.

**49.** The use according to claim 48, wherein the solid tumor is selected from the group comprising carcinoma, sarcoma, osteoma, fibrosarcoma, and chondrosarcoma.

**50.** The use according to claim 47, wherein the neoplastic cell proliferative disorder is selected from the group comprising breast cancer, prostate cancer, colon cancer, brain cancer, lung cancer, pancreatic cancer, gastric cancer, bladder cancer and kidney cancer.

**51.** The use according to claim 47, wherein the non-neoplastic cell proliferative disorder is a fibrotic disorder, preferably the fibrotic disorder is fibrosis.

**52.** The use according to claim 47, wherein the non-neoplastic cell proliferative disorder is selected from the group comprising prostatic hypertrophy, endometriosis, psoriasis, tissue repair and wound healing.

**53.** The use according to claim 36, wherein the immune based/inflammatory disease is an autoimmune disease or disorder.

**54.** The use according to claim 36, wherein the immune based/inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease and dermatomyositis.

**55.** The use according to claim 36, wherein the immune based and/or inflammatory disease is an inflammatory condition.

**56.** The use according to claim 36, wherein the immune based and/or inflammatory disease is selected from the group comprising inflammation associated with bums, lung injury, myocardial infarction, coronary thrombosis, vascular occlusion, post-surgical vascular reocclusion, atherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

**57.** Use of a compound as a pharmaceutical in drug potentiation applications, whereby the compound is a compound as described in any of claims 1 to 26, or a pharmaceutically acceptable salt or a prodrug thereof.

**58.** The use according to claim 57, whereby a drug potentiation application is the manufacture of a medicament for use in drug potentiation applications, whereby preferably the medicament is for the treatment of any of the diseases as described in any of the preceding claims.

**59.** The use according to any of the preceding claims, wherein the medicament comprises the compound or a pharmaceutically acceptable salt or prodrug thereof in a therapeutically effective amount together with a pharmaceutically acceptable carrier and/or diluent and/or adjuvant.

**60.** The use according to any of the preceding claims, wherein the medicament for the administration to a patient is selected from the group comprising humans, canine, bovine, feline, porcine, caprine, equine, ovine animals, domesticated animals, reptiles, birds, lagomorphs, rodents, amphibians, fish, arthropods, valuable non-domesticated animals, and zoo animals.

**61.** The use according to any of the preceding claims wherein the medicament is for the administration to a mammal, preferably to a human being.

**62.** The use according to any of the preceding claims wherein the medicament is for administration via an administration route which is selected from the groups injection, infusion, suppository, tablet, transdermal patch, aerosol, or administered orally, subcutaneously, intravenously, intranasally, transdermally, intraperitoneally, intramuscularly, intrapulmonary, vaginally, rectally, via an implanted reservoir, or intraocularly.

**63.** A method of treating a patient, suffering from a disease as described in any of the preceding claims by administering a pharmaceutical formulation as described in any of the preceding claims.

**64.** A method of treating a patient for drug potentiation by administering a pharmaceutical formulation or medicaments according to any of the preceding claims.

**65.** A compound as described in any of claims 1 to 26, which is selected from the group comprising

3-(1*H*-Indol-3-yl)-2-(3-ethyl-thioureido)-propionic acid cyanomethyl-amide,
3-(1*H*-Indol-3-yl)-2-(3-isopropyl-thioureido)-propionic acid cyanomethyl-amide,
3-(1*H*-Indol-3-yl)-2-[3-(4-nitro-phenyl)-thioureido]-propionic acid cyanomethyl-amide,
3-(1*H*-Indol-3-yl)-2-(3-phenyl-thioureido)-propionic acid cyanomethyl-amide,
3-(1*H*-Indol-3-yl)-2-[3-(4-trifluoromethoxy-phenyl)-thioureido]-propionic acid cyanomethyl-amide,
3-(1*H*-Indol-3-yl)-2-{3-[4-(2,2,2-trifluoro-ethyl)-phenyl)-thioureido}-propionic acid cyanomethyl-amide,
3-(1*H*-Indol-3-yl)-2-[3-(4-methoxy-phenyl)-thioureido]-propionic acid cyanomethyl-amide,

3-(4-Hydroxy-phenyl)-2-(3-ethyl-thioureido)-propionic acid cyanomethyl-amide,
3-(4-Hydroxy-phenyl)-2-(3-isopropyl-thioureido)-propionic acid cyanomethyl-amide,
3-(4-Hydroxy-phenyl)-2-[3-(4-nitro-phenyl)-thioureido]-propionic acid cyanomethyl-amide,
3-(4-Hydroxy-phenyl)-2-(3-phenyl-thioureido)-propionic acid cyanomethyl-amide,
3-(4-Hydroxy-phenyl)-2-[3-(4-trifluoromethoxy-phenyl)-thioureido]-propionic acid cyanomethyl-amide,
3-(4-Hydroxy-phenyl)-2-{3-[4-(2,2,2-trifluoro-ethyl)-phenyl]-thioureido}-propionic acid cyanomethyl-amide,
3-(4-Hydroxy-phenyl)-2-[3-(4-methoxy-phenyl)-thioureido]-propionic acid cyanomethyl-amide
3-Phenyl-2-(3-ethyl-thioureido)-propionic acid cyanomethyl-amide,
3-Phenyl-2-(3-isopropyl-thioureido)-propionic acid cyanomethyl-amide,
3-Phenyl-2-[3-(4-nitro-phenyl)-thioureido]-propionic acid cyanomethyl-amide,
3-Phenyl-2-(3-phenyl-thioureido)-propionic acid cyanomethyl-amide,
3-Phenyl-2-[3-(4-trifluoromethoxy-phenyl)-thioureido]-propionic acid cyanomethyl-amide,
3-Phenyl-2-{3-[4-(2,2,2-trifluoro-ethyl)-phenyl]-thioureido}-propionic acid cyanomethyl-amide,
3-Phenyl-2-[3-(4-methoxy-phenyl)-thioureido]-propionic acid cyanomethyl-amide
3-Methylsulfanyl-2-(3-ethyl-thioureido)-propionic acid cyanomethyl-amide,
3-Methylsulfanyl-2-(3-isopropyl-thioureido)-propionic acid cyanomethyl-amide,
3-Methylsulfanyl-2-[3-(4-nitro-phenyl)-thioureido]-propionic acid cyanomethyl-amide,
3-Methylsulfanyl-2-(3-phenyl-thioureido)-propionic acid cyanomethyl-amide,
3-Methylsulfanyl-2-[3-(4-trifluoromethoxy-phenyl)-thioureido]-propionic acid cyanomethyl-amide,
3-Methylsulfanyl-2-{3-[4-(2,2,2-trinuoro-ethyl)-phenyl]-thioureido}-propionic acid cyanomethyl-amide,
3-Methylsulfanyl-2-[3-(4-methoxy-phenyl)-thioureido]-propionic acid cyanomethyl-amide
3-Methanesulfonyl-2-(3-ethyl-thioureido)-propionic acid cyanomethyl-amide,
3-Methanesulfonyl-2-(3-isopropyl-thioureido)-propionic acid cyanomethyl-amide,
3-Methanesulfonyl-2-[3-(4-nitro-phenyl)-thioureido]-propionic acid cyanomethyl-amide,
3-Methanesulfonyl-2-(3-phenyl-thioureido)-propionic acid cyanomethyl-amide,
3-Methanesulfonyl-2-[3-(4-trifluoromethoxy-phenyl)-thioureido]-propionic acid cyanomethyl-amide,
3-Methanesulfonyl-2-{3-[4-(2,2,2-trifluoro-ethyl)-phenyl]-thioureido}-propionic acid cyanomethyl-amide,
3-Methanesulfonyl-2-[3-(4-methoxy-phenyl)-thioureido]-propionic acid cyanomethyl-amide
3-Naphthalen-2-yl-2-(3-ethyl-thioureido)-propionic acid cyanomethyl-amide,
3-Naphthalen-2-yl-2-(3-isopropyl-thioureido)-propionic acid cyanomethyl-amide,
3-Naphthalen-2-yl-2-[3-(4-nitro-phenyl)-thioureido]-propionic acid cyanomethyl-amide,
3-Naphthalen-2-yl-2-(3-phenyl-thioureido)-propionic acid cyanomethyl-amide,
3-Naphthalen-2-yl-2-[3-(4-trifluoromethoxy-phenyl)-thioureido]-propionic acid cyanomethyl-amide,
3-Naphthalen-2-yl-2-{3-[4-(2,2,2-trifluoro-ethyl)-phenyl]-thioureido}-propionic acid cyanomethyl-amide,
3-Naphthalen-2-yl-2-[3-(4-methoxy-phenyl)-thioureido]-propionic acid cyanomethyl-amide
3-Benzo[b]thiophen-3-yl-2-(3-ethyl-thioureido)-propionic acid cyanomethyl-amide,
3-Benzo[b]thiophen-3-yl-2-(3-isopropyl-thioureido)-propionic acid cyanomethyl-amide,
3-Benzo[b]thiophen-3-yl-2-[3-(4-nitro-phenyl)-thioureido]-propionic acid cyanomethyl-amide,
3-Benzo[b]thiophen-3-yl-2-(3-phenyl-thioureido)-propionic acid cyanomethyl-amide,
3-Benzo[b]thiophen-3-yl-2-[3-(4-trifluoromethoxy-phenyl)-thioureido]-propionic acid cyanomethyl-amide,
3-Benzo[b]thiophen-3-yl-2-{3-[4-(2,2,2-trifluoro-ethyl)-phenyl]-thioureido}-propionic acid cyanomethyl-amide,
3-Benzo[b]thiophen-3-yl-2-[3-(4-methoxy-phenyl)-thioureido]-propionic acid cyanomethyl-amide,

or a pharmaceutically acceptable salt or a prodrug thereof.

Scheme 1

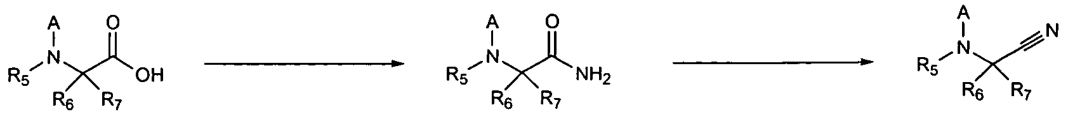

1 2 3

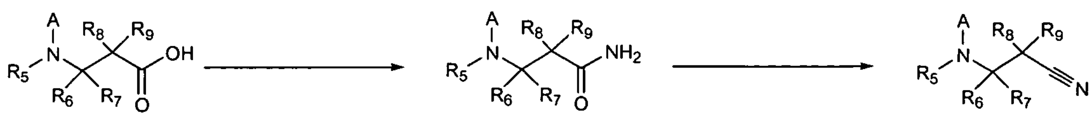

4 5 6

Fig. 1

Scheme 2

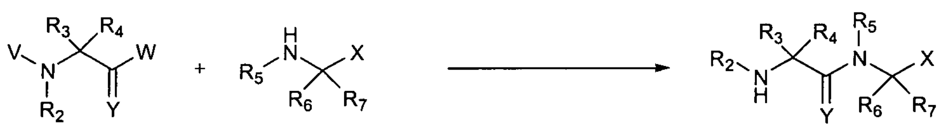

7 8 9

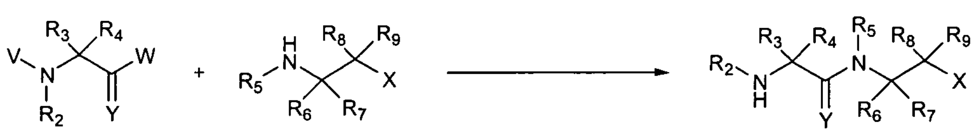

7 10 11

Fig. 2

Scheme 3 a

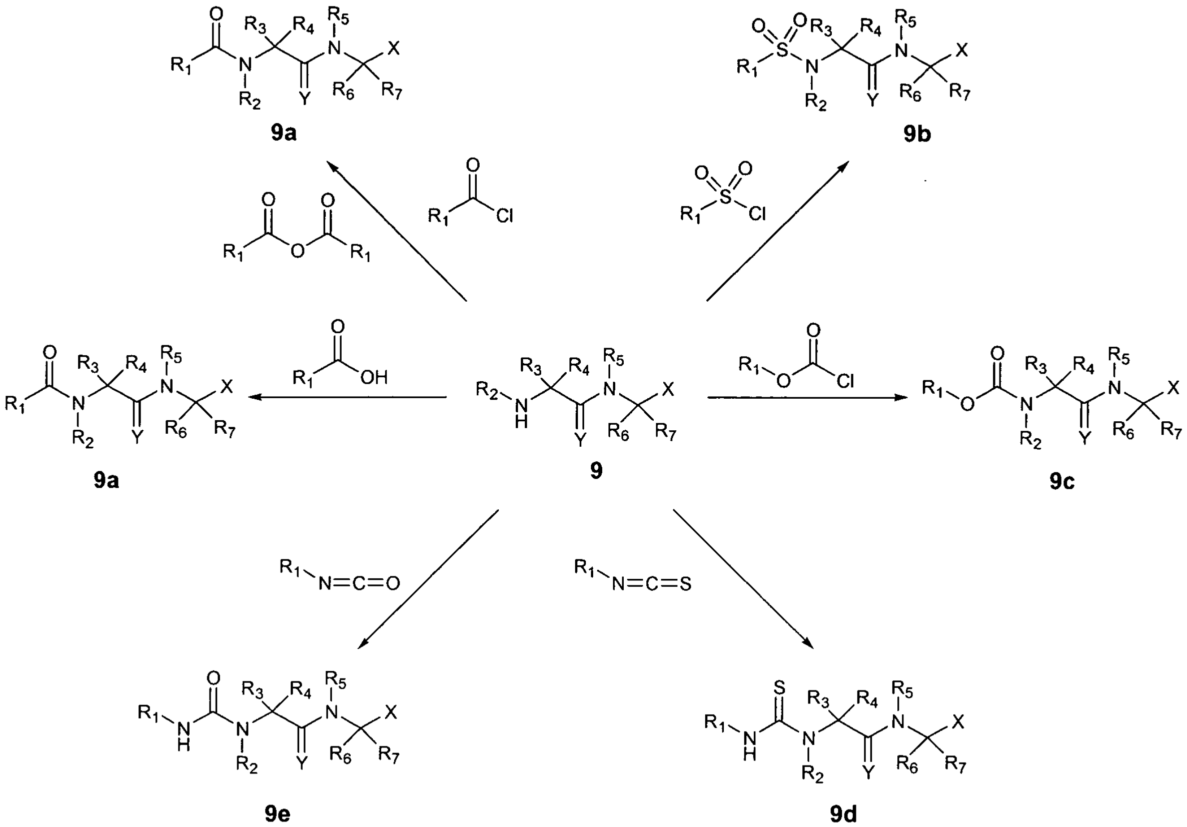

9a

9b

9a

9

9c

9e

9d

Fig. 3a

Scheme 3 b

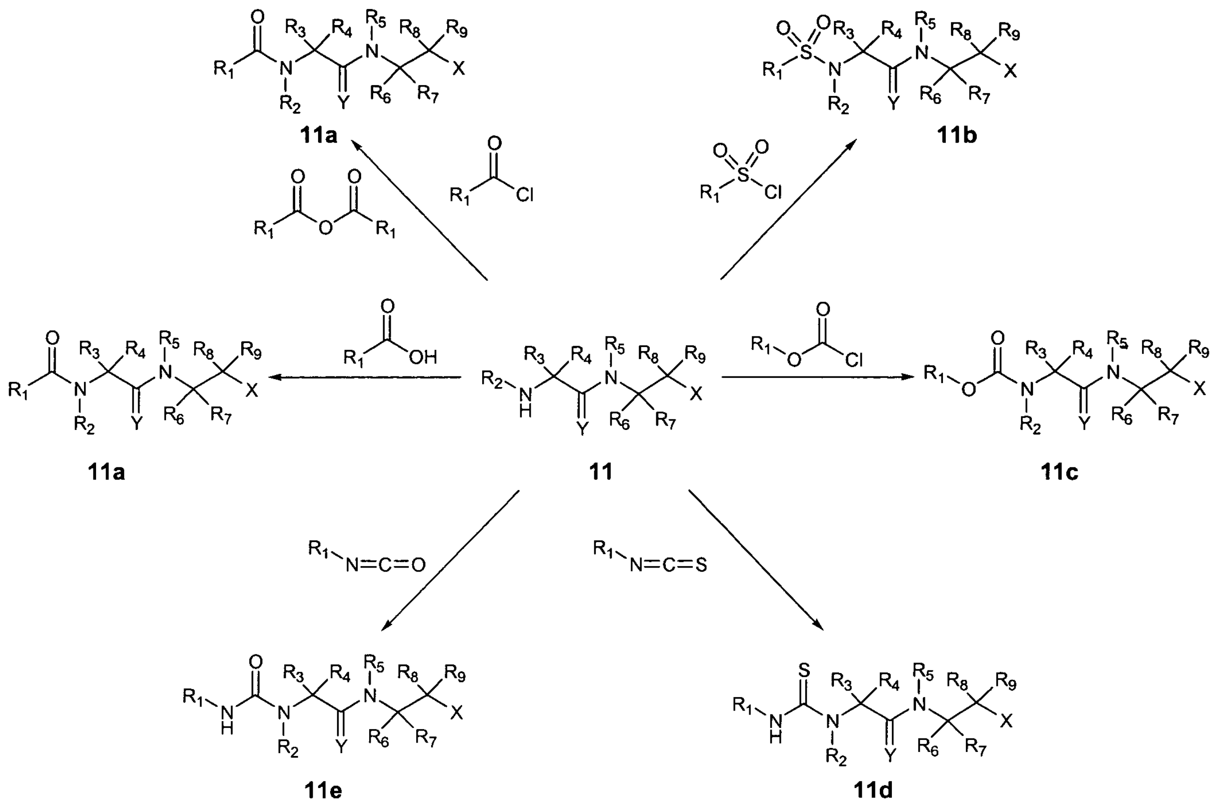

11a

11b

11a

11

11c

11e

11d

Fig. 3b

Scheme 4

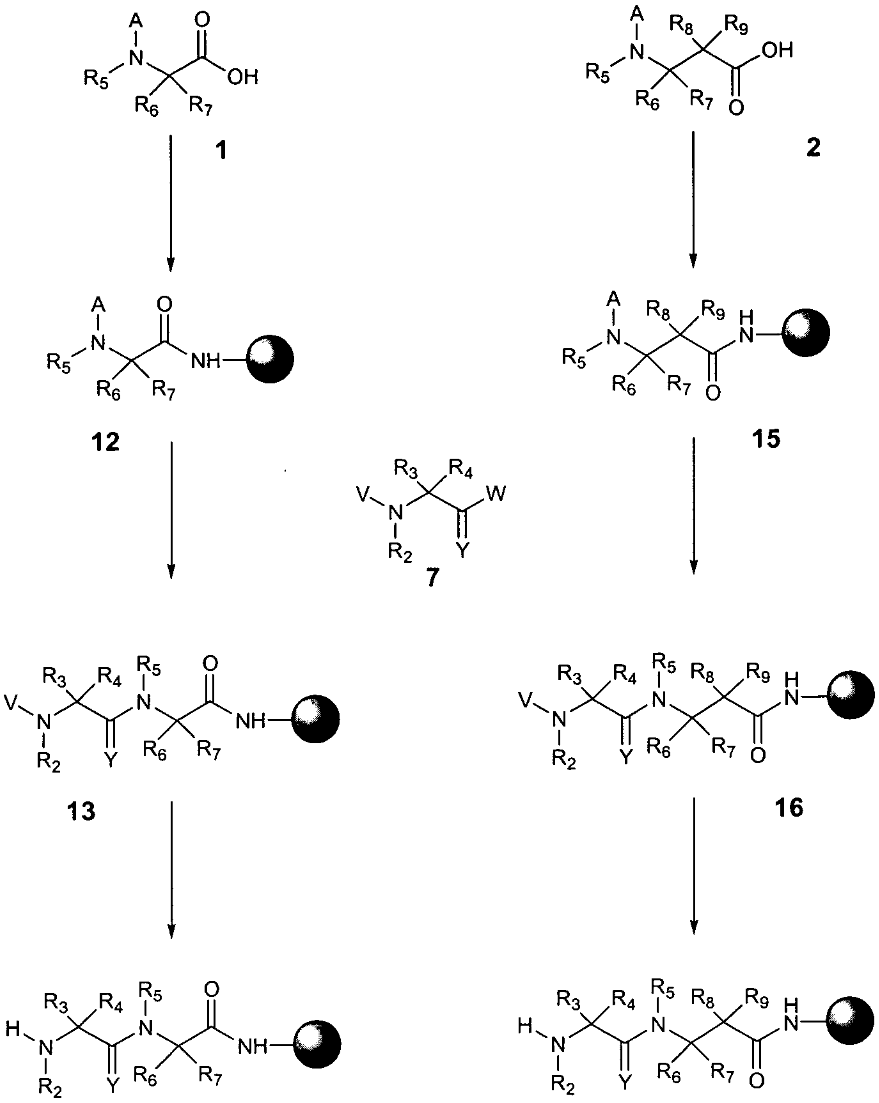

1

2

12

15

7

13

16

14

17

Fig. 4

Scheme 5 a

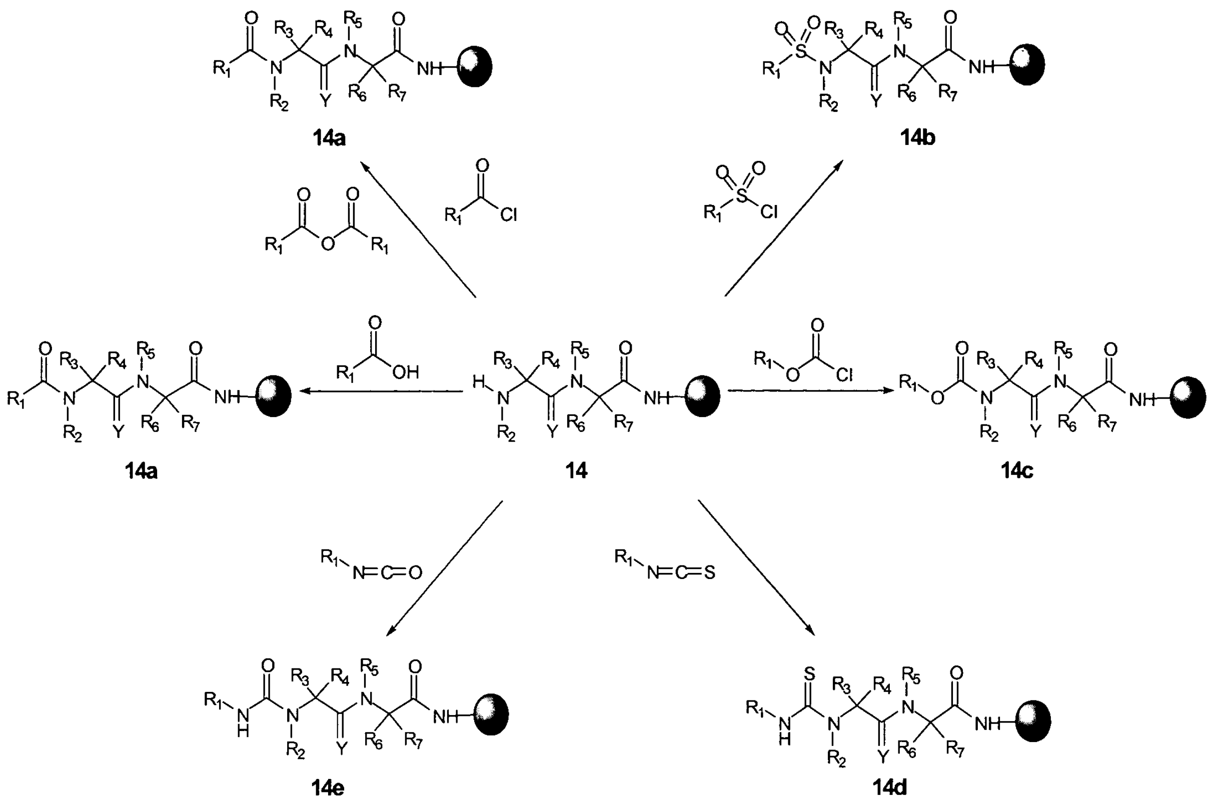

14a
14b
14a
14
14c
14e
14d

Fig. 5a

Scheme 5 b

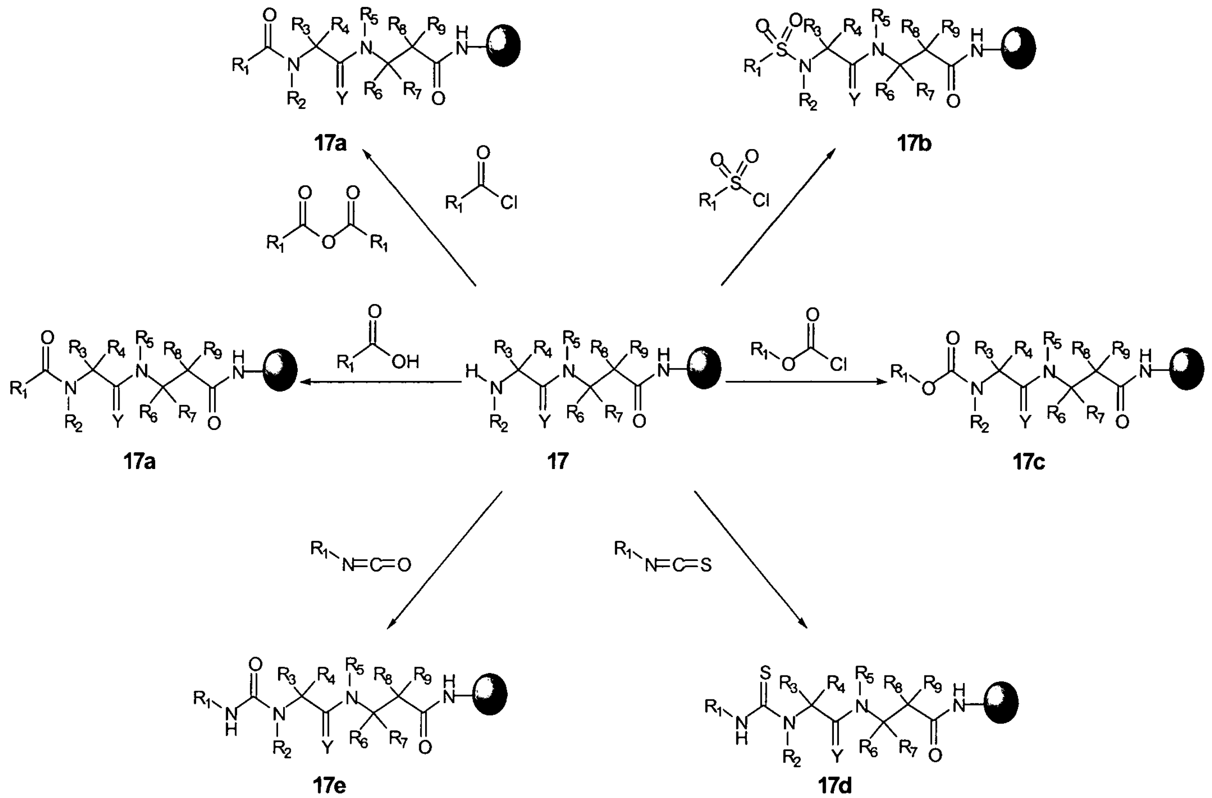

17a 17b

17a 17 17c

17e 17d

Fig. 5b

Scheme 6 a

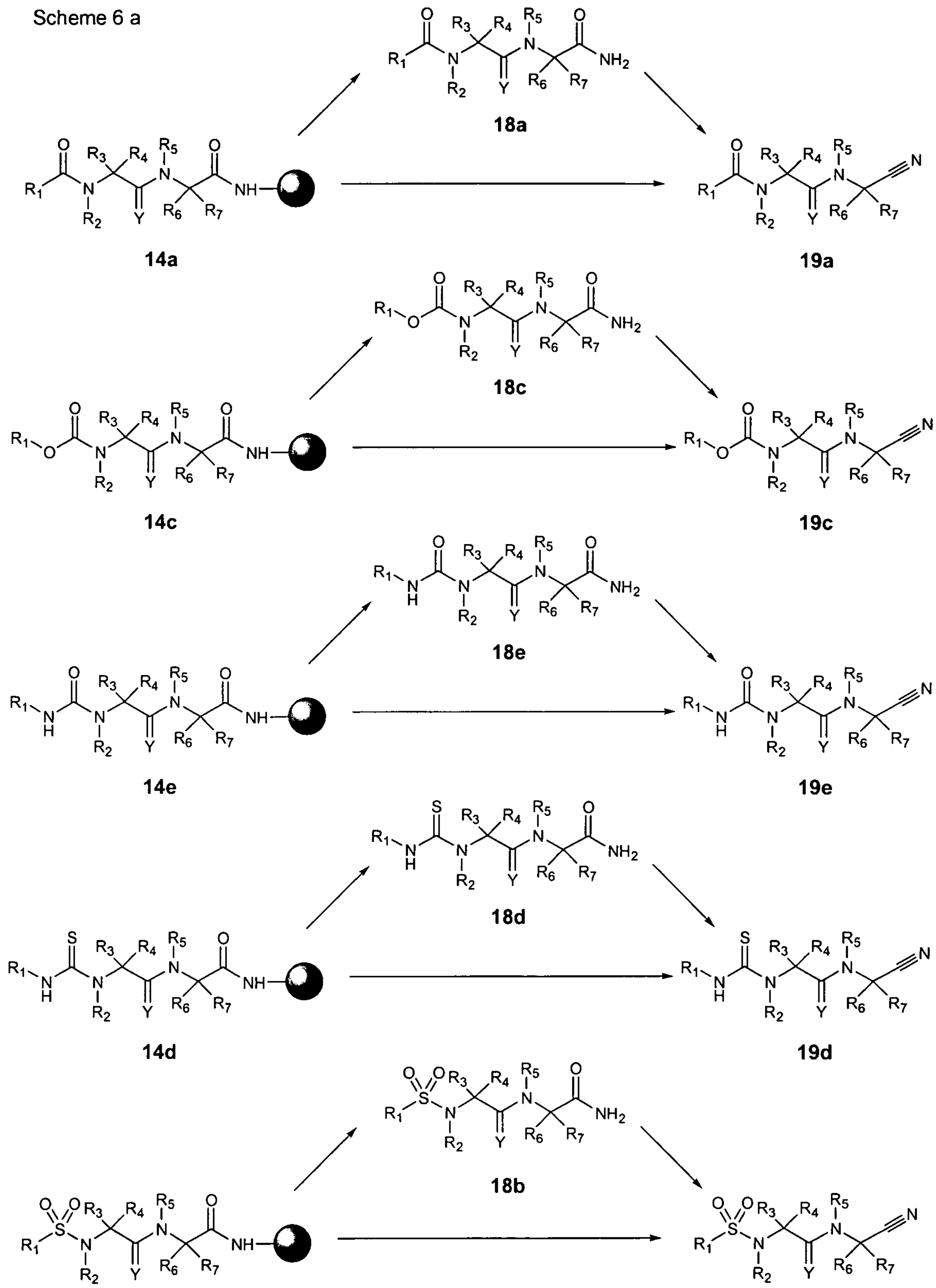

18a

14a

19a

18c

14c

19c

18e

14e

19e

18d

14d

19d

18b

14b

19b

Fig. 6a

Scheme 6 b

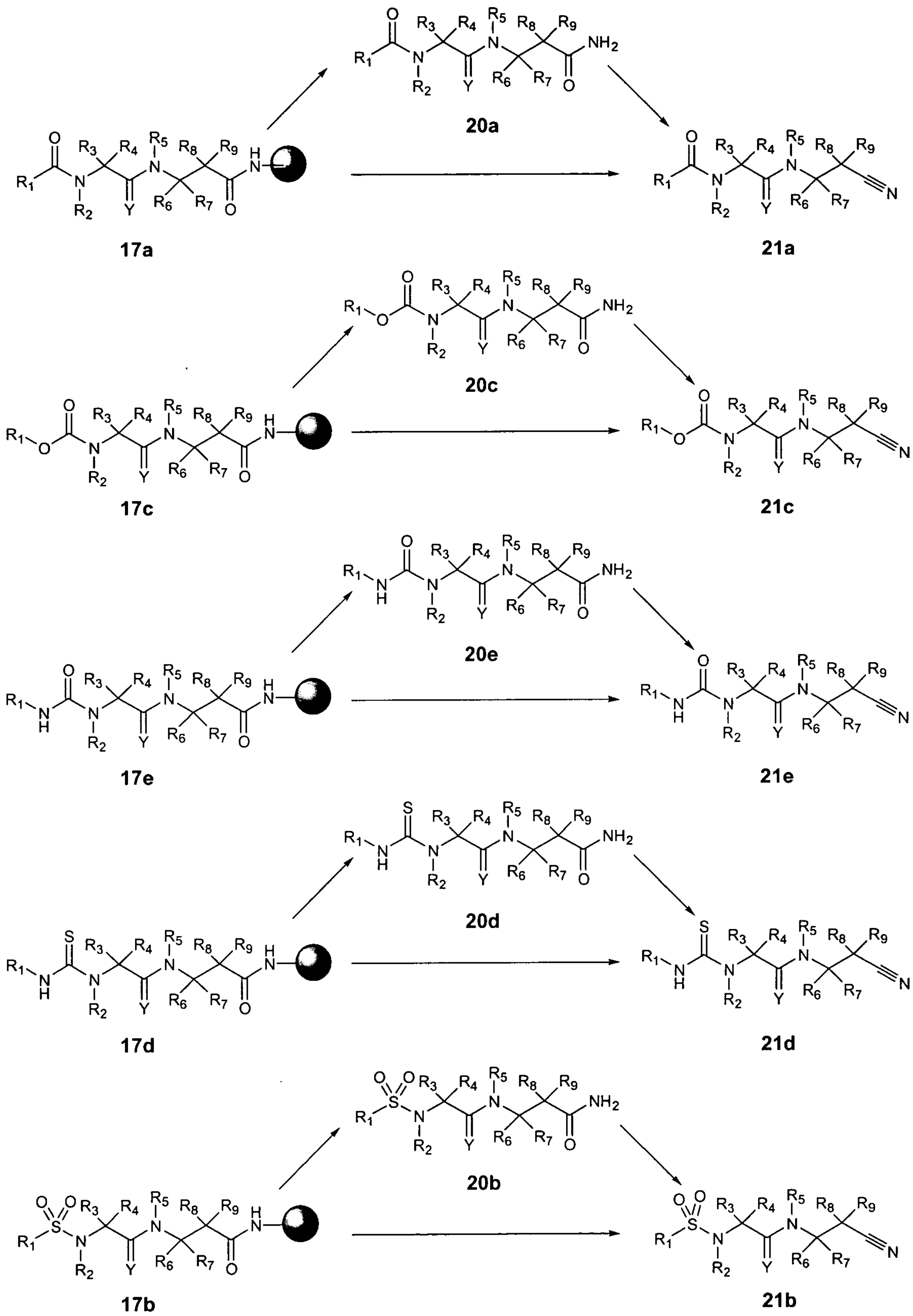

20a

17a

21a

20c

17c

21c

20e

17e

21e

20d

17d

21d

20b

17b

21b

Fig. 6b

European Patent Office

# PARTIAL EUROPEAN SEARCH REPORT

which under Rule 45 of the European Patent Convention shall be considered, for the purposes of subsequent proceedings, as the European search report

Application Number
EP 02 02 8699

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 00 27811 A (BRISTOL-MEYERS SQUIBB COMPANY) 18 May 2000 (2000-05-18)<br>* page 61 - page 64; claim 1 *<br>--- | 1 | C07C255/25<br>A61K31/16<br>A61P43/00<br>G01N33/00 |
| A | US 2002/049193 A1 (GREGORY S. HAMILTON ET AL.) 25 April 2002 (2002-04-25)<br>* claims *<br>----- | 1 | C07C271/22<br>C07C323/44<br>C07C335/16<br>C07C307/10<br>C07C255/24<br>C07C255/58<br>C07D317/66<br>C07C335/12<br>C07C335/18<br>C07C311/10<br>C07D333/34<br>C07D215/36<br>C07D209/42<br>C07D333/38 |

TECHNICAL FIELDS SEARCHED (Int.Cl.7)

C07C
C07D
A61K
A61P
G01N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 13 May 2003 | Van Bijlen, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C07)

European Patent Office

**INCOMPLETE SEARCH SHEET C**

Application Number
EP 02 02 8699

Although claims 1-65 are directed to a method of treatment of the human/animal body (Article 52(4) EPC), the search has been carried out and based on the alleged effects of the compound/composition.Although claims 63-64 are directed to a method of treatment of the human/animal body (Article 52(4) EPC), the search has been carried out and based on the alleged effects of the compound/composition.

--------------------

Claim(s) searched incompletely:
1-65

Reason for the limitation of the search:

Present claims 1-65 relate to a specific use ( as an inhibitor of rotamase ) of an extremely large number of possible compounds. In fact, the claims contain so many options and variables that a lack of clarity within the meaning of Article 84 EPC arises to such an extent as to render a meaningful search of the claims impossible. Also the scope of e.g. claim 1 , in as far as the expression derivative and prodrug is concerned, is so unclear ( Article 84 EPC ) that a meaningful search is impossible with regard to these expressions.
Consequently, the search and the search report can only be considered comprehensive for the use of a compound as an inhibitor of rotamase in as far as the compound is one of the compounds of the examples on page 90-136 of the present application.
Similarly the search and the search report can be considered comprehensive for claim 65 ( compounds per se ) in as far as the compounds listed in this claim are limited to the examples on page 90-136 of the present application.

European Patent Office

# PARTIAL EUROPEAN SEARCH REPORT

Application Number
EP 02 02 8699

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| | | | C07D209/18<br>C07D333/60<br>C07D409/12<br>C07D207/16 |
| | | | TECHNICAL FIELDS SEARCHED (Int.Cl.7) |
| | | | |

EPO FORM 1503 03.82 (P04C10)

## ANNEX TO THE EUROPEAN SEARCH REPORT ON EUROPEAN PATENT APPLICATION NO. EP 02 02 8699

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2003

| Patent document cited in search report | Publication date | Patent family member(s) | Publication date |
|---|---|---|---|
| WO 0027811 A | 18-05-2000 | AU 751541 B2 | 22-08-2002 |
| | | AU 1720500 A | 29-05-2000 |
| | | CA 2350429 A1 | 18-05-2000 |
| | | EP 1129070 A1 | 05-09-2001 |
| | | JP 2002529449 T | 10-09-2002 |
| | | WO 0027811 A1 | 18-05-2000 |
| | | US 6228872 B1 | 08-05-2001 |
| US 2002049193 A1 | 25-04-2002 | US 6294551 B1 | 25-09-2001 |
| | | US 6121273 A | 19-09-2000 |
| | | US 5874449 A | 23-02-1999 |
| | | BR 9713732 A | 25-01-2000 |
| | | CA 2239926 A1 | 30-06-1998 |
| | | EA 2593 B1 | 27-06-2002 |
| | | EP 0957913 A1 | 24-11-1999 |
| | | JP 2002515051 T | 21-05-2002 |
| | | NO 992268 A | 30-08-1999 |
| | | NZ 335480 A | 27-04-2001 |
| | | SK 57799 A3 | 14-08-2000 |
| | | AU 741100 B2 | 22-11-2001 |
| | | AU 5385498 A | 31-07-1998 |
| | | BG 103407 A | 31-01-2000 |
| | | CN 1246792 A | 08-03-2000 |
| | | CZ 9901545 A3 | 17-11-1999 |
| | | HU 0002974 A2 | 29-01-2001 |
| | | KR 2000057489 A | 15-09-2000 |
| | | PL 334214 A1 | 14-02-2000 |
| | | WO 9829116 A1 | 09-07-1998 |
| | | ZA 9711703 A | 10-02-1999 |

EPO FORM P0459

For more details about this annex : see Official Journal of the European Patent Office, No. 12/82